# EUROPEAN PATENT APPLICATION

(11) **EP 4 810 518 A2**
(43) Date of publication of application: **23.09.2026**
(21) Application number: 26194051.4
(22) Date of filing: 03.04.2018
(51) Int. Cl.: C07K 16/30

(54) **ANTIBODY THAT BINDS ERBB-2 AND ERBB-3**

(30) Priority: 31.03.2017 EP 17164396; 31.03.2017 US 201715476260
(62) Document number(s) of the earlier application(s) in accordance with Art. 76 EPC: 18717732.4 / 3 600 412
(71) Applicant: Merus B.V., 3584 CT Utrecht (NL)
(72) Inventor: THROSBY, Mark, 3584 CT Utrecht (NL); GEUIJEN, Cecilia Anna Wilhelmina, 3584 CT Utrecht (NL); MAUSSANG-DETAILLE, David Andre Baptiste, 3584 CT Utrecht (NL); LOGTENBERG, Ton, 3584 CT Utrecht (NL)
(74) Representative: V.O.

(57) **Abstract**

The invention relates among others to antibodies comprising a first antigen-binding site that binds ErbB-2 and a second antigen-binding site that binds ErbB-3. The antibodies can typically reduce a ligand-induced receptor function of ErbB-3 on a ErbB-2 and ErbB-3 positive cell. Also described are method for the treatment and use of the antibodies in imaging and in the treatment of subjects having an ErbB-2, ErbB-3 or ErbB-2/3 positive tumor.

## Description

This application claims priority to EP Application No. 17164396.8, filed March 31, 2017, and US 15/476260, filed March 31, 2017 the contents of which are hereby incorporated by reference.

The invention relates to the field of antibodies. In particular it relates to the field of therapeutic (human) antibodies for the treatment of diseases involving aberrant cells. More in particular it relates to antibodies that bind ErbB-2 and ErbB-3 and their use in the binding of ErbB-2 and ErbB-3 positive cells, particularly tumor cells.

The human epidermal growth factor receptor family (HER, also collectively referred to as the ErbB signaling network) is a family of transmembrane receptor tyrosine kinases (RTK). The family includes the epidermal growth factor receptor (EGFR), also known as ErbB-1 (or HERl), and the homologous receptors ErbB-2 (HER2), ErbB-3 (HER3) and ErbB-4 (HER4). The receptors (reviewed in Yarden and Pines 2012) are widely expressed on epithelial cells. Upregulation of HER receptors or their ligands, such as heregulin (HRG) or epidermal growth factor (EGF), is a frequent event in human cancer (Wilson, Fridlyand et al. 2012). Overexpression of ErbB-1 and ErbB-2 in particular occurs in epithelial tumors and is associated with tumor invasion, metastasis, resistance to chemotherapy, and poor prognosis (Zhang, Berezov et al. 2007). In the normal breast, ErbB-3 has been shown to be important in the growth and differentiation of luminal epithelium. For instance, loss/inhibition of ErbB-3 results in selective expansion of the basal over the luminal epithelium (Balko, Miller et al. 2012). Binding of ligand to the extracellular domain of the RTKs induces receptor dimerization, both between the same (homodimerization) and different (heterodimerization) receptor subtypes. Dimerization can activate the intracellular tyrosine kinase domains, which undergo autophosphorylation and, in turn, can activate a number of downstream pro-proliferative signaling pathways, including those mediated by mitogen-activated protein kinases (MAPK) and the prosurvival pathway Akt (reviewed in Yarden and Pines, 2012). No specific endogenous ligand has been identified for ErbB-2, which is therefore assumed to normally signal through heterodimerization (Sergina, Rausch et al. 2007). ErbB-3 can be activated by engagement of its ligands. These ligands include but are not limited to neuregulin (NRG) and heregulin (HRG).

Various modes of activation of signaling of the ErbB receptor family have been identified. Among these are ligand dependent and ligand independent activation of signaling. Over-expressed ErbB-2 is able to generate oncogenic signaling through the ErbB-2:ErbB-3 heterodimer even in the absence of the ErbB-3 ligand (Junttila, Akita et al. 2009). ErbB-2 activity can be inhibited by ErbB-2 specific antibodies. Such ErbB-2 specific antibodies are for instance used in the treatment of ErbB-2 positive (HER2+) tumors. A problem with such treatments is that often tumors escape the ErbB-2 specific treatment and continue to grow even in the presence of the inhibiting antibody. It has been observed that ErbB-2 positive tumors, such as breast, ovarian, cervical and gastric tumors can escape treatment by the selective outgrowth of a subpopulation of tumor cells that exhibit upregulated ErbB-3 expression (Ocana, Vera-Badillo et al. 2013) and/or ErbB-3 ligand expression (Wilson, Fridlyand et al. 2012). Also activating mutations in the ErbB-3 receptor have been identified.

The anti-ErbB-2 monoclonal antibody trastuzumab (Herceptin) and the ErbB-1 specific cetuximab (Erbitux) are among several monoclonal antibodies approved for clinical application. Trastuzumab has a proven survival benefit in metastatic breast cancer (Arteaga, Sliwkowski et al. 2011). The precise mechanism of action of trastuzumab has not been unequivocally established. Suggested modes of action are the inhibition of RTK signaling and the recruitment of antibody dependent cellular cytotoxicity (ADCC). Other mechanisms of action that have been described include blocking proteolytic cleavage of the ErbB-2 extracellular domain, inhibition of angiogenic factors and enhancement of receptor endocytosis. Other agents that interfere with ErbB-2 signaling have been approved or are under development for treatment of breast and other ErbB-2 overexpression cancers. For example, the chemical compound lapatinib inhibits both ErbB-1 and ErbB-2 tyrosine kinase activity and is used in first line treatment of ErbB-2 amplified breast cancer.

In patients with HER2+ metastatic breast cancer, resistance to trastuzumab either as single-agent or in combination with chemotherapy, commonly occurs within months of starting therapy. Only a fraction of patients with HER2+ metastatic breast cancer respond to single agent trastuzumab, suggesting *de novo* mechanisms of resistance in advanced cancers. These mechanisms include, among others, signaling from other HER family of receptors and compensatory signaling from RTKs outside of the HER family (Thery et al., Resistance to human epidermal growth factor receptor type 2-targeted therapies, Eur J Cancer (2014), Vol. 50, Issue 5, pages 892-901 (ttp://dx.doi.org/10.1016/j.ejca.2014.01.003)). For example, overexpression of HER3 or its ligands along with HER2 leads to the formation of HER-2/HER-3 heterodimers and acquired resistance to trastuzumab. Thus, the antibody trastuzumab is thought to be ineffective in blocking signaling driven by ErbB-3 ligands (Wehrman, Raab et al. 2006, Junttila, Akita et al. 2009, Thery et al. 2014).

Recently the monoclonal antibody pertuzumab was approved for use in combination with trastuzumab on the basis of an extra 5 months progression-free survival benefit (Baselga, Cortes et al. 2012). Pertuzumab also binds ErbB-2 but at a different position than trastuzumab.

Other strategies to treat ErbB-2 positive tumors are directed towards ErbB-3. ErbB-3 binding monoclonal antibodies have demonstrated activity in preclinical studies (Schoeberl, Faber et al. 2010). Some ErbB-3 binding monoclonal antibodies can inhibit proliferation and growth of a variety of cancers.

Another strategy involves binding of both the ErbB-2 and ErbB-3 receptor. The molecule MM-111, is an artificial biological molecule containing two single chain Fv (scFv) fragments that bind ErbB-2 and ErbB-3. The two scFv are associated with a mutated human serum albumin (HSA) protein to increase the half-life of the molecule. In preclinical testing the molecule was shown to inhibit ErbB-3 signaling and proliferation. This effect was predominantly measured on ErbB-3 positive cell lines that expressed relatively high amounts of ErbB-2.

### SUMMARY OF THE INVENTION

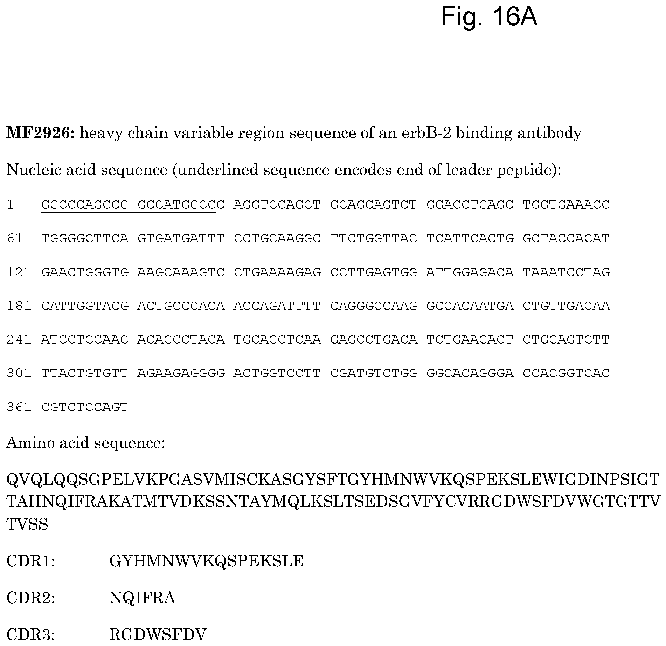

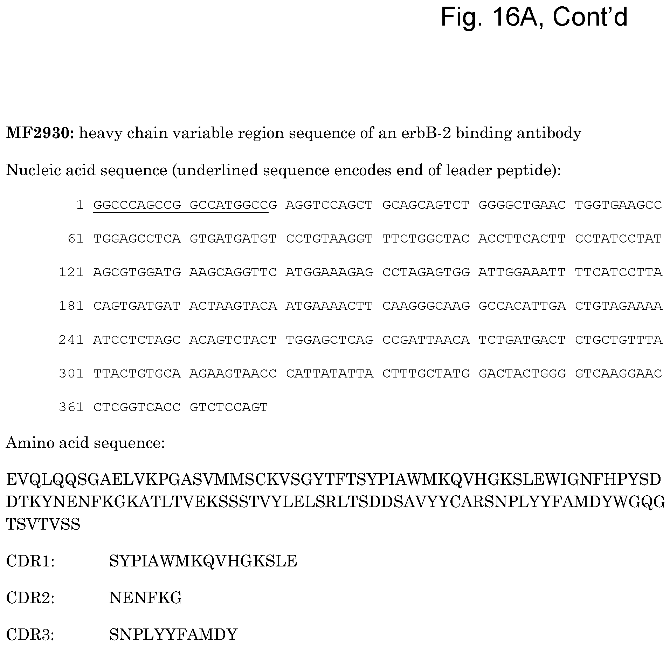

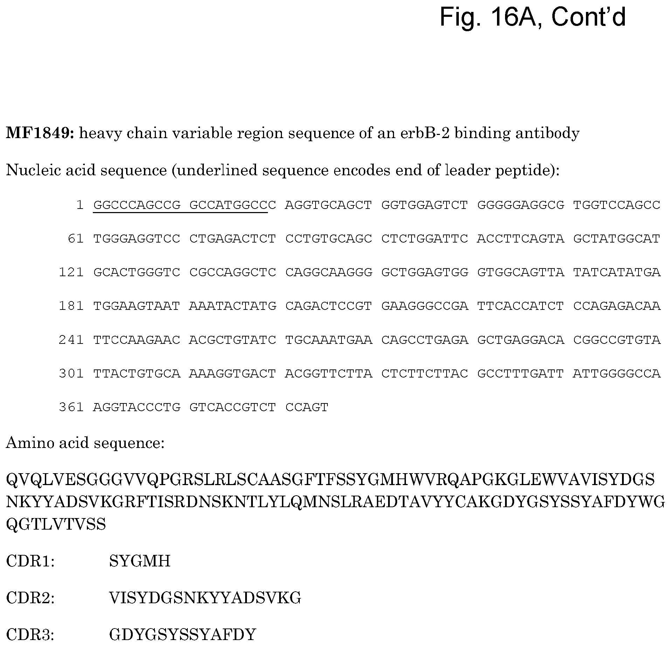

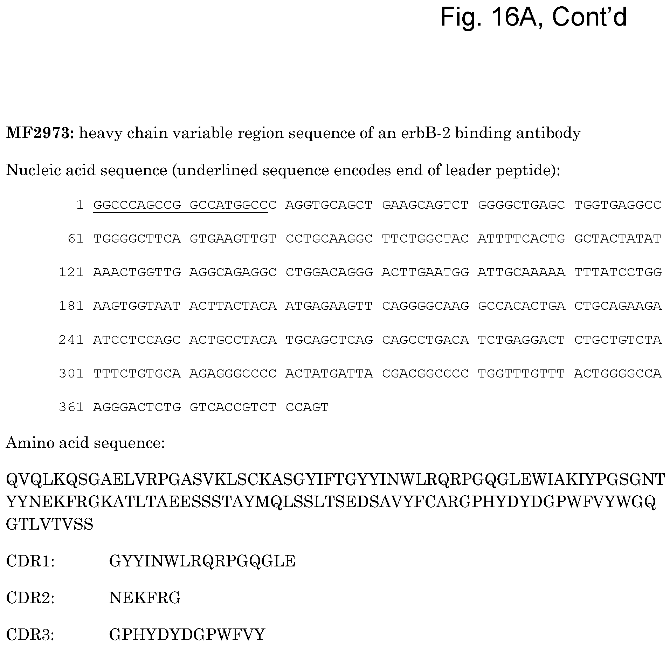

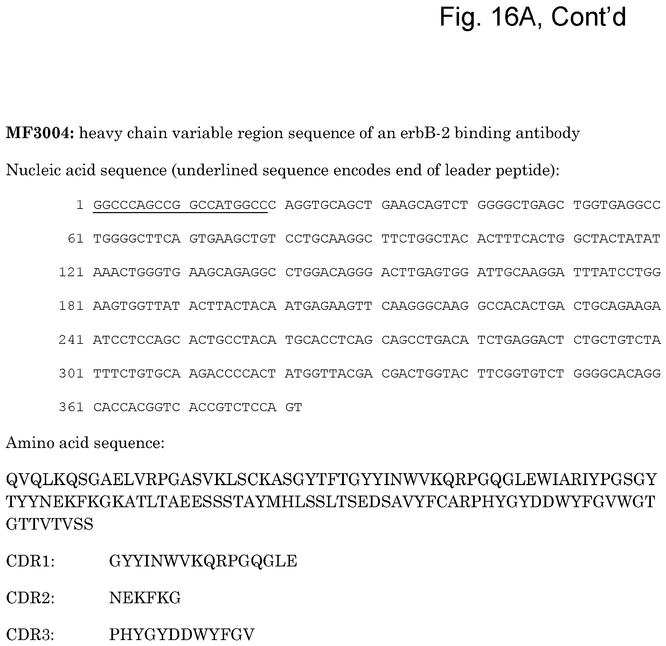

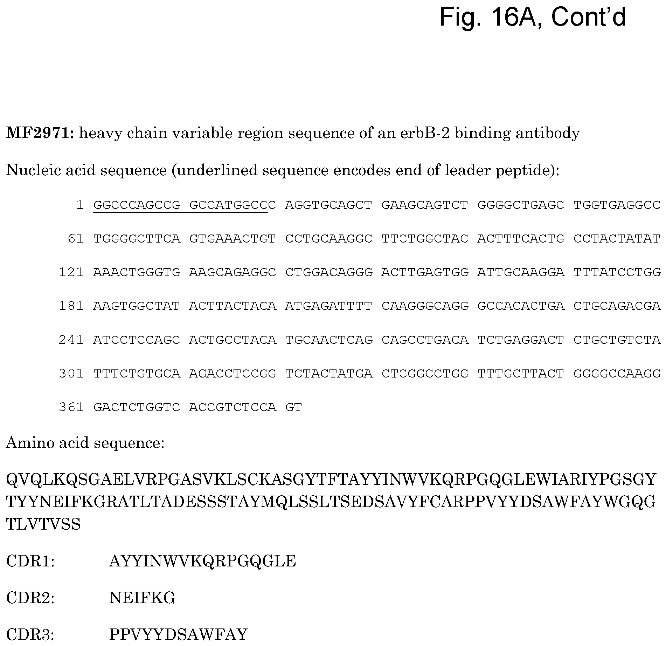

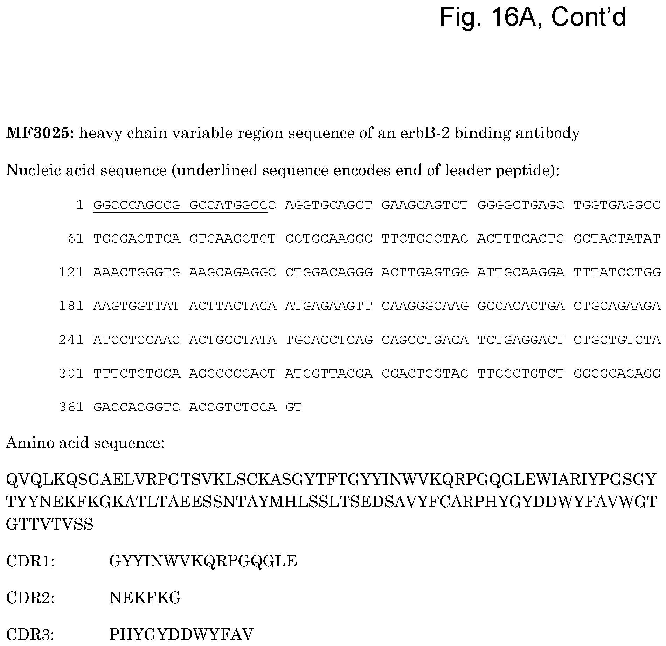

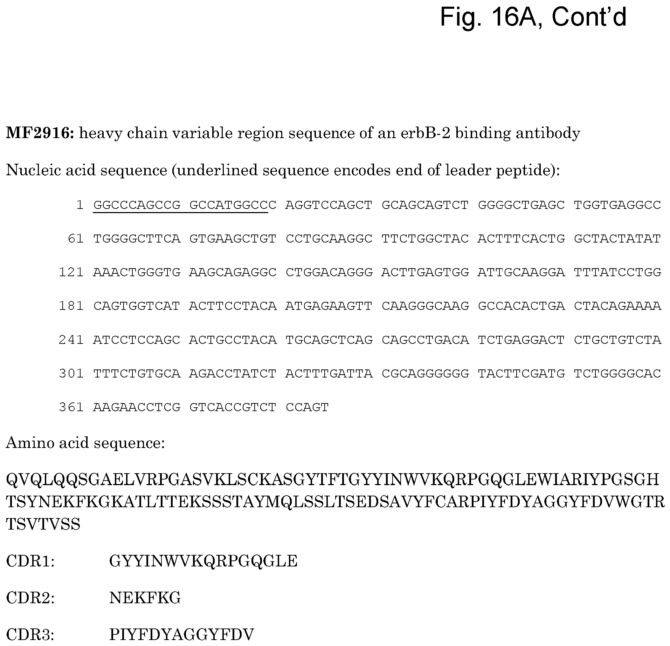

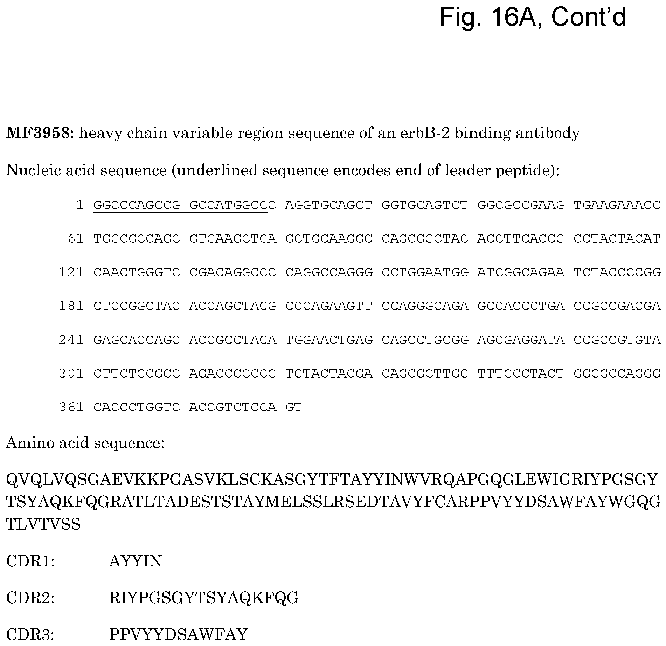

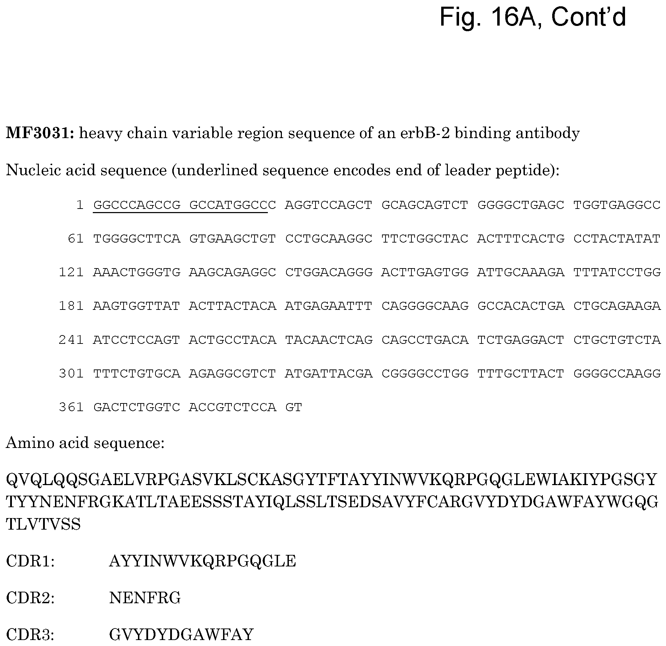

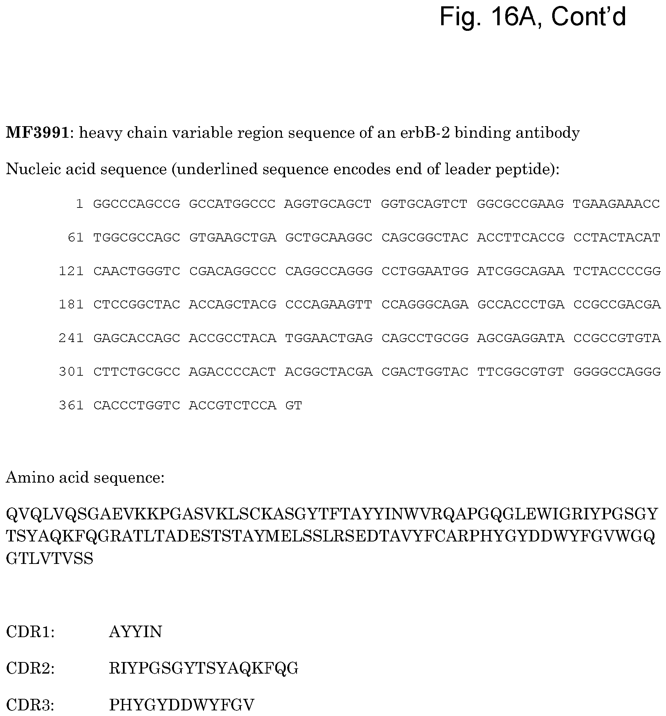

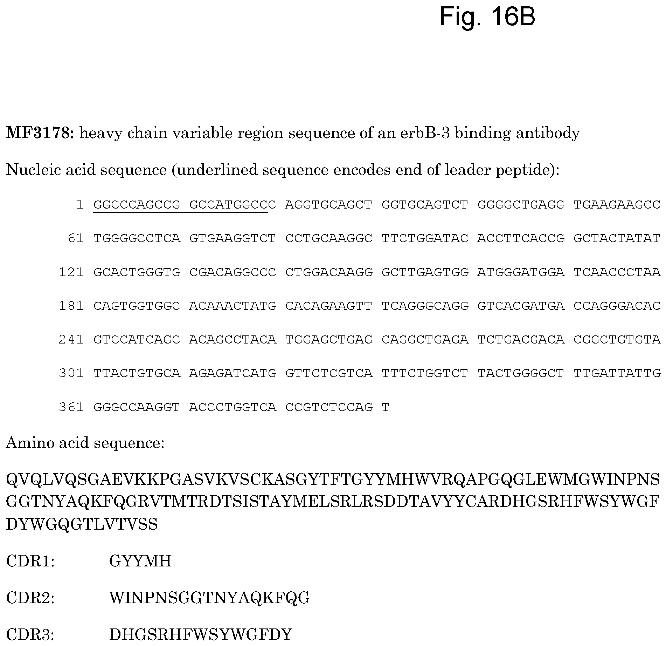

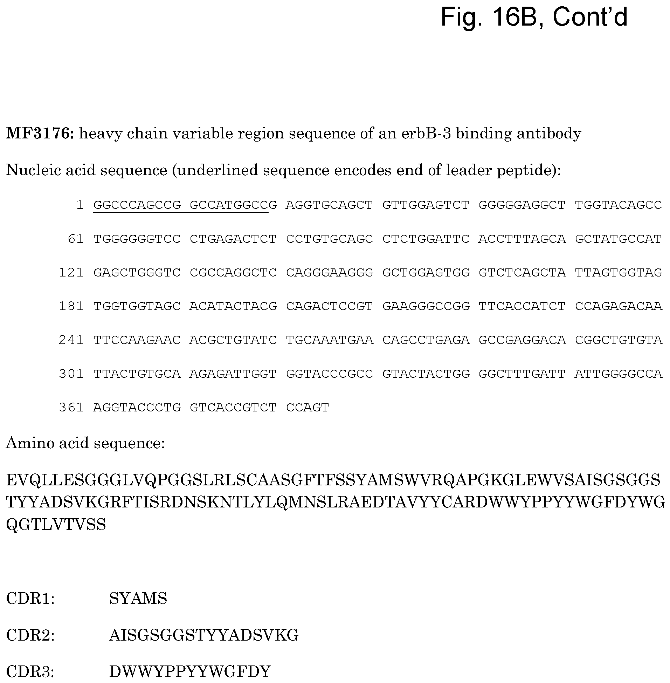

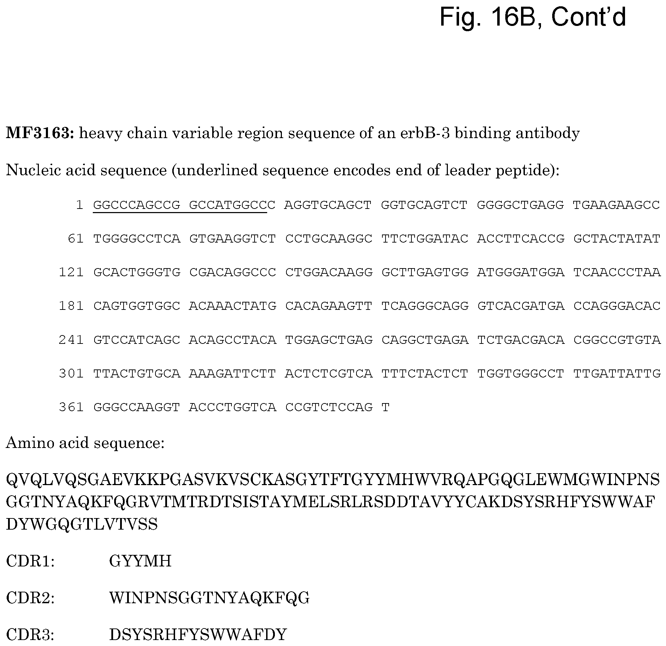

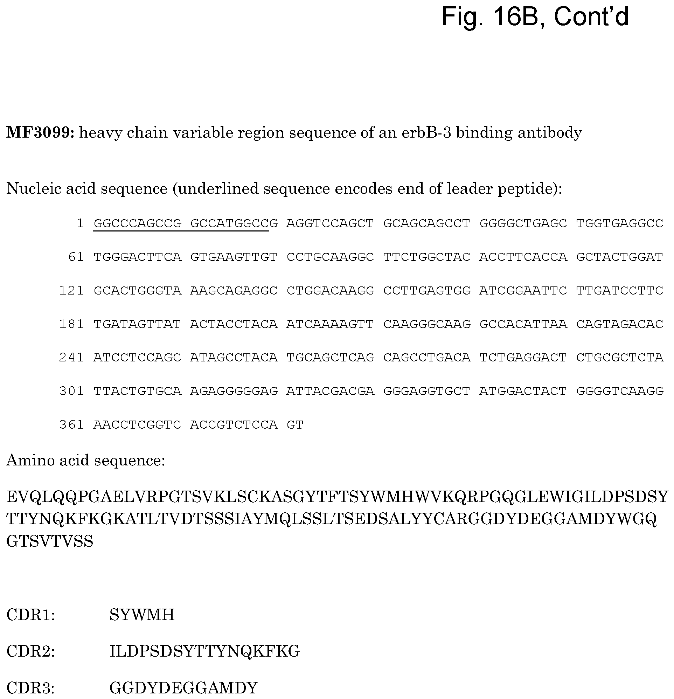

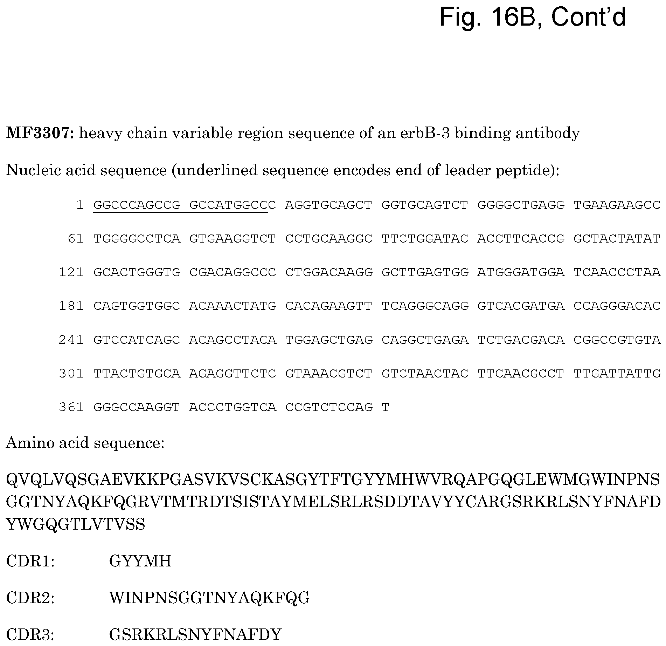

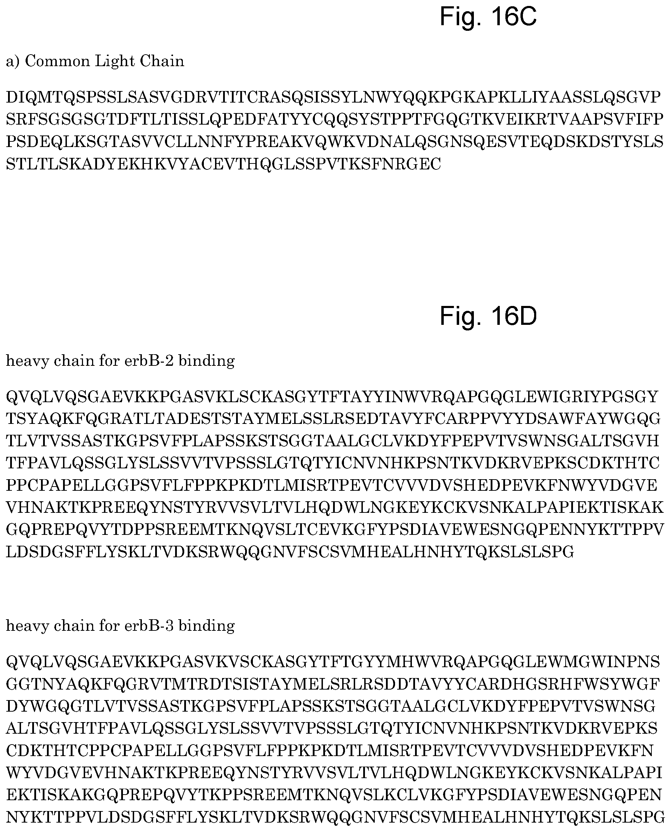

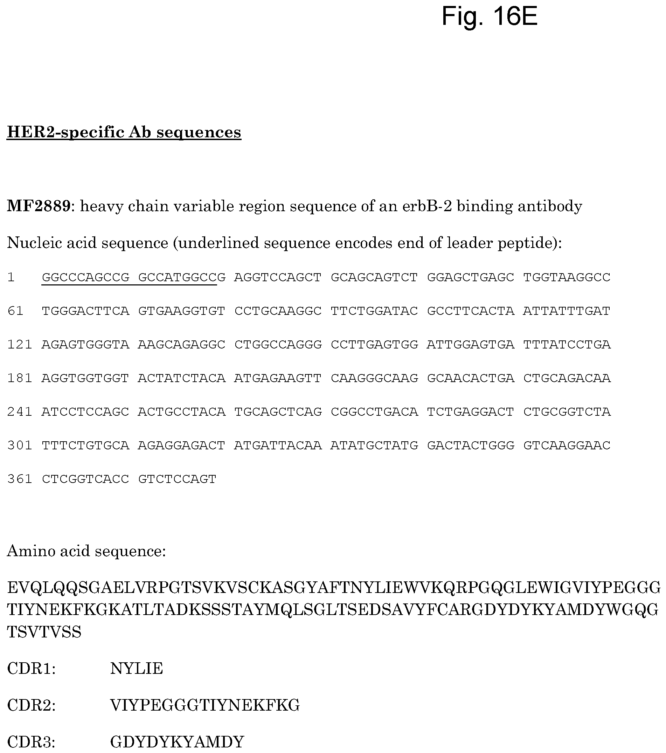

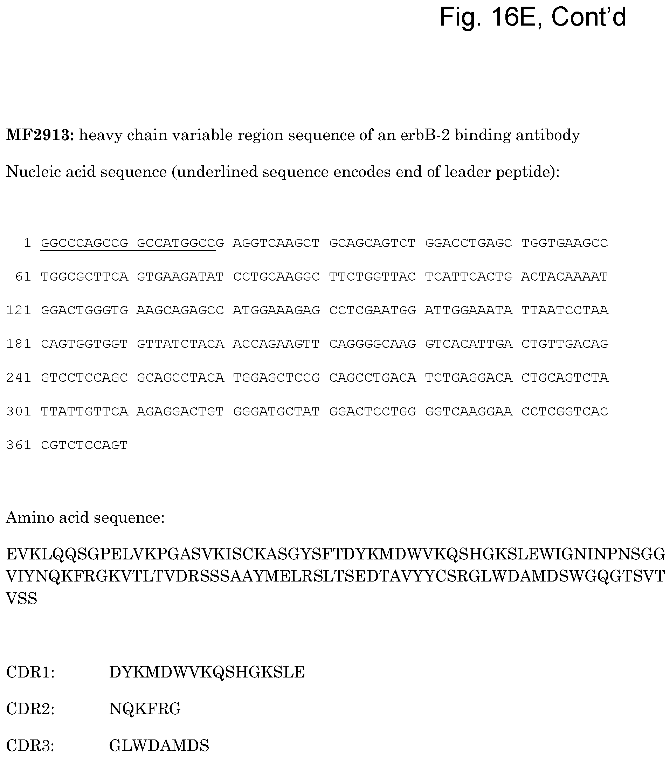

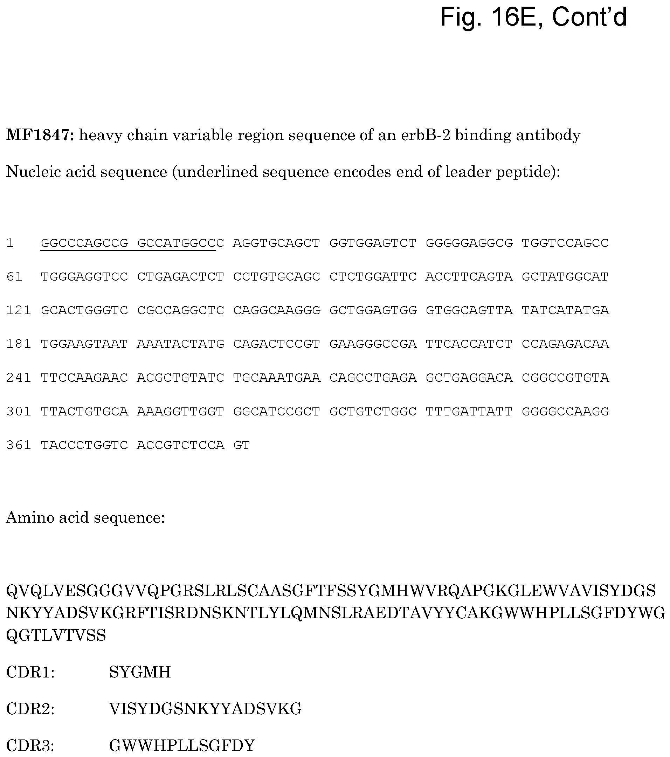

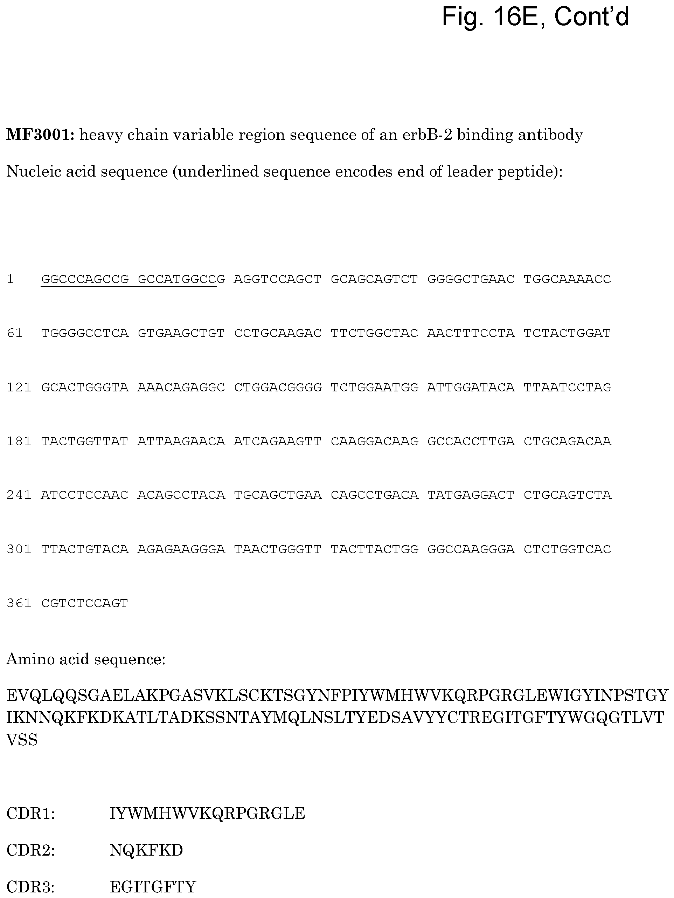

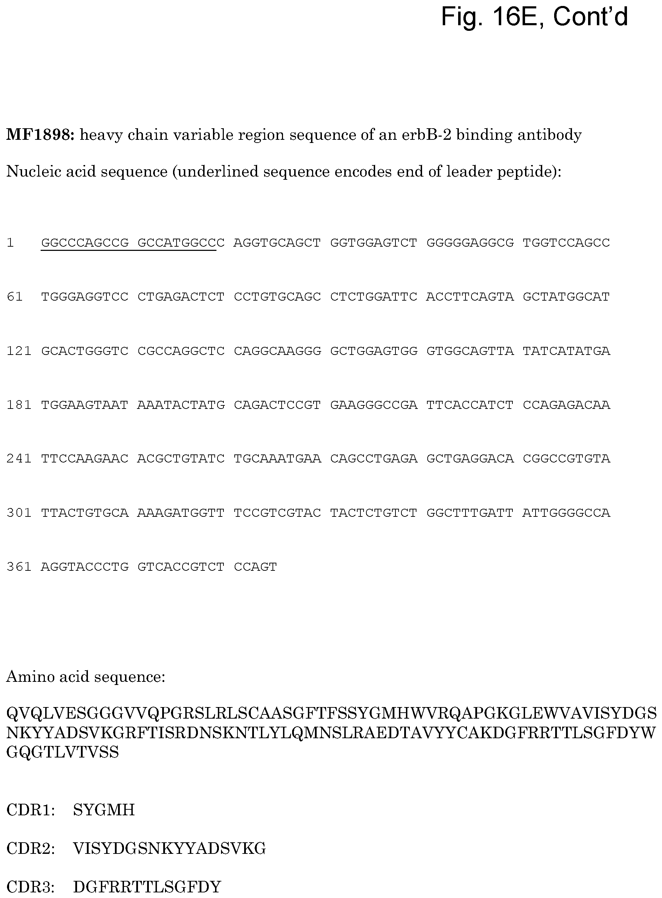

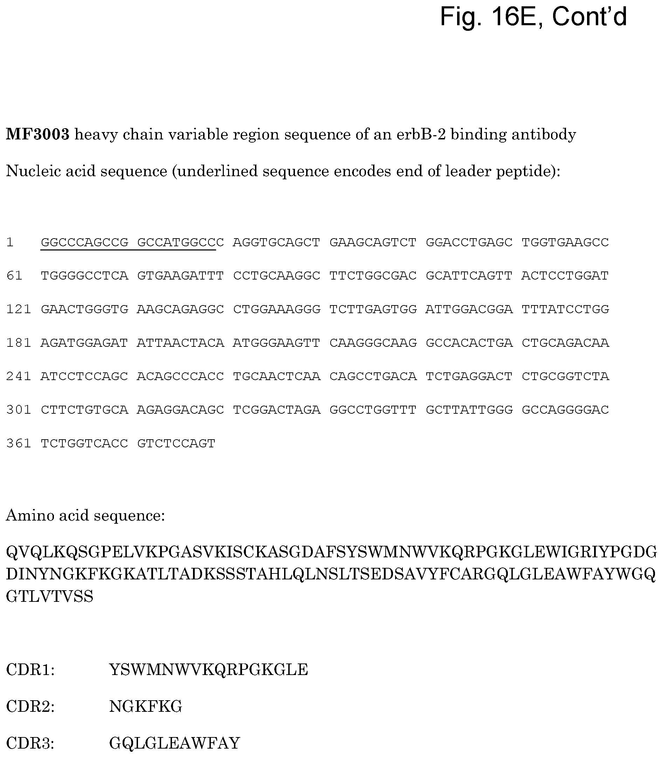

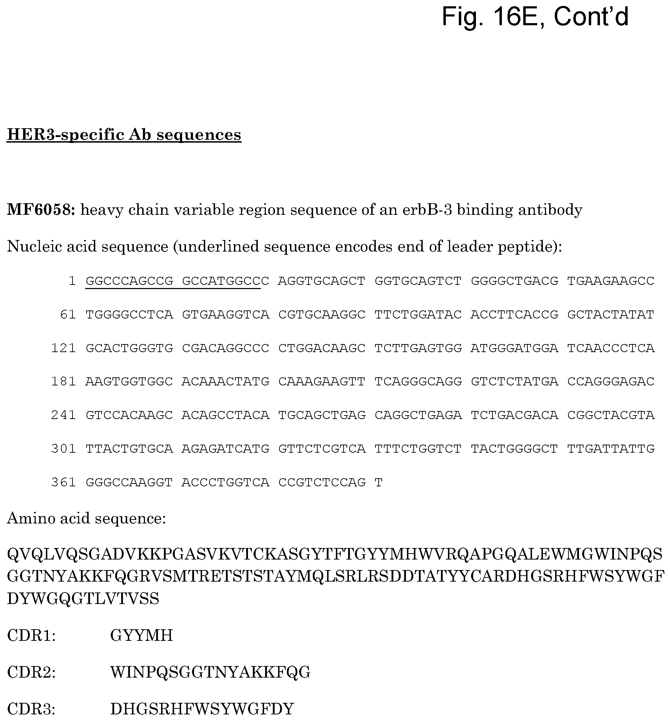

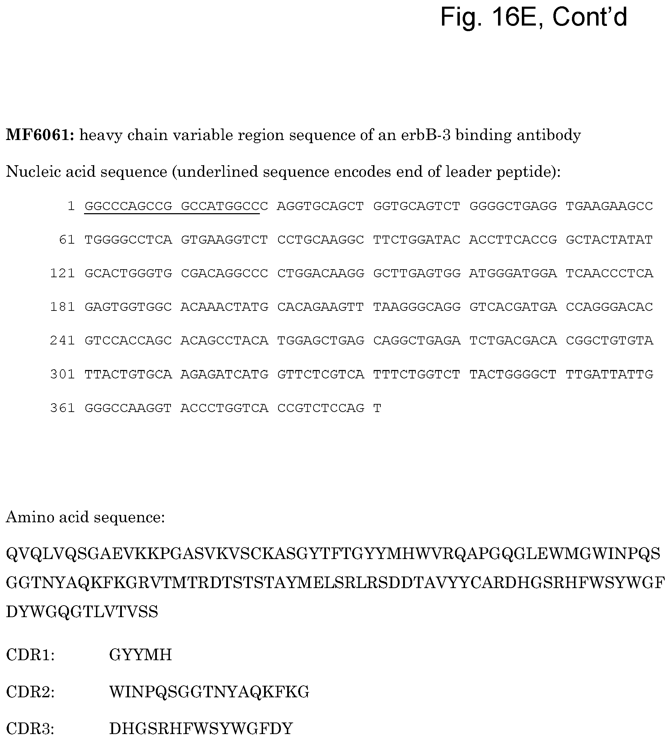

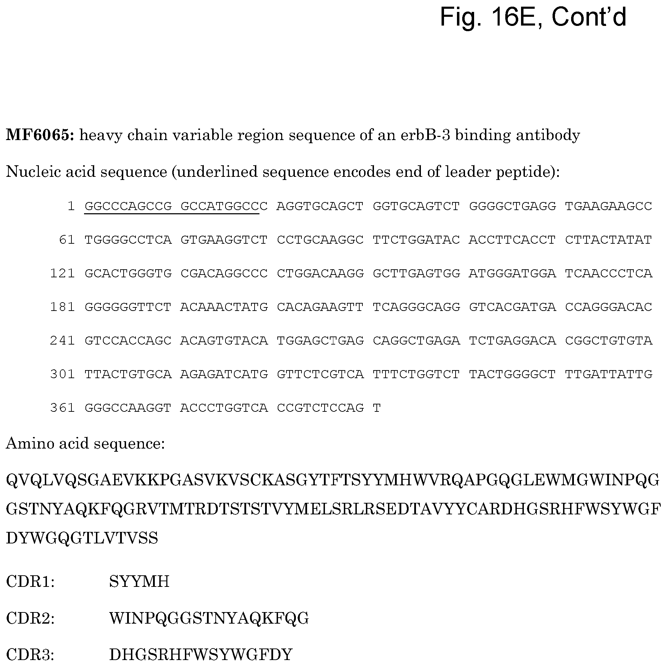

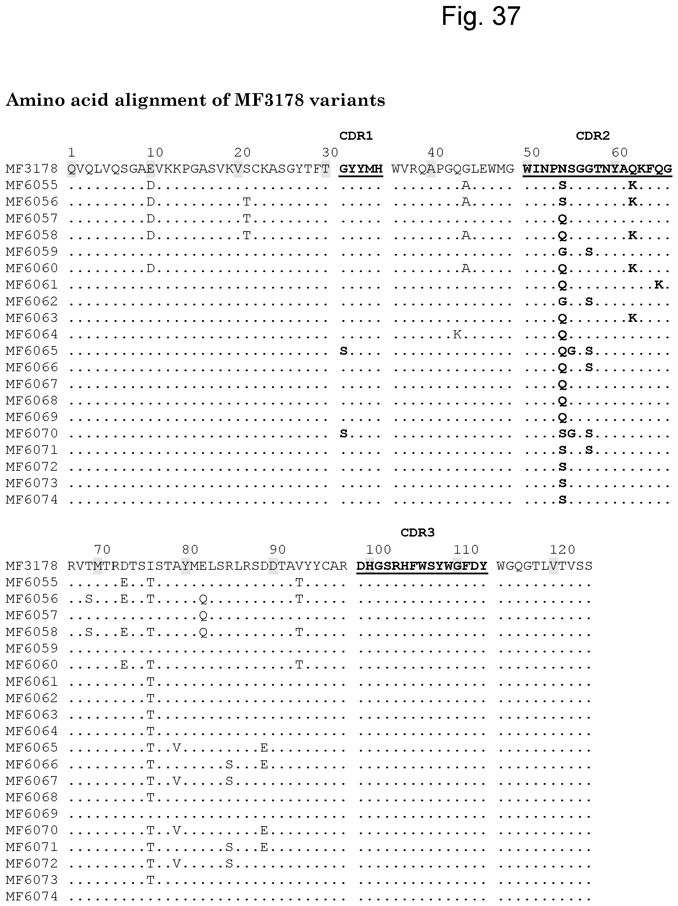

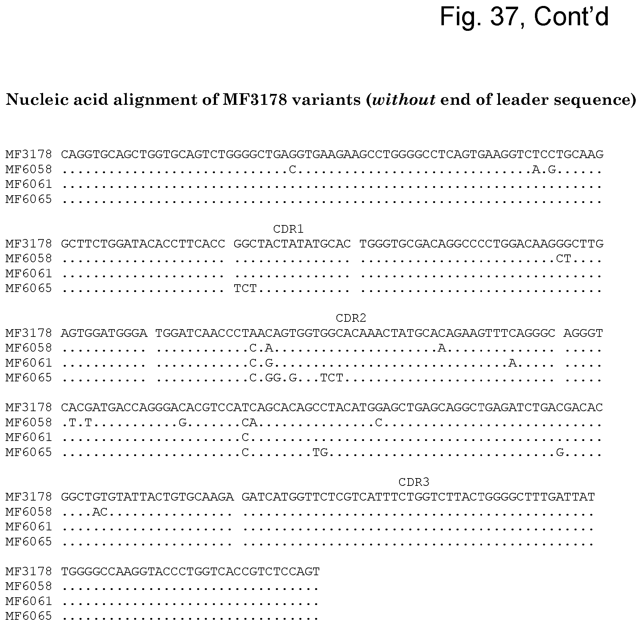

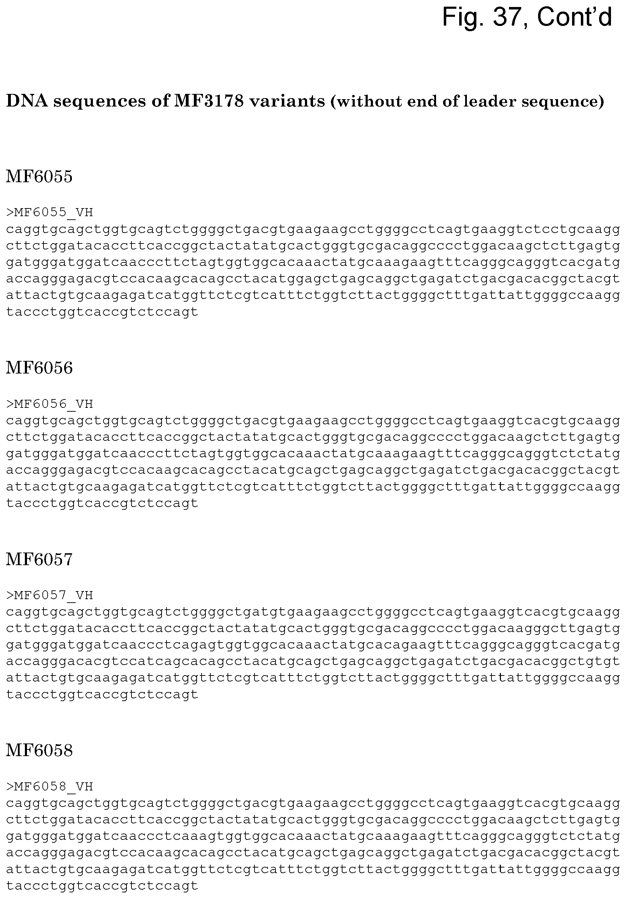

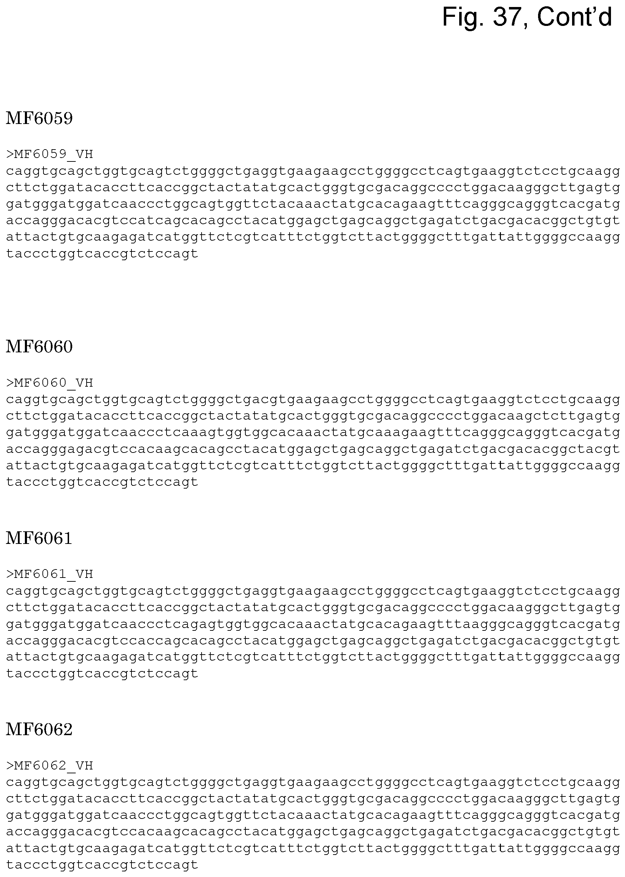

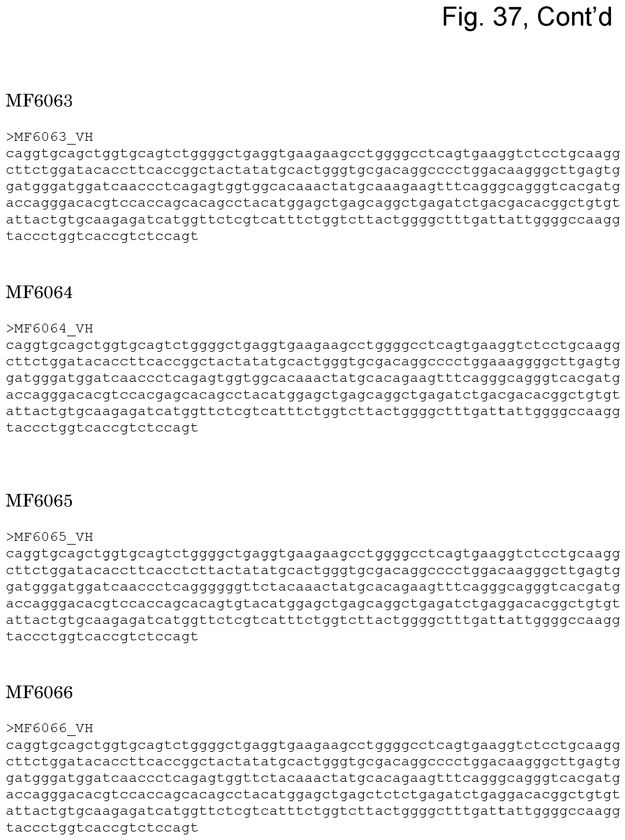

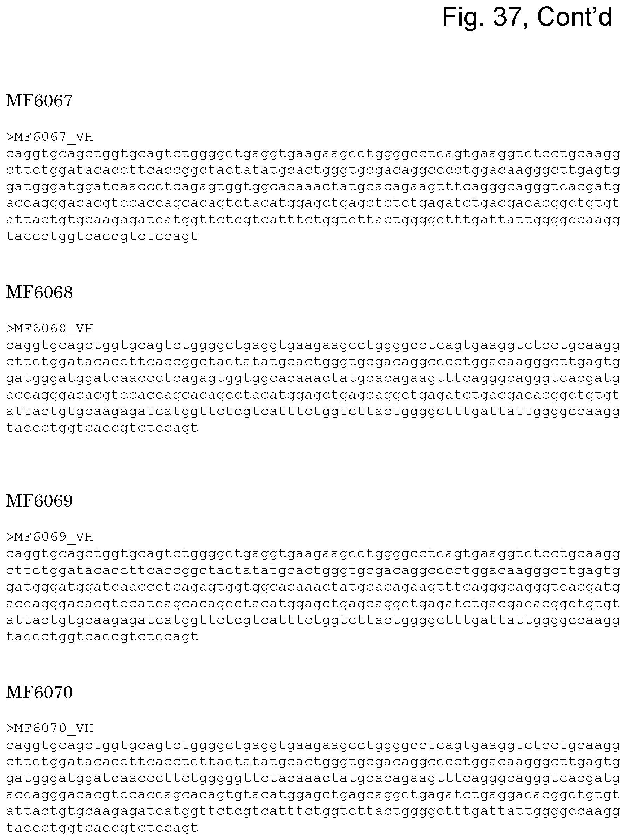

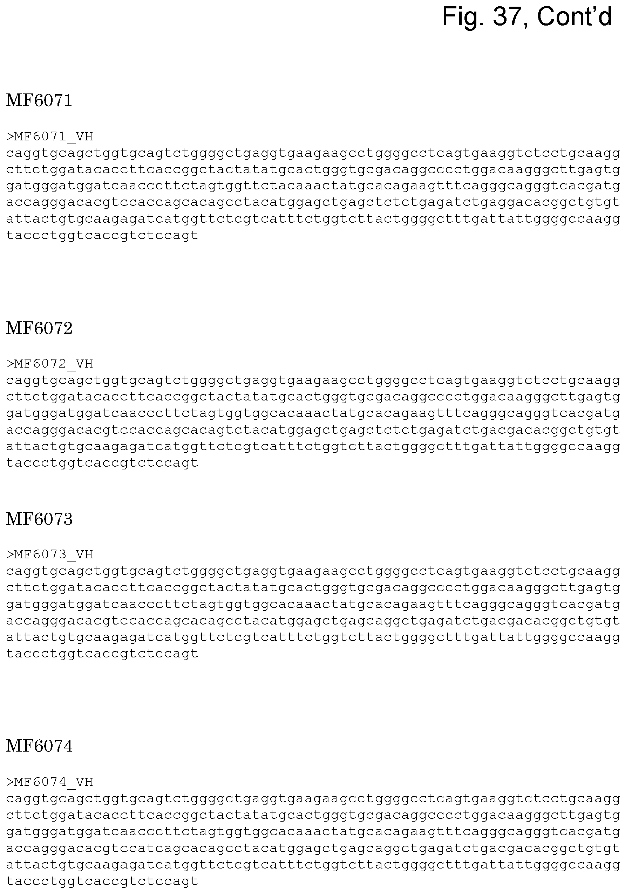

The invention provides a bispecific antibody comprising a first antigen-binding site that binds ErbB-2 and a second antigen-binding site that binds ErbB-3, and wherein the antibody can reduce a ligand-induced receptor function of ErbB-3 on a ErbB-2 and ErbB-3 positive cell. Said first antigen-binding site is preferably present in a variable domain comprising a VH chain with the amino acid sequence of VH chain MF2926; MF2930; MF1849; MF2973; MF3004; MF3958 (is humanized MF2971); MF2971; MF3025; MF2916; MF3991 (is humanized MF3004); MF3031; MF2889; MF2913; MF1847; MF3001; MF3003 or MF1898 as depicted in Figure 16A or Figure 16E. Said second antigen-binding site is preferably present in a variable domain comprising a VH chain with the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 as depicted in Figure 16B or Figure 16E or Figure 37. The immunoglobulin light chain in the variable domain preferably comprises the amino acid sequence of figure 16C.

An antibody of the invention is, unless otherwise specifically specified, preferably a bispecific antibody.

The invention further provides a pharmaceutical composition comprising an antibody according to the invention.

Further provided is an antibody according to the invention that further comprises a label, preferably a label for *in vivo* imaging.

The invention also provides a method for the treatment of a subject having a ErbB-2, ErbB-3 or ErbB-2/ErbB-3 positive cell, including a deleterious cell, or tumor or a subject at risk of having said tumor comprising administering to the subject a bispecific antibody according to the invention. Also provided is a bispecific antibody according to the invention for use in the treatment of a subject having or at risk of having an ErbB-2, ErbB-3 or ErbB-2/ErbB-3 positive tumor.

The invention further provides a method of treatment of an individual that has an ErbB-2 positive tumor or is at risk of developing an ErbB-2, ErbB-3 or ErbB-2/ErbB-3 positive tumor the method comprising administering to the individual in need thereof, a ErbB-2 targeting agent, including an inhibitor or binding agent of ErbB-2, for example a bivalent monospecific antibody that comprises an antigen binding site that can bind an epitope on an extracellular part of ErbB-2, and a bispecific antibody that comprises an antigen-binding site that can bind an epitope on an extracellular part of ErbB-2 and an antigen-binding site that can bind an epitope on an extracellular part of ErbB-3.

Also provided is a combination of a ErbB-2 targeting agent, including an inhibitor or binding agent of ErbB-2, for example a bivalent monospecific antibody that comprises antigen binding sites that can bind an epitope on an extracellular part of ErbB-2, and a bispecific antibody that comprises an antigen-binding site that can bind an epitope on extracellular part of ErbB-2 and an antigen-binding site that can bind an epitope on extracellular part of ErbB-3, for use in a method treatment of an individual that has an ErbB-2, ErbB-3 or ErbB-2/ErbB-3 positive tumor or is at risk of developing said tumor.

Further provided is a pharmaceutical composition comprising a ErbB-2 targeting agent, including an inhibitor or binding agent of ErbB-2, for example a bivalent monospecific antibody that comprises antigen binding sites that can bind an epitope on an extracellular part of ErbB-2 and a bispecific antibody that comprises an antigen-binding site that can bind an epitope on an extracellular part of ErbB-2 and an antigen-binding site that can bind an epitope on an extracellular part of ErbB-3.

Also provided is a kit of parts comprising a ErbB-2 targeting agent, including an inhibitor or binding agent of ErbB-2, for example a bivalent monospecific antibody that comprises antigen binding sites that can bind an epitope on an extracellular part of ErbB-2 and a bispecific antibody that comprises an antigen-binding site that can bind an epitope on an extracellular part of ErbB-2 and an antigen-binding site that can bind an epitope on an extracellular part of ErbB-3.

Also provided is a method of treatment of an individual that has an ErbB-2 positive and ErbB-3 positive tumor in the brain or is at risk of developing an ErbB-2 positive and ErbB-3 positive tumor in the brain the method comprising administering to the individual in need thereof a bispecific antibody that comprises an antigen-binding site that can bind an epitope on an extracellular part of ErbB-2 and an antigen-binding site that can bind an epitope on an extracellular part of ErbB-3.

Also provided is a bispecific antibody that comprises an antigen-binding site that can bind an epitope on an extracellular part of ErbB-2 and an antigen-binding site that can bind an epitope on an extracellular part of ErbB-3 for use in the treatment of an individual that has an ErbB-2 positive and ErbB-3 positive tumor in the brain or is at risk of developing an ErbB-2 positive and ErbB-3 positive tumor in the brain.

### DETAILED DESCRIPTION OF THE INVENTION

The invention provides a bispecific antibody comprising a first antigen-binding site that binds ErbB-2 and a second antigen-binding site that binds ErbB-3, wherein the bispecific antibody reduces or can reduce a ligand-induced receptor function of ErbB-3 on a ErbB-2 and ErbB-3 positive cell.

As used herein, the term "antigen-binding site" refers to a site derived from and preferably as present on a bispecific antibody which is capable of binding to antigen. An unmodified antigen-binding site is typically formed by and present in the variable domain of the antibody. The variable domain contains said antigen-binding site. A variable domain that binds an antigen is a variable domain comprising an antigen-binding site that binds the antigen.

In one embodiment an antibody variable domain of the invention comprises a heavy chain variable region (VH) and a light chain variable region (VL). The antigen-binding site can be present in the combined VH/VL variable domain, or in only the VH region or only the VL region. When the antigen-binding site is present in only one of the two regions of the variable domain, the counterpart variable region can contribute to the folding and/or stability of the binding variable region, but does not significantly contribute to the binding of the antigen itself.

As used herein, antigen-binding refers to the typical binding capacity of an antibody to its antigen. An antibody comprising an antigen-binding site that binds to ErbB-2, binds to ErbB-2 and, under otherwise identical conditions, at least 100-fold lower to the homologous receptors ErbB-1 and ErbB-4 of the same species. An antibody comprising an antigen-binding site that binds to ErbB-3, binds to ErbB-3 and, under otherwise identical conditions, not to the homologous receptors ErbB-1 and ErbB-4 of the same species. Considering that the ErbB-family is a family of cell surface receptors, the binding is typically assessed on cells that express the receptor(s). Binding of an antibody to an antigen can be assessed in various ways. One way is to incubate the antibody with the antigen (preferably cells expressing the antigen), removing unbound antibody (preferably by a wash step) and detecting bound antibody by means of a labeled antibody that binds to the bound antibody.

Antigen binding by an antibody is typically mediated through the complementarity regions of the antibody and the specific three-dimensional structure of both the antigen and the variable domain allowing these two structures to bind together with precision (an interaction similar to a lock and key), as opposed to random, non-specific sticking of antibodies. As an antibody typically recognizes an epitope of an antigen, and as such epitope may be present in other compounds as well, antibodies according to the present invention that bind ErbB-2 and/or ErbB-3 may recognize other proteins as well, if such other compounds contain the same epitope. Hence, the term "binding" does not exclude binding of the antibodies to another protein or protein(s) that contain the same epitope. Such other protein(s) is preferably not a human protein. An ErbB-2 antigen-binding site and an ErbB-3 antigen-binding site as defined in the present invention typically do not bind to other proteins on the membrane of cells in a post-natal, preferably adult human. A bispecific antibody according to the present invention is typically capable of binding ErbB-2 and ErbB-3 with a binding affinity of at least 1x10e-6 M, as outlined in more detail below.

The term "interferes with binding" as used herein means that the antibody is directed to an epitope on ErbB-3 and the antibody competes with ligand for binding to ErbB-3. The antibody may diminish ligand binding, displace ligand when this is already bound to ErbB-3 or it may, for instance through steric hindrance, at least partially prevent that ligand can bind to ErbB-3.

The term "antibody" as used herein means a proteinaceous molecule, preferably belonging to the immunoglobulin class of proteins, containing one or more variable domains that bind an epitope on an antigen, where such domains are derived from or share sequence homology with the variable domain of an antibody. Antibodies for therapeutic use are preferably as close to natural antibodies of the subject to be treated as possible (for instance human antibodies for human subjects). Antibody binding can be expressed in terms of specificity and affinity. The specificity determines which antigen or epitope thereof is specifically bound by the binding domain. The affinity is a measure for the strength of binding to a particular antigen or epitope. Specific binding, is defined as binding with affinities (KD) of at least 1x10e-6 M, more preferably 1x10e-7 M, more preferably higher than 1x10e-9 M. Typically, antibodies for therapeutic applications have affinities of up to 1x10e-10 M or higher. Antibodies such the bispecific antibodies of the present invention comprise the constant domains (Fc part) of a natural antibody. An antibody of the invention is typically a bispecific full length antibody, preferably of the human IgG subclass. Preferably, an antibody of the present invention is of the human IgG1 subclass. Such antibodies of the invention have good ADCC properties, have favorable half life upon in vivo administration to humans and CH3 engineering technology exists that can provide for modified heavy chains that preferentially form heterodimers over homodimers upon co-expression in clonal cells.

An antibody of the invention is preferably a "full length" antibody. The term 'full length' according to the invention is defined as comprising an essentially complete antibody, which however does not necessarily have all functions of an intact antibody. For the avoidance of doubt, a full length antibody contains two heavy and two light chains. Each chain contains constant (C) and variable (V) regions, which can be broken down into domains designated CH1, CH2, CH3, VH, and CL, VL. An antibody binds to antigen via the variable domains contained in the Fab portion, and after binding can interact with molecules and cells of the immune system through the constant domains, mostly through the Fc portion. The terms 'variable domain', 'VH/VL pair', 'VH/VL' are used herein interchangeably. Full length antibodies according to the invention encompass antibodies wherein mutations may be present that provide desired characteristics. Such mutations should not be deletions of substantial portions of any of the regions. However, antibodies wherein one or several amino acid residues are deleted, without essentially altering the binding characteristics of the resulting antibody are embraced within the term "full length antibody". For instance, an IgG antibody can have 1-20 amino acid residue insertions, deletions or a combination thereof in the constant region. For instance, ADCC activity of an antibody can be improved when the antibody itself has a low ADCC activity, by slightly modifying the constant region of the antibody (Junttila, T. T., K. Parsons, et al. (2010). "Superior In vivo Efficacy of Afucosylated Trastuzumab in the Treatment of HER2-Amplified Breast Cancer." Cancer Research 70(11): 4481-4489)

Full length IgG antibodies are preferred because of their favourable half life and the need to stay as close to fully autologous (human) molecules for reasons of immunogenicity. An antibody of the invention is preferably a bispecific IgG antibody, preferably a bispecific full length IgG1 antibody. IgG1 is favoured based on its long circulatory half life in man. In order to prevent any immunogenicity in humans it is preferred that the bispecific IgG antibody according to the invention is a human IgG1.

The term 'bispecific' (bs) means that one part of the antibody (as defined above) binds to one epitope on an antigen whereas a second part binds to a different epitope. The different epitope is typically present on a different antigen. According to the present invention, said first and second antigens are in fact two different proteins. A preferred bispecific antibody is an antibody that comprises parts of two different monoclonal antibodies and consequently binds to two different types of antigen. One arm of the bispecific antibody typically contains the variable domain of one antibody and the other arm contains the variable domain of another antibody. The heavy chain variable regions of the bispecific antibody of the invention are typically different from each other, whereas the light chain variable regions are preferably the same in the bispecific antibodies of the invention. A bispecific antibody wherein the different heavy chain variable regions are associated with the same, or a common, light chain is also referred to as a bispecific antibody with a common light chain. Further provided is therefore a bispecific antibody according to the invention, wherein both arms comprise a common light chain.

Preferred bispecific antibodies can be obtained by co-expression of two different heavy chains and a common light chain in a single cell. When wildtype CH3 domains are used, co-expression of two different heavy chains and a common light chain will result in three different species, AA, AB and BB. To increase the percentage of the desired bispecific product (AB) CH3 engineering can be employed, or in other words, one can use heavy chains with compatible heterodimerization domains, as defined hereunder.

The term 'compatible heterodimerization domains' as used herein refers to protein domains that are engineered such that engineered domain A' will preferentially form heterodimers with engineered domain B' and vice versa, whereas homodimerization between A'-A' and B'-B' is diminished.

The term 'common light chain' according to the invention refers to light chains which may be identical or have some amino acid sequence differences while the binding specificity of the full length antibody is not affected . It is for instance possible within the scope of the definition of common light chains as used herein, to prepare or find light chains that are not identical but still functionally equivalent, e.g., by introducing and testing conservative amino acid changes, changes of amino acids in regions that do not or only partly contribute to binding specificity when paired with the heavy chain, and the like. The terms 'common light chain', 'common VL', 'single light chain', 'single VL', with or without the addition of the term 'rearranged' are all used herein interchangeably. It is an aspect of the present invention to use as common light chain a human light chain that can combine with different heavy chains to form antibodies with functional antigen binding domains (WO2004/009618, WO2009/157771, Merchant et al. 1998 and Nissim et al. 1994). Preferably, the common light chain has a germline sequence. A preferred germline sequence is a light chain variable region that is frequently used in the human repertoire and has good thermodynamic stability, yield and solubility. A preferred germline light chain is O12, preferably the rearranged germline human kappa light chain IgVκ1-39*01/IGJκ1*01 or a fragment or a functional equivalent (i.e. same IgVκ1-39 gene segment but different IGJκ gene segment) thereof (nomenclature according to the IMGT database worldwide web at imgt.org). Further provided is therefore a bispecific antibody according to the invention, wherein said common light chain is a germline light chain, preferably a rearranged germline human kappa light chain comprising the IgVKl-39 gene segment, most preferably the rearranged germline human kappa light chain IgVKl-39*01/IGJKl*01. The terms rearranged germline human kappa light chain IgVκ1-39*01/1GJκ1*01, IGKV1-39/IGKJ1, huVκ1-39 light chain or in short huVκ1-39 are used interchangeably throughout the application. Obviously, those of skill in the art will recognize that "common" also refers to functional equivalents of the light chain of which the amino acid sequence is not identical. Many variants of said light chain exist wherein mutations (deletions, substitutions, additions) are present that do not materially influence the formation of functional binding regions. The light chain of the present invention can also be a light chain as specified herein above, having 1-5 amino acid insertions, deletions, substitutions or a combination thereof.

Also contemplated are antibodies wherein a VH is capable of specifically recognizing a first antigen and the VL, paired with the VH in a immunoglobulin variable domain, is capable of specifically recognizing a second antigen. The resulting VH/VL pair will bind either antigen 1 or antigen 2. Such so called "two-in-one antibodies", described in for instance WO 2008/027236, WO 2010/108127 and Schaefer et al (Cancer Cell 20, 472-486, October 2011), are different from bispecific antibodies of the invention and are further referred to as "two-in-one" antibodies. Such "two-in-one" antibodies have identical arms and are not antibodies of the present invention.

The term 'ErbB-2' as used herein refers to the protein that in humans is encoded by the ERBB-2 gene. Alternative names for the gene or protein include CD340; HER-2; HER-2/neu; MLN 19; NEU; NGL; TKR1. The ERBB-2 gene is frequently called HER2 (from human epidermal growth factor receptor 2). Where reference is made herein to ErbB-2, the reference refers to human ErbB-2. An antibody comprising an antigen-binding site that binds ErbB-2, binds human ErbB-2. The ErbB-2 antigen-binding site may, due to sequence and tertiary structure similarity between human and other mammalian orthologs, also bind such an ortholog but not necessarily so. Database accession numbers for the human ErbB-2 protein and the gene encoding it are (NP_001005862.1, NP_004439.2 NC_000017.10 NT_010783.15 NC_018928.2). The accession numbers are primarily given to provide a further method of identification of ErbB-2 as a target, the actual sequence of the ErbB-2 protein bound the antibody may vary, for instance because of a mutation in the encoding gene such as those occurring in some cancers or the like. The ErbB-2 antigen binding site binds ErbB-2 and a variety of variants thereof, such as those expressed by some ErbB-2 positive tumor cells.

The term "binding agent of ErbB-2" as used herein refers to any molecule or compound capable of binding to ErbB-2. An "inhibitor of ErbB-2" as used herein refers to any molecule or compound capable of reducing or attenuating, either directly or indirectly, an activity of ErbB-2. Such an inhibitor may be a small molecule or may be a biologic, for example an antibody.

The term 'ErbB-3' as used herein refers to the protein that in humans is encoded by the ERBB-3 gene. Alternative names for the gene or protein are HER3; LCCS2; MDA-BF-1; c-ErbB-3; c-erbb-3; erbb-3-S; p180-Erbb-3; p45-sErbb-3; and p85-sErbb-3. Where reference is made herein to ErbB-3, the reference refers to human ErbB-3. An antibody comprising an antigen-binding site that binds ErbB-3, binds human ErbB-3. The ErbB-3 antigen-binding site, may, due to sequence and tertiary structure similarity between human and other mammalian orthologs, also bind such an ortholog but not necessarily so. Database accession numbers for the human ErbB-3 protein and the gene encoding it are (NP_001005915.1 NP_001973.2, NC_000012.11 NC_018923.2 NT_029419.12 ). The accession numbers are primarily given to provide a further method of identification of ErbB-3 as a target, the actual sequence of the ErbB-3 protein bound by an antibody may vary, for instance because of a mutation in the encoding gene such as those occurring in some cancers or the like. The ErbB-3 antigen binding site binds ErbB-3 and a variety of variants thereof, such as those expressed by some ErbB-2 positive tumor cells.
The term "binding agent of ErbB-3" as used herein refers to any molecule or compound capable of binding to ErbB-3. An "inhibitor of ErbB-3" as used herein refers to any molecule or compound capable of reducing or attenuating, either directly or indirectly, an activity of ErbB-3. Such an inhibitor may be an antibody, for example patritumab, MM-121 (seribantumab), lumretuzumab.

A bispecific antibody of the invention that comprises a first antigen-binding site that binds ErbB-2 and a second antigen-binding site that binds ErbB-3, can reduce or reduces a ligand-induced receptor function of ErbB-3 on an ErbB-2 and ErbB-3 positive cell. In the presence of excess ErbB-2, ErbB-2/ErbB-3 heterodimers may provide a growth signal to the expressing cell in the absence of detectable ligand for the ErbB-3 chain in the heterodimer. This ErbB-3 receptor function is herein referred as a ligand-independent receptor function of ErbB-3. The ErbB-2/ErbB-3 heterodimer also provide a growth signal to the expressing cell in the presence of an ErbB-3 ligand. This ErbB-3 receptor function is herein referred to as a ligand-induced receptor function of ErbB-3.

The term "ErbB-3 ligand" as used herein refers to polypeptides which bind and activate ErbB-3. Examples of ErbB-3 ligands include, but are not limited to neuregulin 1 (NRG) and neuregulin 2, betacellulin, heparin-binding epidermal growth factor, and epiregulin. The term includes biologically active fragments and/or variants of a naturally occurring polypeptide.

In a preferred embodiment of the invention the ligand-induced receptor function of ErbB-3 is ErbB-3 ligand-induced growth of an ErbB-2 and ErbB-3 positive cell. In a preferred embodiment said cell is an MCF-7 cell (ATCC^{®} HTB-22^{™}); an SKBR3 (ATCC^{®} HTB-30^{™}) cell; an NCI-87 (ATCC^{®} CRL-5822^{™}) cell; a BxPC-3-luc2 cell (Perkin Elmer 125058), a BT-474 cell (ATCC^{®} HTB-20^{™}) or a JIMT-1 cell (DSMZ no.: ACC 589).

In a preferred embodiment the ErbB-2 and ErbB-3 positive cell comprises at least 50.000 ErbB-2 receptors on the cell surface. In a preferred embodiment at least 100.000 ErbB-2 receptors. In one preferred embodiment, the ErbB-2 and ErbB-3 positive cell comprises at least 1.000.000 ErbB-2 receptors on the cell surface. In another preferred embodiment the ErbB-2 and ErbB-3 positive cell comprises no more than 1.000.000 ErbB-2 receptors on the cell surface. Currently used therapies such as trastuzumab (Herceptin) and pertuzumab are only prescribed for patients with malignant ErbB-2 positive cells that have more than 1.000.000 ErbB-2 receptors on their cell surface, in order to obtain a clinical response. Patients with ErbB-2 positive tumor cells with more than 1.000.000 ErbB-2 receptors on their cell surface are typically classified as ErbB-2 [+++]. Patients are for instance classified using immunohistochemistry or fluorescence *in situ* hybridization. The HercepTest^{™} and/or HER2 FISH (pharm Dx^{™}) are marketed both by Dako Denmark A/S, and/or using a HERmark^{®} assay, marketed by Monogram Biosciences. Trastuzumab and pertuzumab are only prescribed to ErbB-2 [+++] patients because patients with lower ErbB-2 concentrations typically do not exhibit a sufficient clinical response when treated with trastuzumab and pertuzumab. The invention, however, provides bispecific antibodies that also have an improved binding affinity for cells with a lower ErbB-2 receptor concentration, as compared to trastuzumab. As shown in the Examples, proliferation of such cells with lower ErbB2 expression is effectively counteracted with an antibody according to the invention. Such lower ErbB-2 receptor concentration is present on malignant cells of patients that are classified as ErbB-2 [++] or ErbB-2 [+]. Also, relapsed ErbB-2 positive tumors often have an ErbB-2 receptor concentration of lower than 1.000.000 receptors per cell. Such ErbB-2 [++] or ErbB-2 [+] patients, as well as patients with a relapsed ErbB-2 positive tumor, are therefore preferably treated with a bispecific antibody according to the present invention. Further provided is therefore a bispecific antibody comprising a first antigen-binding site that binds ErbB-2 and a second antigen-binding site that binds ErbB-3, wherein the antibody can reduce ligand-induced growth of an ErbB-2 and ErbB-3 positive cell that has less than 1.000.000 ErbB-2 cell-surface receptors. Also provided is a method for the treatment of a subject having a ErbB-2, ErbB-3 or ErbB-2/ErbB-3 positive tumor or at risk of having said tumor, wherein said tumor has less than 1.000.000 ErbB-2 cell-surface receptors per cell, the method comprising administering to the subject a bispecific antibody or pharmaceutical composition according to the invention. A bispecific antibody according to the invention for use in the treatment of a subject having or at risk of having an ErbB-2, ErbB-3 or ErbB-2/ErbB-3 positive tumor, wherein said tumor has less than 1.000.000 ErbB-2 cell-surface receptors per cell, is also herewith provided. Said antibody according to the present invention is typically capable of reducing a ligand-induced receptor function, preferably ligand induced growth, of ErbB-3 on a ErbB-2 and ErbB-3 positive cell. Said antibody according to the invention preferably comprises a first antigen-binding site that binds domain I of ErbB-2 and a second antigen-binding site that binds domain III of ErbB-3. In one preferred embodiment, the affinity of said second antigen-binding site for an ErbB-3 positive cell is equal to, or higher than, the affinity of said first antigen-binding site for an ErbB-2 positive cell, as explained herein below in more detail. The affinity of said second antigen-binding site for an ErbB-3 positive cell is preferably lower than or equal to 2.0 nM, more preferably lower than or equal to 1.39 nM, more preferably lower than or equal to 0.99 nM. The affinity of said first antigen-binding site for an ErbB-2 positive cell is preferably lower than or equal to 5.0 nM, preferably lower than or equal to 4.5 nM preferably lower than or equal to 4.0 nM.

In one preferred embodiment, said antibody according to the invention comprises an antigen-binding site that binds at least one amino acid of domain I of ErbB-2 selected from the group consisting of T144, T164, R166, P172, G179, S180 and R181, and surface-exposed amino acid residues that are located within about 5 amino acid positions from T144, T164, R166, P172, G179, S180 or R181.
In one preferred embodiment, said antibody according to the invention preferably comprises an antigen-binding site that binds at least one amino acid of domain III of ErbB-3 selected from the group consisting of R426 and surface-exposed amino acid residues that are located within 11.2 Å from R426 in the native ErbB-3 protein.

To establish whether a tumor is positive for ErbB-3 the skilled person can for instance determine the ErbB-3 amplification and/or staining in immunohistochemistry. At least 10% tumor cells in a biopt should be positive. The biopt can also contain 20%, 30% 40% 50% 60% 70% or more positive cells.

As used herein the ligand-induced receptor function is reduced by at least 20%, preferably at least 30, 40, 50 60, or at least 70% in a particularly preferred embodiment the ligand-induced receptor function is reduced by 80, more preferably by 90%. The reduction is preferably determined by determining a ligand-induced receptor function in the presence of a bispecific antibody of the invention, and comparing it with the same function in the absence of the antibody, under otherwise identical conditions. The conditions comprise at least the presence of an ErbB-3 ligand. The amount of ligand present is preferably an amount that induces half of the maximum growth of an ErbB-2 and ErbB-3 positive cell line. The ErbB-2 and ErbB-3 positive cell line for this test is preferably the MCF-7 cell line (ATCC^{®} HTB-22^{™}), the SKBR3 cell line (ATCC^{®} HTB-30^{™}) cells, the JIMT-1 cell line (DSMZ ACC 589) or the NCI-87 cell line (ATCC^{®} CRL-5822^{™}). The test and/or the ligand for determining ErbB-3 ligand-induced receptor function is preferably a test for ErbB-3 ligand induced growth reduction as specified in the examples.

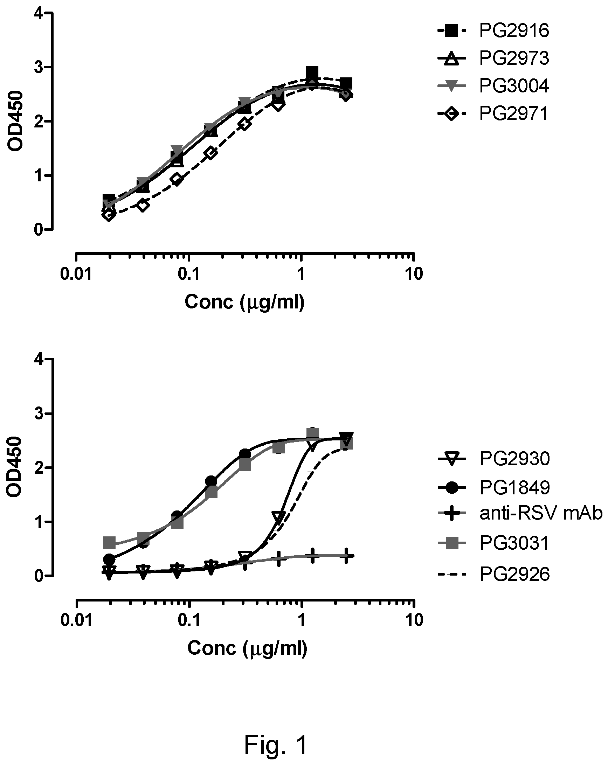

The ErbB-2 protein contains several domains (see for reference figure 1 of Landgraf, R Breast Cancer Res. 2007; 9(1): 202-). The extracellular domains are referred to as domains I-IV. The place of binding to the respective domains of antigen-binding sites of antibodies described herein has been mapped (see examples). A bispecific antibody of the invention with an antigen-binding site (first antigen-binding site) that binds domain I or domain IV of ErbB-2 (first antigen-binding site) comprises a heavy chain variable region that maintains significant binding specificity and affinity for ErbB-2 when combined with various light chains. Bispecific antibodies with an antigen-binding site (first antigen-binding site) that binds domain I or domain IV of ErbB-2 (first antigen-binding site) and an antigen-binding site for ErbB-3 (second antigen-binding site) were found to be more effective in reducing a ligand-induced receptor function of ErbB-3 when compared to a bispecific antibody comprising an antigen-binding site (first antigen-binding site) that binds to another extra-cellular domain of ErbB-2. A bispecific antibody comprising an antigen-binding site (first antigen-binding site) that binds ErbB-2, wherein said antigen-binding site binds to domain I or domain IV of ErbB-2 is preferred. Preferably said antigen-binding site binds to domain IV of ErbB-2. A bispecific antibody with an antigen-binding site (first antigen-binding site) that binds ErbB-2, and that further comprises ADCC was found to be more effective than other ErbB-2 binding antibodies that did not have significant ADCC activity, particularly *in vivo.* A bispecific antibody according to the invention which exhibits ADCC is therefore preferred. It was found that antibodies wherein said first antigen-binding site binds to domain IV of ErbB-2 had intrinsic ADCC activity. A domain I binding ErbB-2 binding antibody that has low intrinsic ADCC activity can be engineered to enhance the ADCC activity Fc regions mediate antibody function by binding to different receptors on immune effector cells such as macrophages, natural killer cells, B-cells and neutrophils. Some of these receptors, such as CD16A (FcyRIIIA) and CD32A (FcyRIIA), activate the cells to build a response against antigens. Other receptors, such as CD32B, inhibit the activation of immune cells. By engineering Fc regions (through introducing amino acid substitutions) that bind to activating receptors with greater selectivity, antibodies can be created that have greater capability to mediate cytotoxic activities desired by an anti-cancer Mab.

One technique for enhancing ADCC of an antibody is afucosylation. (See for instance Junttila, T. T., K. Parsons, et al. (2010). "Superior In vivo Efficacy of Afucosylated Trastuzumab in the Treatment of HER2-Amplified Breast Cancer." Cancer Research 70(11): 4481-4489). Further provided is therefore a bispecific antibody according to the invention, which is afucosylated. Alternatively, or additionally, multiple other strategies can be used to achieve ADCC enhancement, for instance including glycoengineering (Kyowa Hakko/Biowa, GlycArt (Roche) and Eureka Therapeutics) and mutagenesis (Xencor and Macrogenics), all of which seek to improve Fc binding to low-affinity activating FcyRIIIa, and/or to reduce binding to the low affinity inhibitory FcγRIIb.

Several *in vitro* methods exist for determining the efficacy of antibodies or effector cells in eliciting ADCC. Among these are chromium-51 [Cr51] release assays, europium [Eu] release assays, and sulfur-35 [S35] release assays. Usually, a labeled target cell line expressing a certain surface-exposed antigen is incubated with antibody specific for that antigen. After washing, effector cells expressing Fc receptor CD16 are typically co-incubated with the antibody-labeled target cells. Target cell lysis is subsequently typically measured by release of intracellular label, for instance by a scintillation counter or spectrophotometry. A preferred test is detailed in the Examples.

One advantage of the present invention is the fact that binding of antibodies according to the invention such as for instance PB4188 to ErbB-2 and ErbB-3 positive cells results in internalization that is to the same extent as compared to trastuzumab. If a combination of trastuzumab and pertuzumab is used, internalization of these antibodies is enhanced. This enhanced internalization, however, results in reduced ADCC. An antibody according to the present invention resulting in internalization that is essentially to the same extent as compared to trastuzumab is, therefore, preferred over a combination of trastuzumab and pertuzumab because with such antibody the ADCC activity is better maintained.

An antibody of the invention comprising an antigen-binding site that binds ErbB-3, interferes with binding of an ErbB-3 ligand to ErbB-3. Such antibodies are more effective in reducing a ligand-induced receptor function of ErbB-3 on an ErbB-2 and ErbB-3 positive cell line, particularly in the context of an bi-specific antibody that also comprises an antigen-binding site that binds ErbB-2.

Preferred embodiments of the current invention provide a bispecific antibody comprising a first antigen-binding site that binds ErbB-2 and a second antigen-binding site that binds ErbB-3, wherein said first antigen-binding site binds domain I of ErbB-2. As shown in the Examples, bispecific antibodies having these characteristics are well capable of binding ErbB-2 and ErbB-3 positive cells and counteracting their activity (such as the ligand-induced receptor function of ErbB-3 and the ligand-induced growth of an ErbB-2 and ErbB3 positive cell). Moreover, bispecific antibodies according to the invention comprising a first antigen-binding site that binds domain I of ErbB-2 are particularly suitable for use in combination with existing anti-ErbB-2 therapies like trastuzumab and pertuzumab, because trastuzumab and pertuzumab bind different domains of ErbB-2. Trastuzumab binds domain IV of ErbB-2 and pertuzumab binds domain II of ErbB-2. Hence, bispecific antibodies according to the invention that bind domain I of ErbB-2 are preferred because they do not compete with trastuzumab and pertuzumab for the same epitope.

Another preferred embodiment provides a bispecific antibody comprising a first antigen-binding site that binds ErbB-2 and a second antigen-binding site that binds ErbB-3, wherein said second antigen-binding site binds domain III of ErbB-3. Such antibody according to the invention is particularly suitable for combination therapy with currently used anti- ErbB-3 binding molecules that do not bind domain III of ErbB-3, such as MM-121 (Merrimack Pharmaceuticals; also referred to as #Ab6) and RG7116 (Roche) that bind domain I of ErbB-3, because then the different binding molecules do not compete with each other for the same epitope.

Preferably, a bispecific antibody is provided that comprises a first antigen-binding site that binds ErbB-2 and a second antigen-binding site that binds ErbB-3, wherein said first antigen-binding site binds domain I of ErbB-2 and said second antigen-binding site binds domain III of ErbB-3. Such antibody is particularly suitable for combination therapy with anti- ErbB-2 binding molecules that do not bind domain I of ErbB-2, such as trastuzumab and pertuzumab, and with anti- ErbB-3 binding molecules that do not bind domain III of ErbB-3, such as MM-121 (#Ab6) and RG7116.

One preferred embodiment provides a bispecific antibody that comprises a first antigen-binding site that binds ErbB-2 and a second antigen-binding site that binds ErbB-3, wherein said first antigen-binding site binds domain I of ErbB-2 and said second antigen-binding site binds domain III of ErbB-3 and wherein the antibody can reduce a ligand-induced receptor function of ErbB-3 on a ErbB-2 and ErbB-3 positive cell. Said antibody can preferably reduce ligand-induced growth of an ErbB-2 and ErbB-3 positive cell.

Further embodiments of the invention provide a bispecific antibody comprising a first antigen-binding site that binds ErbB-2 and a second antigen-binding site that binds ErbB-3, wherein the affinity (KD) of said second antigen-binding site for an ErbB-3 positive cell is equal to, or higher than, the affinity of said first antigen-binding site for an ErbB-2 positive cell. Contrary to prior art bispecific compounds such as for instance MM-111 from Merrimack Pharmaceuticals, which have a higher affinity for ErbB-2 than for ErbB-3, the present invention provides bispecific antibodies which have an ErbB-3-specific arm with an affinity for ErbB-3 on cells that is higher than the affinity of the ErbB-2-specific arm for ErbB-2 on cells. Such bispecific antibodies are better capable of binding ErbB-3, despite the low cell surface concentration of ErbB-3. This provides the advantage that the functional activity against ErbB-3 is enhanced as compared to prior art compounds, meaning that these bispecific antibodies according to the invention are better capable of counteracting ErbB-3 activity (such as ligand-induced growth).

As used herein, the term "affinity" refers to the KD value.

The affinity (KD) of said second antigen-binding site for an ErbB-3 positive cell is preferably lower than or equal to 2.0 nM, more preferably lower than or equal to 1.5 nM, more preferably lower than or equal to 1.39 nM, more preferably lower than or equal to 0.99 nM. In one preferred embodiment, the affinity of said second antigen-binding site for ErbB-3 on SK-BR-3 cells is lower than or equal to 2.0 nM, more preferably lower than or equal to 1.5 nM, more preferably lower than or equal to 1.39 nM, preferably lower than or equal to 0.99 nM. In one embodiment, said affinity is within the range of 1.39-0.59 nM. In one preferred embodiment, the affinity of said second antigen-binding site for ErbB-3 on BT-474 cells is lower than or equal to 2.0 nM, more preferably lower than or equal to 1.5 nM, more preferably lower than or equal to 1.0 nM, more preferably lower than 0.5 nM, more preferably lower than or equal to 0.31 nM, more preferably lower than or equal to 0.23 nM. In one embodiment, said affinity is within the range of 0.31-0.15 nM. The above-mentioned affinities are preferably as measured using steady state cell affinity measurements, wherein cells are incubated at 4°C using radioactively labeled antibody, where after cell-bound radioactivity is measured, as described in the Examples.

The affinity (KD) of said first antigen-binding site for an ErbB-2 positive cell is preferably lower than or equal to 5.0 nM, more preferably lower than or equal to 4.5 nM, more preferably lower than or equal to 3.9 nM. In one preferred embodiment, the affinity of said first antigen-binding site for ErbB-2 on SK-BR-3 cells is lower than or equal to 5.0 nM, preferably lower than or equal to 4.5 nM, more preferably lower than or equal to 4.0 nM, more preferably lower than or equal to 3.5 nM, more preferably lower than or equal to 3.0 nM, more preferably lower than or equal to 2.3 nM. In one embodiment, said affinity is within the range of 3.0-1.6 nM. In one preferred embodiment, the affinity of said first antigen-binding site for ErbB-2 on BT-474 cells is lower than or equal to 5.0 nM, preferably lower than or equal to 4.5 nM, more preferably lower than or equal to 3.9 nM. In one embodiment, said affinity is within the range of 4.5-3.3 nM. The above-mentioned affinities are preferably as measured using steady state cell affinity measurements, wherein cells are incubated at 4°C using radioactively labeled antibody, where after cell-bound radioactivity is measured, as described in the Examples.

In one preferred embodiment, a bispecific antibody according to the invention is provided, wherein the affinity (KD) of said bispecific antibody for BT-474 cells is lower than or equal to 5.0 nM, preferably lower than or equal to 4.5 nM, more preferably lower than or equal to 4.0 nM, more preferably lower than or equal to 3.5 nM, more preferably lower than or equal to 3.7 nM, preferably lower than or equal to 3.2 nM. In one embodiment, said affinity is within the range of 3.7-2.7 nM. In one preferred embodiment, a bispecific antibody according to the invention is provided, wherein the affinity of said bispecific antibody for SK-BR-3 cells is lower than or equal to 5.0 nM, preferably lower than or equal to 4.5 nM, more preferably lower than or equal to 4.0 nM, more preferably lower than or equal to 3.5 nM, more preferably lower than or equal to 3.0 nM, preferably lower than or equal to 2.5 nM, more preferably lower than or equal to 2.0 nM. In one embodiment, said affinity is within the range of 2.4-1.6 nM. Again, the above-mentioned affinities are preferably as measured using steady state cell affinity measurements, wherein cells are incubated at 4°C using radioactively labeled antibody, where after cell-bound radioactivity is measured, as described in the Examples.

Further preferred embodiments of the invention provide a bispecific antibody comprising a first antigen-binding site that binds ErbB-2 and a second antigen-binding site that binds ErbB-3, wherein the affinity (KD) of said second antigen-binding site for an ErbB-3 positive cell is equal to, or higher than, the affinity of said first antigen-binding site for an ErbB-2 positive cell, and wherein the antibody can reduce a ligand-induced receptor function of ErbB-3 on a ErbB-2 and ErbB-3 positive cell. Said antibody can preferably reduce ligand-induced growth of an ErbB-2 and ErbB-3 positive cell.

The above-mentioned antibodies according to the invention with a high affinity for ErbB-3 preferably bind domain I of ErbB2 and/or domain III of ErbB-3. Further provided is, therefore, a bispecific antibody according to the invention that comprises a first antigen-binding site that binds domain I of ErbB-2 and a second antigen-binding site that binds ErbB-3, wherein the affinity (KD) of said second antigen-binding site for an ErbB-3 positive cell is equal to, or higher than, the affinity of said first antigen-binding site for an ErbB-2 positive cell. Also provided is a bispecific antibody according to the invention that comprises a first antigen-binding site that binds ErbB-2 and a second antigen-binding site that binds domain III of ErbB-3, wherein the affinity of said second antigen-binding site for an ErbB-3 positive cell is equal to, or higher than, the affinity of said first antigen-binding site for an ErbB-2 positive cell. In a particularly preferred embodiment a bispecific antibody according to the invention is provided that comprises a first antigen-binding site that binds domain I of ErbB-2 and a second antigen-binding site that binds domain **III** of ErbB-3, wherein the affinity of said second antigen-binding site for an ErbB-3 positive cell is equal to, or higher than, the affinity of said first antigen-binding site for an ErbB-2 positive cell.

Said second antigen-binding site preferably binds domain III of ErbB-3 and has an affinity (KD) for an ErbB-3 positive cell that is lower than or equal to 2.0 nM, more preferably lower than or equal to 1.5 nM, preferably lower than or equal to 1.39 nM, more preferably lower than or equal to 0.99 nM. In one preferred embodiment, said second antigen-binding site binds domain III of ErbB-3 and has an affinity for ErbB-3 on SK-BR-3 cells that is lower than or equal to 2.0 nM, more preferably lower than or equal to 1.5 nM, preferably lower than or equal to 1.39 nM, more preferably lower than or equal to 0.99 nM. In one embodiment, said affinity is within the range of 1.39-0.59 nM. In one preferred embodiment, said second antigen-binding site binds domain III of ErbB-3 and has an affinity for ErbB-3 on BT-474 cells that is lower than or equal to 2.0 nM, more preferably lower than or equal to 1.5 nM, more preferably lower than or equal to 1.0 nM, more preferably lower than or equal to 0.5 nM, more preferably lower than or equal to 0.31 nM, more preferably lower than or equal to 0.23 nM. In one embodiment, said affinity is within the range of 0.31-0.15 nM.

Said first antigen-binding site preferably binds domain I of ErbB-2 and has an affinity (KD) for an ErbB-2 positive cell that is lower than or equal to 5.0 nM, more preferably lower than or equal to 4.5 nM, more preferably lower than or equal to 3.9 nM. In one preferred embodiment, said first antigen-binding site binds domain I of ErbB-2 and has an affinity for ErbB-2 on SK-BR-3 cells that is lower than or equal to 5.0 nM, more preferably lower than or equal to 4.5 nM, more preferably lower than or equal to 4.0 nM, more preferably lower than or equal to 3.5 nM, more preferably lower than or equal to 3.0 nM, more preferably lower than or equal to 2.5 nM, more preferably lower than or equal to 2.3 nM. In one embodiment, said affinity is within the range of 3.0-1.6 nM. The affinity of said bispecific antibody for SK-BR-3 cells is preferably lower than or equal to 5.0 nM, more preferably lower than or equal to 4.5 nM, more preferably lower than or equal to 4.0 nM, more preferably lower than or equal to 3.5 nM, more preferably lower than or equal to 3.0 nM, more preferably lower than or equal to 2.5 nM, more preferably lower than or equal to 2.4 nM, more preferably lower than or equal to 2.0 nM. In one embodiment, said affinity is within the range of 2.4-1.6 nM.

In one preferred embodiment, said first antigen-binding site binds domain I of ErbB-2 and has an affinity (KD) for ErbB-2 on BT-474 cells that is lower than or equal to 5.0 nM, more preferably lower than or equal to 4.5 nM, preferably lower than or equal to 3.9 nM. In one embodiment, said affinity is within the range of 4.5-3.3 nM. The affinity of said bispecific antibody for BT-474 cells is preferably lower than or equal to 5.0 nM, more preferably lower than or equal to 4.5 nM, more preferably lower than or equal to 4.0 nM, more preferably lower than or equal to 3.7 nM, more preferably lower than or equal to 3.2 nM. In one embodiment, said affinity is within the range of 3.7-2.7 nM.

Again, the above-mentioned affinities are preferably as measured using steady state cell affinity measurements, wherein cells are incubated at 4°C using radioactively labeled antibody, where after cell-bound radioactivity is measured, as described in the Examples.

Another preferred embodiment provides a bispecific antibody according to the invention comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell, wherein said bispecific antibody does not significantly affect the survival of cardiomyocytes. Cardiotoxicity is a known risk factor in ErbB-2 targeting therapies and the frequency of complications is increased when trastuzumab is used in conjunction with anthracyclines thereby inducing cardiac stress. For instance, the combination of doxycycline (DOX) with trastuzumab induces severe cardiac side effects. Clinical studies have estimated that 5% to 10% of patients who receive trastuzumab in the adjuvant setting of breast cancer develop cardiac dysfunction (Guarneri et al., J Clin Oncol., 1985, 3:818-26; Ewer MS et al., Nat Rev Cardiol 2010;7:564-75). However, in a retrospective study, it was demonstrated that the risk for developing asymptomatic cardiac dysfunction is actually as high as about 25% when trastuzumab is used in the adjuvant setting with DOX (Wadhwa et al., Breast Cancer Res Treat 2009;117:357-64). As shown in the Examples, the present invention provides antibodies that target ErbB-2 and that do not, or to a significantly lesser extent as compared to trastuzumab and pertuzumab, affect the survival of cardiomyocytes. This provides an important advantage since cardiotoxicity is reduced. This is already advantageous for people who do not suffer from an impaired cardiac function, and even more so for people who do suffer from an impaired cardiac function, or who are at risk thereof, such as for instance subjects suffering from congestive heart failure (CHF), left ventricular dysfunction (LVD) and/or a ≥ 10% decreased Left Ventricular Ejection Fraction (LVEF), and/or subjects who have had a myocardial infarction. Antibodies according to the invention that do not significantly affect the survival of cardiomyocytes are, therefore, preferred. *In vitro,* the function of cardiomyocytes is for instance measured by determining the viability of cardiomyocytes, by determining BNP (B-type natriuretic peptide, which is a cardiac biomarker), by determining QT prolongation, and/or by determining mitochondrial membrane potential.

Said antibody according to the invention preferably comprises a first antigen-binding site that binds domain I of ErbB-2 and a second antigen-binding site that binds domain III of ErbB-3. One embodiment provides an antibody according to the invention that does not significantly affect the survival of cardiomyocytes, comprising a first antigen-binding site that binds ErbB-2 and a second antigen-binding site that binds ErbB-3, wherein the affinity of said second antigen-binding site for an ErbB-3 positive cell is equal to, or higher than, the affinity of said first antigen-binding site for an ErbB-2 positive cell. The affinity of said second antigen-binding site for an ErbB-3 positive cell is preferably lower than or equal to 2.0 nM, more preferably lower than or equal to 1.39 nM, more preferably lower than or equal to 0.99 nM. The affinity of said first antigen-binding site for an ErbB-2 positive cell is preferably lower than or equal to 5.0 nM, preferably lower than or equal to 4.5 nM preferably lower than or equal to 4.0 nM.

In one preferred embodiment said antibody that does not significantly affect the survival of cardiomyocytes comprises:
- at least the CDR3 sequence, preferably at least the CDR1, CDR2 and CDR3 sequences, or at least the heavy chain variable region sequence, of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or a heavy chain variable region sequence that differs in at most 15 amino acids, preferably in at most 1, 2, 3, 4, 5, 6, 7, 8, 9 or 10 amino acids, more preferably in at most 1, 2, 3, 4 or 5 amino acids, from the recited heavy chain variable region sequences; and/or
- at least the CDR3 sequence, preferably at least the CDR1, CDR2 and CDR3 sequences, or at least the heavy chain variable region sequence, of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or a heavy chain variable region sequence that differs in at most 15 amino acids, preferably in at most 1, 2, 3, 4, 5, 6, 7, 8, 9 or 10 amino acids, more preferably in at most 1, 2, 3, 4 or 5 amino acids, from the recited heavy chain variable region sequences. In one preferred embodiment, said antibody is PB4188.

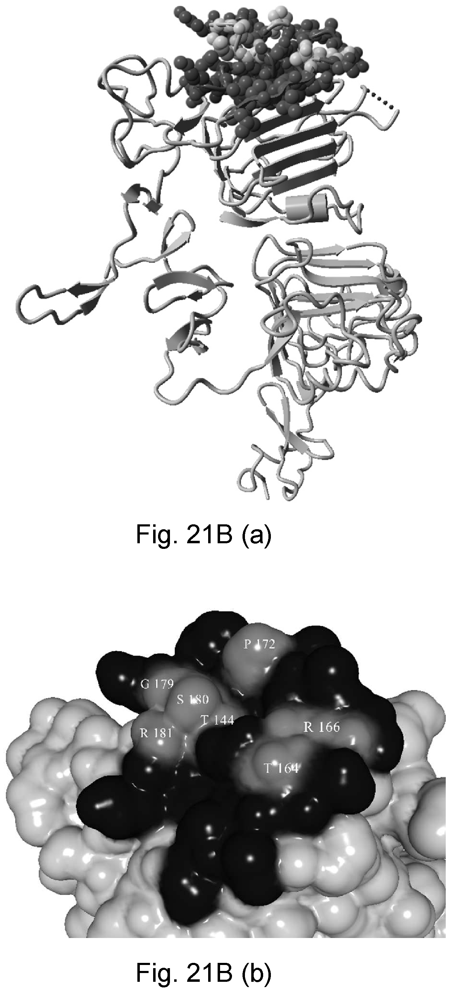

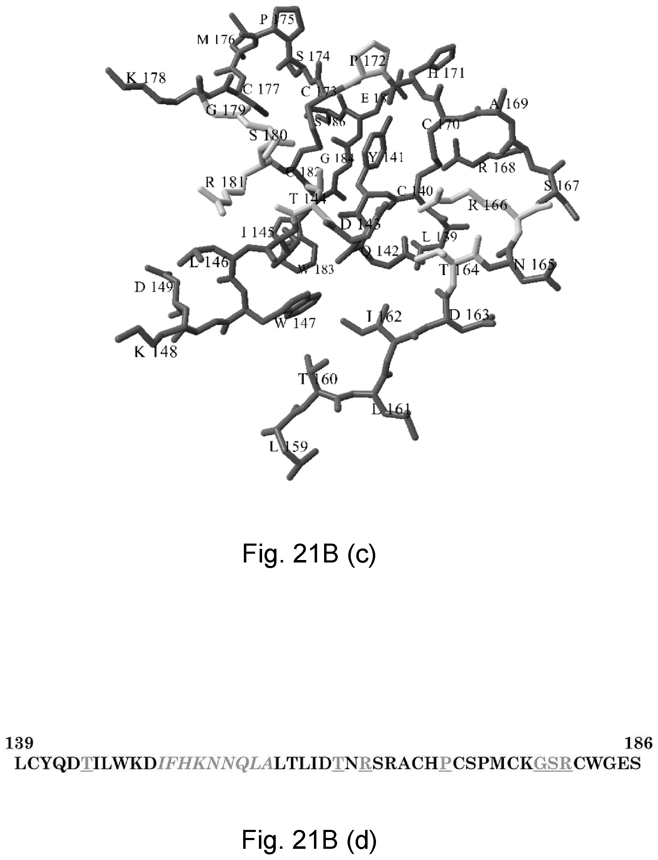

Another aspect of the present invention provides an antibody according to the invention, comprising a first antigen-binding site that binds ErbB-2 and a second antigen-binding site that binds ErbB-3, wherein said antibody comprises an antigen-binding site that binds at least one amino acid residue of domain I of ErbB-2 selected from the group consisting of T144, T164, R166, P172, G179, S180 and R181, and surface-exposed amino acid residues that are located within about 5 amino acid positions from T144, T164, R166, P172, G179, S180 or R181. The amino acid residue numbering is that of Protein Data Bank (PDB) ID #1S78. As shown in the Examples, antibodies binding this region of domain I of ErbB-2 exhibit particularly good binding characteristics and they are capable of counteracting the activity of ErbB-2 positive cells (such as ligand-induced receptor function of ErbB-3 on a ErbB-2 and ErbB-3 positive cell, and/or ligand-induced growth of such cell). Moreover, such antibodies are particularly suitable for combination therapy with currently known anti-ErbB-2 monoclonal antibodies like trastuzumab (that binds domain IV of ErbB-2) and pertuzumab (that binds domain II of ErbB-2) because they bind different domains of ErbB-2. Hence, these antibodies can be used simultaneously without competition for the same epitope. The term "surface-exposed amino acid residues that are located within about 5 amino acid positions from T144, T164, R166, P172, G179, S180 or R181" refers to amino acid residues that are in the primary amino acid sequence located within about the first five amino acid residues adjacent to the recited residues and that are at least in part exposed to the outside of the protein, so that they can be bound by antibodies (see for instance Figure 21B). Preferably, said amino acid residue located within about 5 amino acid positions from T144, T164, R166, P172, G179, S180 or R181 is selected from the group consisting of L139, C140, Y141, Q142, D143, I145, L146, W147, K148, D149, L159, T160, L161, I162, D163, N165, S167, R168, A169, C170, H171, C173, S174, P175, M176, C177, K178, C182, W183, G184, E185 and S186. Preferably, said antibody comprises an antigen-binding site that binds at least 2 or at least 3 amino acid residues of domain I of ErbB-2 selected from the group consisting of T144, T164, R166, P172, G179, S180 and R181, and surface-exposed amino acid residues that are located within 5 amino acid positions from T144, T164, R166, P172, G179, S180 or R181.

In one preferred embodiment, a bispecific antibody according to the invention is provided, wherein said antibody comprises an antigen-binding site that binds at least T144, R166 and R181 of domain I of ErbB-2. Another embodiment provides a bispecific antibody according to the invention, wherein said antibody comprises an antigen-binding site that binds at least T144, R166, P172, G179 and R181 of domain I of ErbB-2. Another embodiment provides a bispecific antibody according to the invention, wherein said antibody comprises an antigen-binding site that binds at least T144, T164, R166, P172, G179, S180 and R181 of domain I of ErbB-2.

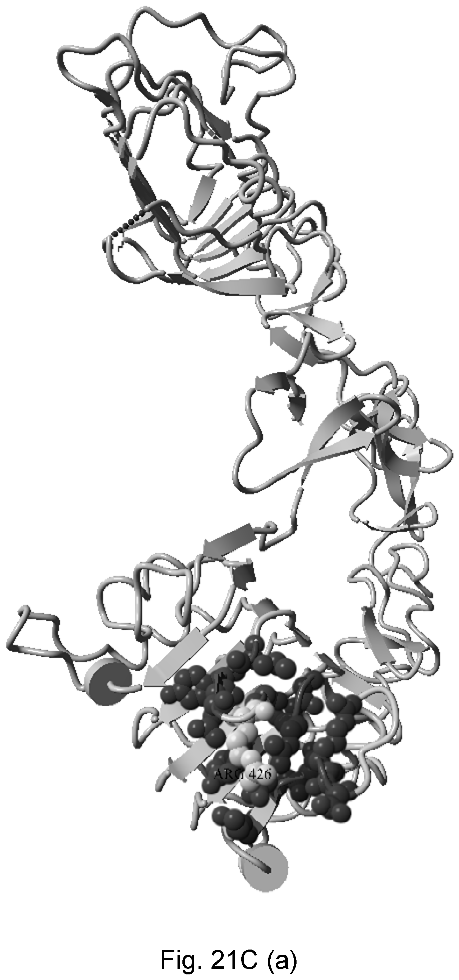

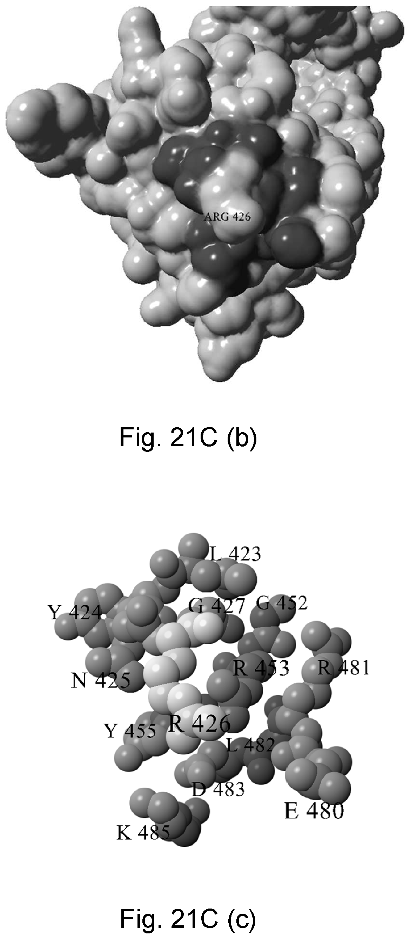

Another aspect of the present invention provides an antibody comprising a first antigen-binding site that binds ErbB-2 and a second antigen-binding site that binds ErbB-3, wherein said antibody comprises an antigen-binding site that binds at least one amino acid of domain III of ErbB-3 selected from the group consisting R426 and surface-exposed amino acid residues that are located within 11.2 Å from R426 in the native ErbB-3 protein. The amino acid residue numbering is that of Protein Data Bank (PDB) ID #4P59. As shown in the Examples, antibodies binding this region of domain III of ErbB-3 exhibit particularly good binding characteristics and they are capable of counteracting the activity of ErbB-3 positive cells (such as ligand-induced receptor function of ErbB-3 on a ErbB-2 and ErbB-3 positive cell, and/or ligand-induced growth of such cell). The term "surface-exposed amino acid residues that are located within 11.2 Å from R426 in the native ErbB-3 protein" refers to amino acid residues that are in the tertiary structure of the ErbB-3 protein spationally positioned within 11.2 Å from R426 and that are at least in part exposed to the outside of the protein, so that they can be bound by antibodies. Preferably, said amino acid residues that are located within 11.2 Å from R426 in the native ErbB-3 protein are selected from the group consisting of L423, Y424, N425, G427, G452, R453, Y455, E480, R481, L482, D483 and K485 (see for instance Figure 21C and Table 15). In one preferred embodiment, a bispecific antibody according to the invention is provided, wherein said antibody comprises an antigen-binding site that binds at least R426 of domain III of ErbB-3. Preferably, said antibody comprises an antigen-binding site that binds at least R426 of domain III of ErbB-3.

A bispecific antibody of the invention is preferably afucosylated in order to enhance ADCC activity. A bispecific antibody of the invention preferably comprises a reduced amount of fucosylation of the N-linked carbohydrate structure in the Fc region, when compared to the same antibody produced in a normal CHO cell.

A bispecific antibody of the present invention is preferably used in humans. To this end a bispecific antibody of the invention is preferably a human or humanized antibody.

Tolerance of a human to a polypeptide is governed by many different aspects. Immunity, be it T-cell mediated, B-cell mediated or other is one of the variables that are encompassed in tolerance of the human for a polypeptide. The constant region of a bispecific antibody of the present invention is preferably a human constant region. The constant region may contain one or more, preferably not more than 10, preferably not more than 5 amino-acid differences with the constant region of a naturally occurring human antibody. It is preferred that the constant part is entirely derived from a naturally occurring human antibody. Various antibodies produced herein are derived from a human antibody variable domain library. As such these variable domains are human. The unique CDR regions may be derived from humans, be synthetic or derived from another organism. The variable region is considered a human variable region when it has an amino acid sequence that is identical to an amino acid sequence of the variable region of a naturally occurring human antibody, but for the CDR region. The variable region of an ErbB-2 binding VH, an ErbB-3 binding VH, or a light chain in an antibody of the invention may contain one or more, preferably not more than 10, preferably not more than 5 amino-acid differences with the variable region of a naturally occurring human antibody, not counting possible differences in the amino acid sequence of the CDR regions. Such mutations occur also in nature in the context of somatic hypermutation.

Antibodies may be derived from various animal species, at least with regard to the heavy chain variable region. It is common practice to humanize such e.g. murine heavy chain variable regions. There are various ways in which this can be achieved among which there are CDR-grafting into a human heavy chain variable region with a 3D-structure that matches the 3-D structure of the murine heavy chain variable region; deimmunization of the murine heavy chain variable region, preferably done by removing known or suspected T- or B- cell epitopes from the murine heavy chain variable region. The removal is typically by substituting one or more of the amino acids in the epitope for another (typically conservative) amino acid, such that the sequence of the epitope is modified such that it is no longer a Tor B-cell epitope.

Such deimmunized murine heavy chain variable regions are less immunogenic in humans than the original murine heavy chain variable region. Preferably a variable region or domain of the invention is further humanized, such as for instance veneered. By using veneering techniques, exterior residues which are readily encountered by the immune system are selectively replaced with human residues to provide a hybrid molecule that comprises either a weakly immunogenic or substantially non-immunogenic veneered surface. An animal as used in the invention is preferably a mammal, more preferably a primate, most preferably a human.

A bispecific antibody according to the invention preferably comprises a constant region of a human antibody. According to differences in their heavy chain constant domains, antibodies are grouped into five classes, or isotypes: IgG, IgA, IgM, IgD, and IgE. These classes or isotypes comprise at least one of said heavy chains that is named with a corresponding Greek letter. In a preferred embodiment the invention provides an antibody according to the invention wherein said constant region is selected from the group of IgG, IgA, IgM, IgD, and IgE constant regions, more preferably said constant region comprises an IgG constant region, more preferably an IgG1 constant region, preferably a mutated IgG1 constant region. Some variation in the constant region of IgG1 occurs in nature, such as for instance the allotypes G1m1, 17 and G1m3, and/or is allowed without changing the immunological properties of the resulting antibody. Typically between about 1-10 amino acid insertions, deletions, substitutions or a combination thereof are allowed in the constant region.

The invention in one embodiment provides an antibody comprising a variable domain that binds ErbB-2, wherein said antibody comprises at least the CDR3 sequence of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or wherein said antibody comprises a heavy chain CDR3 sequence that differs in at most three, preferably in at most two, preferably in no more than one amino acid from a CDR3 sequence of a VH selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E. Said antibody preferably comprises at least the CDR3 sequence of MF1849, MF2971, MF3958, MF3004 or MF3991, most preferably at least the CDR3 sequence of MF3958.

Said antibody preferably comprises at least the CDR1, CDR2 and CDR3 sequences of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or heavy chain CDR1, CDR2 and CDR3 sequences that differ in at most three, preferably in at most two, preferably in at most one amino acid from the CDR1, CDR2 and CDR3 sequences of MF2926, MF2930, MF1849; MF2973, MF3004, MF3958, MF2971, MF3025, MF2916, MF3991, MF3031, MF2889, MF2913, MF1847, MF3001, MF3003 or MF1898. Said antibody preferably comprises at least the CDR1, CDR2 and CDR3 sequences of MF1849, MF2971, MF3958, MF3004 or MF3991, most preferably at least the CDR1, CDR2 and CDR3 sequences of MF3958.

The invention also provides an antibody comprising a variable domain that binds ErbB-3, wherein said antibody comprises at least the CDR3 sequence of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or wherein said antibody comprises a heavy chain CDR3 sequence that differs in at most three, preferably in at most two, preferably in no more than one amino acid from a CDR3 sequence of a VH selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37. Said antibody preferably comprises at least the CDR3 sequence of MF3178, MF3176, MF3163, MF6058, MF6061 or MF6065, most preferably at least the CDR3 sequence of MF3178.

Said antibody preferably comprises at least the CDR1, CDR2 and CDR3 sequences of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or heavy chain CDR1, CDR2 and CDR3 sequences that differ in at most three, preferably in at most two, preferably in at most one amino acid from the CDR1, CDR2 and CDR3 sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074. Said antibody preferably comprises at least the CDR1, CDR2 and CDR3 sequences of MF3178, MF3176, MF3163, MF6058, MF6061 or MF6065, most preferably at least the CDR1, CDR2 and CDR3 sequence of MF3178.

The invention in one embodiment provides a bispecific antibody comprising a first antigen-binding site that binds ErbB-2 and a second antigen-binding site that binds ErbB-3, wherein said first antigen-binding site comprises at least the CDR3 sequence of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or a heavy chain CDR3 sequence that differs in at most three, preferably in at most two, preferably in no more than one amino acid from a CDR3 sequence of a VH selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, and wherein said second antigen-binding site comprises at least the CDR3 sequence of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or a heavy chain CDR3 sequence that differs in at most three, preferably in at most two, preferably in no more than one amino acid from a CDR3 sequence of a VH selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37. Said first antigen-binding site preferably comprises at least the CDR3 sequence of MF1849, MF2971, MF3958, MF3004 or MF3991, most preferably at least the CDR3 sequence of MF3958 and said second antigen-binding site preferably comprises at least the CDR3 sequence of MF3178, MF3176, MF3163, MF6058, MF6061 or MF6065, most preferably at least the CDR3 sequence of MF3178.

Said first antigen-binding site preferably comprises at least the CDR1, CDR2 and CDR3 sequences of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or heavy chain CDR1, CDR2 and CDR3 sequences that differ in at most three, preferably in at most two, preferably in at most one amino acid from the CDR1, CDR2 and CDR3 sequences of MF2926, MF2930, MF1849; MF2973, MF3004, MF3958, MF2971, MF3025, MF2916, MF3991, MF3031, MF2889, MF2913, MF1847, MF3001, MF3003 or MF1898, and said second antigen-binding site preferably comprises at least the CDR1, CDR2 and CDR3 sequences of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or heavy chain CDR1, CDR2 and CDR3 sequences that differ in at most three, preferably in at most two, preferably in at most one amino acid from the CDR1, CDR2 and CDR3 sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 as depicted in Figure 16B or Figure 16E or Figure 37. Said first antigen-binding site preferably comprises at least the CDR1, CDR2 and CDR3 sequences of MF1849, MF2971, MF3958, MF3004 or MF3991, most preferably at least the CDR1, CDR2 and CDR3 sequences of MF3958, and said second antigen-binding site preferably comprises at least the CDR1, CDR2 and CDR3 sequences of MF3178, MF3176, MF3163, MF6058, MF6061 or MF6065, most preferably at least the CDR1, CDR2 and CDR3 sequence of MF3178.

One preferred embodiment provides a bispecific antibody comprising a first antigen-binding site that binds ErbB-2 and a second antigen-binding site that binds ErbB-3, wherein said first antigen-binding site comprises at least the CDR3 sequence of MF3958, or a CDR3 sequence that differs in at most three, preferably in at most two, preferably in no more than one amino acid from the CDR3 sequence of MF3958, and wherein said second antigen-binding site comprises at least the CDR3 sequence of MF3178, or a CDR3 sequence that differs in at most three, preferably in at most two, preferably in no more than one amino acid from the CDR3 sequence of MF3178.

The invention in one embodiment provides a bispecific antibody comprising a first antigen-binding site that binds ErbB-2 and a second antigen-binding site that binds ErbB-3, wherein said first antigen-binding site comprises at least the CDR1, CDR2 and CDR3 sequences of MF3958, or CDR1, CDR2 and CDR3 sequences that differ in at most three, preferably in at most two, preferably in at most one amino acid from the CDR1, CDR2 and CDR3 sequences of MF3958, and wherein said second antigen-binding site comprises at least the CDR1, CDR2 and CDR3 sequence of MF3178, or CDR1, CDR2 and CDR3 sequences that differ in at most three, preferably in at most two, preferably in at most one amino acid from the CDR1, CDR2 and CDR3 sequences of MF3178.

The invention in one embodiment provides a bispecific antibody comprising a first antigen-binding site that binds ErbB-2 and a second antigen-binding site that binds ErbB-3, wherein said first antigen-binding site comprises at least the CDR3 sequence of MF3958 and wherein said second antigen-binding site comprises at least the CDR3 sequence of MF3178.

The invention in one embodiment provides a bispecific antibody comprising a first antigen-binding site that binds ErbB-2 and a second antigen-binding site that binds ErbB-3, wherein said first antigen-binding site comprises at least the CDR1, CDR2 and CDR3 sequences of MF3958 and wherein said second antigen-binding site comprises at least the CDR1, CDR2 and CDR3 sequence of MF3178.

CDR sequences are for instance varied for optimization purposes, preferably in order to improve binding efficacy or the stability of the antibody. Optimization is for instance performed by mutagenesis procedures where after the stability and/or binding affinity of the resulting antibodies are preferably tested and an improved ErbB-2 or ErbB-3 -specific CDR sequence is preferably selected. A skilled person is well capable of generating antibody variants comprising at least one altered CDR sequence according to the invention. For instance, conservative amino acid substitution is applied. Examples of conservative amino acid substitution include the substitution of one hydrophobic residue such as isoleucine, valine, leucine or methionine for another hydrophobic residue, and the substitution of one polar residue for another polar residue, such as the substitution of arginine for lysine, glutamic acid for aspartic acid, or glutamine for asparagine.

The invention in one embodiment provides an antibody comprising a variable domain that binds ErbB-2, wherein the VH chain of said variable domain comprises the amino acid sequence of VH chain MF2926; MF2930; MF1849; MF2973; MF3004; MF3958 (is humanized MF2971); MF2971; MF3025; MF2916; MF3991 (is humanized MF3004); MF3031; MF2889; MF2913; MF1847; MF3001, MF3003 or MF1898 as depicted in Figure 16A or Figure 16E; or comprises the amino acid sequence of VH chain MF2926; MF2930; MF1849; MF2973; MF3004; MF3958 (is humanized MF2971); MF2971; MF3025; MF2916; MF3991 (is humanized MF3004); MF3031; MF2889; MF2913; MF1847; MF3001, MF3003 or MF1898 as depicted in Figure 16A or Figure 16E having at most 15, preferably 1, 2, 3, 4, 5, 6, 7, 8, 9 or 10 more preferably at most 1, 2, 3, 4 or 5, amino acid insertions, deletions, substitutions or a combination thereof with respect to the above mentioned VH chain sequence of Figure 16A or Figure 16E. The VH chain of the variable domain that binds ErbB-2 preferably comprises the amino acid sequence of:
- MF1849; or
- MF2971 or a humanized version thereof, wherein said humanized version preferably comprises the amino acid sequence of MF3958; or
- MF3004 or a humanized version thereof, wherein said humanized version preferably comprises the amino acid sequence of MF3991;
as depicted in Figure 16A. In one embodiment, the VH chain of the variable domain that binds ErbB-2 comprises the amino acid sequence of VH chain MF1849; or MF2971 or a humanized version thereof, wherein said humanized version preferably comprises the amino acid sequence of MF3958; or MF3004 or a humanized version thereof, wherein said humanized version preferably comprises the amino acid sequence of MF3991, wherein the recited VH sequences have at most 15, preferably 1, 2, 3, 4, 5, 6, 7, 8, 9 or 10, more preferably at most 1, 2, 3, 4 or 5, amino acid insertions, deletions, substitutions or a combination thereof with respect to the respective sequence depicted in Figure 16A. In a preferred embodiment the VH chain of the variable domain that binds ErbB-2 comprises the amino acid sequence of MF3958; or comprises the amino acid sequence of MF3958 depicted in figure 16A having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence. The antibody comprising a variable domain that binds ErbB-2 is preferably a bispecific antibody that preferably further comprises a variable domain that binds ErbB-3. The VH chain of the variable domain that binds Erb-B3 preferably comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 as depicted in Figure 16B or Figure 16E or Figure 37; or comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 as depicted in Figure 16B or Figure 16E or Figure 37 having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence of Figure 16B or Figure 16E or Figure 37. The VH chain of the variable domain that binds Erb-B3 preferably comprises the amino acid sequence of MF3178, MF3176, MF3163, MF6058, MF6061 or MF6065; or comprises the amino acid sequence of MF3178, MF3176, MF3163, MF6058, MF6061 or MF6065 having at most 15, preferably 1, 2, 3, 4, 5, 6, 7, 8, 9 or 10, more preferably in at most 1, 2, 3, 4 or 5, amino acid insertions, deletions, substitutions or a combination thereof with respect to the respective VH chain sequence of Figure 16B or Figure 37. In a preferred embodiment the VH chain of the variable domain that binds ErbB-3 comprises the amino acid sequence of MF3178; or comprises the amino acid sequence of MF3178 depicted in Figure 16B having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence. Preferably, the above-mentioned amino acid insertions, deletions and substitutions are not present in the CDR3 region. The above-mentioned amino acid insertions, deletions and substitutions are also preferably not present in the CDR1 and CDR2 regions. The above-mentioned amino acid insertions, deletions and substitutions are also preferably not present in the FR4 region.

The invention further provides an antibody comprising a variable domain that binds ErbB-3, wherein the VH chain of said variable region comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 as depicted in Figure 16B or Figure 16E or Figure 37, or comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 as depicted in Figure 16B or Figure 16E or Figure 37 having at most 15, preferably 1, 2, 3, 4, 5, 6, 7, 8, 9 or 10, more preferably at most 1, 2, 3, 4 or 5, amino acid insertions, deletions, substitutions or a combination thereof with respect to said VH chain sequence. The VH chain of the variable domain that binds ErbB3 preferably comprises the amino acid sequence of VH chain MF3178, MF3176, MF3163, MF6058, MF6061 or MF6065; or comprises the amino acid sequence of VH chain MF3178, MF3176, MF3163, MF6058, MF6061 or MF6065 having at most 15, preferably 1, 2, 3, 4, 5, 6, 7, 8, 9 or 10, more preferably at most 1, 2, 3, 4 or 5, amino acid insertions, deletions, substitutions or a combination thereof with respect to said VH chain sequence. In a preferred embodiment the VH chain of the variable domain that binds ErbB-3 comprises the amino acid sequence of VH chain MF3178 depicted in Figure 16B; or comprises the amino acid sequence of VH chain MF3178 depicted in Figure 16B having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence. The antibody comprising a variable domain that binds ErbB-3, is preferably a bispecific antibody that preferably further comprises a variable domain that binds ErbB-2. The VH chain of the variable domain that binds ErbB-2 preferably comprises the amino acid sequence of a VH chain of Figure 16A or Figure 16E. The VH chain of the variable domain that binds ErbB-2 preferably comprises the amino acid sequence of MF1849; or MF2971 or a humanized version thereof, wherein said humanized version preferably comprises the amino acid sequence of MF3958; or MF3004 or a humanized version thereof, wherein said humanized version preferably comprises the amino acid sequence of MF3991 as depicted in Figure 16A. In one embodiment, the recited Erb-B2 binding VH sequences have at most 15, preferably 1, 2, 3, 4, 5, 6, 7, 8, 9 or 10, more preferably at most 1, 2, 3, 4 or 5, amino acid insertions, deletions, substitutions or a combination thereof with respect to the respective sequence depicted in Figure 16A. In one preferred embodiment, said ErbB-2 binding VH chain of Figure 16A comprises the amino acid sequence of MF3958; or comprises the amino acid sequence of MF3958 having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence. Preferably, the above-mentioned amino acid insertions, deletions and substitutions are not present in the CDR3 region. The above-mentioned amino acid insertions, deletions and substitutions are also preferably not present in the CDR1 and CDR2 regions. The above-mentioned amino acid insertions, deletions and substitutions are also preferably not present in the FR4 region.

Further provided is an antibody according to the invention, wherein said antibody comprises an ErbB-2 specific heavy chain variable region sequence selected from the group consisting of the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or wherein said antibody comprises a heavy chain variable region sequence that differs in at most 15, preferably in 1, 2, 3, 4, 5, 6, 7, 8, 9 or 10, more preferably in at most 1, 2, 3, 4 or 5, amino acids from the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 or MF1898.

Further provided is an antibody according to the invention, wherein said antibody comprises an ErbB-3 specific heavy chain variable region sequence selected from the group consisting of the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or wherein said antibody comprises a heavy chain variable region sequence that differs in at most 15, preferably in 1, 2, 3, 4, 5, 6, 7, 8, 9 or 10, more preferably in at most 1, 2, 3, 4 or 5, amino acids from the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074.

The invention in one embodiment provides an antibody comprising two antigen-binding sites that bind ErbB-2, wherein at least one of said antigen-binding sites binds domain I of ErbB-2. Preferably, both antigen-binding sites bind domain I of ErbB-2. Such antibody according to the invention is particularly suitable for combination therapy with currently used anti- ErbB-2 binding molecules that do not bind domain I of ErbB-2, such as trastuzumab that binds domain IV of ErbB-2 and pertuzumab that binds domain II of ErbB-2, because then the different binding molecules do not compete with each other for the same epitope.

Further provided is an antibody comprising two antigen-binding sites that bind ErbB-2, wherein at least one of said antigen-binding sites binds domain I of ErbB-2 and wherein the affinity (KD) of said at least one antigen-binding site for an ErbB-2 positive cell is lower than or equal to 5.0 nM, preferably lower than or equal to 4.5 nM, more preferably lower than or equal to 3.9 nM. Preferably, both antigen-binding sites bind domain I of ErbB-2. In one preferred embodiment, the affinity of said at least one antigen-binding site for ErbB-2 on SK-BR-3 cells is lower than or equal to 5.0 nM, preferably lower than or equal to 4.5 nM, more preferably lower than or equal to 4.0 nM, more preferably lower than or equal to 3.5 nM, more preferably lower than or equal to 3.0 nM, more preferably lower than or equal to 2.3 nM. In one embodiment, said affinity is within the range of 3.0-1.6 nM. In one preferred embodiment, the affinity of said at least one antigen-binding site for ErbB-2 on BT-474 cells is lower than or equal to 5.0 nM, preferably lower than or equal to 4.5 nM, more preferably lower than or equal to 3.9 nM. In one embodiment, said affinity is within the range of 4.5-3.3 nM.

The above-mentioned affinities are preferably as measured using steady state cell affinity measurements, wherein cells are incubated at 4°C using radioactively labeled antibody, where after cell-bound radioactivity is measured, as described in the Examples.

The invention further provides an antibody comprising two variable domains that bind ErbB-2, wherein a VH chain of said variable domains comprises the amino acid sequence of the VH chain MF2926; MF2930; MF1849; MF2973; MF3004; MF3958 (is humanized MF2971); MF2971; MF3025; MF2916; MF3991 (is humanized MF3004); MF3031; MF2889; MF2913; MF1847; MF3001, MF3003 or MF1898 as depicted in Figure 16A or Figure 16E; or the amino acid sequence of the VH chain MF2926; MF2930; MF1849; MF2973; MF3004; MF3958 (is humanized MF2971); MF2971; MF3025; MF2916; MF3991 (is humanized MF3004); MF3031; MF2889; MF2913; MF1847; MF3001, MF3003 or MF1898 VH-chains as depicted in Figure 16A or Figure 16E, having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the respective sequence depicted in Figure 16A or Figure 16E. Said VH preferably comprises the amino acid sequence of VH chain MF1849; or MF2971 or a humanized version thereof, wherein said humanized version preferably comprises the amino acid sequence of MF3958; or MF3004 or a humanized version thereof, wherein said humanized version preferably comprises the amino acid sequence of MF3991 as depicted in Figure 16A; or comprises the amino acid sequence of VH chain MF1849; or MF2971 or a humanized version thereof, wherein said humanized version preferably comprises the amino acid sequence of MF3958; or MF3004 or a humanized version thereof, wherein said humanized version preferably comprises the amino acid sequence of MF3991 as depicted in Figure 16A having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the respective sequence depicted in Figure 16A. The variable domains of the antibody preferably comprise identical VH chains, preferably having a sequence as depicted in Figure 16A or Figure 16E. An antibody with variable domains with identical VH chains is not a bispecific antibody. VH chains are identical for the present invention if they comprise the same VH chain sequence as depicted in Figure 16A or Figure 16E or Figure 37, or the same VH chain sequence but for 1, 2, 3, 4 or 5 amino acid insertions, deletions, substitutions or a combination thereof with respect to the respective sequence depicted in Figure 16A or Figure 16E or Figure 37.

The invention in one embodiment provides an antibody comprising two antigen-binding sites that bind ErbB-3, wherein at least one of said antigen-binding sites binds domain III of ErbB-3. Preferably, both antigen-binding sites bind domain III of ErbB-3. Such antibody according to the invention is particularly suitable for combination therapy with currently used anti- ErbB-3 binding molecules that do not bind domain III of ErbB-3, such as MM-121 (#Ab6) and RG7116 that bind domain I of ErbB-3, because then the different binding molecules do not compete with each other for the same epitope.

Further provided is an antibody comprising two antigen-binding sites that bind ErbB-3, wherein at least one of said antigen-binding sites binds domain III of ErbB-3 and wherein the affinity (KD) of said at least one antigen-binding site for an ErbB-3 positive cell is lower than or equal to 2.0 nM, preferably lower than or equal to 1.5 nM, more preferably lower than or equal to 1.39 nM, more preferably lower than or equal to 0.99 nM. Preferably, both antigen-binding sites bind domain III of ErbB-3. In one preferred embodiment, the affinity of said at least one antigen-binding site for ErbB-3 on SK-BR-3 cells is lower than or equal to 2.0 nM, preferably lower than or equal to 1.5 nM, more preferably lower than or equal to 1.39 nM, more preferably lower than or equal to 0.99 nM. In one embodiment, said affinity is within the range of 1.39-0.59 nM. In one preferred embodiment, the affinity of said at least one antigen-binding site for ErbB-3 on BT-474 cells is lower than or equal to 2.0 nM, more preferably lower than or equal to 1.5 nM, more preferably lower than or equal to 1.0 nM, more preferably lower than or equal to 0.5 nM, more preferably lower than or equal to 0.31 nM, more preferably lower than or equal to 0.23 nM. In one embodiment, said affinity is within the range of 0.31-0.15 nM.

Again, the above-mentioned affinities are preferably as measured using steady state cell affinity measurements, wherein cells are incubated at 4°C using radioactively labeled antibody, where after cell-bound radioactivity is measured, as described in the Examples.

The invention further provides an antibody comprising two variable domains that each bind ErbB3 wherein a VH of the variable domains comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 as depicted in Figure 16B or Figure 16E or Figure 37; or comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 having at most 15, preferably 1, 2, 3, 4, 5, 6, 7, 8, 9 or 10, more preferably at most 1, 2, 3, 4 or 5, amino acid insertions, deletions, substitutions or a combination thereof with respect to any of said VH chain sequences. Said VH preferably comprises the amino acid sequence of VH chain MF3178, MF3176, MF3163, MF6058, MF6061 or MF6065; or comprises the amino acid sequence of VH chain MF3178, MF3176, MF3163, MF6058, MF6061 or MF6065 having at most 15, preferably 1, 2, 3, 4, 5, 6, 7, 8, 9 or 10, more preferably at most 1, 2, 3, 4 or 5, amino acid insertions, deletions, substitutions or a combination thereof with respect to any of said VH chain sequences. Said VH preferably comprises the amino acid sequence of VH chain MF3178; or comprises the amino acid sequence of VH chain MF3178 depicted in Figure 16B having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the MF3178 VH chain sequence. The variable domains of the antibody preferably comprise identical VH chains, preferably having a sequence as depicted in Figure 16B or Figure 16E or Figure 37. An antibody with variable domains with identical VH chains is not a bispecific antibody. The VH chains are identical if they comprise the same VH chain sequence as depicted in Figure 16B or Figure 16E or Figure 37, or the same VH chain sequence but for 1, 2, 3, 4 or 5 amino acid insertions, deletions, substitutions or a combination thereof with respect to the VH chain sequence of Figure 16B or Figure 16E or Figure 37.

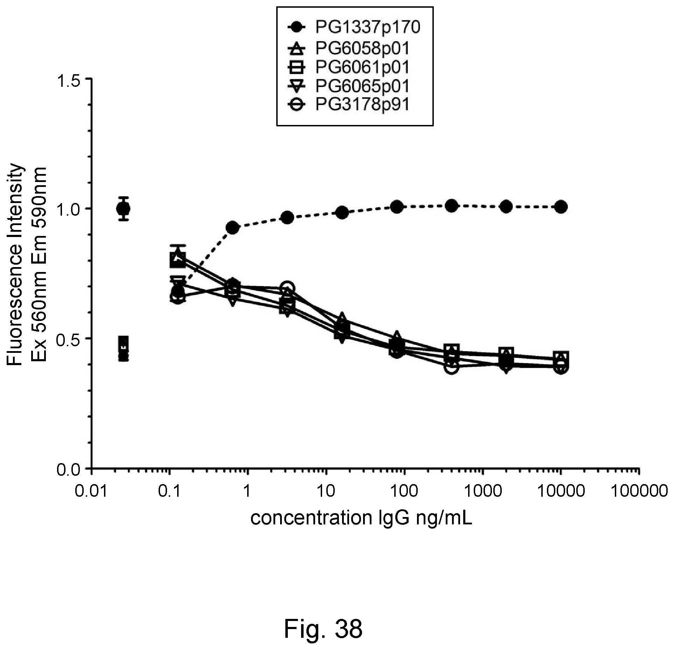

Monospecific antibodies according to the present invention that are specific for ErbB-3 have the advantage that they have a better functional activity against ErbB-3, as compared to prior art compounds such as for instance MM-121 (#Ab6), meaning that these antibodies according to the invention are better capable of counteracting ErbB-3 activity (such as a ligand-induced receptor function of ErbB-3 and/or ligand-induced growth of an ErbB-2 and ErbB-3 positive cell). This is for instance shown in Table 7 and Figure 38.

In a preferred embodiment the invention provides a bispecific antibody comprising a variable domain that binds ErbB-2, wherein the VH chain of said variable domain comprises
- the amino acid sequence of VH chain MF1849; or MF2971 or a humanized version thereof, wherein said humanized version preferably comprises the amino acid sequence of MF3958; or MF3004 or a humanized version thereof, wherein said humanized version preferably comprises the amino acid sequence of MF3991, as depicted in Figure 16A; or comprises
- the amino acid sequence of VH chain MF1849 or MF2971 or a humanized version thereof, wherein said humanized version preferably comprises the amino acid sequence of MF3958; or MF3004 or a humanized version thereof, wherein said humanized version preferably comprises the amino acid sequence of MF3991, as depicted in Figure 16A having at most 15, preferably 1, 2, 3, 4, 5, 6, 7, 8, 9 or 10, more preferably at most 1, 2, 3, 4 or 5, amino acid insertions, deletions, substitutions or a combination thereof with respect to said VH. Such bispecific antibody according to this embodiment further preferably comprises a variable domain that binds ErbB-3. The VH chain of the variable domain that binds ErbB-3 preferably comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 as depicted in Figure 16B or Figure 16E or Figure 37, or most preferably comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 as depicted in Figure 16B or Figure 16E or Figure 37, having at most 15, preferably 1, 2, 3, 4, 5, 6, 7, 8, 9 or 10, more preferably at most 1, 2, 3, 4 or 5, amino acid insertions, deletions, substitutions or a combination thereof with respect to any of said VH chain sequences of Figure 16B or Figure 16E or Figure 37. The VH chain of the variable domain that binds ErbB-3 preferably comprises the amino acid sequence of VH chain MF3178 as depicted in Figure 16B or comprises the amino acid sequence of VH chain MF3178 depicted in Figure 16B having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence of Figure 16B.

The invention preferably provides a bispecific antibody comprising a variable domain that binds ErbB-2 and a variable domain that binds ErbB-3, wherein the VH chain of the variable domain that binds ErbB-2 comprises
- the amino acid sequence of VH chain MF3958 as depicted in Figure 16A; or
- the amino acid sequence of VH chain MF3958 as depicted in Figure 16A having at most 15, preferably 1, 2, 3, 4, 5, 6, 7, 8, 9 or 10, more preferably at most 1, 2, 3, 4 or 5, amino acid insertions, deletions, substitutions or a combination thereof with respect said VH; and
   wherein the VH chain of the variable domain that binds ErbB-3 comprises
- the amino acid sequence of VH chain MF3178 as depicted in Figure 16B; or
- the amino acid sequence of VH chain MF3178 depicted in Figure 16B having at most 15, preferably 1, 2, 3, 4, 5, 6, 7, 8, 9 or 10 more preferably at most 1, 2, 3, 4 or 5, amino acid insertions, deletions, substitutions or a combination thereof with respect to the VH chain sequence of Figure 16B.

The invention preferably provides a bispecific antibody comprising a variable domain that binds ErbB-2 and a variable domain that binds ErbB-3, wherein the VH chain of the variable domain that binds ErbB-2 comprises
- the amino acid sequence of VH chain MF3991 as depicted in Figure 16A; or
- the amino acid sequence of VH chain MF3991 as depicted in Figure 16A having at most 15, preferably 1, 2, 3, 4, 5, 6, 7, 8, 9 or 10, more preferably at most 1, 2, 3, 4 or 5, amino acid insertions, deletions, substitutions or a combination thereof with respect said VH; and
   wherein the VH chain of the variable domain that binds ErbB-3 comprises
- the amino acid sequence of VH chain MF3178 as depicted in Figure 16B; or
- the amino acid sequence of VH chain MF3178 depicted in Figure 16B having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence of Figure 16B.

When compared to the sequence in Figure 16, the behavior of a VH chain typically starts to become noticeably different when it has more than 15 amino acid changes with respect to the amino acid sequence of a VH chain as depicted in Figure 16. A VH chain having at most 15, preferably 1, 2, 3, 4, 5, 6, 7, 8, 9 or 10 amino acid insertions, deletions, substitutions or a combination thereof with respect to the VH chain depicted in Figure 16, preferably has 1, 2, 3, 4 or 5 amino acid insertions, deletions, substitutions or a combination thereof with respect to the VH chain depicted in Figure 16, preferably 1, 2, 3 or 4 insertions, deletions, substitutions or a combination thereof, preferably 1, 2 or 3 insertions, deletions, substitutions or a combination thereof, more preferably 1 or 2 insertions, deletions, substitutions or a combination thereof, and preferably 1 insertion, deletion, substitution or a combination thereof with respect to the VH chain depicted in Figure 16. The one or more amino acid insertions, deletions, substitutions or a combination thereof are preferably not in the CDR1, CDR2 and CDR3 region of the VH chain. They are also preferably not present in the FR4 region. An amino acid substitution is preferably a conservative amino acid substitution.

In a preferred embodiment the invention provides a bispecific antibody comprising an amino acid sequence as depicted in Figure 16D, or a bispecific antibody of Figure 16D having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the sequence of Figure 16D, wherein the at most 15, preferably 1, 2, 3, 4, 5, 6, 7, 8, 9 or 10 amino acid substitutions are preferably conservative amino acid substitutions. The insertions, deletions, substitutions or a combination thereof are preferably not in the CDR3 region of the VH chain, preferably not in the CDR1, CDR2 and CDR3 region of the VH chain, and preferably not in the FR4 region.

Rational methods have evolved toward minimizing the content of non-human residues in the human context. Various methods are available to successfully graft the antigen-binding property of a bispecific antibody onto another antibody. The binding properties of antibodies rest predominantly in the exact sequence of the CDR3 region, often supported by the sequence of the CDR1 and CDR2 regions in the variable domain combined with the appropriate structure of the variable domain as a whole. Various methods are presently available to graft CDR regions onto a suitable variable domain of another antibody. Some of these methods are reviewed in J.C. Almagro1 and J. Fransson (2008) Frontiers in Bioscience 13, 1619-1633, which is included by reference herein. The invention therefore further provides a human or humanized bispecific antibody comprising a first antigen-binding site that binds ErbB-2 and a second antigen-binding site that binds ErbB-3, wherein the variable domain comprising the ErbB-2 binding site comprises a VH CDR3 sequence as depicted in Figure 16A or Figure 16E, and wherein the variable domain comprising the ErbB-3 binding site comprises a VH CDR3 region as depicted in Figure 16B or Figure 16E or Figure 37. The VH variable region comprising the ErbB-2 binding site preferably comprises the sequence of the CDR1 region, CDR2 region and the CDR3 region of a VH chain in Figure 16A or Figure 16E. The VH variable region comprising the ErbB-3 binding site preferably comprises the sequence of the CDR1 region, CDR2 region and the CDR3 region of a VH chain in Figure 16B or Figure 16E or Figure 37. CDR grafting may also be used to produce a VH chain with the CDR regions of a VH of Figure 16 or Figure 37, but having a different framework. The different framework may be of another human VH, or a different mammal.

The mentioned at most 15, preferably 1, 2, 3, 4, 5, 6, 7, 8, 9 or 10 amino acid substitutions are preferably conservative amino acid substitutions. The insertions, deletions, substitutions or a combination thereof are preferably not in the CDR3 region of the VH chain, preferably not in the CDR1, CDR2 or CDR3 region of the VH chain and preferably not in the FR4 region.

The light chain of a variable domain comprising a variable heavy chain sequence as depicted in Figure 16 or Figure 37, is preferably germline light chain O12, preferably the rearranged germline human kappa light chain IgVκ1-39*01/IGJκ1*01 or a fragment or a functional derivative thereof (nomenclature according to the IMGT database worldwide web at imgt.org). The terms rearranged germline human kappa light chain IgVκ1-39*01/IGJκ1*01, IGKV1-39/IGKJ1, huVκ1-39 light chain or in short huVκ1-39 are used. The light chain can have 1, 2, 3, 4 or 5 amino acid insertions, deletions, substitutions or a combination thereof. The mentioned 1, 2, 3, 4 or 5 amino acid substitutions are preferably conservative amino acid substitutions, the insertions, deletions, substitutions or a combination thereof are preferably not in the CDR3 region of the VL chain, preferably not in the CDR1, CDR2 or CDR3 region or FR4 region of the VL chain.

Various methods are available to produce bispecific antibodies. One method involves the expression of two different heavy chains and two different light chains in a cell and collecting antibody that is produced by the cell. Antibody produced in this way will typically contain a collection of antibodies with different combinations of heavy and light chains, some of which are the desired bispecific antibody. The bispecific antibody can subsequently be purified from the collection. The ratio of bispecific to other antibodies that are produced by the cell can be increased in various ways. In a preferred embodiment of the invention, the ratio is increased by expressing not two different light chains but two essentially identical light chains in the cell. This concept is in the art also referred to as the "common light chain" method. When the essentially identically light chains work together with the two different heavy chains allowing the formation of variable domains with different antigen-binding sites and concomitant different binding properties, the ratio of bispecific antibody to other antibody that is produced by the cell is significantly improved over the expression of two different light chains. The ratio of bispecific antibody that is produced by the cell can be further improved by stimulating the pairing of two different heavy chains with each other over the pairing of two identical heavy chains. The art describes various ways in which such heterodimerization of heavy chains can be achieved. One way is to generate 'knob into hole' bispecific antibodies. See US Patent Application 20030078385 (Arathoon et al. - Genentech). Another and preferred method is described in US provisional application 61/635,935, which has been followed up by US regular application No. 13/866,747 and PCT application No. PCT/NL2013/050294 (WO 2013/157954 A1), which are incorporated herein by reference. Methods and means are disclosed for producing bispecific antibodies from a single cell, whereby means are provided that favor the formation of bispecific antibodies over the formation of monospecific antibodies. These methods can also be favorably employed in the present invention. Thus the invention provides a method for producing a bispecific antibody according to the invention (from a single cell), wherein said bispecific antibody comprises two CH3 domains that are capable of forming an interface, said method comprising providing in said cell a) a first nucleic acid molecule encoding a 1st CH3 domain comprising heavy chain, b) a second nucleic acid molecule encoding a 2nd CH3 domain comprising heavy chain, wherein said nucleic acid molecules are provided with means for preferential pairing of said 1st and 2nd CH3 domain comprising heavy chains, said method further comprising the step of culturing said host cell and allowing for expression of said two nucleic acid molecules and harvesting said bispecific antibody from the culture. Said first and second nucleic acid molecules may be part of the same nucleic acid molecule, vector or gene delivery vehicle and may be integrated at the same site of the host cell's genome. Alternatively, said first and second nucleic acid molecules are separately provided to said cell.

A preferred embodiment provides a method for producing a bispecific antibody according to the invention (from a single cell), wherein said bispecific antibody comprises two CH3 domains that are capable of forming an interface, said method comprising providing:
- a cell having a) a first nucleic acid molecule encoding a heavy chain comprising an antigen binding site that binds ErbB-2 and that contains a 1st CH3 domain, and b) a second nucleic acid molecule encoding a heavy chain comprising an antigen-binding site that binds ErbB-3 and that contains a 2nd CH3 domain, wherein said nucleic acid molecules are provided with means for preferential pairing of said 1st and 2nd CH3 domains,
said method further comprising the step of culturing said cell and allowing for expression of said two nucleic acid molecules and harvesting said bispecific IgG antibody from the culture. In a particularly preferred embodiment, said cell also has a third nucleic acid molecule encoding a common light chain. Said first, second and third nucleic acid molecule may be part of the same nucleic acid molecule, vector or gene delivery vehicle and may be integrated at the same site of the host cell's genome. Alternatively, said first, second and third nucleic acid molecules are separately provided to said cell. A preferred common light chain is O12, preferably the rearranged germline human kappa light chain IgVκ1 39*01/IGJκ1*01, as described above. Means for preferential pairing of said 1^{st} and said 2^{nd} CH3 domain are preferably the corresponding mutations in the CH3 domain of the heavy chain coding regions. The preferred mutations to produce essentially only bispecific antibodies are the amino acid substitutions L351K and T366K (numbering according to Kabat) in the first CH3 domain and the amino acid substitutions L351D and L368E in the second CH3 domain, or vice versa. Further provided is therefore a method according to the invention for producing a bispecific antibody, wherein said first CH3 domain comprises the amino acid substitutions L351K and T366K (numbering according to Kabat) and wherein said second CH3 domain comprises the amino acid substitutions L351D and L368E, said method further comprising the step of culturing said cell and allowing for expression of said nucleic acid molecules and harvesting said bispecific antibody from the culture. Also provided is a method according to the invention for producing a bispecific antibody, wherein said first CH3 domain comprises the amino acid substitutions L351D and L368E (numbering according to Kabat) and wherein said second CH3 domain comprises the amino acid substitutions L351K and T366K, said method further comprising the step of culturing said cell and allowing for expression of said nucleic acid molecules and harvesting said bispecific antibody from the culture. Antibodies that can be produced by these methods are also part of the present invention. The CH3 heterodimerization domains are preferably IgG1 heterodimerization domains. The heavy chain constant regions comprising the CH3 heterodimerization domains are preferably IgG1 constant regions.

In one embodiment the invention provides a nucleic acid molecule encoding an antibody heavy chain variable region according to the invention. The nucleic acid molecule (typically an *in vitro,* isolated or recombinant nucleic acid) preferably encodes a heavy chain variable region as depicted in Figure 16A or Figure 16B or Figure 37, or a heavy chain variable region as depicted in Figure 16A or Figure 16B or Figure 37 having 1, 2, 3, 4 or 5 amino acid insertions, deletions, substitutions or a combination thereof. In a preferred embodiment the nucleic acid molecule comprises a sequence as depicted in Figure 16 or Figure 37. In another preferred embodiment the nucleic acid molecule encodes the same amino acid sequence as the nucleic acid depicted in Figure 16 or Figure 37, but has a different sequence because it encodes one or more different codons. For instance, such nucleic acid molecule is codon optimized for antibody producer cells, such as for instance Chinese hamster ovary (CHO) cells, NS0 cells or PER-C6^{™} cells. The invention further provides a nucleic acid sequence encoding a heavy chain of Figure 16D or Figure 37.

A nucleic acid molecule as used in the invention is typically but not exclusively a ribonucleic acid (RNA) or a deoxyribonucleic acid (DNA). Alternative nucleic acids are available for a person skilled in the art. A nucleic acid according to the invention is for instance comprised in a cell. When said nucleic acid is expressed in said cell, said cell produces an antibody according to the invention. Therefore, the invention in one embodiment provides a cell comprising an antibody according to the invention and/or a nucleic acid according to the invention. Said cell is preferably an animal cell, more preferably a mammal cell, more preferably a primate cell, most preferably a human cell. For the purposes of the invention a suitable cell is any cell capable of comprising and preferably of producing an antibody according to the invention and/or a nucleic acid according to the invention.

The invention further provides a cell comprising an antibody according to the invention. Preferably said cell (typically an *in vitro,* isolated or recombinant cell) produces said antibody. In a preferred embodiment said cell is a hybridoma cell, a CHO cell, an NS0 cell or a PER-C6^{™} cell. In a particularly preferred embodiment said cell is a CHO cell. Further provided is a cell culture comprising a cell according to the invention. Various institutions and companies have developed cell lines for the large scale production of antibodies, for instance for clinical use. Non-limiting examples of such cell lines are CHO cells, NS0 cells or PER.C6^{™} cells. These cells are also used for other purposes such as the production of proteins. Cell lines developed for industrial scale production of proteins and antibodies are herein further referred to as industrial cell lines. Thus in a preferred embodiment the invention provides the use of a cell line developed for the large scale production of antibody for the production of an antibody of the invention.

The invention further provides a method for producing an antibody comprising culturing a cell of the invention and harvesting said antibody from said culture. Preferably said cell is cultured in a serum free medium. Preferably said cell is adapted for suspension growth. Further provided is an antibody obtainable by a method for producing an antibody according to the invention. The antibody is preferably purified from the medium of the culture. Preferably said antibody is affinity purified.

A cell of the invention is for instance a hybridoma cell line, a CHO cell, an NS0 cell or another cell type known for its suitability for antibody production for clinical purposes. In a particularly preferred embodiment said cell is a human cell. Preferably a cell that is transformed by an adenovirus E1 region or a functional equivalent thereof. A preferred example of such a cell line is the PER.C6^{™} cell line or equivalent thereof. In a particularly preferred embodiment said cell is a CHO cell or a variant thereof. Preferably a variant that makes use of a Glutamine synthetase (GS) vector system for expression of an antibody.

The invention further provides a composition, preferably a pharmaceutical composition, comprising an antibody according to the invention. The pharmaceutical composition preferably comprises a (pharmaceutically acceptable) excipient or carrier. In a preferred embodiment the pharmaceutical composition comprises 5-50 mM Histidine, 100-300 mM Trehalose, 0.1-03 g/L PolySorbate20 or a combination thereof. The pH is preferably set at pH = 5.5 - 6.5. In a preferred embodiment the pharmaceutical composition comprises 25 mM Histidine, 220 mM Trehalose, 0.2 g/L PolySorbate20 or a combination thereof. The pH is preferably set at pH = 5.5 - 6.5, most preferably at pH = 6.

An antibody of the invention preferably further comprises a label, preferably a label for *in vivo* imaging. Such a label is typically not necessary for therapeutic applications. In for instance a diagnostic setting, a label can be helpful. For instance in visualizing target cells in the body. Various labels are suited and many are well known in the art. In a preferred embodiment the label is a radioactive label for detection. In another preferred embodiment, the label is an infrared label. Preferably the infrared label is suited for *in vivo* imaging. Various infrared labels are available to the person skilled in the art. Preferred infrared labels are for instance, IRDye 800; IRDye 680RD; IRDye 680LT; IRDye 750; IRDye 700DX; IRDye 800RS IRDye 650; IRDye 700 phosphoramidite; IRDye 800 phosphoramidite (LI-COR USA; 4647 Superior Street; Lincoln, Nebraska).

The invention further provides a method for the treatment of a subject having a ErbB-2, ErbB-3 or ErbB-2/ErbB-3 positive tumor or at risk of having said tumor comprising administering to the subject an antibody or pharmaceutical composition according to the invention. Before start of said treatment, the method preferably comprises determining whether said subject has, or is at risk of, such ErbB-2, ErbB-3 or ErbB-2/ErbB-3 positive tumor. In some embodiments, the subject is classified as [+] or [++] for ErbB-2. In another embodiment the subject is classified as [+++] for ErbB-2. The invention further provides an antibody of the invention for use in the treatment of a subject having or at risk of having an ErbB-2, ErbB-3 or ErbB-2/ErbB-3 positive tumor. Alternatively formulated, the invention provides a use of an antibody according to the invention for the manufacture of a medicament or prophylactic agent for the treatment of an ErbB-2, ErbB-3 or ErbB-2/ErbB-3 positive tumor. As used herein, the term treatment encompasses prophylaxis.

The tumor is preferably an ErbB-2, ErbB-3 or ErbB-2/ErbB-3 positive cancer. Preferably said positive cancer is a breast cancer, such as early-stage breast cancer. However, the invention can be applied to a wide range of ErbB-2, ErbB-3 or ErbB-2/ErbB-3 positive cancers, like gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, melanoma, and the like. Said antibody according to the present invention is typically capable of reducing a ligand-induced receptor function, preferably ligand induced growth, of ErbB-3 on a ErbB-2 and ErbB-3 positive cell. Said antibody according to the invention preferably comprises a first antigen-binding site that binds domain I of ErbB-2 and a second antigen-binding site that binds domain III of ErbB-3. In one preferred embodiment, the affinity (KD) of said second antigen-binding site for an ErbB-3 positive cell is equal to, or higher than, the affinity of said first antigen-binding site for an ErbB-2 positive cell. Further provided is therefore an antibody comprising a first antigen-binding site that binds ErbB-2 and a second antigen-binding site that binds ErbB-3 for use in the treatment of a subject having or at risk of having an ErbB-2, ErbB-3 or ErbB-2/ErbB-3 positive tumor, preferably breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma, wherein the affinity of said second antigen-binding site for an ErbB-3 positive cell is equal to, or higher than, the affinity of said first antigen-binding site for an ErbB-2 positive cell. The affinity of said second antigen-binding site for an ErbB-3 positive cell is preferably lower than or equal to 2.0 nM, more preferably lower than or equal to 1.39 nM, more preferably lower than or equal to 0.99 nM. The affinity of said first antigen-binding site for an ErbB-2 positive cell is preferably lower than or equal to 5.0 nM, preferably lower than or equal to 4.5 nM preferably lower than or equal to 4.0 nM. In one preferred embodiment, said antibody is antibody PB4188.

In one preferred embodiment, said antibody according to the invention comprises an antigen-binding site that binds at least one amino acid of domain I of ErbB-2 selected from the group consisting of T144, T164, R166, P172, G179, S180 and R181, and surface-exposed amino acid residues that are located within about 5 amino acid positions from T144, T164, R166, P172, G179, S180 or R181.

In one preferred embodiment, said antibody according to the invention preferably comprises an antigen-binding site that binds at least one amino acid of domain III of ErbB-3 selected from the group consisting R426 and surface-exposed amino acid residues that are located within 11.2 Å from R426 in the native ErbB-3 protein.

Further provided is therefore an antibody comprising a first antigen-binding site that binds ErbB-2 and a second antigen-binding site that binds ErbB-3 for use in the treatment of a subject having or at risk of having an ErbB-2, ErbB-3 or ErbB-2/ErbB-3 positive tumor, preferably breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma, wherein said antibody according to the invention comprises an antigen-binding site that binds at least one amino acid of domain I of ErbB-2 selected from the group consisting of T144, T164, R166, P172, G179, S180 and R181, and surface-exposed amino acid residues that are located within about 5 amino acid positions from T144, T164, R166, P172, G179, S180 or R181, and/or wherein said antibody according to the invention preferably comprises an antigen-binding site that binds at least one amino acid of domain III of ErbB-3 selected from the group consisting of R426 and surface-exposed amino acid residues that are located within 11.2 Å from R426 in the native ErbB-3 protein.

The subject is preferably a human subject. The subject is preferably a subject eligible for monoclonal antibody therapy using an ErbB-2 specific antibody such as trastuzumab. In a preferred embodiment the subject comprises a tumor, preferably an ErbB-2/ErbB-3 positive cancer, preferably a tumor/cancer with an ErbB-2 therapy resistant phenotype and/or a heregulin resistance phenotype, preferably a monoclonal antibody resistant phenotype. A tumor involving such phenotype can escape treatment with a current anti-HER2 regimen, such as (but not limited to) monoclonal antibody therapy against ErbB-2.

The amount of antibody according to the invention to be administered to a patient is typically in the therapeutic window, meaning that a sufficient quantity is used for obtaining a therapeutic effect, while the amount does not exceed a threshold value leading to an unacceptable extent of side-effects. The lower the amount of antibody needed for obtaining a desired therapeutic effect, the larger the therapeutic window will typically be. An antibody according to the invention exerting sufficient therapeutic effects at low dosage is, therefore, preferred. The dosage can be in the range of the dosing regime for trastuzumab or lower.

The present invention describes among others antibodies that target the ErbB-2 and ErbB-3 receptors and result in potent proliferation inhibition of cancer cell lines *in vitro* and tumor growth inhibition *in vivo,* even in the presence of an escape mechanism such as for instance upregulation of NRG1-β1. A diverse panel of human and murine Fab binding arms specific for either ErbB-2 or ErbB-3 were identified. These were produced as bispecific antibodies by cloning them into complementary expression vectors that contain mutations in the CH3 region that drives heterodimerization of heavy chains. More than 500 bispecific antibodies were produced at small scale and tested in binding and functional assays on three different cancer cell lines. Various bispecific antibodies were selected and tested in an orthotopic xenograft model using the BxPC3 cell line. This cell line expresses both the ErbB-2 and ErbB-3 receptors and is partially dependent on the ErbB-3 ligand for growth. BxPC3 models are a robust and stringent screening model. Furthermore, a strong anti-tumor activity *in vivo* has been confirmed using a xenograft model using the JIMT-1 cell line. JIMT-1 cells are derived from a pleural metastasis of a 62-year old patient with breast cancer who was clinically resistant to trastuzumab. JIMT-1 cells grow as an adherent monolayer and form xenograft tumors in nude mice. JIMT-1 cells have an amplified HER-2 oncogene, which showed no identifiable mutations in its coding sequence. JIMT-1 cells overexpress HER-2 mRNA and protein, and the levels of HER-1, HER-3, and HER-4 mRNA and protein are similar to the trastuzumab-sensitive cell line SKBR-3 (Tanner et al, Mol Cancer Ther 2004).

Importantly, a better anti-tumor effect was obtained using an antibody according to the invention as compared to the currently used monoclonal antibodies trastuzumab and pertuzumab, as well as the chemical compound lapatinib.

Antibodies of the invention can be produced at levels > 50 mg/L after transient transfection in suspension 293F cells. The bispecific antibodies can be purified to greater than 98% purity with yields > 70%. Analytical characterization studies show bispecific IgGl antibody profiles that are comparable to bivalent monospecific IgG1. In terms of functional activity a bispecific antibody of the invention can demonstrate superior potency compared to trastuzumab + pertuzumab *in vitro* and *in vivo.*

Preferred embodiments of the invention provide combination therapy. In one embodiment, an antibody according to the invention is combined with a ErbB-2 targeting agent, including an ErbB-2 inhibitor or binding agent.

Exemplary ErbB-2 targeting agents for use in combination therapy with a ErbB-2, ErbB-3-binding bispecific antibody, includes any ErbB-2 targeting agent, for example a binding agent or inhibitor of Erb-B2. The ErbB-2 targeting agent may be a small molecule HER2 tyrosine kinase inhibitor, such as lapatinib (Tyverb/Tykerb^{®}), neratinib afatinib, tucatinib or AZD8931

The ErbB-2 targeting agent may be an antibody. Trastuzumab or pertuzumab, for example, may be preferred since these antibodies bind different ErbB-2 epitopes so that they do not compete for the same epitope with an antibody according to the invention, as shown in the Examples.

The ErbB-2 targeting agent may be an antibody drug conjugate, for example trastuzumab emtansine or DS-8201.

In another embodiment, an antibody according to the invention is combined with MM-121 (#Ab6) or RG7116 (Roche), since these antibodies bind different ErbB-3 epitopes so that they do not compete for the same epitope with an antibody according to the invention, as shown in the Examples.

In another preferred embodiment, a binding compound that is specific for ErbB-2 and ErbB-3 is combined with an inhibitor of a component of the PI3Kinase pathway and/ or with an inhibitor of a component of the MAPK pathway, such as for instance with a tyrosine kinase inhibitor, a PI3Ka inhibitor, an Akt inhibitor, an mTOR inhibitor or an Src inhibitor. In one embodiment a binding compound that is specific for ErbB-2 and ErbB-3 is combined with a microtubuli disrupting drug or with an inhibitor of a histone deacetylase (HDAC). Surprisingly, the inventors have found a synergistic effect when these combinations are used. Further provided is therefore a method for the treatment of a subject having a ErbB-2, ErbB-3 or ErbB-2/ErbB-3 positive tumor or at risk of having said tumor, the method comprising administering to the subject:
- a binding compound that is specific for ErbB-2 and ErbB-3, and
- one or more compounds selected from the group consisting of an inhibitor of a component of the PI3Kinase pathway, an inhibitor of a component of the MAPK pathway, a microtubuli disrupting drug, and an inhibitor of a histone deacetylase (HDAC). Said inhibitor preferably comprises a tyrosine kinase inhibitor, a PI3Ka inhibitor, an Akt inhibitor, an mTOR inhibitor or an Src inhibitor. Said tyrosine kinase inhibitor is preferably afatinib, lapatinib and/or neratinib. Said PI3Ka inhibitor is preferably BYL719. In one embodiment, said Akt inhibitor is MK-2206. In one preferred embodiment, said mTOR inhibitor is everolimus. In one preferred embodiment, said Src inhibitor is saracatinib. In one preferred embodiment, said microtubuli disrupting drug is paclitaxel. In one preferred embodiment, said HDAC inhibitor is vorinostat. In one preferred embodiment, said binding compound that is specific for ErbB-2 and ErbB-3 is MM-111 (Merrimack Pharmaceuticals). In one preferred embodiment, said binding compound that is specific for ErbB-2 and ErbB-3 is a bispecific antibody. In one preferred embodiment , said binding compound that is specific for ErbB-2 and ErbB-3 is a bispecific antibody according to the invention.

Further provided is therefore a method for the treatment of a subject having a ErbB-2, ErbB-3 or ErbB-2/ErbB-3 positive tumor or at risk of having said tumor, the method comprising administering to the subject:
- a bispecific antibody comprising a first antigen-binding site that binds ErbB-2 and a second antigen-binding site that binds ErbB-3, and
- one or more compounds selected from the group consisting of an inhibitor of a component of the PI3Kinase pathway, an inhibitor of a component of the MAPK pathway, a microtubuli disrupting drug, and an HDAC inhibitor.

Also provided is a bispecific antibody comprising a first antigen-binding site that binds ErbB-2 and a second antigen-binding site that binds ErbB-3 for use in the treatment of a ErbB-2, ErbB-3 or ErbB-2/ErbB-3 positive tumor, wherein said treatment comprises administering said bispecific antibody and at least one compound selected from the group consisting of an inhibitor of a component of the PI3Kinase pathway, an inhibitor of a component of the MAPK pathway, a microtubuli disrupting drug, and an HDAC inhibitor to a subject having a ErbB-2, ErbB-3 or ErbB-2/ErbB-3 positive tumor. Preferably, a bispecific antibody according to the invention having a first antigen-binding site that binds domain I of ErbB-2 and a second antigen-binding site that binds domain III of ErbB-3 is combined with one or more compounds selected from the group consisting of an inhibitor of a component of the PI3Kinase pathway, an inhibitor of a component of the MAPK pathway, a microtubuli disrupting drug, and an HDAC inhibitor. Said inhibitor preferably comprises a tyrosine kinase inhibitor, a PI3Ka inhibitor, an Akt inhibitor, an mTOR inhibitor or an Src inhibitor. Said tyrosine kinase inhibitor is preferably afatinib, lapatinib and/or neratinib. Said PI3Ka inhibitor is preferably BYL719. In one embodiment, said Akt inhibitor is MK-2206. In one preferred embodiment, said mTOR inhibitor is everolimus. In one preferred embodiment, said Src inhibitor is saracatinib. In one preferred embodiment, said microtubuli disrupting drug is paclitaxel. In one preferred embodiment, said HDAC inhibitor is vorinostat.

Said ErbB-2, ErbB-3 or ErbB-2/ErbB-3 positive tumor is preferably breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma. Most preferably, said tumor is breast cancer. In one embodiment, said ErbB-2, ErbB-3 or ErbB-2/ErbB-3 positive tumor has less than 1.000.000 ErbB-2 cell-surface receptors per tumor cell.

In one embodiment, an antibody according to the present invention that is combined with one or more compounds selected from the group consisting of an inhibitor of a component of the PI3Kinase pathway, an inhibitor of a component of the MAPK pathway, a microtubuli disrupting drug and an HDAC inhibitor, preferably with at least one compound selected from the group consisting of a tyrosine kinase inhibitor, a PI3Ka inhibitor, an Akt inhibitor, an mTOR inhibitor, an Src inhibitor, vorinostat and paclitaxel, more preferably with at least one compound selected from the group consisting of afatinib, lapatinib, neratinib, BYL719, MK-2206, everolimus, saracatinib, vorinostat and paclitaxel, is typically capable of reducing a ligand-induced receptor function, preferably ligand induced growth, of ErbB-3 on a ErbB-2 and ErbB-3 positive cell. Said antibody according to the invention preferably comprises a first antigen-binding site that binds domain I of ErbB-2 and a second antigen-binding site that binds domain III of ErbB-3. In one preferred embodiment, the affinity (KD) of said second antigen-binding site for an ErbB-3 positive cell is equal to, or higher than, the affinity of said first antigen-binding site for an ErbB-2 positive cell. The affinity of said second antigen-binding site for an ErbB-3 positive cell is preferably lower than or equal to 2.0 nM, more preferably lower than or equal to 1.39 nM, more preferably lower than or equal to 0.99 nM. The affinity of said first antigen-binding site for an ErbB-2 positive cell is preferably lower than or equal to 5.0 nM, preferably lower than or equal to 4.5 nM preferably lower than or equal to 4.0 nM.

In one preferred embodiment, an antibody according to the invention that is combined with one or more compounds selected from the group consisting of an inhibitor of a component of the PI3Kinase pathway, an inhibitor of a component of the MAPK pathway, a microtubuli disrupting drug and an HDAC inhibitor, preferably with at least one compound selected from the group consisting of a tyrosine kinase inhibitor, a PI3Ka inhibitor, an Akt inhibitor, an mTOR inhibitor, an Src inhibitor, vorinostat and paclitaxel, more preferably with at least one compound selected from the group consisting of afatinib, lapatinib, neratinib, BYL719, MK-2206, everolimus, saracatinib, vorinostat and paclitaxel, comprises an antigen-binding site that binds at least one amino acid of domain I of ErbB-2 selected from the group consisting of T144, T164, R166, P172, G179, S180 and R181, and surface-exposed amino acid residues that are located within about 5 amino acid positions from T144, T164, R166, P172, G179, S180 or R181.

In one preferred embodiment, an antibody according to the invention that is combined with one or more compounds selected from the group consisting of an inhibitor of a component of the PI3Kinase pathway, an inhibitor of a component of the MAPK pathway, a microtubuli disrupting drug and an HDAC inhibitor, preferably with at least one compound selected from the group consisting of a tyrosine kinase inhibitor, a PI3Ka inhibitor, an Akt inhibitor, an mTOR inhibitor, an Src inhibitor, vorinostat and paclitaxel, more preferably with at least one compound selected from the group consisting of afatinib, lapatinib, neratinib, BYL719, MK-2206, everolimus, saracatinib, vorinostat and paclitaxel, comprises an antigen-binding site that binds at least one amino acid of domain III of ErbB-3 selected from the group consisting of R426 and surface-exposed amino acid residues that are located within 11.2 Å from R426 in the native ErbB-3 protein.

Preferably, a bispecific antibody according to the invention comprising at least the CDR3 sequence, preferably at least the CDR1, CDR2 and CDR3 sequences, of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, and/or comprising at least the CDR3 sequence, preferably at least the CDR1, CDR2 and CDR3 sequences, of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37 is combined with one or more compounds selected from the group consisting of an inhibitor of a component of the PI3Kinase pathway, an inhibitor of a component of the MAPK pathway, a microtubuli disrupting drug and an HDAC inhibitor, preferably with at least one compound selected from the group consisting of a tyrosine kinase inhibitor, a PI3Ka inhibitor, an Akt inhibitor, an mTOR inhibitor, an Src inhibitor, vorinostat and paclitaxel, more preferably with at least one compound selected from the group consisting of afatinib, lapatinib, neratinib, BYL719, MK-2206, everolimus, saracatinib, vorinostat and paclitaxel

In one preferred embodiment a bispecific antibody according to the invention comprising:
- an ErbB-2 specific heavy chain variable region sequence selected from the group consisting of the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or comprising an ErbB-2 specific heavy chain variable region sequence that differs in at most 15 amino acids, preferably in at most 1, 2, 3, 4, 5, 6, 7, 8, 9 or 10 amino acids, more preferably in at most 1, 2, 3, 4 or 5 amino acids, from the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 or MF1898, and
- an ErbB-3 specific heavy chain variable region sequence selected from the group consisting of the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or comprising an ErbB-3 specific heavy chain variable region sequence that differs in at most 15 amino acids, preferably in at most 1, 2, 3, 4, 5, 6, 7, 8, 9 or 10 amino acids, more preferably in at most 1, 2, 3, 4 or 5 amino acids, from the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074, is combined with one or more compounds selected from the group consisting of an inhibitor of a component of the PI3Kinase pathway, an inhibitor of a component of the MAPK pathway, a microtubuli disrupting drug and an HDAC inhibitor, preferably with at least one compound selected from the group consisting of a tyrosine kinase inhibitor, a PI3Ka inhibitor, an Akt inhibitor, an mTOR inhibitor, an Src inhibitor, vorinostat and paclitaxel, more preferably with at least one compound selected from the group consisting of afatinib, lapatinib, neratinib, BYL719, MK-2206, everolimus, saracatinib, vorinostat and paclitaxel. In one preferred embodiment, antibody PB4188 is combined with one or more compounds selected from the group consisting of an inhibitor of a component of the PI3Kinase pathway, an inhibitor of a component of the MAPK pathway, a microtubuli disrupting drug and an HDAC inhibitor, preferably with at least one compound selected from the group consisting of a tyrosine kinase inhibitor, a PI3Ka inhibitor, an Akt inhibitor, an mTOR inhibitor, an Src inhibitor, vorinostat and paclitaxel, more preferably with at least one compound selected from the group consisting of afatinib, lapatinib, neratinib, BYL719, MK-2206, everolimus, saracatinib, vorinostat and paclitaxel.

Preferred embodiments of the invention provide uses of antibodies according to the invention under heregulin stress conditions. Heregulin is a growth factor that is involved in growth of ErbB-3 positive tumor cells. Typically, when the tumor cells express high levels of heregulin (referred to as heregulin stress), currently known therapies like trastuzumab, pertuzumab and lapatinib are no longer capable of inhibiting tumor growth. This phenomenon is called heregulin resistance. Surprisingly, however, an antibody according to the invention is also capable of counteracting growth of tumor cells that express high levels of heregulin. As used herein, an expression level of heregulin is considered high if a cell has a heregulin expression level that is at least 60%, preferably at least 70%, more preferably at least 80%, more preferably at least 85%, more preferably at least 90% or 95% of the heregulin expression level of BXPC3 or MCF7 cells. Heregulin expression levels are for instance measured using qPCR with tumor RNA (such as for instance described in Shames et al. PLOS ONE, February 2013, Vol.8, Issue 2, pp 1-10 and in Yonesaka et al., Sci.transl.Med., Vol.3, Issue 99 (2011); pp1-11), or using protein detection methods, like for instance ELISA, preferably using blood, plasma or serum samples (such as for instance described in Yonesaka et al., Sci.transl.Med., Vol.3, Issue 99 (2011); pp1-11). Further provided is therefore an antibody according to the invention for use in the treatment of a subject having or at risk of having an ErbB-2, ErbB-3 or ErbB-2/ErbB-3 positive tumor, wherein said cells of said tumor have a heregulin expression level that is at least 60%, preferably at least 70%, more preferably at least 80%, more preferably at least 85%, more preferably at least 90% or 95% of the heregulin expression level of BXPC3 or MCF7 cells. Said antibody according to the invention preferably comprises a first antigen-binding site that binds domain I of ErbB-2. Also provided is a method for the treatment of a subject having a ErbB-2, ErbB-3 or ErbB-2/ErbB-3 positive tumor, wherein cells of said tumor have a heregulin expression level that is at least 60%, preferably at least 70%, more preferably at least 80%, more preferably at least 85%, more preferably at least 90% or 95% of the heregulin expression level of BXPC3 or MCF7 cells, the method comprising administering to the subject an antibody or pharmaceutical composition according to the invention. One preferred embodiment provides a use of an antibody according to the invention for the preparation of a medicament for the treatment of an ErbB-2, ErbB-3 or ErbB-2/ErbB-3 positive tumor, wherein cells of said tumor have a heregulin expression level that is at least 60%, preferably at least 70%, more preferably at least 80%, more preferably at least 85%, more preferably at least 90% or 95% of the heregulin expression level of BXPC3 or MCF7 cells. Said ErbB-2, ErbB-3 or ErbB-2/ErbB-3 positive tumor is preferably breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma. Most preferably, said tumor is breast cancer. Further provided is therefore an antibody according to the invention for use in the treatment of a subject having or at risk of having breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma, preferably breast cancer, wherein cells of said cancer have a heregulin expression level that is at least 60%, preferably at least 70%, more preferably at least 80%, more preferably at least 85%, more preferably at least 90% or 95% of the heregulin expression level of BXPC3 or MCF7 cells. Said antibody according to the invention preferably comprises a first antigen-binding site that binds domain I of ErbB-2.

High heregulin levels are typically present during the formation of metastases (i.e. the migration, invasion, growth and/or differentiation of tumor cells or tumor initiating cells). Typically, tumor initiating cells are identified based on stem cell markers such as for instance CD44, CD24, CD133 and/or ALDH1. These processes can therefore barely be counteracted with currently known therapies like trastuzumab and pertuzumab. Since an antibody according to the invention is capable of counteracting growth and/or differentiation of tumor cells or tumor initiating cells that express high levels of heregulin, such antibody according to the invention is also particularly suitable for counteracting the formation of metastases. Further provided is therefore a method for counteracting the formation of a metastasis in a subject having a ErbB-2, ErbB-3 or ErbB-2/ErbB-3 positive tumor, wherein said ErbB-2, ErbB-3 or ErbB-2/ErbB-3 positive tumor cell has a heregulin expression level that is at least 60%, preferably at least 70%, more preferably at least 80%, more preferably at least 85%, more preferably at least 90% or 95% of the heregulin expression level of BXPC3 or MCF7 cells, comprising administering to the subject a bispecific antibody comprising a first antigen-binding site that binds ErbB-2 and a second antigen-binding site that binds ErbB-3. Also provided is a bispecific antibody comprising a first antigen-binding site that binds ErbB-2 and a second antigen-binding site that binds ErbB-3 for use in the treatment or prevention of the formation of metastases, wherein said ErbB-2, ErbB-3 or ErbB-2/ErbB-3 positive tumor cell has a heregulin expression level that is at least 60%, preferably at least 70%, more preferably at least 80%, more preferably at least 85%, more preferably at least 90% or 95% of the heregulin expression level of BXPC3 or MCF7 cells. Further provided is a use of a bispecific antibody according to the invention for the preparation of a medicament for the treatment or prevention of the formation of metastases, wherein said ErbB-2, ErbB-3 or ErbB-2/ErbB-3 positive tumor cell has a heregulin expression level that is at least 60%, preferably at least 70%, more preferably at least 80%, more preferably at least 85%, more preferably at least 90% or 95% of the heregulin expression level of BXPC3 or MCF7 cells. Said ErbB-2, ErbB-3 or ErbB-2/ErbB-3 positive tumor is preferably breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma. Most preferably, said tumor is breast cancer. Further provided is therefore a bispecific antibody according to the invention comprising a first antigen-binding site that binds ErbB-2 and a second antigen-binding site that binds ErbB-3 for use in the treatment or prevention of the formation of metastases of breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma cells, preferably breast cancer cells, wherein said cells have a heregulin expression level that is at least 60%, preferably at least 70%, more preferably at least 80%, more preferably at least 85%, more preferably at least 90% or 95% of the heregulin expression level of BXPC3 or MCF7 cells. Said antibody according to the present invention is typically capable of reducing a ligand-induced receptor function, preferably ligand induced growth, of ErbB-3 on a ErbB-2 and ErbB-3 positive cell. Said antibody according to the invention preferably comprises a first antigen-binding site that binds domain I of ErbB-2 and a second antigen-binding site that binds domain III of ErbB-3. In one preferred embodiment, the affinity (KD) of said second antigen-binding site for an ErbB-3 positive cell is equal to, or higher than, the affinity of said first antigen-binding site for an ErbB-2 positive cell. The affinity of said second antigen-binding site for an ErbB-3 positive cell is preferably lower than or equal to 2.0 nM, more preferably lower than or equal to 1.39 nM, more preferably lower than or equal to 0.99 nM. The affinity of said first antigen-binding site for an ErbB-2 positive cell is preferably lower than or equal to 5.0 nM, preferably lower than or equal to 4.5 nM preferably lower than or equal to 4.0 nM.

In one preferred embodiment, said antibody according to the invention comprises an antigen-binding site that binds at least one amino acid of domain I of ErbB-2 selected from the group consisting of T144, T164, R166, P172, G179, S180 and R181, and surface-exposed amino acid residues that are located within about 5 amino acid positions from T144, T164, R166, P172, G179, S180 or R181.

In one preferred embodiment, said antibody according to the invention preferably comprises an antigen-binding site that binds at least one amino acid of domain III of ErbB-3 selected from the group consisting of R426 and surface-exposed amino acid residues that are located within 11.2 Å from R426 in the native ErbB-3 protein.

One preferred embodiment provides a method according to the invention for the treatment of a subject having a ErbB-2, ErbB-3 or ErbB-2/ErbB-3 positive tumor wherein cells of said tumor have a heregulin expression level that is at least 60%, preferably at least 70%, more preferably at least 80%, more preferably at least 85%, more preferably at least 90% or 95% of the heregulin expression level of BXPC3 or MCF7 cells, or an antibody according to the invention for use in such treatment, wherein said antibody comprises at least the CDR3 sequence, preferably at least the CDR1, CDR2 and CDR3 sequences, or at least the heavy chain variable region sequence, of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E.

One preferred embodiment provides a method according to the invention for the treatment of a subject having a ErbB-2, ErbB-3 or ErbB-2/ErbB-3 positive tumor wherein cells of said tumor have a heregulin expression level that is at least 60%, preferably at least 70%, more preferably at least 80%, more preferably at least 85%, more preferably at least 90% or 95% of the heregulin expression level of BXPC3 or MCF7 cells, or an antibody according to the invention for use in such treatment, wherein said antibody comprises at least the CDR3 sequence, preferably at least the CDR1, CDR2 and CDR3 sequences, or at least the heavy chain variable region sequence, of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37. One embodiment provides antibody PB4188 for use in the treatment of a subject having a ErbB-2, ErbB-3 or ErbB-2/ErbB-3 positive tumor, wherein cells of said tumor have a heregulin expression level that is at least 60%, preferably at least 70%, more preferably at least 80%, more preferably at least 85%, more preferably at least 90% or 95% of the heregulin expression level of BXPC3 or MCF7 cells.

As already described, antibodies according to the present invention are particularly suitable for treating ErbB-2 positive tumor cells with less than 1.000.000 ErbB-2 receptors on their cell surface. Patients with such tumors, who are typically classified as ErbB-2 [++] or ErbB-2 [+], include patients with primary tumors as well as patients with relapsed ErbB-2 positive tumors. Currently used therapies such as trastuzumab (Herceptin) and pertuzumab are only prescribed for patients with malignant ErbB-2 positive cells that have more than 1.000.000 ErbB-2 receptors on their cell surface, which are classified as ErbB-2 [+++]. Patients that are classified as ErbB-2 [++] or ErbB-2 [+] are therefore preferably treated with an antibody according to the present invention. Further provided is therefore a method or antibody for use according to the invention, wherein said subject has an ErbB-2 or ErbB-2/ErbB-3 positive tumor that has less than 1.000.000 ErbB-2 cell-surface receptors per tumor cell. One preferred embodiment provides a bispecific antibody according to the invention comprising a first antigen-binding site that binds ErbB-2 and a second antigen-binding site that binds ErbB-3 for use in the treatment or prevention of the formation of metastases, wherein said ErbB-2, ErbB-3 or ErbB-2/ErbB-3 positive tumor cell has a heregulin expression level that is at least 60%, preferably at least 70%, more preferably at least 80%, more preferably at least 85%, more preferably at least 90% or 95% of the heregulin expression level of BXPC3 or MCF7 cells, and wherein said tumor cell has less than 1.000.000 ErbB-2 cell-surface receptors.

In another preferred embodiment, an antibody according to the invention is used for counteracting an ErbB-2, ErbB-3 or ErbB-2/ErbB-3 positive tumor in a subject who has an impaired cardiac function, or who is at risk thereof. With an impaired cardiac function is meant that the subject has a cardiac function, such as for instance the left ventricular ejection fraction (LVEF), that is lower than 90%, preferably lower than 85% or lower than 80%, preferably lower than 75% or lower than 70%, as compared to a healthy cardiac function. Said healthy cardiac function is, for instance, the average cardiac function (such as for instance the average LVEF) of the healthy population. Alternatively, said healthy cardiac function is the function (such as the LVEF) as measured in a patient before the start of anti-tumor therapy with an antibody according to the invention.

Cardiac function is for instance monitored by a physical examination of the subject and by an examination of the LVEF, using for instance an echocardiogram or a MUGA scan.

ErbB-2 is involved in growth, repair, and survival of adult cardiomyocytes as part of a signalling network that involves the heregulin receptor complex HER2:HER4. As described herein before, cardiotoxicity is a known risk factor in ErbB-2 targeting therapies and the frequency of complications is increased when trastuzumab is used in conjunction with anthracyclines thereby inducing cardiac stress. For instance, the combination of doxycycline with trastuzumab induces severe cardiac side effects. Despite the increasing number of clinical cases of trastuzumab-induced cardiac dysfunction, its mechanism of action is unknown. In view of the cardiotoxicity of currently known therapies against ErbB-2, ErbB-3 or ErbB-2/ErbB-3 positive tumors, it is of particular advantage to use an antibody according to the invention. As shown in the Examples, antibodies have now been provided that do not, or to a significantly lesser extent as compared to trastuzumab and pertuzumab, affect the survival of cardiomyocytes. This provides an important advantage since cardiotoxicity is reduced. This is already advantageous for people who do not suffer from an impaired cardiac function, and even more so for people who do suffer from an impaired cardiac function, such as for instance subjects suffering from congestive heart failure (CHF), left ventricular dysfunction (LVD) and/or a decreased Left Ventricular Ejection Fraction (LVEF), and/or subjects who have had a myocardial infarction. Further provided is therefore a bispecific antibody according to the invention for use in the treatment of a subject having or at risk of having an ErbB-2, ErbB-3 or ErbB-2/ErbB-3 positive tumor, wherein said subject has a cardiac function that is lower than 90%, preferably lower than 85% or lower than 80% or lower than 75% or lower than 70%, as compared to a healthy cardiac function. Said cardiac function preferably includes the LVEF. Said ErbB-2, ErbB-3 or ErbB-2/ErbB-3 positive tumor is preferably breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma. Most preferably, said tumor is breast cancer. Said antibody according to the invention preferably comprises a first antigen-binding site that binds domain I of ErbB-2 and a second antigen-binding site that binds domain III of ErbB-3. One preferred embodiment provides a method according to the invention for the treatment of a subject having a ErbB-2, ErbB-3 or ErbB-2/ErbB-3 positive tumor wherein the subject has a cardiac function that is lower than 90%, preferably lower than 85%, preferably lower than 80%, preferably lower than 75% or lower than 70%, as compared to a healthy cardiac function, or an antibody according to the invention for use in such treatment, wherein said antibody comprises:
- at least the CDR3 sequence, preferably at least the CDR1, CDR2 and CDR3 sequences, or at least the heavy chain variable region sequence, of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or a heavy chain variable region sequence that differs in at most 15 amino acids, preferably in at most 1, 2, 3, 4, 5, 6, 7, 8, 9 or 10 amino acids, more preferably in at most 1, 2, 3, 4 or 5 amino acids, from the recited heavy chain variable region sequences; and/or
- at least the CDR3 sequence, preferably at least the CDR1, CDR2 and CDR3 sequences, or at least the heavy chain variable region sequence, of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or a heavy chain variable region sequence that differs in at most 15 amino acids, preferably in at most 1, 2, 3, 4, 5, 6, 7, 8, 9 or 10 amino acids, more preferably in at most 1, 2, 3, 4 or 5 amino acids, from the recited heavy chain variable region sequences. In one preferred embodiment, said antibody is PB4188.

In one embodiment, said bispecific antibody is for use in the treatment of a subject under heregulin stress conditions, as explained in more detail elsewhere. Further provided is therefore a bispecific antibody according to the invention for use in the treatment of a subject having or at risk of having an ErbB-2, ErbB-3 or ErbB-2/ErbB-3 positive tumor, wherein said subject has a cardiac function that is lower than 90%, preferably lower than 85%, preferably lower than 80%, preferably lower than 75% or lower than 70%, as compared to a healthy cardiac function, and wherein said cells of said tumor have a heregulin expression level that is at least 60%, preferably at least 70%, more preferably at least 80%, more preferably at least 85%, more preferably at least 90% or 95% of the heregulin expression level of BXPC3 or MCF7 cells. Said cardiac function preferably includes the LVEF. Also provided is a method for the treatment of a subject having a ErbB-2, ErbB-3 or ErbB-2/ErbB-3 positive tumor, wherein the subject has a cardiac function that is lower than 90%, preferably lower than 85%, preferably lower than 80%, preferably lower than 75%, preferably lower than 70%, as compared to a healthy cardiac function, and wherein cells of said tumor have a heregulin expression level that is at least 60%, preferably at least 70%, more preferably at least 80%, more preferably at least 85%, more preferably at least 90% or 95% of the heregulin expression level of BXPC3 or MCF7 cells, the method comprising administering to the subject a bispecific antibody or pharmaceutical composition according to the invention. One preferred embodiment provides a use of a bispecific antibody according to the invention for the preparation of a medicament for the treatment of an ErbB-2, ErbB-3 or ErbB-2/ErbB-3 positive tumor in a subject who has a cardiac function, preferably a LVEF, that is lower than 90%, preferably lower than 85%, preferably lower than 80%, preferably lower than 75% or lower than 70%, as compared to a healthy cardiac function, preferably a healthy LVEF, wherein cells of said tumor have a heregulin expression level that is at least 60%, preferably at least 70%, more preferably at least 80%, more preferably at least 85%, more preferably at least 90% or 95% of the heregulin expression level of BXPC3 or MCF7 cells.

Also provided is a bispecific antibody comprising a first antigen-binding site that binds ErbB-2 and a second antigen-binding site that binds ErbB-3 for use in the treatment or prevention of the formation of metastases, wherein said subject has a cardiac function that is lower than 90%, preferably lower than 85%, preferably lower than 80%, preferably lower than 75%, preferably lower than 70% as compared to a healthy cardiac function. Further provided is a use of a bispecific antibody according to the invention for the preparation of a medicament for the treatment or prevention of the formation of metastases, wherein said subject has a cardiac function that is lower than 90%, preferably lower than 85%, preferably lower than 80%, preferably lower than 75%, preferably lower than 70% as compared to a healthy cardiac function. Said ErbB-2, ErbB-3 or ErbB-2/ErbB-3 positive tumor is preferably breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma. Most preferably, said tumor is breast cancer. Said cardiac function preferably includes the LVEF. In one preferred embodiment, said antibody is antibody PB4188.

In another embodiment, use is made of antibodies according to the invention for counteracting phosphorylation of various factors of the prosurvival pathway Akt (also referred to as the PI3 kinase pathway) and the MAP kinase pathway. These are downstream pro-proliferative signaling pathways of HER3. Surprisingly, the inventors have succeeded in significantly inhibiting phosphorylation of Akt, ERK1/2 and S6 ribosomal protein (S6-RP) with an antibody according to the present invention, whereas trastuzumab and pertuzumab do not have these strong anti-phosphorylation effects. Counteracting phosphorylation of factors of the pro-proliferative PI3 kinase and MAP kinase pathways is advantageous, since this counteracts growth of an ErbB-3 positive tumor cell. Further provided is therefore a use of an antibody according to the invention for counteracting, preferably inhibiting, phosphorylation of Akt, ERK1/2 and/or S6-RP. Importantly, phosphorylation of Akt can be significantly reduced or even completely blocked with an antibody of the invention, both *in vitro* and *in vivo,* as shown in the Examples. A preferred embodiment therefore provides a use of an antibody according to the invention for counteracting, preferably inhibiting, phosphorylation of Akt. Also provided is a use of an antibody according to the invention for counteracting the formation of a HER3-p85 complex. Since the formation of a HER3-p85 complex is the first phase in Akt activation, it is advantageous to counteracting the formation of said HER3-p85 complex. Said antibody according to the invention is preferably a bispecific antibody comprising a first antigen-binding site that binds domain I ErbB-2 and a second antigen-binding site that binds domain III of ErbB-3. Said antibody preferably comprises an antigen-binding site that binds at least one amino acid of domain I of ErbB-2 selected from the group consisting of T144, T164, R166, P172, G179, S180 and R181, and surface-exposed amino acid residues that are located within about 5 amino acid positions from T144, T164, R166, P172, G179, S180 or R181. Additionally, or alternatively, said antibody preferably comprises an antigen-binding site that binds at least one amino acid of domain III of ErbB-3 selected from the group consisting of F409 and R426 and surface-exposed amino acid residues that are located within 11.2 Å from R426 in the native ErbB-3 protein. In one embodiment, said antibody comprises at least one CDR1, CDR2 and CDR3 sequence, or at least one VH sequence, as depicted in Figure 16 or Figure 37. In one embodiment, said antibody is PB4188.

The invention also provides a method of treatment of an individual that has an ErbB-2 positive tumor or is at risk of developing an ErbB-2 positive tumor the method comprising administering to the individual in need thereof an ErbB-2 targeting agent, including an ErbB-2 inhibitor or binding agent, such as a bivalent monospecific antibody that comprises an antigen binding site that can bind an epitope on an extracellular part of ErbB-2, and a bispecific antibody that comprises an antigen-binding site that can bind an epitope on an extracellular part of ErbB-2 and an antigen-binding site that can bind an epitope on an extracellular part of ErbB-3.

Where the ErbB-2 inhibitor is a monospecific antibody, the monospecific antibody and the bispecific antibody preferably bind different epitopes on ErbB-2. The different ErbB-2 epitopes are preferably on different extracellular ErbB-2 domains. The monospecific antibody can preferably bind an epitope on ErbB-2 extracellular domain IV, domain II or domain III. The bispecific antibody can preferably bind an epitope on ErbB-2 extracellular domain I.

The ErbB-2 targeting agent may comprise a drug conjugate, in particular where the ErbB-2 inhibitor is a monospecific antibody, the monospecific antibody preferably comprises a drug conjugate, for example, ado-trastuzumab emtansine (Kadcyla^{®}).

The drug conjugate can also be on the bispecific antibody or on both the bispecific antibody and the targeting agent of ErbB2. The drug conjugate preferably comprises emtansine. Antibody-drug conjugates or ADCs are an important class of highly potent biopharmaceutical drugs designed as a targeted therapy for the treatment of people with cancer. Unlike chemotherapy, ADCs are intended to target and kill only the cancer cells and spare healthy cells. A drug conjugate is an antibody linked to a biologically active cytotoxic (anticancer) payload or drug. By combining the unique capabilities of monoclonal antibodies with the cancer-killing ability of cytotoxic drugs, antibody-drug conjugates allow sensitive discrimination between healthy and diseased tissue. This means that, in contrast to traditional chemotherapeutic agents, antibody-drug conjugates target and attack the cancer cell so that healthy cells are less severely affected. Antibody drug conjugates are described in DiJoseph et al; Blood. 2004 103(5):1807-14. Mullard A. Nature Reviews Drug Discovery 12, 329-332 (2013); Zolot RS et al; Nature Reviews Drug Discovery 12, 259-260 (2013); Merten et al; Bioconjug Chem 2015; 26:2176-2185; Schlom et al; Cancer Res. 1992; 52(5):1067-72. Rohrer T. Journal of Antibody-drug Conjugates. June 21, 2013. Suitable drugs for incorporation into an ADC are the Auristatins (Tubulin polymerase inhibitors); Maytansines (Tubulin depolymerisation); Calicheamicins (DNA cleavage); Duocarymycins (DNA minor groove alkylating agents); PBD dimers (DNA minor groove cross-linkers); and α-Amanitin (RNA polymerase II inhibitor). In a preferred embodiment the drug is emtansine.

Where the ErbB-2 inhibitor is a monospecific antibody, the monospecific antibody is preferably trastuzumab (CAS Number 180288-69-1). It can be replaced or combined with pertuzumab (CAS Number 380610-27-5) In a particularly preferred embodiment the monospecific antibody is trastuzumab- emtansine (T-DM1 also marketed under the name Kadcyla^{®}).

The bispecific antibody preferably comprises a first antigen-binding site that binds ErbB-2 and a second antigen-binding site that binds ErbB-3, wherein said first antigen-binding site comprises at least the CDR3 sequence of MF3958, or a CDR3 sequence that differs in at most three, preferably in at most two, preferably in no more than one amino acid from the CDR3 sequence of MF3958, and wherein said second antigen-binding site comprises at least the CDR3 sequence of MF3178, or a CDR3 sequence that differs in at most three, preferably in at most two, preferably in no more than one amino acid from the CDR3 sequence of MF3178. The bispecific antibody preferably comprises a first antigen-binding site that binds ErbB-2 and a second antigen-binding site that binds ErbB-3, wherein said first antigen-binding site comprises at least the CDR3 sequence of MF3958 and wherein said second antigen-binding site comprises at least the CDR3 sequence of MF3178.

In a preferred embodiment the bispecific antibody comprises a first antigen-binding site that binds ErbB-2 and a second antigen-binding site that binds ErbB-3, wherein said first antigen-binding site comprises at least the CDR1, CDR2 and CDR3 sequences of MF3958, or CDR1, CDR2 and CDR3 sequences that differ in at most three, preferably in at most two, preferably in at most one amino acid from the CDR1, CDR2 and CDR3 sequences of MF3958, and wherein said second antigen-binding site comprises at least the CDR1, CDR2 and CDR3 sequence of MF3178, or CDR1, CDR2 and CDR3 sequences that differ in at most three, preferably in at most two, preferably in at most one amino acid from the CDR1, CDR2 and CDR3 sequences of MF3178.

In a preferred embodiment the bispecific antibody comprises a first antigen-binding site that binds ErbB-2 and a second antigen-binding site that binds ErbB-3, wherein said first antigen-binding site comprises at least the CDR1, CDR2 and CDR3 sequences of MF3958 and wherein said second antigen-binding site comprises at least the CDR1, CDR2 and CDR3 sequence of MF3178.

In a preferred embodiment the bispecific antibody comprises a variable domain that binds ErbB-2 and a variable domain that binds ErbB-3,
wherein the VH chain of the variable domain that binds ErbB-2 comprises
   - the amino acid sequence of VH chain MF3958 as depicted in Figure 16A; or
   - the amino acid sequence of VH chain MF3958 as depicted in Figure 16A having at most 15, preferably 1, 2, 3, 4, 5, 6, 7, 8, 9 or 10, more preferably at most 1, 2, 3, 4 or 5, amino acid insertions, deletions, substitutions or a combination thereof with respect said VH; and
wherein the VH chain of the variable domain that binds ErbB-3 comprises
   - the amino acid sequence of VH chain MF3178 as depicted in Figure 16B; or
   - the amino acid sequence of VH chain MF3178 depicted in Figure 16B having at most 15, preferably 1, 2, 3, 4, 5, 6, 7, 8, 9 or 10 more preferably at most 1, 2, 3, 4 or 5, amino acid insertions, deletions, substitutions or a combination thereof with respect to the VH chain sequence of Figure 16B.

The bispecific antibody is preferably antibody PB4188. The treatment can be combined with a chemotherapy drug. Thus the treatment preferably further comprises administering a chemotherapy drug to the individual in need thereof. Many different chemotherapy drugs have been developed for the treatment of cancer. Invariably some or more active than others in combatting particular tumors.

The chemotherapy drug may be, for example, vinorelbine, paclitaxel, docetaxel, gemcitabine, eribulin, capecitabine or carboplatin.

The invention further provides a combination of an ErbB-2 a ErbB-2 targeting agent, including an inhibitor or binding agent, such as a bivalent monospecific antibody, that comprises antigen binding sites that can bind an epitope on an extracellular part of ErbB-2; and a bispecific antibody that comprises an antigen-binding site that can bind an epitope on extracellular part of ErbB-2 and an antigen-binding site that can bind an epitope on extracellular part of ErbB-3, for use in a method treatment of an individual that has an ErbB-2 positive tumor or is at risk of developing an ErbB-2 positive tumor.

Further provided is a pharmaceutical composition comprising a ErbB-2 targeting agent, including an ErbB-2 inhibitor or binding agent, such as a bivalent monospecific antibody that comprises antigen binding sites that can bind an epitope on an extracellular part of ErbB-2 and a bispecific antibody that comprises an antigen-binding site that can bind an epitope on an extracellular part of ErbB-2 and an antigen-binding site that can bind an epitope on an extracellular part of ErbB-3. Also provided is a kit of parts comprising a ErbB-2 targeting agent, including an ErbB-2 inhibitor or binding agent, such as a bivalent monospecific antibody that comprises antigen binding sites that can bind an epitope on an extracellular part of ErbB-2 and a bispecific antibody that comprises an antigen-binding site that can bind an epitope on an extracellular part of ErbB-2 and an antigen-binding site that can bind an epitope on an extracellular part of ErbB-3.

The invention further provides a method of treatment of an individual that has an ErbB-2 positive and ErbB-3 positive tumor in the brain or is at risk of developing an ErbB-2 positive and ErbB-3 positive tumor in the brain the method comprising administering to the individual in need thereof an antibody that comprises an antigen-binding site that can bind an epitope on an extracellular part of ErbB-2 and an antigen-binding site that can bind an epitope on an extracellular part of ErbB-3. The tumor is preferably a metastasis of a breast tumor. Preferably the antibody can bind an epitope on ErbB-2 extracellular domain I. Preferably the antibody can bind an epitope on ErbB-3 extracellular domain III. The method preferably further comprises administration of a ErbB-2 targeting agent, including an ErbB-2 inhibitor or binding agent, such as a monospecific bivalent antibody with antigen-binding sites that can bind an epitope on an extracellular part of ErbB-2. Preferably the method further comprises administration of a ErbB-2 targeting agent, including an ErbB-2 inhibitor or binding agent, such as a monospecific bivalent antibody with antigen-binding sites that can bind an epitope on an extracellular part of ErbB-3. An ErbB-2 inihibitor, such as a monospecific bivalent antibody with antigen-binding sites that can bind an epitope on an extracellular part of ErbB-2 or an epitope on an extracellular part of ErbB-3, may comprise a drug conjugate. The drug preferably comprises emtansine. The monospecific bivalent antibody with antigen-binding sites that can bind an epitope on an extracellular part of ErbB-2 is preferably trastuzumab, pertuzumab or a biosimilar with the same variable domain amino acid sequence. The antibody that comprises an antigen-binding site that can bind an epitope on an extracellular part of ErbB-2 and an antigen-binding site that can bind an epitope on an extracellular part of ErbB-3 is preferably a bispecific antibody. The bispecific antibody is preferably antibody PB4188. Further provided is an antibody that comprises an antigen-binding site that can bind an epitope on an extracellular part of ErbB-2 and an antigen-binding site that can bind an epitope on an extracellular part of ErbB-3 for use in the treatment of an individual that has an ErbB-2 positive and ErbB-3 positive tumor in the brain or is at risk of developing an ErbB-2 positive and ErbB-3 positive tumor in the brain.

An individual is at risk of developing a tumor as indicated herein if the individual has had a tumor and the tumor responded well to treatment provided to the individual. Particularly when the invidual has entered into complete remission such that the number of tumor cells in the individual is not measurable with conventional techniques such as regular MRI or CT scan imaging. Such an individual has, unfortunately, a much higher risk of developing a tumor either at the site of the original tumor (recurrent tumor) at a distant site (metastatic tumor) or develop a tumor of new orgin (for instance treatment induced). An individual at risk is thus preferably an individual that has had a tumor and is in complete remission thereof.

Provided is a bispecific antibody comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell. The antibody preferably can reduce ligand-induced growth of an ErbB-2 and ErbB-3 positive cell. The antibody can preferably reduce ligand-induced growth of an ErbB-2 and ErbB-3 positive cell, wherein said cell has at least 100.000 ErbB-2 cell-surface receptors per cell. Preferably said cell is an MCF-7 cell, an SKBR-3 cell, NCI-N87 cell, an BxPC-3 cell, an BT-474 cell or a JIMT-1 cell. The first antigen-binding site can preferably bind to domain I or domain IV of ErbB-2. The second antigen-binding site preferably interferes with binding of an ErbB-3 ligand to ErbB-3. Also provided is a bispecific antibody comprising a first antigen-binding site that binds ErbB-2 and a second antigen-binding site that binds ErbB-3, wherein said first antigen-binding site binds domain I of ErbB-2 and said second antigen-binding site binds domain III of ErbB-3. Further provided is an a bispecific antibody comprising a first antigen-binding site that binds ErbB-2 and a second antigen-binding site that binds ErbB-3, wherein the affinity (KD) of said second antigen-binding site for an ErbB-3 positive cell is equal to, or higher than, the affinity of said first antigen-binding site for an ErbB-2 positive cell. The antibody can preferably reduce a ligand-induced receptor function of ErbB-3 on a ErbB-2 and ErbB-3 positive cell. The antibody can preferably reduce ligand-induced growth of an ErbB-2 and ErbB-3 positive cell. The affinity (KD) of said second antigen-binding site for an ErbB-3 positive cell is preferably lower than or equal to 2.0 nM, preferably lower than or equal to 1.39 nM, more preferably lower than or equal to 0.99 nM. The affinity (KD) of said first antigen-binding site for an ErbB-2 positive cell is preferably lower than or equal to 5.0 nM, preferably lower than or equal to 4.5 nM preferably lower than or equal to 4.0 nM. The affinity (KD) of said bispecific antibody for BT 474 cells is preferably lower than or equal to 5.0 nM, preferably lower than or equal to 4.0 nM, more preferably lower than or equal to 3.2 nM, and/or wherein the affinity of said bispecific antibody for SK BR 3 cells is lower than or equal to 5.0 nM, preferably lower than or equal to 3.0 nM, more preferably lower than or equal to 2.0 nM. Further provided is an antibody comprising two antigen-binding sites that bind ErbB-2, wherein at least one of said antigen-binding sites binds domain I of ErbB-2. The affinity (KD) of at least one of said antigen-binding sites for an ErbB-2 positive cell is preferably lower than or equal to 5.0 nM, preferably lower than or equal to 4.0 nM, more preferably lower than or equal to 4.0 nM. Also provided is an antibody that comprises two antigen-binding sites that bind ErbB-3, wherein at least one of said antigen-binding sites binds domain III of ErbB-3. The affinity (KD) of at least one of said antigen-binding sites for an ErbB-3 positive cell is preferably lower than or equal to 2.0 nM, preferably lower than or equal to 1.39 nM, more preferably lower than or equal to 0.99 nM. Said ErbB-3 positive cell and/or said ErbB-2 positive cell is preferably a BT 474 cell or a SK BR 3 cell. The antibody preferably comprises an antigen-binding site that binds at least one amino acid of domain I of ErbB-2 selected from the group consisting of T144, T164, R166, P172, G179, S180 and R181, and surface-exposed amino acid residues that are located within about 5 amino acid positions from T144, T164, R166, P172, G179, S180 or R181. It preferably comprises an antigen-binding site that binds at least one amino acid of domain III of ErbB-3 selected from the group consisting and R426 and surface-exposed amino acid residues that are located within 11.2 Å from R426 in the native ErbB-3 protein. Said antibody preferably comprises at least the CDR3 sequence of an ErbB 2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E. Said antibody preferably comprises at least the CDR3 sequence of an ErbB 3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37. Said antibody preferably comprises at least the CDR1, CDR2 and CDR3 sequences of an ErbB 2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or wherein said antibody comprises CDR sequences that differ in at most 3 amino acids, preferably in at most 2 amino acids, preferably in at most 1 amino acid from the CDR1, CDR2 and CDR3 sequences of MF2926, MF2930, MF1849; MF2973, MF3004, MF3958, MF2971, MF3025, MF2916, MF3991, MF3031, MF2889, MF2913, MF1847, MF3001, MF3003 or MF1898. Said antibody preferably comprises at least the CDR1, CDR2 and CDR3 sequences of an ErbB 3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or wherein said antibody comprises CDR sequences that differ in at most 3 amino acids, preferably in at most 2 amino acids, preferably in at most 1 amino acid from the CDR1, CDR2 and CDR3 sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074. Said antibody preferably comprises an ErbB 2 specific heavy chain variable region sequence selected from the group consisting of the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 and MF1898 as depicted in Figure 16A or Figure 16E, or wherein said antibody comprises a heavy chain variable region sequence that differs in at most 15 amino acids from the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 or MF1898. Said antibody preferably comprises an ErbB 3 specific heavy chain variable region sequence selected from the group consisting of the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074 as depicted in Figure 16B or Figure 16E or Figure 37, or wherein said antibody comprises a heavy chain variable region sequence that differs in at most 15 amino acids from the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074. The antibody preferably exhibits antibody-dependent cell-mediated cytotoxicity (ADCC). The antibody is preferably afucosylated in order to enhance ADCC. It is preferably a human or humanized antibody. The antibody preferably comprises two different immunoglobulin heavy chains with compatible heterodimerization domains. Said compatible heterodimerization domains are preferably compatible immunoglobulin heavy chain CH3 heterodimerization domains. Preferably both arms comprise a common light chain. Said common light chain is preferably a germline light chain, preferably a rearranged germline human kappa light chain comprising the IgVKl-39 gene segment, most preferably the rearranged germline human kappa light chain IgVKl-39*01/IGJKl*01. The antibody preferably further comprises a label, preferably a label for in vivo imaging. Also provided is a pharmaceutical composition comprising a bispecific antibody as indicated herein. Also provided is a method for the treatment of a subject having a ErbB-2, ErbB-3 or ErbB-2/ErbB-3 positive tumor or at risk of having said tumor comprising administering to the subject an antibody or pharmaceutical composition according to the invention. Also provided is an antibody of the invention for use in the treatment of a subject having or at risk of having an ErbB-2, ErbB-3 or ErbB-2/ErbB-3 positive tumor. The bispecific antibody preferably does not significantly affect the survival of cardiomyocytes. Said bispecific antibody is for use for a subject who has a cardiac function that is lower than 90% as compared to a healthy cardiac function. Also provided is a method for the treatment of a subject having a ErbB-2, ErbB-3 or ErbB-2/ErbB-3 positive tumor or at risk of having said tumor comprising administering to the subject:
- a bispecific antibody comprising a first antigen-binding site that binds ErbB-2 and a second antigen-binding site that binds ErbB-3, and
- one or more compounds selected from the group consisting of an inhibitor of a component of the PI3Kinase pathway, an inhibitor of a component of the MAPK pathway, a microtubuli disrupting drug and an HDAC inhibitor, preferably one or more compounds selected from the group consisting of a tyrosine kinase inhibitor, a PI3Ka inhibitor, an Akt inhibitor, an mTOR inhibitor, an Src inhibitor, vorinostat and paclitaxel. Also provided is a bispecific antibody comprising a first antigen-binding site that binds ErbB-2 and a second antigen-binding site that binds ErbB-3 for use in the treatment of a ErbB-2, ErbB-3 or ErbB-2/ErbB-3 positive tumor, wherein said treatment comprises administering said bispecific antibody and at least one compound selected from the group consisting of an inhibitor of a component of the PI3Kinase pathway, an inhibitor of a component of the MAPK pathway, a microtubuli disrupting drug and an HDAC inhibitor, preferably administering said bispecific antibody and at least one compound selected from the group consisting of a tyrosine kinase inhibitor, a PI3Ka inhibitor, an Akt inhibitor, an mTOR inhibitor, an Src inhibitor, vorinostat and paclitaxel, to a subject having a ErbB-2, ErbB-3 or ErbB-2/ErbB-3 positive tumor. Said tyrosine kinase inhibitor preferably comprises afatinib, lapatinib and/or neratinib. Said PI3K inhibitor is preferably BYL719. Said Akt inhibitor is preferably MK 2206. Said mTOR inhibitor is preferably everolimus. Said Src inhibitor is preferably saracatinib. Said microtubuli targeting drug is preferably Paclitaxel. Said HDAC inhibitor is preferably vorinostat. Also provided is a method for counteracting the formation of a metastasis in a subject having a ErbB-2, ErbB-3 or ErbB-2/ErbB-3 positive tumor, wherein said ErbB-2, ErbB-3 or ErbB-2/ErbB-3 positive tumor cell has a heregulin expression level that is at least 60%, preferably at least 70%, more preferably at least 80%, more preferably at least 85%, more preferably at least 90% or 95% of the heregulin expression level of BXPC3 or MCF7 cells, comprising administering to the subject a bispecific antibody comprising a first antigen-binding site that binds ErbB-2 and a second antigen-binding site that binds ErbB-3. Also provided is a bispecific antibody comprising a first antigen-binding site that binds ErbB-2 and a second antigen-binding site that binds ErbB-3 for use in the treatment or prevention of the formation of a metastasis of a ErbB-2, ErbB-3 or ErbB-2/ErbB-3 positive tumor cell, wherein said ErbB-2, ErbB-3 or ErbB-2/ErbB-3 positive tumor cell has a heregulin expression level that is at least 60%, preferably at least 70%, more preferably at least 80%, more preferably at least 85%, more preferably at least 90% or 95% of the heregulin expression level of BXPC3 or MCF7 cells. Provided is a method or antibody for use according to the invention, wherein said subject has an ErbB-2 or ErbB-2/ErbB-3 positive tumor that has less than 1.000.000 ErbB-2 cell-surface receptors per cell. Said antibody is preferably an antibody according to the invention. Said tumor cell is preferably a breast cancer, gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, or melanoma cell. Said subject preferably has a cardiac function that is lower than 90% as compared to a healthy cardiac function. Said cardiac function preferably comprises the Left Ventricular Ejection Fraction (LVEF). Said subject preferably suffers from congestive heart failure (CHF), left ventricular dysfunction (LVD) and/or a ≥ 10% decreased Left Ventricular Ejection Fraction (LVEF), and/or wherein said subject has had a myocardial infarction. Also provided is the use of an antibody of the invention for counteracting, preferably inhibiting, phosphorylation of Akt, ERK and/or S6 ribosomal protein.

Further embodiments of the invention are summarized in the following list of embodiments:
1. A combination of a ErbB-2 targeting agent; and a bispecific antibody that comprises an antigen-binding site that can bind an epitope on extracellular part of ErbB-2 and an antigen-binding site that can bind an epitope on extracellular part of ErbB-3, for use in a method treatment of an individual that has an ErbB-2, ErbB-3 or ErbB-2/ErbB-3 positive tumor or is at risk of developing said tumor.
2. The combination for use of embodiment 1, wherein the targeting agent of ErbB-2 is an ErbB-2 binding agent inhibitor.
3. The combination for use of embodiment 1 or 2, wherein the targeting agent of ErbB-2 is an ErbB-2 inhibitor.
4. The combination for use of embodiment 3, wherein the inhibitor of ErbB-2 is a bivalent monospecific antibody that comprises antigen binding sites that can bind an epitope on an extracellular part of ErbB-2.
5. The combination for use of embodiment 4, wherein the monospecific antibody and the bispecific antibody bind different epitopes on ErbB-2.
6. The combination for use of embodiment 5, wherein the different ErbB-2 epitopes are on different extracellular ErbB-2 domains.
7. The combination for use of any one of embodiments 5-6, wherein the monospecific antibody can bind an epitope on ErbB-2 extracellular domain IV, domain III and/or domain II.
8. The combination for use of any one of embodiments 1-7, wherein the bispecific antibody can bind an epitope on ErbB-2 extracellular domain I.
9. The combination for use of any one of embodiments 4-8, wherein the monospecific antibody comprises a drug conjugate.
10. The combination for use of any one of embodiments 1-9, wherein the bispecific antibody comprises a drug conjugate.
11. The combination for use of embodiment 9 or embodiment 10, wherein the drug conjugate comprises emtansine.
12. The combination for use of any one of embodiments 4-11, wherein the monospecific antibody is Trastuzumab.
13. The combination for use of any one of embodiments 4-11, wherein the monospecific antibody is Trastuzumab emtansine
14. The combination for use of any one of embodiments 1-13, wherein the bispecific antibody comprises antibody PB4188.
15. The combination for use of any one of embodiments 1-14, further comprising administering a chemotherapy drug to the individual in need thereof.
16. A method of treatment of an individual that has an ErbB-2, ErbB-3 or ErbB-2/ErbB-3 positive tumor or is at risk of developing an ErbB-2, ErbB-3 or ErbB-2/ErbB-3 positive tumor the method comprising administering to the individual in need thereof a targeting agent of ErbB-2 and a bispecific antibody that comprises an antigen-binding site that can bind an epitope on an extracellular part of ErbB-2 and an antigen-binding site that can bind an epitope on an extracellular part of ErbB-3.
17. A pharmaceutical composition comprising a targeting agent of ErbB-2and a bispecific antibody that comprises an antigen-binding site that can bind an epitope on an extracellular part of ErbB-2 and an antigen-binding site that can bind an epitope on an extracellular part of ErbB-3.
18. A kit of parts comprising a targeting agent of ErbB-2 and a bispecific antibody that comprises an antigen-binding site that can bind an epitope on an extracellular part of ErbB-2 and an antigen-binding site that can bind an epitope on an extracellular part of ErbB-3.
19. A method of treatment according to embodiment 16, a pharmaceutical composition according to embodiment 17 or a kit of parts according to embodiment 18, wherein the targeting agent of ErbB-2 is a binding agent or inhibitor of ErbB-2.
20. A method of treatment, a pharmaceutical composition or a kit of parts according to embodiment 19, wherein the binding agent or inhibitor of ErbB-2 is lapatinib or neratinib.
21. A method of treatment according to embodiment 16, a pharmaceutical composition according to embodiment 17 or a kit of parts according to embodiment 18, wherein the targeting agent of ErbB-2 is a bivalent monospecific antibody that comprises antigen binding sites that can bind an epitope on an extracellular part of ErbB-2.
22. A bispecific antibody that comprises an antigen-binding site that can bind an epitope on an extracellular part of ErbB-2 and an antigen-binding site that can bind an epitope on an extracellular part of ErbB-3 for use in the treatment of an individual that has an ErbB-2 positive and ErbB-3 positive tumor in the brain or is at risk of developing an ErbB-2 positive and ErbB-3 positive tumor in the brain.
23. The bispecific antibody for use of embodiment 22, wherein the tumor is a metastasis of a breast tumor.
24. The bispecific antibody for use of embodiment 22 or embodiment 23, wherein the bispecific antibody can bind an epitope on ErbB-2 extracellular domain I.
25. The bispecific antibody for use of any one of embodiments 22-24, wherein the bispecific antibody can bind an epitope on ErbB-3 extracellular domain III.
26. The bispecific antibody for use of any one of embodiments 22-25, wherein the method further comprises administration of a targeting agent of ErbB-2.
27. The bispecific antibody for use of embodiment 26, wherein the targeting agent of ErbB-2 is lapatinib or neratinib.
28. The bispecific antibody for use of embodiment 26, wherein the targeting agent of ErbB-2 is a bivalent monospecific antibody that comprises antigen binding sites that can bind an epitope on an extracellular part of ErbB-2.
29. The bispecific antibody for use of any one of embodiments 22-28, wherein the method further comprises administration of a targeting agent of ErbB-3.
30. The bispecific antibody for use of embodiment 29, wherein the targeting agent of ErbB-3 is an antibody, such as patritumab, MM-121 (seribantumab), or lumretuzumab
31. The bispecific antibody for use of embodiment 29, wherein the inhibitor of ErbB-2 is a bivalent monospecific antibody that comprises antigen binding sites that can bind an epitope on an extracellular part of ErbB-3.
32. The bispecific antibody of embodiment 28 or embodiment 31, wherein the monospecific bivalent antibody with antigen-binding sites that can bind an epitope on an extracellular part of ErbB-2 or an epitope on an extracellular part of ErbB-3 comprises a drug conjugate.
33. The bispecific antibody of embodiment 32, wherein the drug comprises emtansine.
34. The bispecific antibody of any one of embodiments 28, wherein the monospecific bivalent antibody with antigen-binding sites that can bind an epitope on an extracellular part of ErbB-2 is trastuzumab, pertuzumab or a biosimilar with the same variable domain amino acid sequence.
35. The bispecific antibody of any one of embodiments 22-34, wherein the bispecific antibody is antibody PB4188.
36. A method of treatment of an individual that has an ErbB-2 positive and ErbB-3 positive tumor in the brain or is at risk of developing an ErbB-2 positive and ErbB-3 positive tumor in the brain the method comprising administering to the individual in need thereof a bispecific antibody that comprises an antigen-binding site that can bind an epitope on an extracellular part of ErbB-2 and an antigen-binding site that can bind an epitope on an extracellular part of ErbB-3.

For the purpose of clarity and a concise description features are described herein as part of the same or separate embodiments, however, it will be appreciated that the scope of the invention may include embodiments having combinations of all or some of the features described.

### BRIEF DESCRIPTION OF THE DRAWINGS

Figure 1 : Antigen titration on monomeric HER2 of a panel of HER2 arms that are also present in active HER2xHER3 bispecific antibodies in combination with one arm of PG3178. All HER2 monoclonals of the HER2xHER3 panel except for PG3025 were tested on an HER2 antigen titration ELISA.
Figure 2: Functional activity of HER2 x HER3 bispecific antibodies on BxPC3 cells with or without ligand stimulation. Dotted lines represent activity of trastuzumab, the reference antibody in this assay, with or without ligand stimulation.
Figure 3: Titration curves of HER2 and HER3 monoclonal antibodies (Upper panel) and HER2 x HER3 bispecific antibodies thereof (Lower panel) in the MCF-7 assay
Figure 4: Antibody treatment effect on BxPC3-luc2 tumor size at day 31 in an orthotopic murine model. BLI, tumor growth as measured by bioluminescence.
Figure 5: Antibody treatment effect on BxPC3-luc2 tumor size at day 31 in an orthotopic murine model. BLI, tumor growth as measured by bioluminescence.
Figure 6: FACS analysis of a bispecific HER2xHER3 antibody and its parental monoclonal antibodies on MCF-7 and BxPC3-luc2 HER2 expressing cells. MFI, mean fluorescence intensity.
Figure 7 : Analytical characterization by HP-SEC and CIEX-HPLC. PB4188 (upper panel), anti-HER2 parental monoclonal antibody (middle panel), anti-RSV monoclonal reference IgG (lower panel).
Figure 8: Inhibition of JIMT-1 cell proliferation in soft agar by a serial titration of antibody.
Figure 9: Inhibition of BT-474 (upper panel) and SKBR3 (lower panel) cell proliferation in matrigel by a serial titration of antibody.
Figure 10a: HRG induced proliferation and branching/invasion of SKBR-3 cells in matrigel.
Figure 10b:Inhibition of HRG induced proliferation and branching/invasion of SKBR-3 cells in matrigel by PB4188 in contrast to the parental monoclonal antibodies.
Figure 10c: Inhibition of HRG induced proliferation and branching/invasion of SKBR-3 cells in matrigel by PB4188 in contrast to anti-HER3 monoclonal antibodies.
Figure 10d: Inhibition of HRG induced proliferation and branching/invasion of SKBR-3 cells in matrigel by PB4188 in contrast to combinations of anti-HER3 monoclonal antibodies with trastuzumab.
Figure 10e: Inhibition of HRG induced proliferation and branching/invasion of SKBR-3 cells in matrigel by PB4188 and the combination PB4188 plus trastuzumab
Figure 11: Superior inhibitory activity of PB4188 in HER2⁺⁺⁺ N87 cells in the presence of 100 ng/ml HRG.
Figure 12: ADCC activity of PB4188 and PB3448 in a dose titration
Figure 13: Increased ADCC activity of bispecific antibody compared to monoclonal parental antibodies or a combination thereof
Figure 14: ADCC activity of afucosylated PB4188 compared to trastuzumab on low (upper panel) and high (lower panel) HER2 expressing cells
Figure 15: ADCC activity of afucosylated PB4188 on SKBR-3 HER2⁺⁺⁺ cells in the presence of reporter cells expressing a high or low FcγR variant
Figure 16: Nucleic acid and amino acid sequences of VH-chains, common light chain and heavy chains of antibodies of the invention. Where in this figure a leader sequence is indicated this is not part of the VH chain or antibody, but is typically cleaved of during processing of the protein in the cell that produces the protein.
Figure 17: Antibody treatment effect on tumor size in a JIMT-1 murine xenograft model. Tumor growth measured by tumor volume caliper measurement of the different treatment groups. Top, tumor growth during 60 days; bottom tumor growth inhibition (TGI) at the end of treatment period (29 days).
Figure 18: Kaplan-Meier survival curves of the different treatment groups in the JIMT-1 murine xenograft model.
Figure 19: Inhibition of N87 ligand driven growth. HRG driven proliferation of N87 can be overcome over a wide range of HRG by PB4188 in contrast to the parental anti-HER3 antibody. Data shown at antibody concentration of 40 ng/ml.
Figure 20: Steady state cell affinity measurements of ¹²⁵I-labeled IgG HER2xHER3 (PB4188) towards BT-474 cells (top; three independent assays) and SK-BR-3 cells (bottom; three independent assays). Non-specific binding was determined using a 100-fold excess of unlabeled HER2xHER3.
Figure 21A: Epitope mapping HER2. Critical residues identified are represented as black spheres on the HER2 crystal structure, secondary critical residues identified are represented as gray spheres (PDB ID #1S78).
Figure 21B
   a) HER2 crystal structure (PDB #1S78) showing verified PG3958 epitope residues as light gray spheres and surrounding residues (+/- five amino acid residues) as dark gray spheres. b) Solvent exposed surface of epitope region showing verified epitope residues in gray and surrounding residues (+/- five residues) in black. c) Detailed view of epitope region with verified epitope residues in light gray and surrounding residues (+/- five residues) in dark gray. d) Primary amino acid sequence of HER2 PG3958 epitope region indicating verified epitope residues (gray underlined), surrounding residues (black) and distant residues (gray italic, not shown in a, b and c). Figures and analyses were made with Yasara (www.yasara.org).
Figure 21C:
   a) HER3 crystal structure (PDB #4P59) showing epitope residue Arg 426 in gray spheres and all surface exposed residues within an 11.2 Å radius from Arg 426 in black spheres. b) Solvent exposed surface of epitope region with Arg 426 and distant residues shown in gray and all surface exposed residues within a 11.2 Å radius from Arg 426 shown in black. c) Residues in the epitope region Arg 426 in light gray and surrounding residues (all labeled) in dark gray. Figures and analyses were made with Yasara (www.yasara.org).

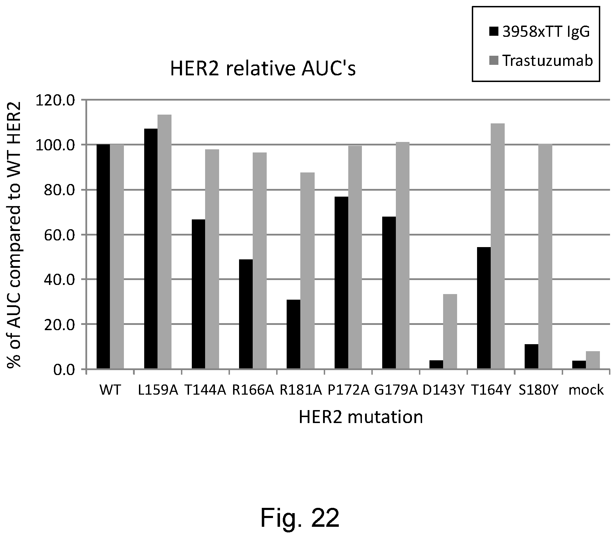

Figure 22: Confirmation of critical binding residues for Fab arm 3958 to HER2. Trastuzumab was included as a control antibody. Binding was determined in a FACS titration and binding is expressed as AUC in comparison to trastuzumab binding. D143Y is not considered to be part of the 3958 epitope as binding of Trastuzumab to this mutant is also blocked.

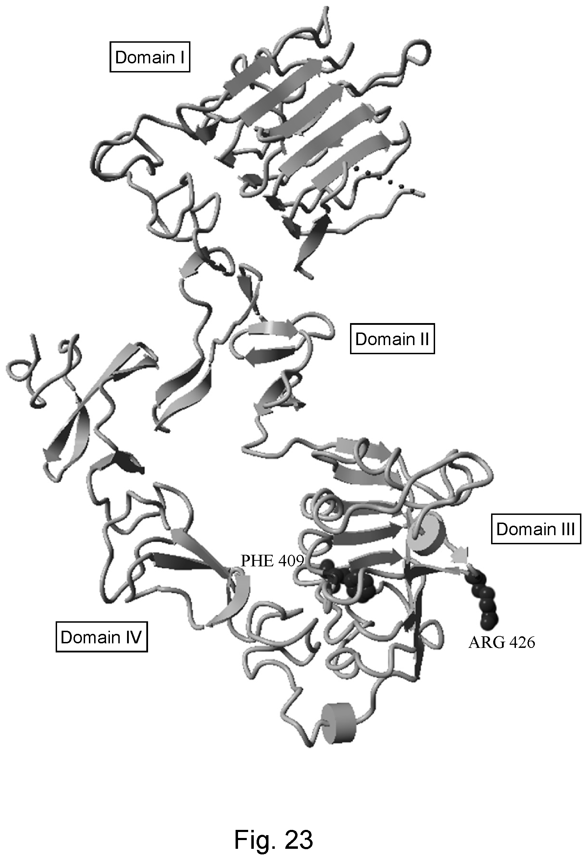

Figure 23: Critical residues for PG3178 binding represented in the HER3 crystal structure. Critical residues identified for PG3178binding are represented as black spheres on the HER3 crystal structure (PDB ID # 4P59).

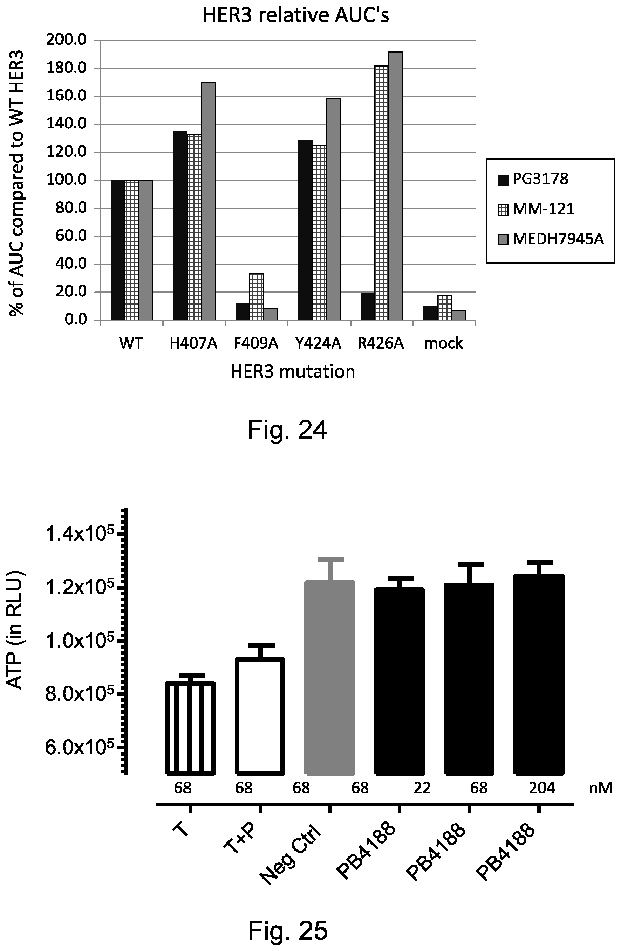

Figure 24: Confirmation of R426 as a critical binding residue for PG3175 to HER3. Two anti-HER3 antibodies were included as control antibodies. Binding was determined in a FACS titration and binding is expressed as AUC in comparison to binding to WT HER3.
Figure 25: Absence of PB4188 toxicity under cardiac stress *in vitro.* Incubation of cardiomyocytes with PB4188 or monospecific benchmark antibodies in the presence 3 µM of the anthracyclin doxorubicin. Viability of the cardiomyocytes was determined by quantification of ATP and expressed in relative light units (RLU). T, trastuzumab; P, pertuzumab.

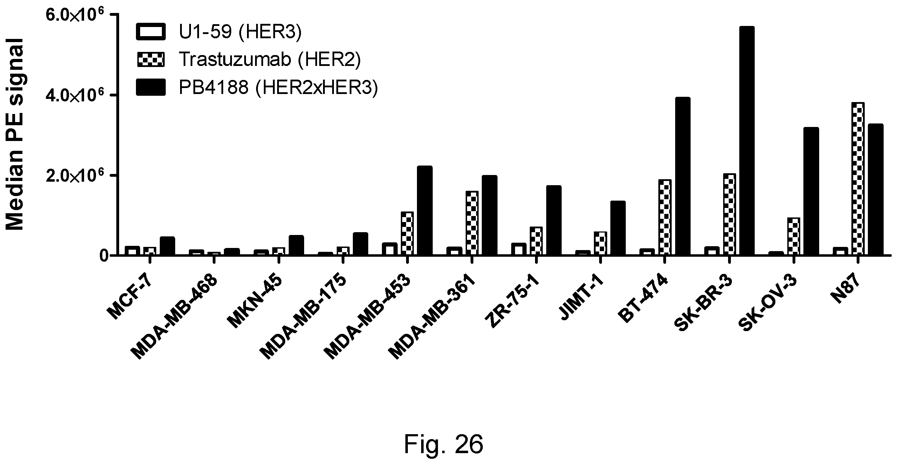

Figure 26: Binding of PB4188 in comparison to trastuzumab and a HER3 antibody to HER2 amplified cells. FACS titrations were performed on the indicated cell lines expressing different HER2 levels. Area under the curve of Median PE signal values were plotted per cell line.

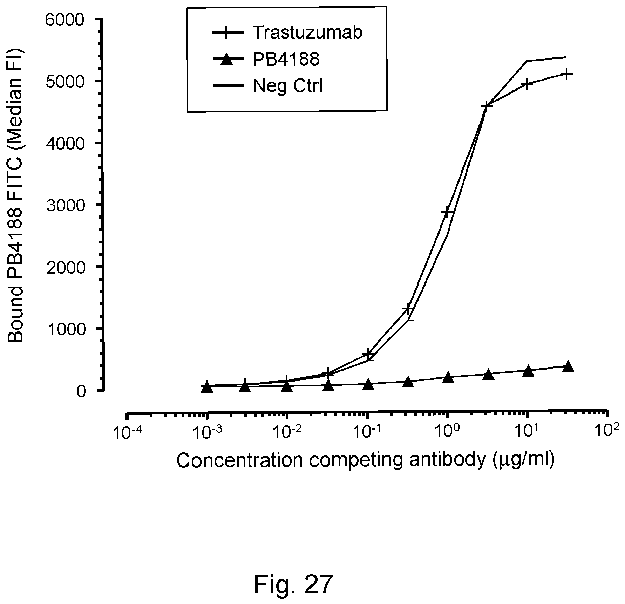

Figure 27: Binding of a serial titration of PB4188^{FITC} to SKBR-3 cells preincubated with a saturated concentration of PB4188, trastuzumab or a negative control antibody. PB4188^{FITC} binds as effectively to SKBR-3 in the presence of trastuzumab or control antibody.

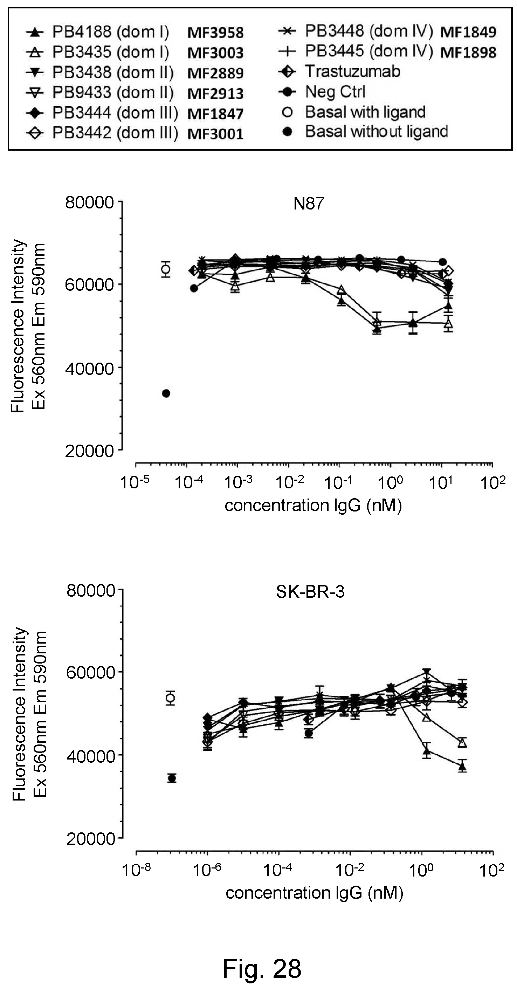

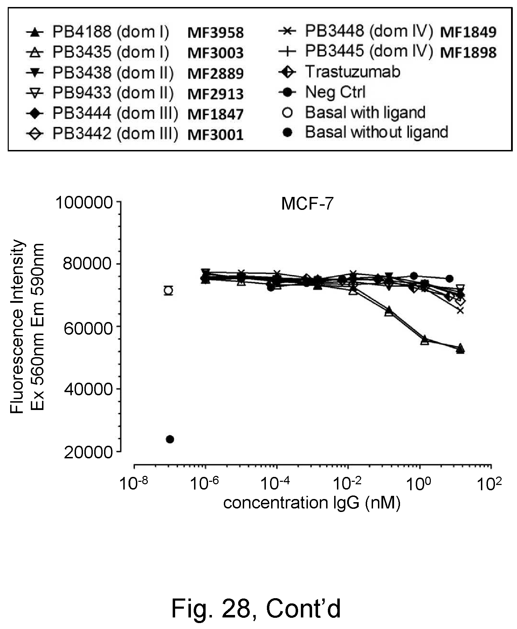

Figure 28: Inhibition of cell proliferation under HRG stress conditions by HER2xHER3 bispecific antibodies composed of the same HER3 Fab arm and different HER2 arms that are directed against the four HER2 domains.

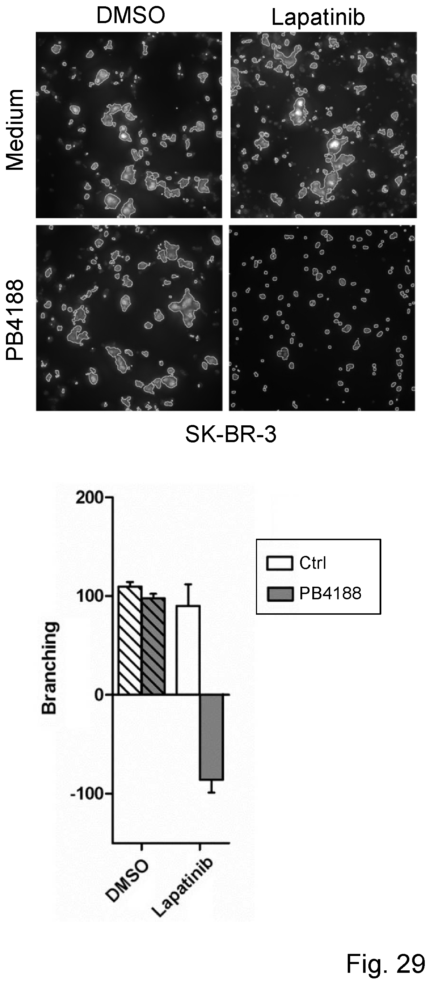

Figure 29: Synergistic combination of PB4188 with lapatinib on the growth and morphology of SKBR-3 cells. Left, microscopical views of cells treated under different conditions; right morphological changes plotted graphically in relation to the treatment conditions

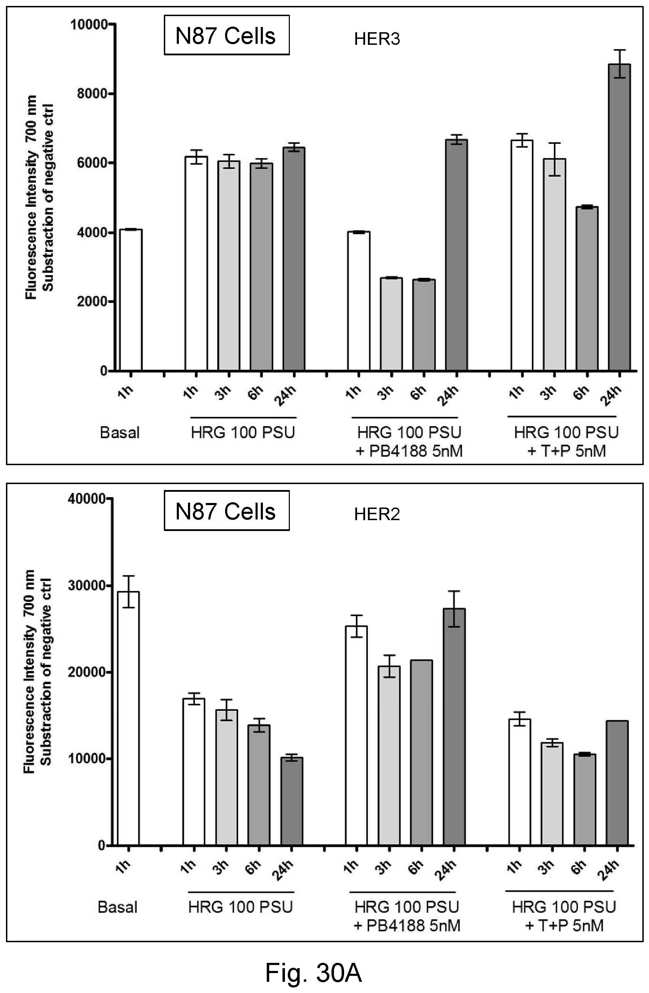

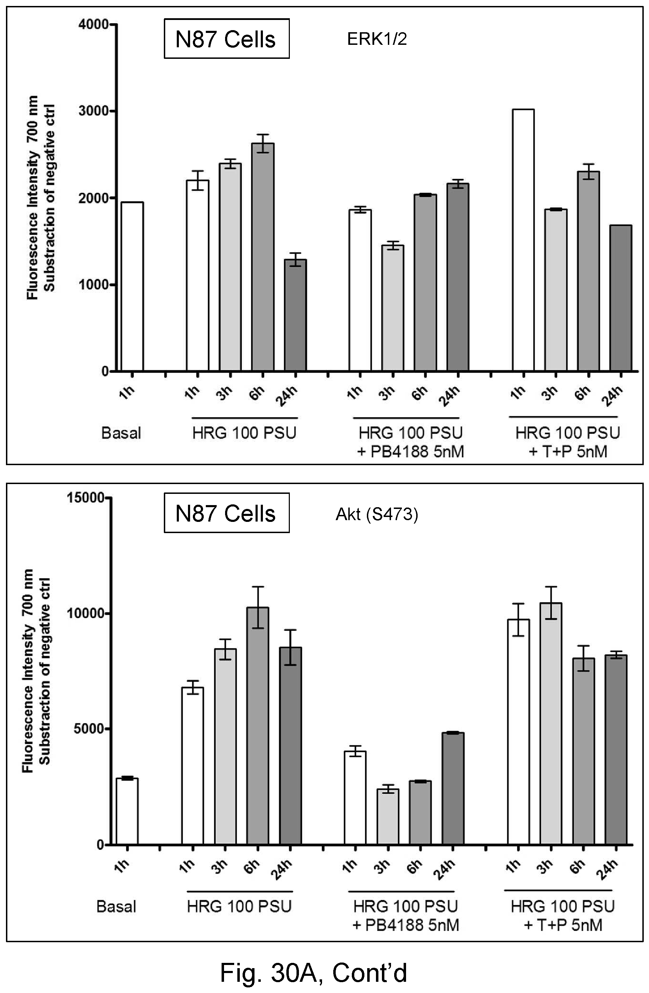

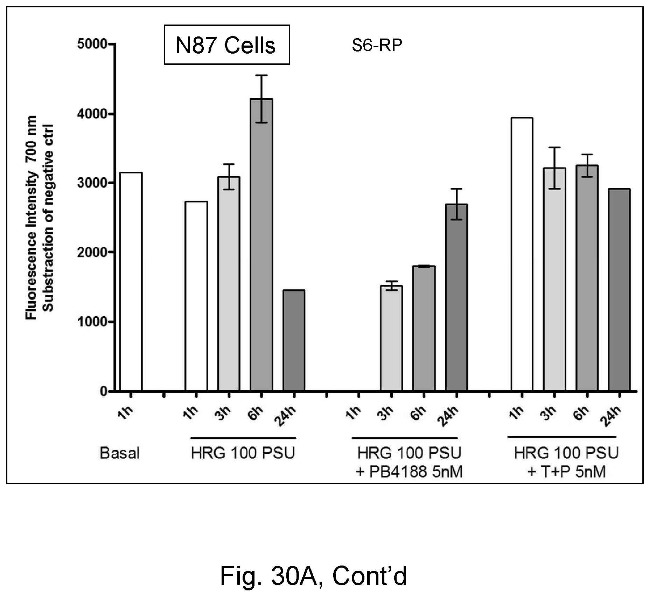

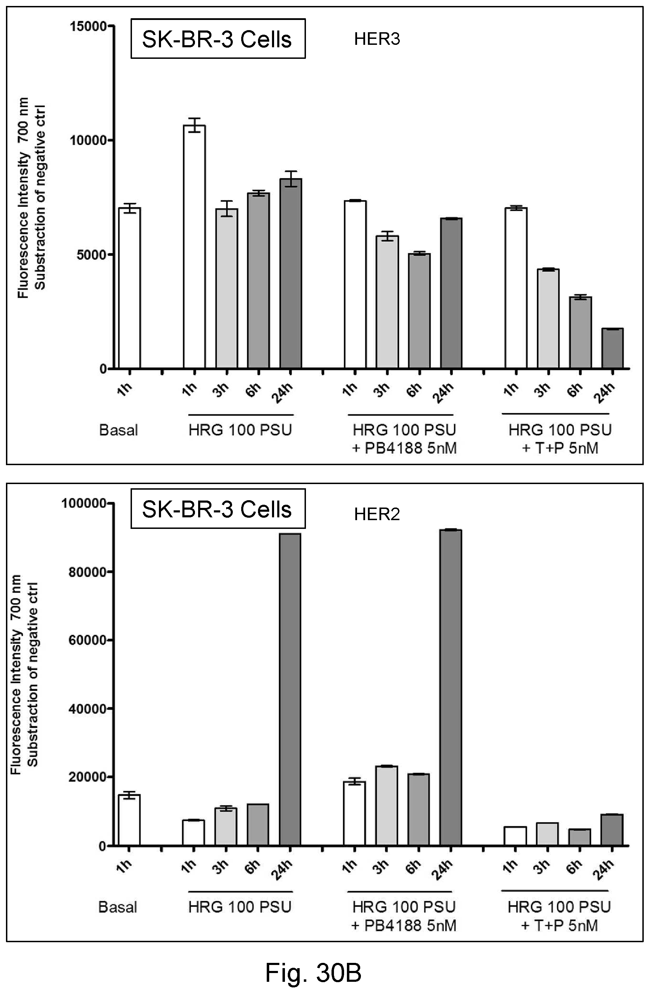

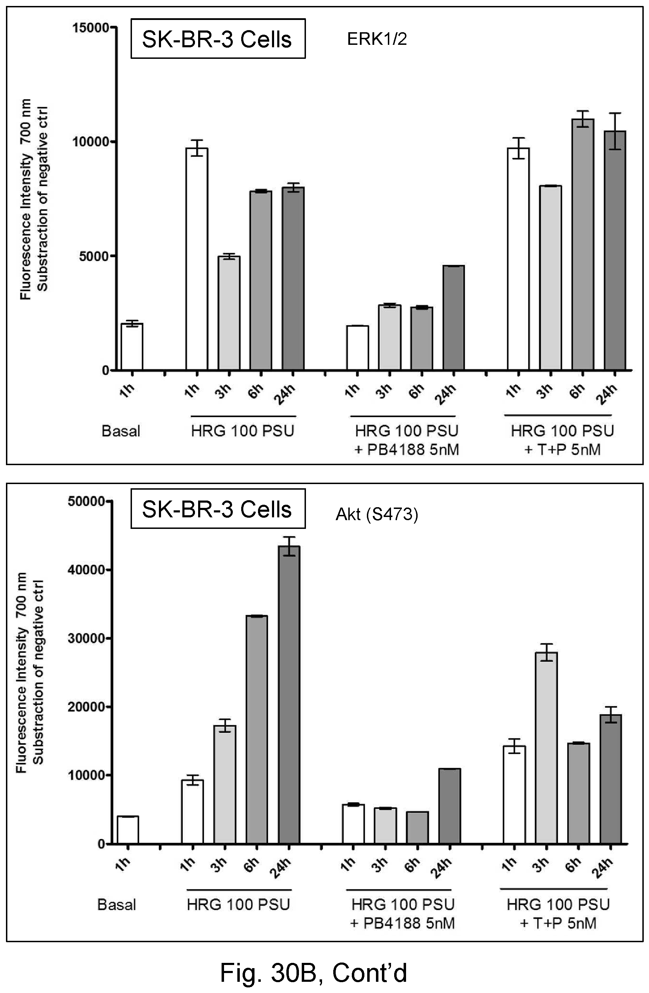

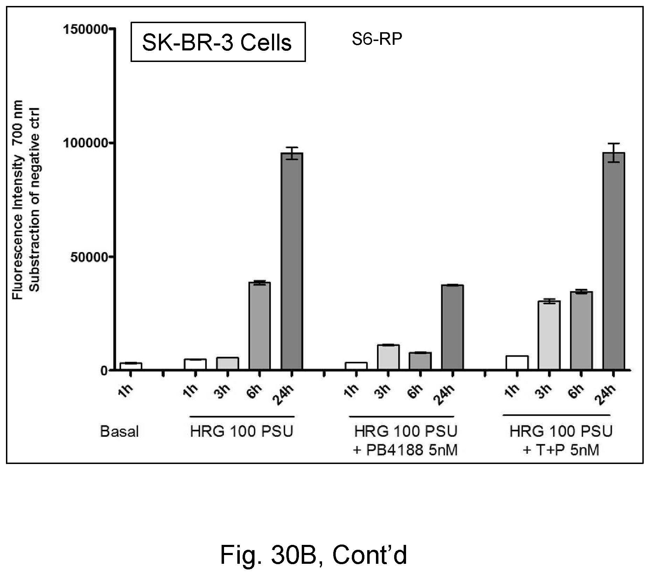

Figure 30A+B: Inhibition of HRG mediated phosphorylation of N87 and SKBR-3 cells by PB4188 in a time course experiment. Trastuzumab + Pertuzumab and HRG alone were included as controls.

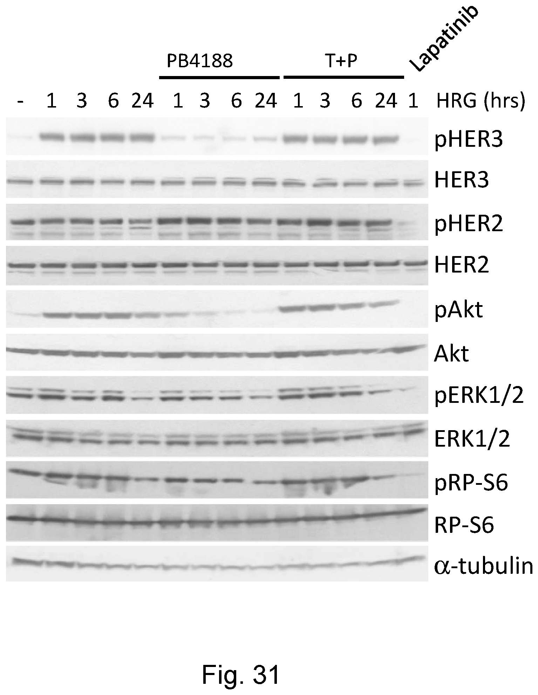

Figure 31: Inhibition of HRG mediated phosphorylation of N87 cells by PB4188 in a time course experiment. Trastuzumab + Pertuzumab and lapatinib were included as controls.

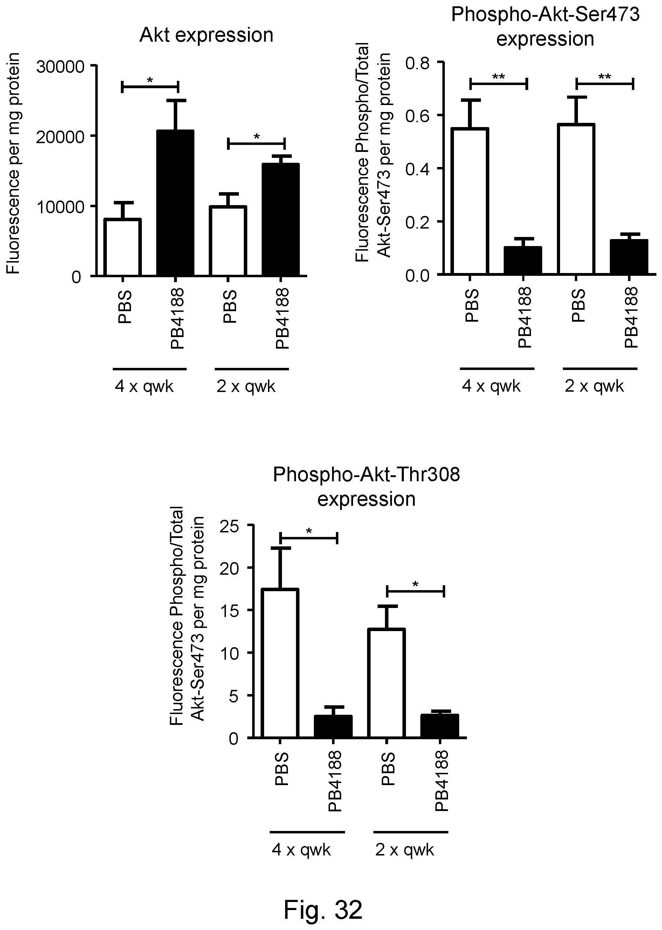

Figure 32: Changes in Akt levels and Akt phosphorylation were assessed 4 H after a two weekly of four weekly dose of PB4188. Phosphorylation levels in tumor lysates were assessed by Luminex assays. Analysis were performed in duplicate and five tumors were analyzed per group.

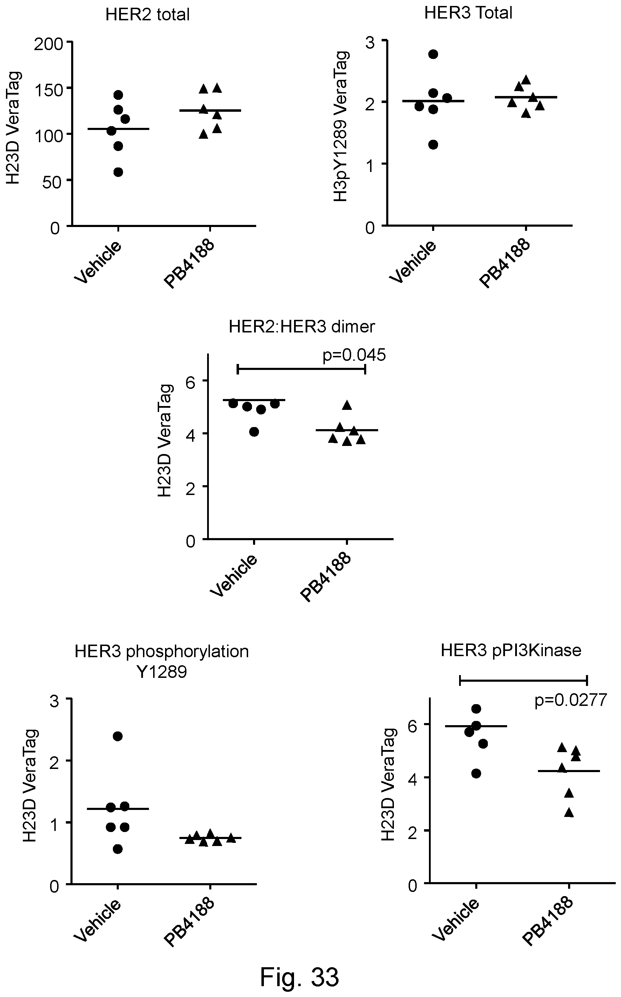

Figure 33: *In vivo* mediated effect of PB4188 on HER2:HER3 mediated signaling as analyzed by Vera Tag analysis on JIMT-1 tumor material. Tumors were analyzed 4H after dosing, tumors derived from PBS treated animals were included as controls.

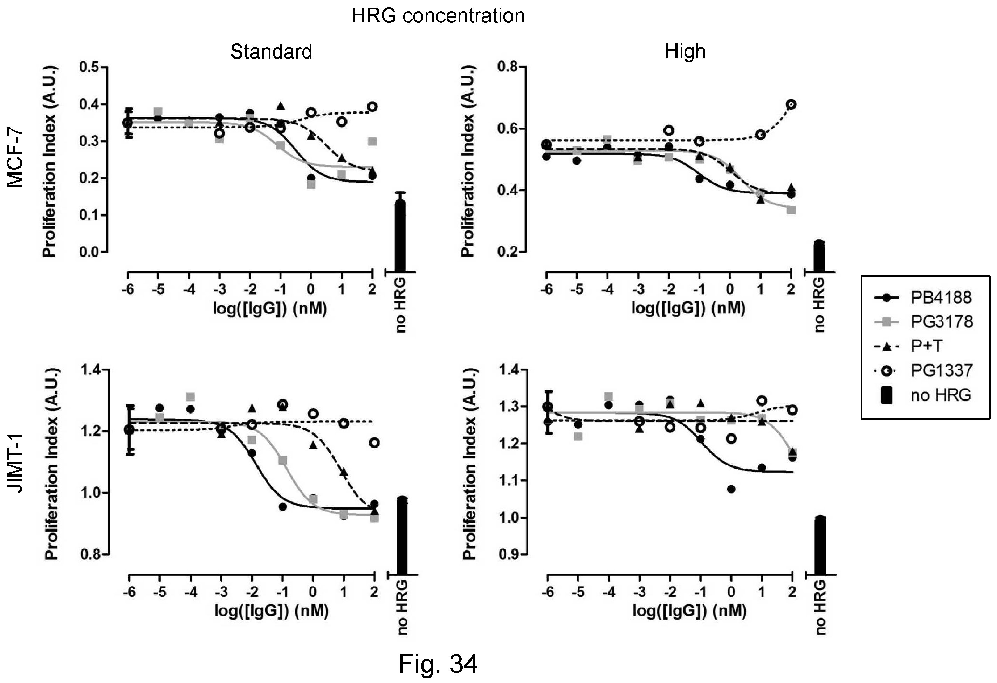

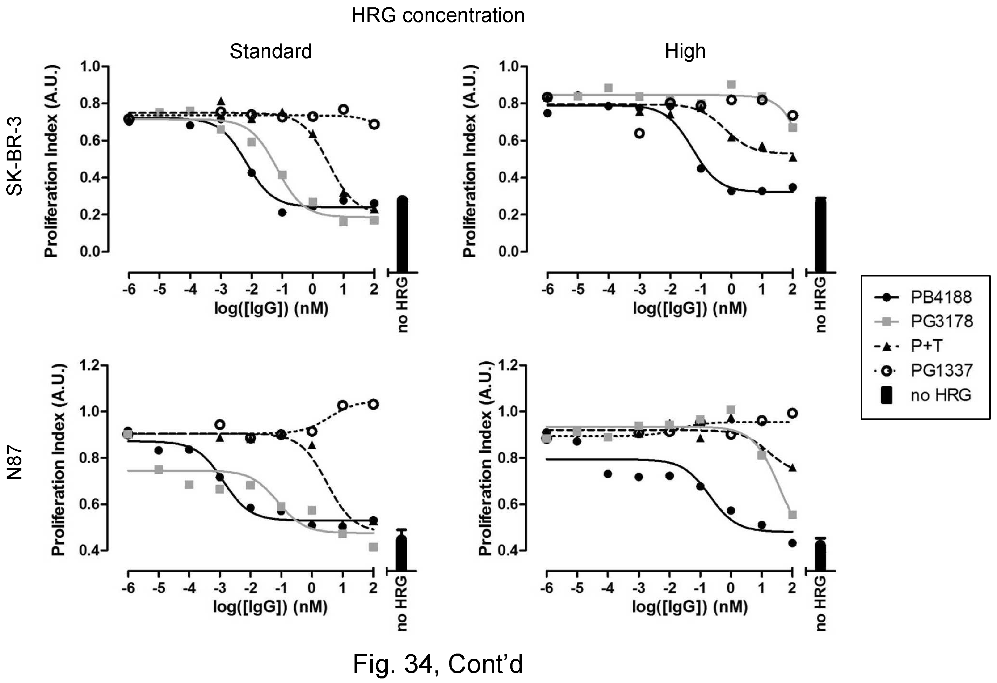

Figure 34: PB4188 reduces cell cycle progression. Cell seeded in assay medium were incubated with titration of antibodies in the presence of a standard (1 ng/ml) or high ( 100 ng/ml) concentration of HRG. 24 hrs later (or 48 hrs for MCF-7 cells), cells were analyzed for their distribution in the different phases of the cell cycle (G0/G1, S or G2/M phases). Proliferation index was calculated as the ratio between the percentage of cells in the S and G2/M phases and the percentage of cells in the GO/G1 phase. P+T, pertuzumab + trastruzumab.

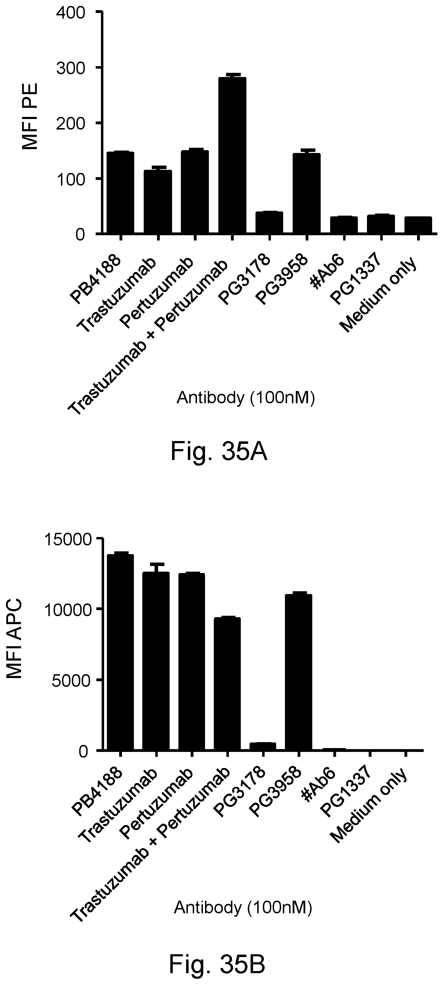

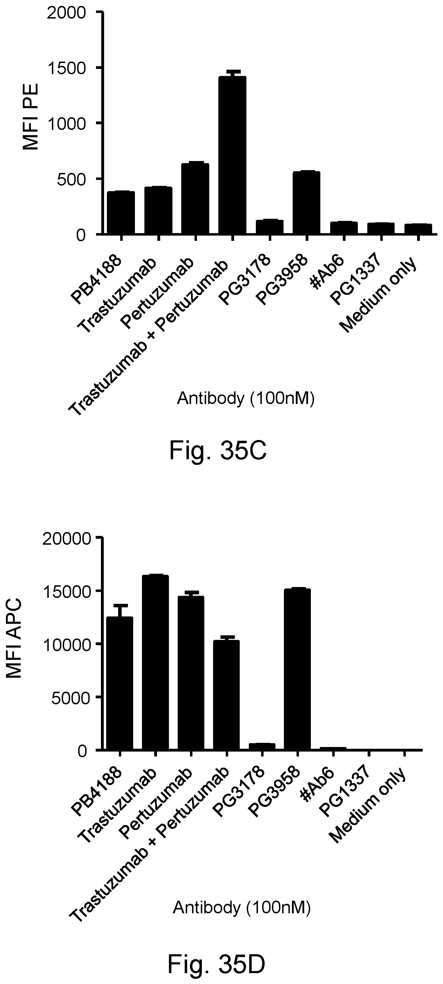

Figure 35: Internalization of antibodies labelled with pH-sensitive dye in HER2-overexpressing cancer cells. N87 (A, B) and SKBR-3 (C, D) seeded in assay medium supplemented with 1 ng/ml HRG were incubated for 24 hrs with 100 nM pH-sensitive dye-labelled antibodies. After harvesting, cells were stained with APC-labelled anti-human IgG secondary antibody to detect cell surface-bound antibodies. Cells were analyzed by FACS for fluorescence in the PE (A, C) to determine internalization and APC (B, D) channels to determine surface binding of the antibodies.

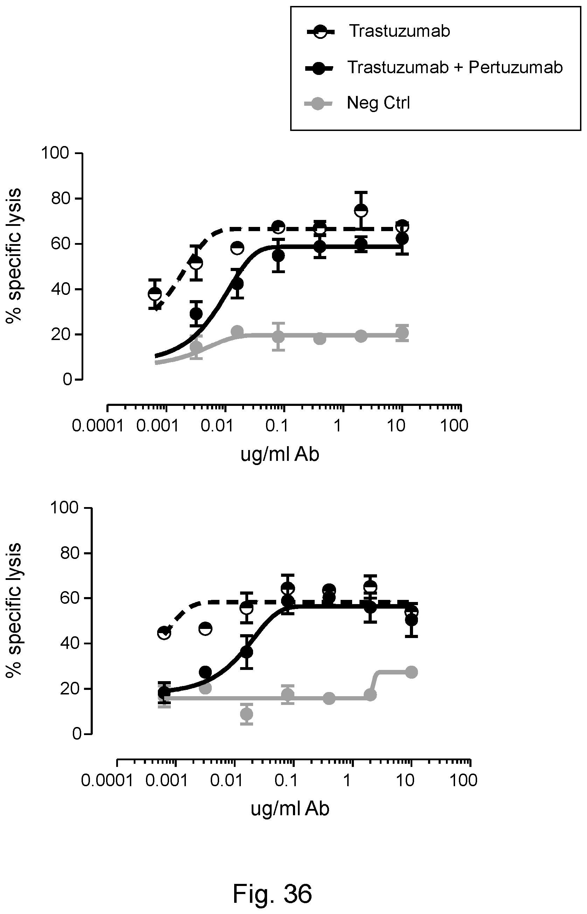

Figure 36: ADCC activity of Trastuzumab versus Trastuzumab + Pertuzumab with cells derived from two different donors.
Figure 37: Amino acid and nucleotide alignments of the F3178 variants. CDR regions are indicated.
Figure 38: Titration curves of HER3 monoclonal antibodies in the HRG dependent N87 assay. PG6058, PG6061 and PG6065 are variants of PG3178. PG1337 is a negative control specific for tetanus toxoid. Data were normalized to basal proliferation with ligand present on each plate.

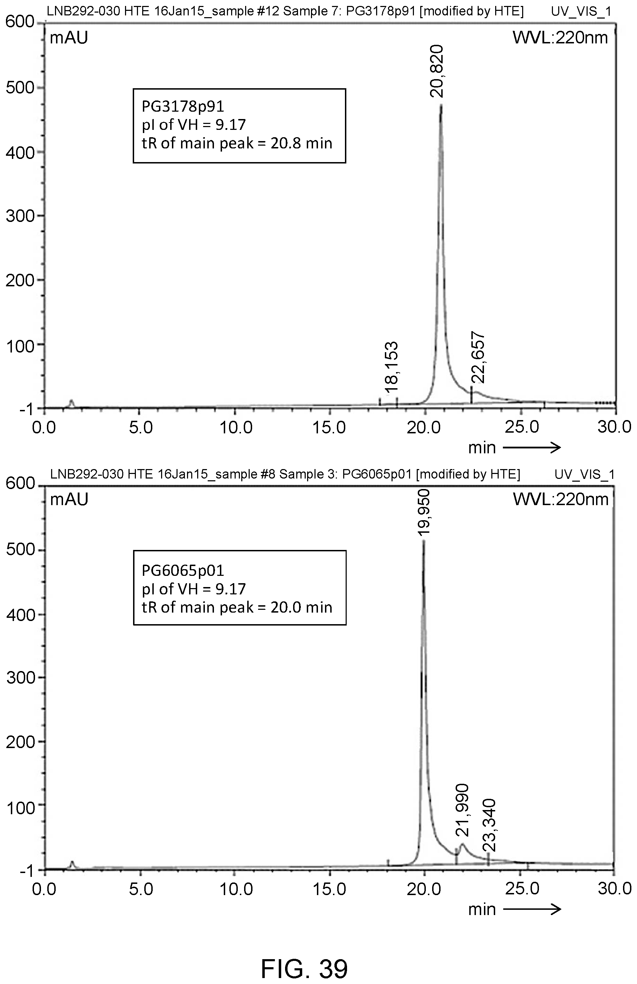

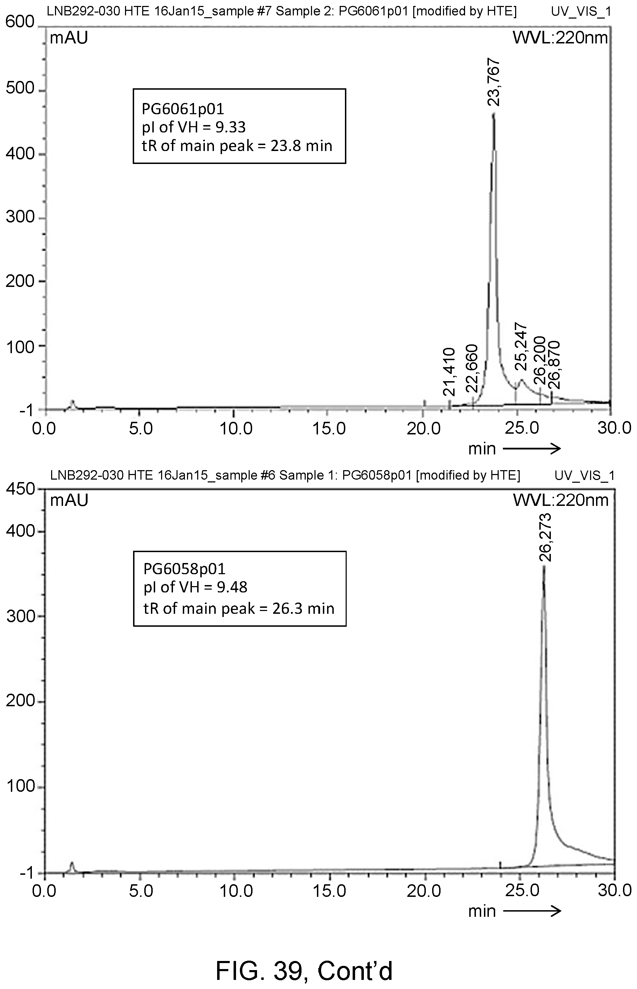

Figure 39: CIEX-HPLC profiles of HER3 monoclonal antibodies. PG6058, PG6061 and PG6065 are variants of PG3178. The calculated iso-electric point (pI) of the VH region and the retention time (tR) of the main peak are given for each antibody.

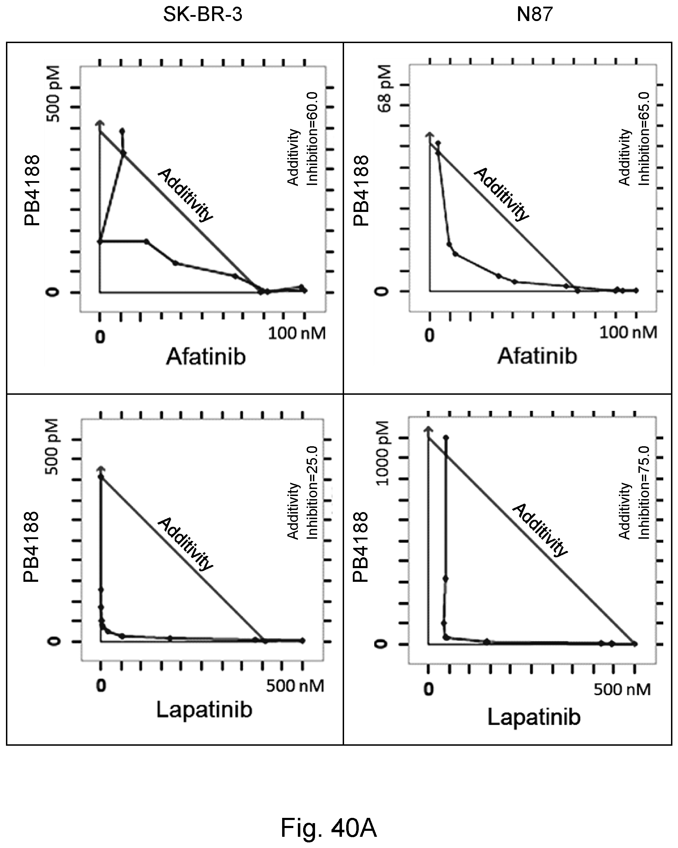

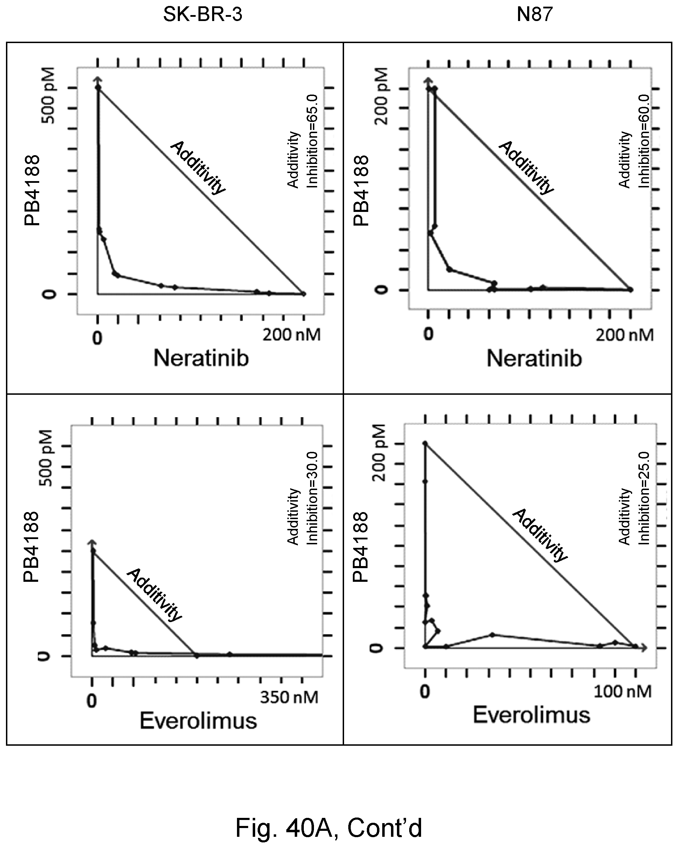

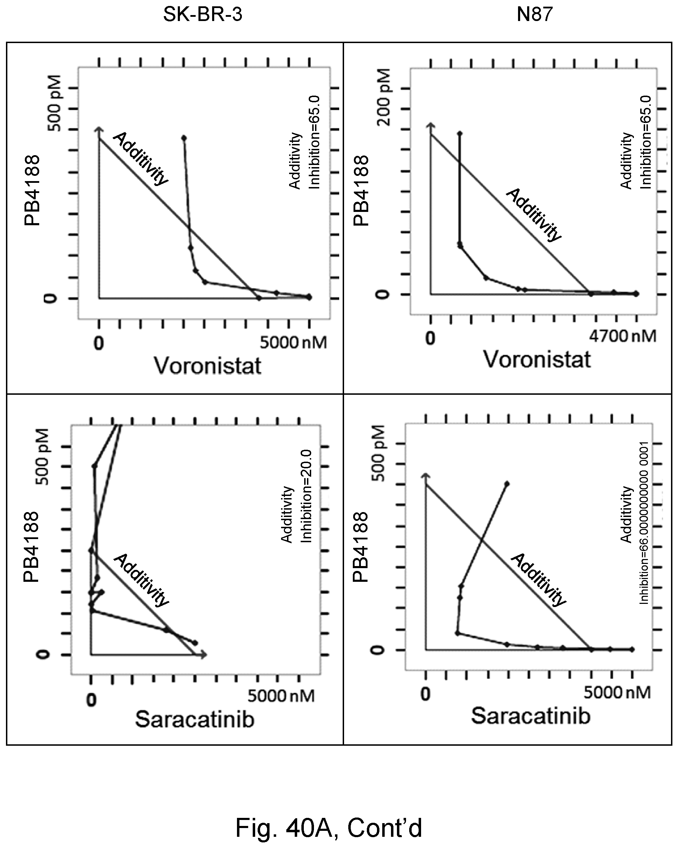

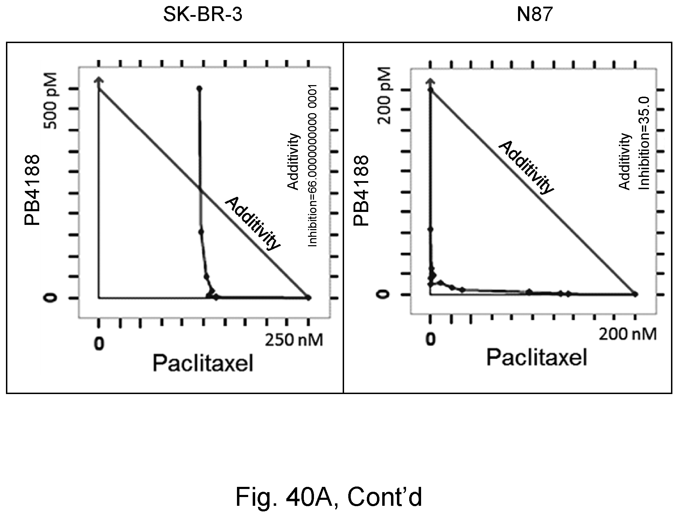

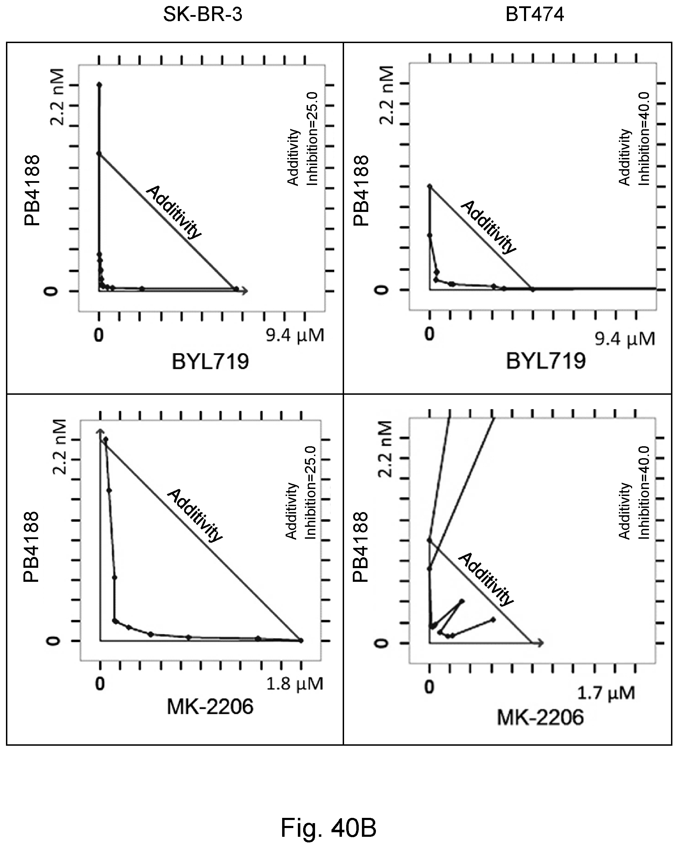

Figure 40: In *vitro* drug combination isobolograms with PB4188 on HER2 amplified cell lines at HRG stress concentrations (A) or grown in matrigel (B).

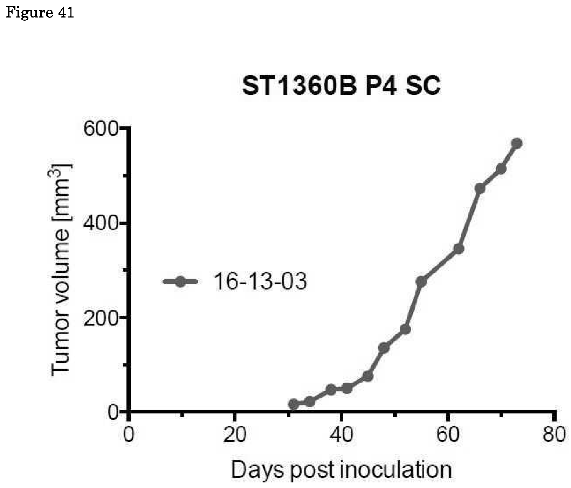

Figure 41: Tumor growth curve for subcutaneous tumor (4th passage) used for intracranial implantation.

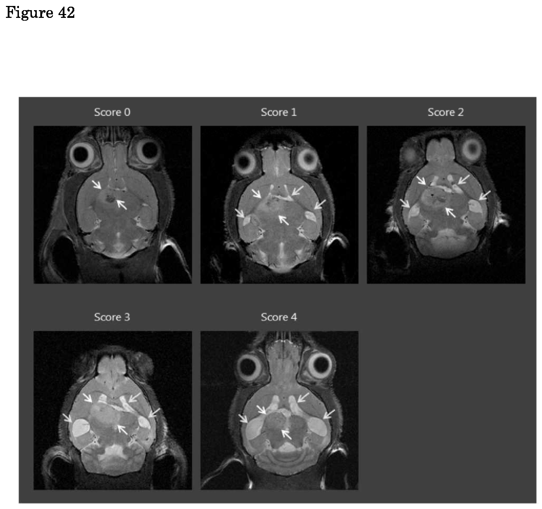

Figure 42 Brain edema scoring system. Representative T2-weighted MR images of examples of the brain edema scores from 0 to 4. White arrows indicate tumor areas and yellow arrows point at edema.

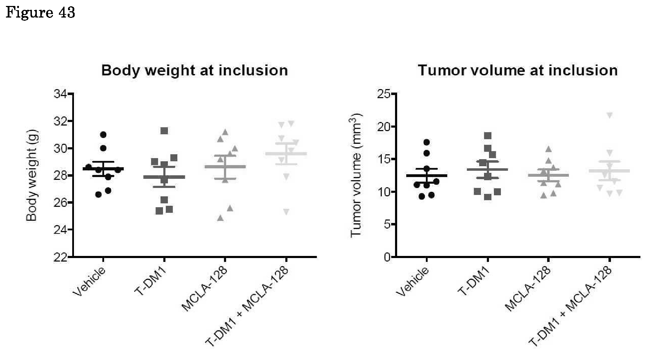

Figure 43: Body weight and tumor volume at inclusion of mice in group AD. No difference in weight or tumor volume was seen between the groups (one-way ANOVA, p = 0.43 (body weight) and p = 0.92 (tumor volume).

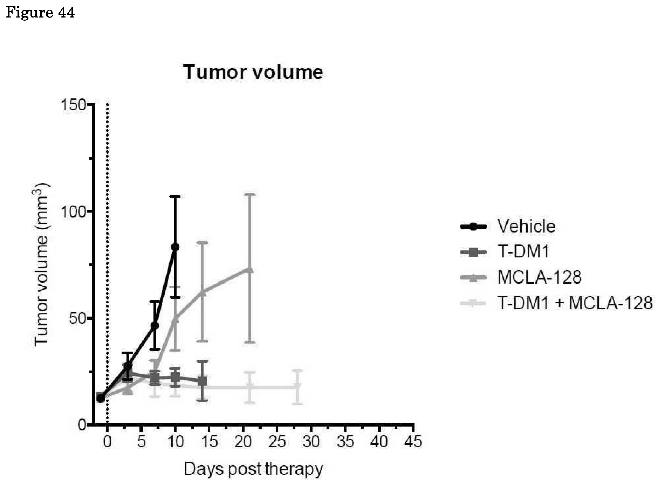

Figure 44: Tumor volume post initiation of therapy for the four treatment groups. Graphed is mean ± SEM, N=8-6/group. For each group, tumor volume is plotted until the time where at least 6 animals were alive.

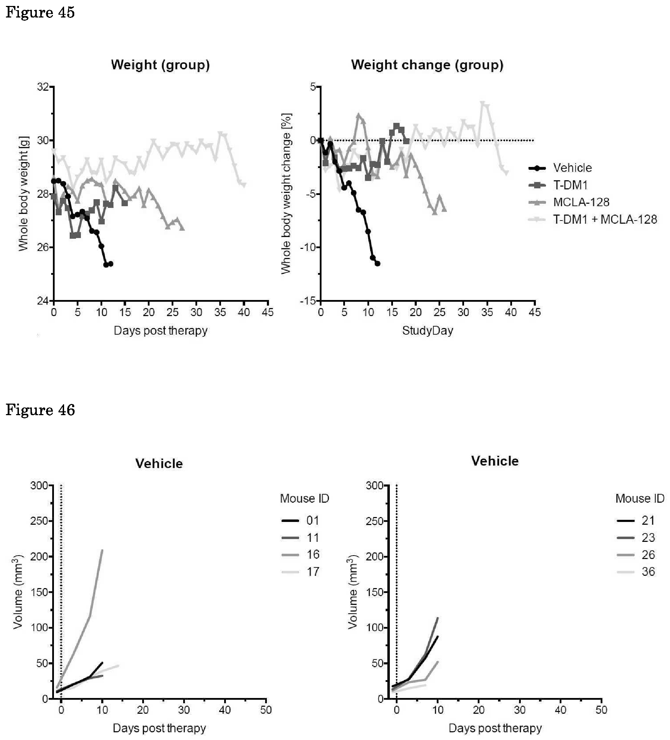

Figure 45: Body weight post initiation of therapy for the four treatment groups expressed in grams (left) and as percentage change from inclusion weight (right). Only the mean is mean graphed, N=8-6/group. For each group, body weight is plotted until the time where at least 6 animals were alive.
Figure 46: Individual tumor volume for mice treated with vehicle measured by T2-weighted MRI.

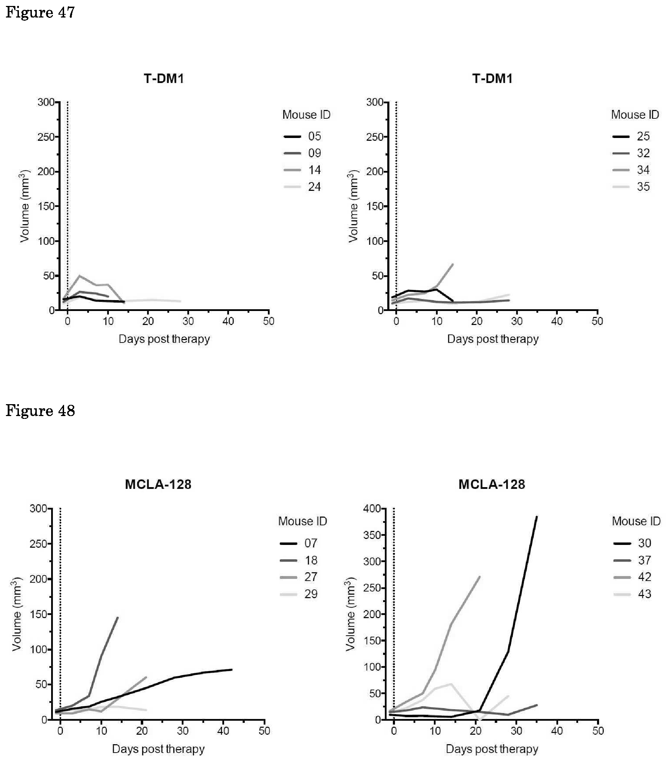

Figure 47: Individual tumor volume for mice treated with T-DM1 measured by T2-weighted MRI.
Figure 48: Individual tumor volume for mice treated with MCLA-128 measured by T2-weighted MRI.

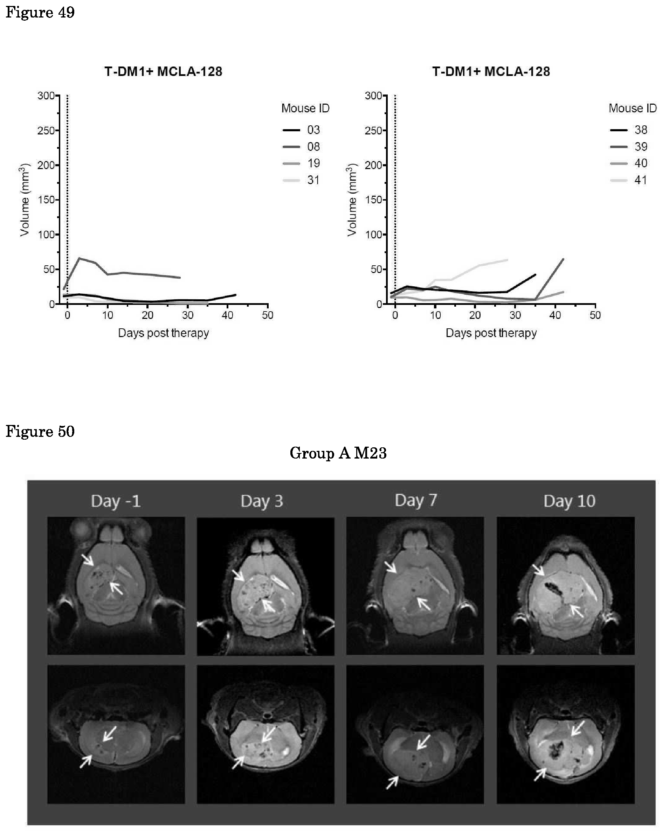

Figure 49: Individual tumor volume for mice treated with T-DM1 + MCLA-128 measured by T2-weighted MRI.
Figure 50: Representative T2-weighted MR images of one mouse from group A (M23). The images show coronal (top) and axial (bottom) slices.

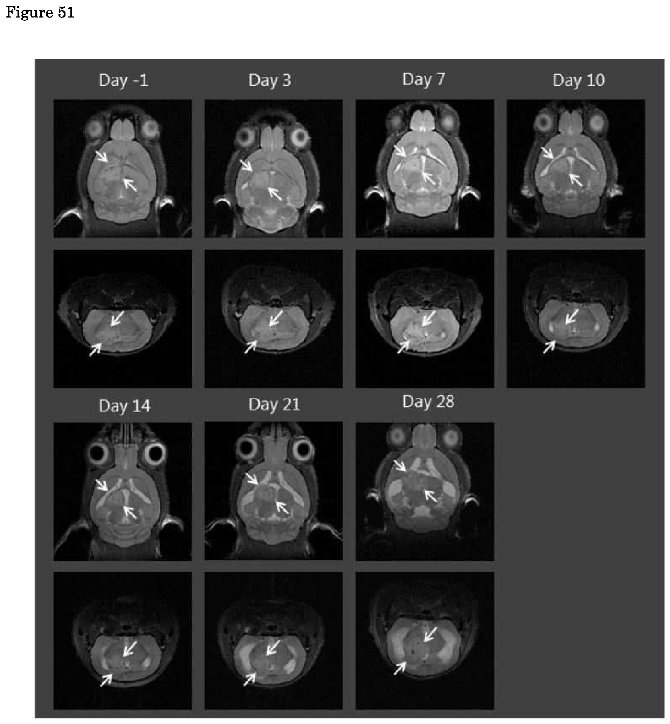

Figure 51: Representative T2-weighted MR images of one mouse from group B (M35). The images show coronal (top) and axial (bottom) slices.

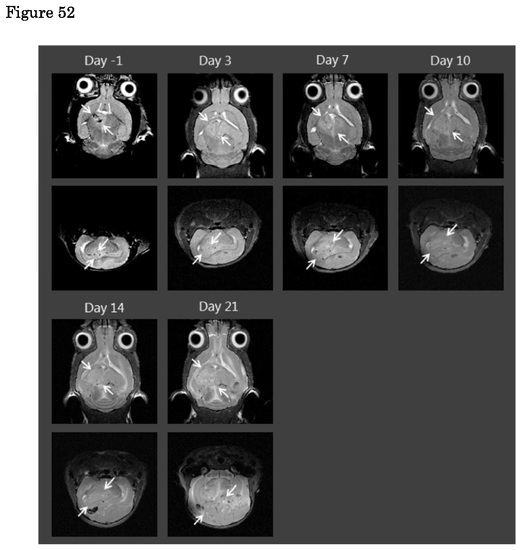

Figure 52: Representative T2-weighted MR images of one mouse from group C (M42). The images show coronal (top) and axial (bottom) slices.

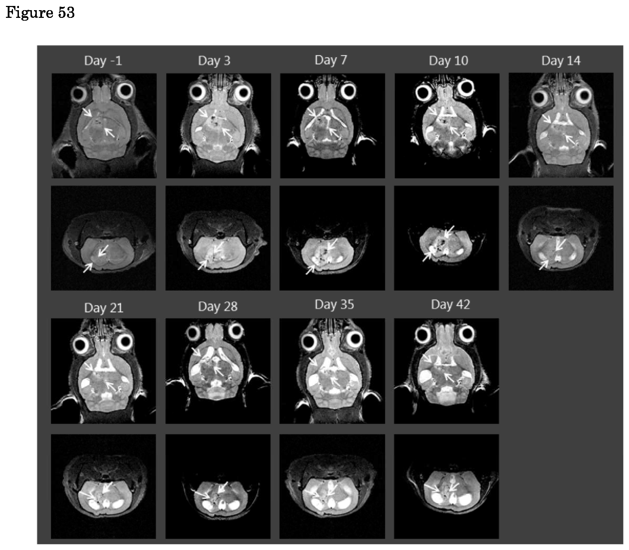

Figure 53: Representative T2-weighted MR images of one mouse from group D (M03). The images show coronal (top) and axial (bottom) slices.

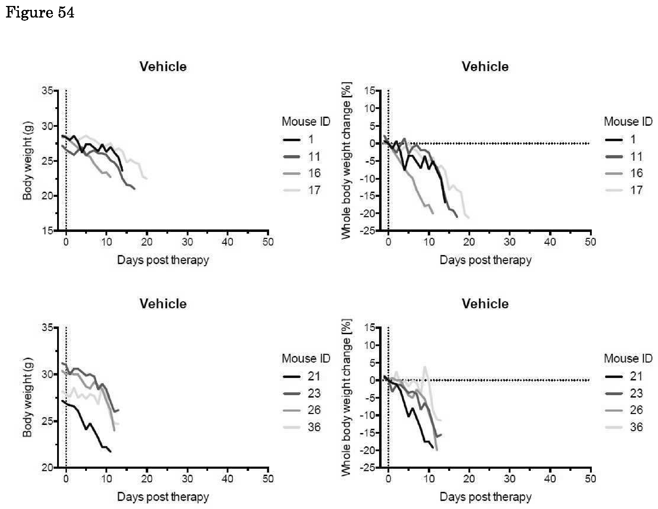

Figure 54: Individual weight measurements for mice treated with vehicle expressed in grams (left) and as percentage change from inclusion weight (right).

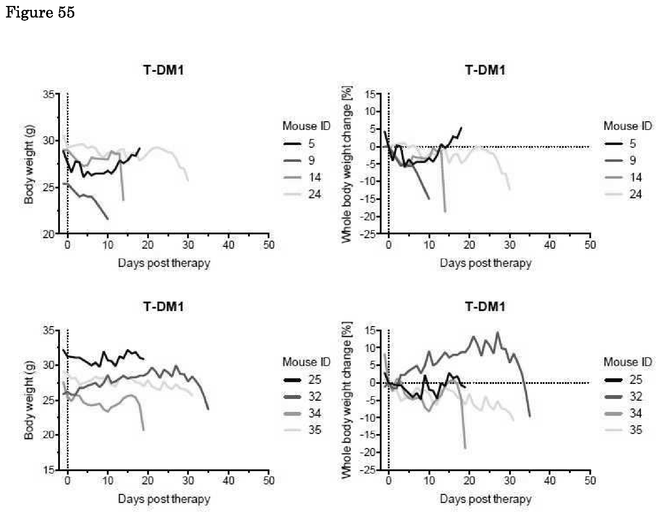

Figure 55: Individual weight measurements for mice treated with T-DM1expressed in grams (left) and as percentage change from inclusion weight (right).

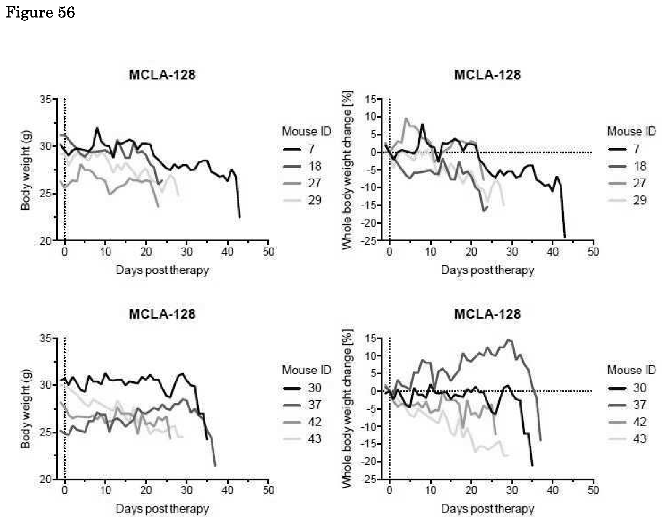

Figure 56: Individual weight measurements for mice treated with MCLA-128 expressed in grams (left) and as percentage change from inclusion weight (right).

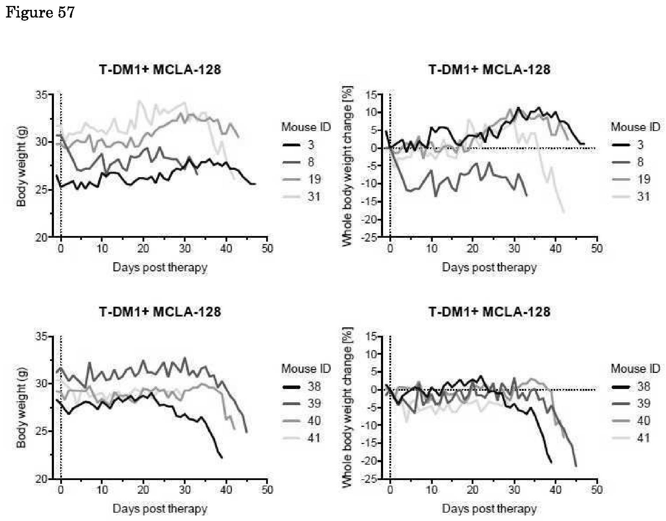

Figure 57: Individual weight measurements for mice treated with T-DM1+ MCLA-128 expressed in grams (left) and as percentage change from inclusion weight (right).

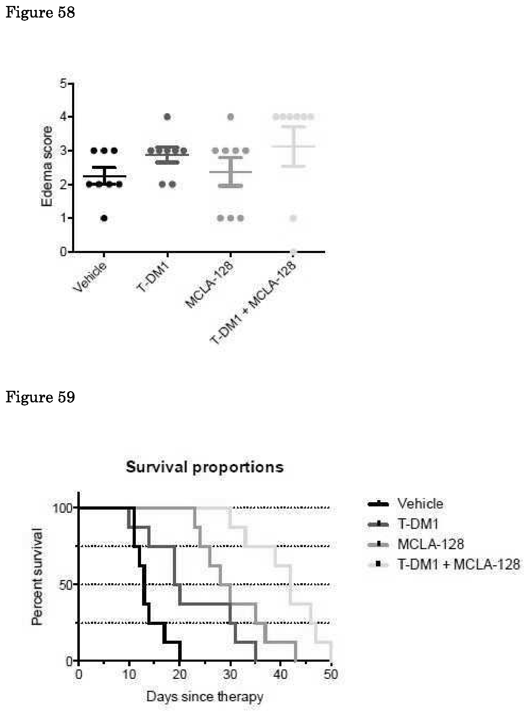

Figure 58: Scoring of edema in the brain on the last MR scan for each mouse. The tumor volume at the time of scoring is different between the groups, and tumor volume may influence the level of brain edema. The average tumor volume of T-DM1 and T-DM1 + MCLA-128 treated animals was smaller compared to vehicle or MCLA-128 treated animals. As such, the results should be interpreted with care.
Figure 59: Kaplan-Meier plot of survival data from all groups. The median survival was 13, 19.5, 29, and 42 days for animals treated with vehicle, TDM1, MCLA-128, and T-DM1 + MCLA-128, respectively. The survival curves were significant different (p<0.0001, Log-rank).

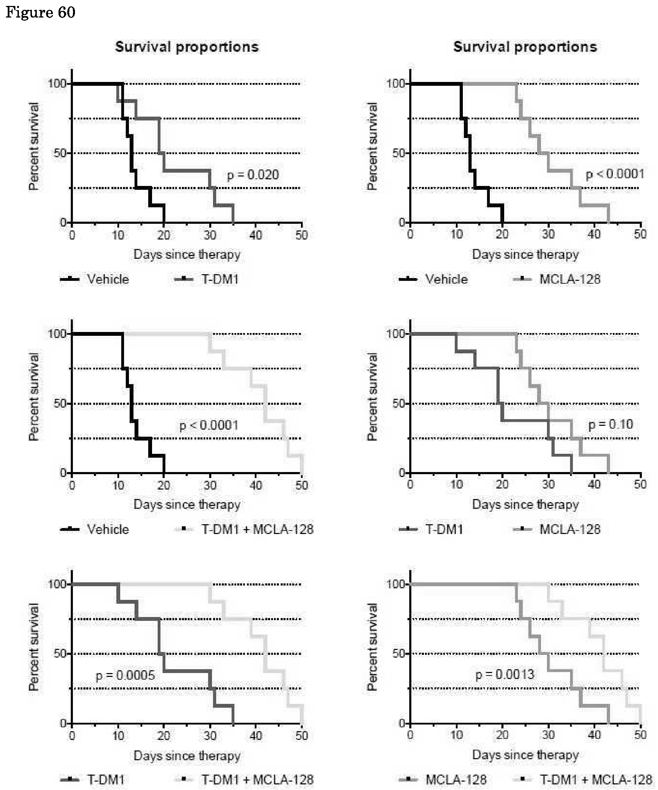

Figure 60: Pair-wise Kaplan-Meier plots. A significant longer median survival was observed for mice treated with T-DM1 (19.5 days), MCLA-128 (29 days), and T-DM1 + MCLA-128 (42 days) compared to vehicle treated mice (13 days). No difference in median survival was seen between T-DM1 and MCLA-128 treated mice. Mice treated with T-DM1 +MCLA-128 has a significant longer median survival compared to mice treated with T-DM1 or MCLA-128 alone.

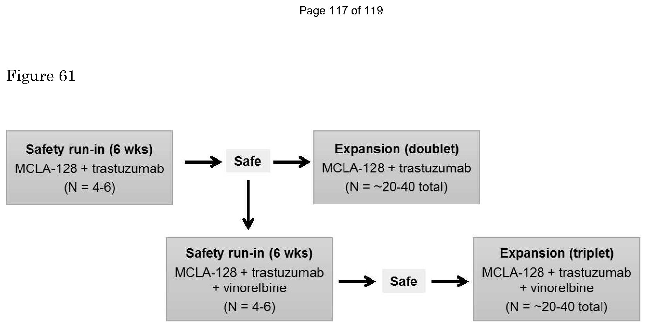

Figure 61: Study design for a combination therapy (doublet and triplet) clinical trial.

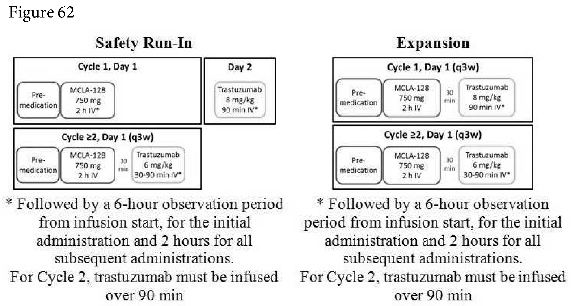

Figure 62: Doublet treatment administration in a combination therapy clinical trial.

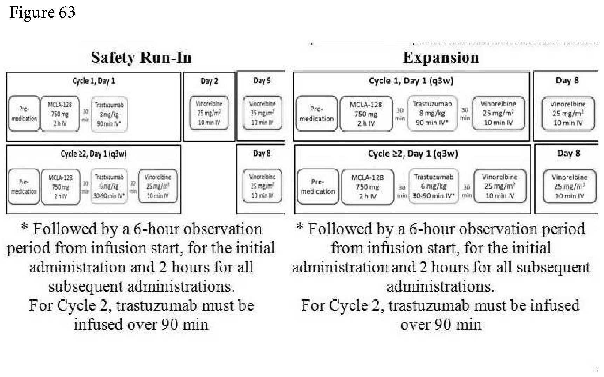

Figure 63: Triplet treatment administration in combination therapy clinical trial.

### EXAMPLES

### Example 1

### Methods, Materials and Screening for Antibodies

### Cell Lines:

BxPC-3-luc2 (Perkin Elmer 125058), N87 (ATCC^{®} CRL-5822^{™}), SK-BR-3 (ATCC^{®} HTB-30^{™}), BT-474 (ATCC^{®} HTB-20^{™}), JIMT-1 (DSMZ ACC 589), L929 (Sigma Aldrich 85011425), K562 (DSMZ ACC10), HEK293T (ATCC^{®} -CRL-11268^{™}), CHO-K1(DSMZ ACC110 ), MCF-7 (DSMZ ACC 115), MDA-MB-468 ( #300279-513, Cell line services) SK-OV-3 (ATCC ^{®} HTB-77^{™}), MDA-MB-175 (ATCC-HTB-25), MDA-MB-453 (ATCC-HTB-131), MDA-MB-361(ATCC-HTB-27), ZR-75-1 (ATCC-CRL-1500) and MKN-45 (DSMZ ACC409) cell lines were purchased from ATCC, DSMZ or Sigma Aldrich and routinely maintained in growth media supplemented with 10% heat inactivated fetal bovine serum (FBS). HEK293F Freestyle cells were obtained from Invitrogen and routinely maintained in 293 FreeStyle medium.

### Generation of Recombinant Human, Chicken, rat and swapped domain vectors (cloning of HER)

*Human HER2.* Full length Human HER2 was amplified by PCR from cDNA derived from RNA isolated from the breast cancer cell line JIMT-1. The primers used for the amplification of human HER2 were as follows. Forward primer: AAGCTGGCTAGCACCATGGAGCTGGCGGCCTTGTGC Reversed primer: AATAATTCTAGACTGGCACGTCCAGACCCAGG. The full-length amplified product was digested with NheI and XbaI and subsequently cloned in the corresponding sites of pcDNA3.1 (Invitrogen).

The sequence was verified by comparison with the NCBI Reference Sequence NM_004448.2. To generate constructs solely expressing the human HER2 extracellular domain (ECD) for transfection and immunization purposes the HER2 transmembrane domain and ECD were PCR amplified and recloned in pVax1. For transfection purposes another construct was generated in pDisplay by amplifying the HER2 ECD domain, in this construct the HER2 ECD domain is fused to the PDGFR transmembrane domain.

*Human HER3*. The full length human cDNA clone of HER3 was obtained from Origene. To generate constructs solely expressing the human HER3 ECD for transfection and immunization purposes the HER3 transmembrane domain and ECD were PCR amplified and recloned in pVax1. In addition another construct was generated in pVax1 whereby the HER3 ECD domain was fused to the PDGFR transmembrane domain. All sequences were verified by comparison with the NCBI Reference NM_001982.3

*Cynomolgus HER2* extracellular domain was PCR amplified from cynomolgus cDNA - Monkey) Normal Colon Tissue (Biochain). The primers used for the amplification of cynomolgus HER2 were as follows:
Forward primer: AAGCTGGCTAGCACCATGGAGCTGGCGGCCTGGTAC Reversed primer: AATAATTCTAGACTGGCACGTCCAGACCCAGG The full -length amplified product was digested with NheI-XbaI and subsequently cloned in the corresponding sites of pcDNA3.1. The clone was sequenced and aligned with sequences available of rhesus monkeys (XM_002800451) to check correctness of the ErbB-2 clone.

*Cynomolgus HER3* extracellular domain was PCR amplified from cynomolgus cDNA - Monkey) Normal Colon Tissue (Biochain). The primers used for the amplification of cynomolgus HER3 were as follows:
Forward primer: AAGCTGGCTAGCACCATGAGGGCGAACGGCGCTCTG, Reversed primer: AATAATTCTAGATTACGTTCTCTGGGCATTAGC The full -length amplified product was digested with NheI-XbaI and subsequently cloned in the corresponding sites of pcDNA3.1. The clone was sequenced and aligned with sequences available of rhesus monkeys (ENSMMUP00000027321) to check correctness of the HER3 clone.

The *chicken HER2* sequence was based on the reference sequence
NM_001044661.1. Chimeric swapped domain constructs were generated by swapping domains I until IV of the chicken HER2 sequence for the human I domains I until IV. Sequences containing a myc tag were optimized for expression in mammalian cells and synthesized at Geneart.

The *rat HER3* sequence was based on the reference sequence NM_001044661.1. Chimeric swapped domain constructs were generated by swapping domains I until IV of the rat HER3 sequence for the human I domains I until IV. Sequences containing a myc tag were optimized for expression in mammalian cells and synthesized at Geneart.

### Generation of HER2 and HER3 over-expressing cell lines

To generate cell lines that express high levels of HER3 on the cell surface a mammalian expression vector was generated by excising the full length HER3 by a *NotI* and *Kpn*I digestion. Subsequently the fragment was cloned in the corresponding sites of the pcDNA3.1(-)/hygro vector. A full length HER2 and HER3 expression vector encoding a neomycin resistance gene was used to generate cell lines that express high levels of HER2 on the cell surface. Prior to transfection the plasmids were linearized by a SSpI and FspI digestion. Both vectors were transfected separately into K562 cells and stable pools were generated following antibiotic selection. The resultant cell lines (K562-HER2 and K562-HER3) expressed high levels of HER2 and HER3 on their cell surface.

### Immunizations

*HER2 immunizations*. Four different immunization strategies were applied. For cohort #A, six C57Bl/6 mice were immunized with 2 x10⁶ L929 cells transiently transfected with HER2 in 200 µl via intraperitoneal injection. Subsequently, mice were boosted with 20 µg Erbb-2-Fc (RND systems) protein dissolved in 125 µl Titermax Gold via intraperitoneal injection on day 14, followed by boosts with 2 x10⁶ L929 cells transiently transfected with HER2 in 200 µl on days 28 and 42. For cohort #C, six C57Bl/6 mice were immunized with 2 x10⁶ L929 cells transiently transfected with HER2 via intraperitoneal injection. Subsequently, mice were boosted with 2 x10⁶ L929 cells transiently transfected with HER2 in 200 µl via intraperitoneal injection on day 14, followed by a protein boosts with 20 µg Erbb-2-Fc protein dissolved in 125 µl Titermax Gold via intraperitoneal injection on day 35 and a final boost with 20 µg Erbb-2-Fc protein dissolved in 200 µl PBS via intraperitoneal injection on day 49. For cohort #E, six C57Bl/6 mice were immunized with 20 µg Erbb-2-Fc protein dissolved in 125 µl Titermax Gold via intraperitoneal injection. Subsequently, protein boosts with 20 µg Erbb-2-Fc protein dissolved in 125 µl Titermax Gold via intraperitoneal injection were made at day 14 and 28 and a final boost with 20 µg Erbb-2-Fc protein dissolved in 200 µl PBS via intraperitoneal injection on day 42. For cohort #G, six C57Bl/6 mice were immunized by DNA vaccination at Genovac (Freiburg, Germany) according to their protocols. The endotoxin-free provided vectors used for the DNA vaccination encoded the transmembrane and extracellular part of HER2 cloned in pVax1. Subsequently, DNA boosts were given at day 14, 28 and 66.

*HER3 immunizations*. Four different immunization strategies were applied. For cohort #B, six (C57Bl/6) mice were immunized with 2 x10⁶ L929 cells transiently transfected with HER3 in 200 µl via intraperitoneal injection. Subsequently, mice were boosted with 2 x10⁶ L929 cells transiently transfected with HER3 in 200 µl on days 14, 28, 49 and 63. For cohort #D, six C57Bl/6 mice were immunized with 2 x10⁶ L929 cells transiently transfected with HER3 via intraperitoneal injection on day 0, 14 and 28. Subsequently, mice were boosted with 20 µg Erbb-3-Fc protein dissolved in 125 µl Titermax Gold via intraperitoneal injection on day 49 and a final boost with 20 µg Erbb-3-Fc protein dissolved in 200 µl PBS via intraperitoneal injection on day 66. For cohort #F, six C57Bl/6 mice were immunized with 20 µg Erbb-3-Fc protein dissolved in 125 µl Titermax Gold via intraperitoneal injection. Subsequently, mice were boosted with 20 µg Erbb-3-Fc protein dissolved in 125 µl Titermax Gold via intraperitoneal injection at day 14 and 28 and a final boost was given with 20 µg Erbb-3-Fc protein dissolved in 200 µl PBS via intraperitoneal injection on day 42. For cohort #H, six C57Bl/6 mice were immunized by DNA vaccination at Genovac (Freiburg, Germany) according to their protocols. The endotoxin-free provided vectors used for the DNA vaccination encoded the transmembrane of PDGFR and extracellular part of HER3 cloned in pVax1. Subsequently, DNA boosts were given at day 14, 28 and 66.

### Determination of antibody titers.

Anti-HER2 titers in the serum from immunized C57Bl/6 mice were determined by ELISA against ECD-Erbb-2 protein (Bendermedsystems) and FACS analysis on the HER2 negative K562, the HER2 low expressing cell line MCF-7 and HER2 amplified SKBR-3 and BT-474 cells. Anti-HER3 titers in the serum from immunized C57Bl/6 mice were determined by ELISA against Erbb-3-Fc protein and FACS analysis on the HER3 negative K562, the HER2 low expressing cell line MCF-7 and HER2 amplified SKBR-3 and BT-474 cells.

Serum titers against HER2 and HER3 before sacrificing the animals are described in Table 1 and Table 2 respectively. Animals in all cohorts developed antibody responses against HER2 or HER3.

### Recovery of lymphoid tissue.

Spleen and draining lymph nodes were removed from all mice vaccinated with DNA (cohorts #G and #H). Single cell suspensions were generated from all tissues and subsequently tissues were lysed in Trizol reagent. From cohorts #A until #F spleens were removed from all mice except for one mouse of cohort #C that died after the first boost. Single cell suspensions were generated from all spleens and the total B cell fraction was isolated using the MACS separation procedure either by CD19 enrichment (cohorts # A, E, F) or depletion of non-B cells (cohorts # B, C, D).

### Generation of phage display libraries from immunized mice

One phage library was built for each mouse. To this end the material from all mice per group (5 or 6 mice per group) was used to prepare phage libraries using the following approach. From each individual mouse RNA was isolated and cDNA was synthesized and VH-family specific PCRs were performed. Subsequently all VH-family PCR products per mouse were purified and the DNA concentration was determined and digested and ligated in a phage-display vector containing the common-light chain to generate a mouse-human chimeric phage library. All phage libraries contained > 10⁶ clones with an insert frequency of > 85%.

### Selection of phages carrying Fab fragments specifically binding to HER2 and HER3

Antibody fragments were selected using antibody phage display libraries. Immunized libraries and synthetic libraries (as described in de Kruif et al. Mol. Biol. (1995), 248, 97-105) were used for selections.

### HER2 phage selection and screening

Phage libraries were rescued with VCS-M13 helper phage (Stratagene) and selected for two rounds in immunotubes (Nunc) coated recombinant protein. In the first round ECD-Erbb-2 protein (Bendermedsystems) was coated onto immunotubes whereas in the second round Erbb-2-Fc (RND systems) was coated onto immunotubes. The immunotubes were blocked with 4% non fat dry milk (ELK). Phage antibody libraries were also blocked with 4% ELK prior to the addition of the phage library to the immunotubes. Incubation with the phage library with the coated protein in the immune tubes was performed for 2 H at room temperature under rotating conditions. Immunotubes were then washed five to ten times with 0.05% Tween-20 in PBS followed by 5 to 10 times in PBS. Bound phages were eluted using 50mM glycine (pH 2.2) and added to E. coli XL-1 Blue and incubated at 37°C for phage infection. Subsequently infected bacteria were plated on agar plates containing Ampicillin, tetracyclin and glucose and incubated at 37°C overnight. After the first round, colonies were scraped off the plates and combined and thereafter rescued and amplified to prepare an enriched first round library. The enriched library was then selected on Erbb-2-Fc (RND systems) using the protocol described above. After the second round selection individual clones were picked and rescued to prepare a phage monoclonal miniprep. Positive phage clones binding Erbb2 were then identified in FACS for binding to the breast cancer cell line BT-474. The VH genes of all Erbb2 specific clones were sequenced. VH gene rearrangements were established with VBASE2 software to identify unique clones. All unique clones were then tested in phage format for binding in FACS to HEK293T cells (negative control), HEK293T cells transiently transfected with ErbB-2 and BT-474 cells.

### HER3 phage selection and screening

Phage libraries were rescued with VCS-M13 helper phage (Stratagene) and selected for two rounds in immunotubes (Nunc) coated with recombinant protein. In both selection rounds round Erbb-3-Fc (RND systems) was coated onto immunotubes. To overcome a selection bias towards the Fc part of the fusion protein, both selection rounds on Erbb-3-Fc were performed in the presence of 150 µg/ml human IgG. The immunotubes were blocked with 4% ELK. Phage antibody libraries were blocked with 4% ELK prior to the addition of the phage library to the immunotubes. Incubation with the phage library was performed for 2 H under rotating conditions. Immunotubes were then washed five to ten times with 0.05% Tween-20 in PBS followed by 5 to 10 times in PBS. Bound phages were eluted using 50mM glycine (pH 2.2) and added to E. coli XL-1 Blue and incubated for phage infection. Subsequently infected bacteria were plated on agar plates containing Ampicillin, tetracyclin and glucose and incubated at 37°C overnight. After the first round, colonies were scraped off the plates and combined and phages were rescued and amplified to prepare an enriched first round library. The enriched library was then selected on Erbb-3-Fc (RND systems) using the protocol described above. After the second round selection individual clones were picked and rescued to prepare a phage monoclonal miniprep. Positive phage clones were identified in FACS for binding to the breast cancer cell line BT-474. The VH genes of all positive clones were sequenced. VH gene rearrangements were established with VBASE2 software to identify unique clones. All unique clones were tested in phage format for binding in FACS to K562 cells (negative control), stable K562-HER3 cells and BT-474 cells.

In total 36 selections were performed on Erbb2 and Erbb3 antigen formats. All selection screening procedures resulted in 89 unique Fab clones directed against HER2 and 137 unique Fab clones directed against HER3. A Fab was considered unique based on its unique HCDR3 sequence, an indication of a unique VDJ recombination event. In some cases clonal variants were obtained, with an identical HCDR3 but differences in the CDR1 and/or CDR2. From the immunized mice libraries clusters of clonal variants containing substitutions in the VH gene reflecting affinity variants were selected.

### Antibody selection/characterization

### Generation of monoclonal antibodies

VH genes of unique antibodies, as judged by VH gene sequence and some sequence variants thereof, derived from the immunized mouse phage libraries were cloned in the backbone IgG1 vector. Two different production cell lines were used during the process; HEK293T and 293F Freestyle cells. Adherent HEK293T cells were cultivated in 6-well plates to a confluency of 80%. The cells were transiently transfected with the individual DNA-FUGENE mixture and further cultivated. Seven days after transfection, supernatant was harvested and medium was refreshed. Fourteen days after transfection supernatants were combined and filtrated through 0.22 µM (Sartorius). The sterile supernatant was stored at 4°C. Suspension adapted 293F Freestyle cells were cultivated in T125 flasks at a shaker plateau until a density of 3.0 x 10⁶ cells/ml. Cells were seeded at a density of 0.3-0.5 x 10⁶ viable cells/ml in each well of a 24-deep well plate. The cells were transiently transfected with the individual sterile DNA: PEl mixture and further cultivated. Seven days after transfection, supernatant was harvested and filtrated through 0.22 µM (Sartorius). The sterile supernatant was stored at 4°C.

### Generation of bispecific antibodies

Bispecific antibodies were generated using the proprietary CH3 technology to ensure efficient hetero-dimerisation and formation of a bispecific antibody. The CH3 technology uses charge-based point mutations in the CH3 region to allow efficient pairing of two different heavy chain molecules as previously described (PCT/NL2013/050294; published as WO 2013/157954 A1).

### IgG purification for functional screening

The purification of IgG was performed at small scale (< 500 µg), medium scale (<10 mg) and large scale (>10 mg) using affinity chromatography. Small scale purifications were performed under sterile conditions in 24 well filter plates using vacuum filtration. First the pH of the medium was adjusted to pH 8.0 and subsequently the small scale productions were incubated with protein A Sepharose CL-4B beads (50% v/v) (Pierce) for 2 H at 25°C on a shaking platform at 600 rpm (Heidolph plate shaker). Next the beads were harvested by vacuum filtration. Beads were washed twice with PBS pH 7.4. IgG was eluted at pH 3.0 with 0.1 M citrate buffer and the IgG fraction was immediately neutralized by Tris pH 8.0. Buffer exchange was performed by centrifugation using multiscreen Ultracel 10 multiplates (Millipore). The samples ended up in a final buffer of PBS pH 7.4

### Validation of HER2/HER3 specific IgGs

Antibodies were tested for binding in FACS to BT-474, HEK293T and HEK293T overexpressing HER2 or HER3. Therefore cells were harvested using trypsin and diluted to 10⁶ cells/ml in FACS buffer (PBS/0.5%BSA/0.5mM EDTA). 1-2 x10⁵cells were added to each well in a U-bottom 96 well plate. Cells were centrifuged for 2 minutes at 300 g at 4°C. Supernatant was discarded by inverting plate(s). 50 µl of each IgG sample was added at a concentration of 10 µg/ml and incubated for 1H on ice. Cells were centrifuged once, supernatant was removed and cells were washed twice with FACS buffer. 50 µl diluted 1:100 mouse anti human IgG PE (Invitrogen) was added and incubated for 30-60 minutes on ice in the dark. After adding FACS buffer, cells were centrifuged once, supernatant was removed and cells were washed twice with FACS buffer. Cells were analysed on a FACSCanto Flow cytometer in a HTS setting. Binding of the antibodies to cells was assessed by mean fluorescence intensity (MFI).

To test for non-specific binding reactivity ELISA assays were used. HER2 and HER3 antibodies were tested for reactivity against the antigens fibrinogen, hemoglobulin and tetanus toxin. To test specific binding to HER2 and HER3, the antibodies were tested for binding to purified recombinant extracellular domains of EGFR, HER2, HER3 and HER4. Antigens were coated overnight to MAXISORP^{™} ELISA plates. Wells of the ELISA plates were blocked with PBS (pH 7.2) containing 5% BSA for 1 hour at 37°C. Selected antibodies were tested in duplo at a concentration of 10 µg/ml diluted in PBS-2% BSA and allowed to bind for 2 hours at 25°C. As a control the procedure was performed simultaneously with an antibody specific for the coated antigens and a negative control antibody. The ELISA plates were washed 5 times with PBS-T (PBS-0.05% v/v Tween 20). Bound IgG was detected with 1:2000 diluted HRP-conjugate (Goat anti-mouse BD) and was allowed to bind for 2 hours at 25°C. The ELISA plates were washed 5 times with PBS-T (PBS-0.05% Tween 20) and bound IgG was detected by means of OD492nm measurement.

### Epitope grouping of HER2/HER3 specific IgGs

The panel of anti-HER2 antibodies was binned based on their reactivity to the HER2 ECD derived from other species (mouse, chicken) and on their binding to specific domains in the HER2 molecule i.e. domains I, II, III and IV using chimeric constructs.

The panel of anti-HER3 antibodies was binned based on their reactivity to the HER3 ECD derived from other species (cyno, rat) and on their binding to specific domains in the HER3 molecule i.e. domains I, II, III and IV using chimeric constructs.

For this purpose CHO-K1 cells were transiently transfected with the relevant constructs using lipofectamin/DNA mixes. In the chimeric swapped domain construct, domains of chicken HER2 or rat HER3 are replaced by the human counterpart. Binding of the specific antibodies was measured by FACS. Expression of the constructs was confirmed using an anti-myc antibody. FACS staining with trastuzumab was included as a control for specific binding to domain IV. Antibodies in each group could be ranked based on the intensity of staining (MFI). The HER2 panel of 65 antibodies could be mapped into seven bins (Table 3).
1. Domain I specific (25)
2. Domain II specific (2)
3. Domain III specific (23)
4. Domain IV specific (7)
5. Domain IV specific and cross reactive to mouse (2)
6. Reactive to all constructs (2)
7. Only reactive to human HER2 (4)

### Competition with trastuzumab

Two antibodies mapped to HER2 domain IV inhibited proliferation of SKBR-3 cells. Both antibodies shared a similar CDR3 except for one amino acid difference. One antibody, PG1849 was investigated for its capacity to compete with trastuzumab in a competition ELISA. In this ELISA Fc-HER2 was coated and incubated with a concentration of 15 µg/ml IgG antibody. After an incubation of 15 minutes phages were allowed to incubate for another hour. Thereafter, phages were detected. Table 4 demonstrates that PG1849 and trastuzumab could bind simultaneously to HER2 since no loss of signal appeared during the ELISA. True competition only was observed when the same phage and antibody were combined in the assay.

The HER3 panel of 124 antibodies could be mapped into five bins (Table 5):
1. High Domain III reactivity, rat and mouse reactive and minor reactivity to domain IV (8)
2. High Domain III reactivity, rat, human and cyno reactive, minor reactivity to domain IV (8)
3. Only reactivity to rat, cyno and human HER3 (43)
4. Only reactive to human HER3 (32)
5. Reactive to all constructs (33)

### Cell line proliferation assays

SK-BR-3 cells were cultured in DMEM-F/12 supplemented with L-glutamine and 10% heat inactivated FBS. BxPC-3-luc2 cells were cultured in RPMI1640 supplemented with 10% heat inactivated FBS. MCF-7 cells were cultured in RPMI1640 supplemented with 100 µM, NEAA1 mM sodium pyruvate, 4 µg/ml insulin and 10% heat inactivated FBS.

For the proliferation assay of SK-BR-3 cells, subconfluent cell cultures were washed with PBS, trypsinized and trypsin was inactivated by adding culture medium. Cells were diluted to 6x10⁴ cells/ml in culture medium. Antibodies were diluted to concentrations of 10 and 1 µg/ml and added in a volume of 100 µl in 96-well black bottom plates (ABgene AB-0932). Cells were added at density of 6000 cells/well. The cells were cultivated for 3 days at 37°C, 5% CO, in 95% relative humidity. Alamar Blue^{™} (Invitrogen) was added according to the manufacturer's instructions and incubated for 6 hours at 37°C, 5% CO, in 95% relative humidity in the dark. Fluorescence was measured at 550 nm excitation and 590 nm emission wavelength. The extent of growth inhibition was compared to that of the same concentration of trastuzumab (Table 6).

For the proliferation assay of MCF-7 and BxPC-3-luc2 cells, subconfluent cell cultures were washed with PBS, trypsinized and trypsin was inactivated by adding culture medium. Cells were washed twice in large volumes of assay medium (RPMI 1640 medium containing 0.05% BSA and 10 µg/ml Holo Transferrin). MCF-7 cells were diluted to 5x10⁴ cells/ml in culture medium. Antibodies were diluted to concentrations of 10 and 1 µg/ml and added in a volume of 100 µl in 96-well black bottom plates (ABgene AB-0932). Cells were added at a density of 5000 cells/well in the presence of 1 ng/ml final concentration human Recombinant Human NRG1-beta 1/HRG1-beta 1 EGF Domain; (396-HB-050 RND). Human NRG1-beta 1/HRG1-beta 1 EGF Domain will hereinafter be referred to as HRG. The cells were cultivated for 5 days at 37°C, 5% CO, in 95% relative humidity. Alamar Blue^{™} (Invitrogen) was added according to the manufacturer's instructions and incubated for 24 hours at 37°C, 5% CO2, in 95% relative humidity in the dark. Fluorescence was measured at 550 nm excitation with 590 nm emission wave length. The extent of growth inhibition was compared to that of the same concentration of #Ab6 (Table 7).

BxPC-3-luc-2 proliferation assays were used to screen the bispecific antibodies. BxPC-3-luc-2 cells were diluted to 8x10⁴ cells/ml in culture medium. Antibodies were diluted to concentrations of 10 and 1 µg/ml and added in a volume of 100 µl in 96-well black bottom plates (ABgene AB-0932). Cells were added at density of 8000 cells/well in the absence or presence of 10 ng/ml final concentration human HRG. The cells were cultivated for 4 days at 37°C, 5% CO, in 95% relative humidity. Alamar Blue^{™} (Invitrogen) was added according to the manufacturer's instructions and incubated for 4 hours at 37°C, 5% CO, in 95% relative humidity in the dark. Fluorescence was measured at 550 nm excitation with 590 nm emission wave length.

To minimalize edge effects, the outer wells of the 96 well plates were fully filled with PBS.

### Affinity ranking of HER2 specific IgGs

We used the method described by Devash (PNAS, 1990) to rank the antibodies in a limited antigen-ELISA. The use of decreased antigen coating concentrations eliminates observed cross-reactivity reactions and can be used to detect high-affinity/avidity antibodies. Therefore the antigen concentration on the solid support was gradually decreased to investigate the weak immunoreactivities. A serial titration of ECD-Erbb-2 protein starting from 2.5 µg/ml until 0.019 µg/ml was coated overnight to MAXISORP^{™} ELISA plates. Wells of the ELISA plates were blocked with PBS (pH 7.2) containing 5% BSA for 1 hour at 37°C. Selected antibodies were tested in duplo at a concentration of 10 µg/ml diluted in PBS-2% BSA and allowed to bind for 2 hours at 25°C. As a control the procedure was performed simultaneously with an antibody specific for the coated antigens and a negative control antibody. The ELISA plates were washed 5 times with PBS-T (PBS-0.05% v/v Tween 20). Bound IgG was detected with 1:2000 diluted HRP-conjugate (Goat anti-mouse IgG, BD Biosciences) and was allowed to bind for 2 hours at 25°C. The ELISA plates were washed 5 times with PBS-T (PBS-0.05% Tween 20) and bound IgG was detected by means of OD492nm measurement. PG1849, PG2916, PG2926, PG2930, PG2971, PG2973, PG3004 and PG3031 were tested in an HER2 antigen titration ELISA (Fig. 1).

### Binding of HER2 VH genes with various kappa light chains

To investigate the binding of HER2 VHs derived from different phage display libraries a panel of HER2 antibodies was cloned and expressed in the context of another VK kappa chain, i.e. the VL of MEHD7945A. Produced IgGs were subjected to FACS analysis on K562 cells and stable K562-HER2 cells. VH genes derived from the combinatorial libraries and non-combinatorial libraries are listed in Table 8. The VH chains MF2971, MF3958, MF2916, MF2973, MF3004, MF3025, MF3031 all could be combined with the MEHD7945A light chain without loosing significant antigen specificity and binding as observed when combined with the common light chain IGKV1-39. VH chain MF1849 was not able to combine with the variant kappa light chain and retain antigen specificity and binding.

### Other HER2 and HER3 antibodies

Antibodies that inhibit the function of HER2 or HER3 are known in the art. Further antibodies were constructed according to published information and expressed in 293F Freestyle cells. The anti-HER2 antibodies pertuzumab and trastuzumab were generated based on the information disclosed in US2006/0212956 A1 (Genentech). The anti-HER3 antibody #Ab6, was based on the information disclosed in WO 2008/100624 (Merrimack Pharmaceuticals, Inc.) and recloned in a IgG1 back bone vector. The information of the 1-53 and U1-59 anti-HER3 antibodies was obtained from US 7,705,103 B2 (U3 Pharma AG). The information of the anti-HER3 LJM716 antibody was obtained from US 2012/0107306. The information for the construction of the two-in-one anti-EGFR anti-HER3 antibody MEHD7945A was obtained from WO2010/108127.

### Screening of HER2xHER3 bispecific antibodies

VH from the HER2 and HER3 antibody panel were recloned into the charged engineered vectors such that upon expression of the antibody heavy chains heterodimerization of heavy chains is forced resulting in the generation of bispecific antibodies after transfection. Three different strategies were used in combining HER2 and HER3 arms in bispecific IgG format:
1. HER2 (blocking ligand independent growth) xHER3 (blocking ligand independent growth)
2. HER2 (blocking ligand independent growth) xHER3 (blocking ligand dependent growth)
3. HER2 from different epitope bins x HER3 (blocking ligand dependent growth)

In some bispecific combinations, antibodies generated in group 2 and 3 overlapped with group 1.

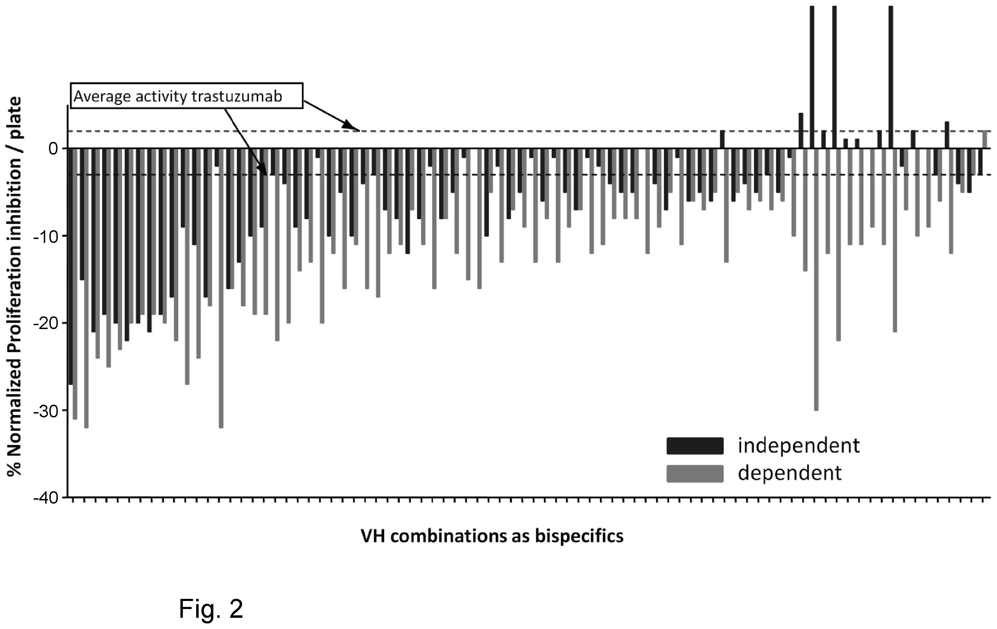

A total of 495 bispecific antibodies was produced in 24-well format and purified. All antibodies were tested for their capacity to inhibit the proliferation of the HER2- and HER3-expressing pancreatic BxPC-3-luc-2 cell line (Caliper). The potency of the antibodies was determined in a HRG-dependent and HRG-independent setting in a black and white screening with antibodies being present at a concentration of 10 and 1 µg/ml. Trastuzumab was included as a reference antibody as well as a negative control antibody at the same concentrations. The functional activity of the top 80 HER2xHER3 bispecifics (based on combined inhibition) at 1 µg/ml is shown in Figure 2.

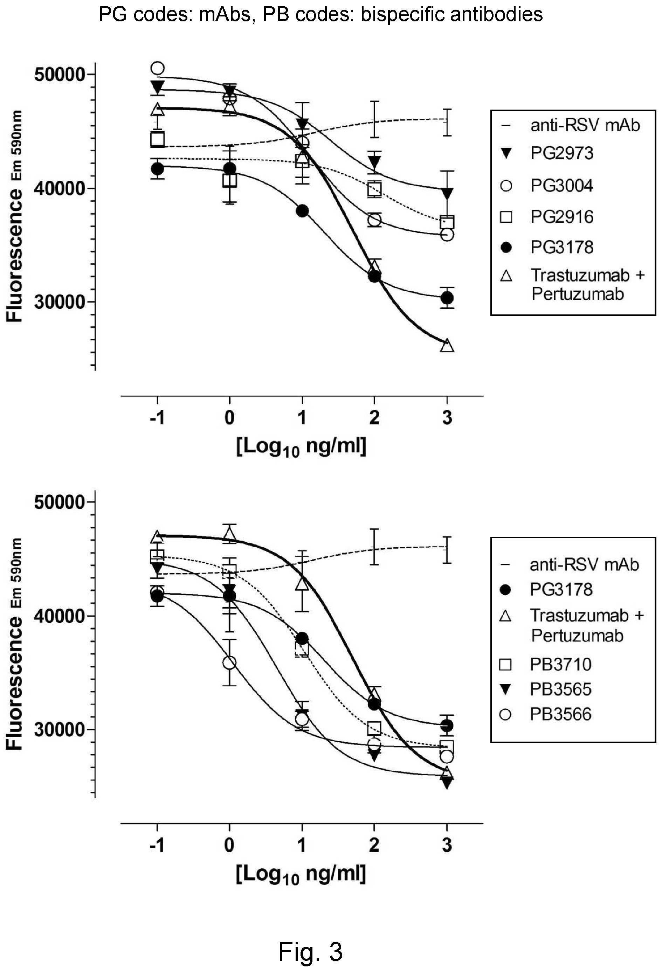

Antibodies (40 in total) that showed a higher inhibitory activity compared to the positive control antibody were selected, reproduced and purified in a 24-well format and tested again in the black-and-white BxPC-3-luc-2 screen at 10 and 1 µg/ml concentrations. These antibodies were further titrated in HRG-dependent MCF-7 assay and compared against the combination of trastuzumab and pertuzumab (1:1) and a negative control antibody. Figure 3 shows an example of titration curves of three bispecific antibodies in comparison to the parental HER3 antibody and the combination of trastuzumab + pertuzumab. The parental monoclonal antibodies are shown in the top panel and the bispecific antibodies are shown in the lower panel. (Figure 3).

The IC₅₀ for the bispecific antibodies, monoclonals and comparator antibodies was calculated using non-linear regression analysis with Prism software. Graph pad software lists the IC₅₀ values of the bispecific antibodies in the MCF-7 assay and their inhibitory activity in the BxPC3 assay for comparison. A panel of 12 HER2xHER3 bispecific antibodies had more potent inhibiting activity compared to trastuzumab + pertuzumab. In addition the bispecific antibodies were equally or more potent than the parental monoclonal PG3178 (Table 9).

The bispecific antibodies that inhibited ligand dependent cell growth were composed of HER2 arms in combination with the HER3 arms 3178, 3163, 3099 and 3176. Both the HER2 and HER3 arms of the most potent bispecifics were as a bivalent monoclonal also capable of inhibiting ligand-independent SKBR-3 proliferation (both the HER2 and HER3 arms) (Table 6) or ligand dependent MCF-7 proliferation (HER3 arms) (Table 7). The majority of the potent antibodies was composed of a HER2 arm recognizing domain I in combination with anti-HER3 antibody 3178.

### Inhibition of BxPC-3-luc2 tumor growth

The antibodies described in Table 9 were tested in a BxPC-3-luc2 pancreatic xenograft model. The BxPC-3-luc2 cell line expresses both HER2 and HER3 and is considered a HER2 low expressing cell line. CB17 SCID female mice, 8-10 weeks old at the beginning of the study were engrafted orthotopically in the pancreas with 1×10⁶ tumor cells in 20µl. To this aim mice were anesthetized and laid on the right side to expose the left side and a 0.5 cm incision was made on the left flank region. The pancreas and spleen were exteriorized and 1×10⁶ tumor cells in 20µl was injected into the sub-capsulary space of the pancreas tail. One week after implantation, bioluminescence (BLI) data were generated. 15 minutes prior to the imaging, all of the mice received i.p. injections of 150 mg/kg Luciferin (D-Luciferin-EF Potassium Salt, Cat. #E6552, Promega). BLI imaging was performed once or twice weekly using the left side view. Outlier animals - based on BLI/tumor volume - were removed and the mice were randomly distributed into groups of 7 mice each. On experimental day 8, the treatment was started. The animals in the antibody treatment group were dosed weekly for 3 consecutive weeks (days 0, 7, 14 and 21) with 30 mg/kg of antibody. At day 0 of the treatment the animals received twice the loading dose, i.e. 60 mg/kg of antibody. The final imaging was carried out at day 31.

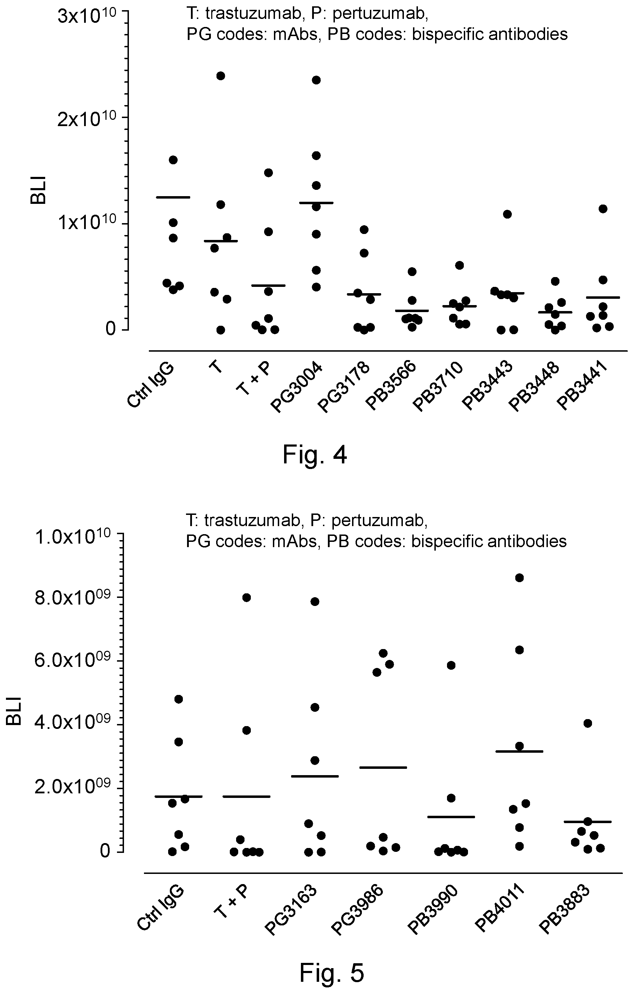

Two BxPC-3-luc2 xenograft models were run with a different panel of bispecific antibodies and parental antibodies In the first BxPC-3-luc2 xenograft model (Figure 4), one group received the negative control anti-RSV antibody (Ctrl IgG), one group received the control antibody trastuzumab and one group received the positive control antibody trastuzumab + pertuzumab (1:1 v/v). The seven remaining groups received one of the monoclonal (PG) or bispecific (PB) antibodies PG3004, PG3178, PB3566, PB3710, PB3443, PB3448 and PB3441. Details of the composition of the bispecific antibodies are depicted in Table 9.

All five bispecific antibodies tested were able to inhibit tumor growth. The mean tumor mass (BLI) of bispecific HER2 x HER3 antibody treated animals was similar to that in the animals treated with the combination of trastuzumab + pertuzumab. (Fig. 4)

In the second BxPC-3-luc2 xenograft model (Figure 5), one group received the negative control anti-RSV antibody (Ctrl IgG) and one group received the positive control antibody combination trastuzumab + pertuzumab (1:1 v/v). The five remaining groups received one of the antibodies PG3163, PB3986, PB3990, PB4011 and PB3883. For details about the bispecific PB antibodies: Table 9. These bispecific antibodies contained three different HER3 binding arms combined with the same HER2 arm MF2971 and an additional HER2 arm combined with the HER3 binding arm MF3163. In this experiment the tumors in the control group did not show the same level of accelerated growth as in the first experiment complicating interpretation of the results. Nevertheless, in comparison to trastuzumab + pertuzumab the PB3883 and PB3990 HER2xHER3 bispecifics had similar inhibitory activities (Fig. 5).

Based on the *in vivo* and *in vitro* data a bispecific panel of antibodies was selected of which the HER2 arms were composed of MF2971, MF3004, MF1849 and the HER3 arm was composed of MF3178. The MF2971 and MF3004 arm were of mouse origin and were humanized.

### Binding of bispecific HER2xHER3 antibody compared to parental monoclonal antibodies

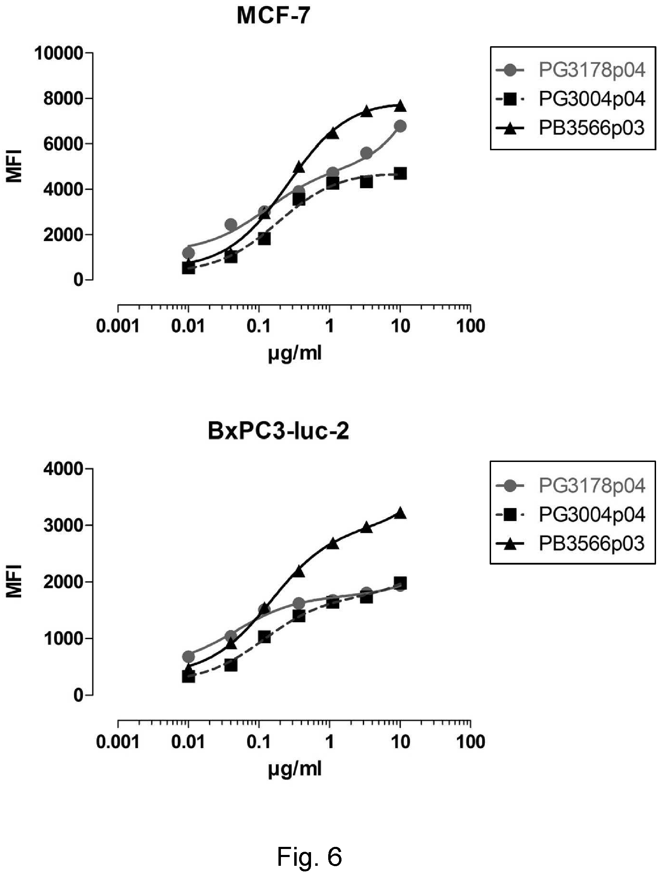

Binding of HER2xHER3 bispecific antibodies as compared to their parental counterparts was determined by FACS analysis. A FACS was performed on BxPC-3-luc2 cells and MCF-7 cells with a serial titration of antibodies ranging from 2,5 µg g/ml - 0, 01 µg g/ml. The tested antibody panel was composed of the bispecific antibody PB3566 and its parental antibodies the anti-HER3 antibody PG3178 and the anti-HER2 antibody PG3004. The MFI data were plotted and the graphs on both cell lines show that the bispecific PB3566 binds more effectively to both tumor cell lines compared to the anti-HER3 antibody PG3178 and the anti-HER2 antibody PG3004. (Fig. 6)

### Humanization of MF2971 and MF3004

MF2971 and MF3004 were humanized according to technology known in the art. A total of seven humanised/de-immunised variant sequences of MF2971 were expressed, validated and characterised in vitro as monoclonal and in bispecific format combination with the HER3-specific antibody MF3178. The same was done for seven variant sequences of MF3004, which were created by replacing the HCDR3 of MF2971 in the seven MF2971 variants with the HCDR3 of MF3004. The expression, integrity, thermal stability and functional activity of all humanized variants was analysed. Based on production, integrity, stability and functionality integrity, a variant of MF2971 (2971-var2) was chosen as the optimal humanized variant of the VH to be used in a bispecific format with MF3178. This 2971-var2 was renamed MF3958. The bispecific HER2xHER3 combination MF3958xMF3178 resulted in PB4188.

### Large scale production, purification and analytical studies of PB4188

Suspension adapted 293F Freestyle cells were cultivated in Erlenmeyer flasks at a shaker plateau until a density of 3.0 x 10⁶ cells/ml. Cells were seeded in a 4 L erlen flasks at a density of 0.3-0.5 x 10⁶ viable cells/ml. The cells were transiently transfected with the individual sterile DNA: PEl mixture and further cultivated. Seven days after transfection, conditioned medium containing bispecific antibody was harvested by low-speed centrifugation, 5 minutes 1000 g, followed by high speed centrifugation, 5 minutes at 4000g. Collected conditioned medium was concentrated over a 5 kDa Satorius hydrosart cassette to about 600 ml and subsequently diafiltrated against 4 L PBS. Antibodies were bound on column to ~35 ml MabSelectSure XL (11°C). A-specifically bound proteins were removed by washing the column in reversed flow mode with 150 ml PBS, 150 ml PBS containing 1 M NaCl, 100 ml PBS. The bound antibodies were eluted using 100 mM citrate pH 3.0 in reversed flow mode and 5 ml fractions were collected in 10 ml tubes containing 4 ml 1Tris pH 8.0 for neutralization. The eluted antibodies were further purified by gel-filtration using superdex 200 50/1000. Thepurified antibody was filter-sterilized using a 0.22 µm syringe filter. IgG concentration was determined by OD280 measurement and the protein concentration was calculated based on the amino acid sequence. Protein was tested for aggregation (HPSEC), purity (SDS-PAGE, nMS, IEX and IEF). Protein samples were stored at -80°C.

### IgG purification for analytical and xenograft studies.

Medium scale purifications were performed on an AKTA 100 Explorer using HiTrap MabSelect Sure columns and HiTrap desalting columns. Samples were loaded at 5 ml/min. The column was washed with 2 column volumes of PBS. IgG was eluted at pH 3.0 with 0.1 M citrate buffer. Next the sample was desalted and ended up in a final buffer of PBS pH 7.4. IgGs were filtered through a 0.45 µM filter (Sartorius). The IgG concentration was measured using Octet with protein A sensors. Protein was tested for aggregation (HPSEC), purity (SDS-PAGE, nMS, IEX and IEF). Protein samples were stored at -80°C.

### Analytical characteristics of PB4188

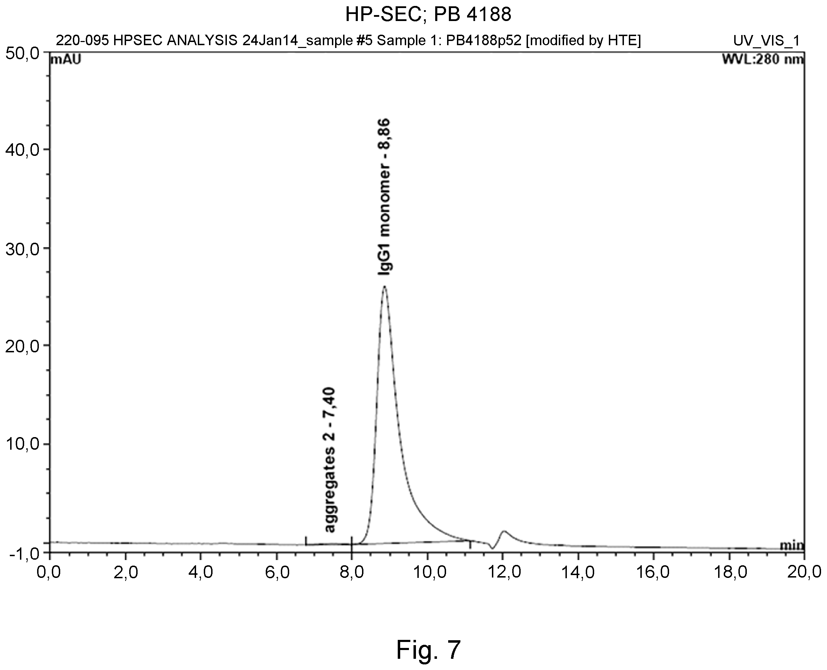

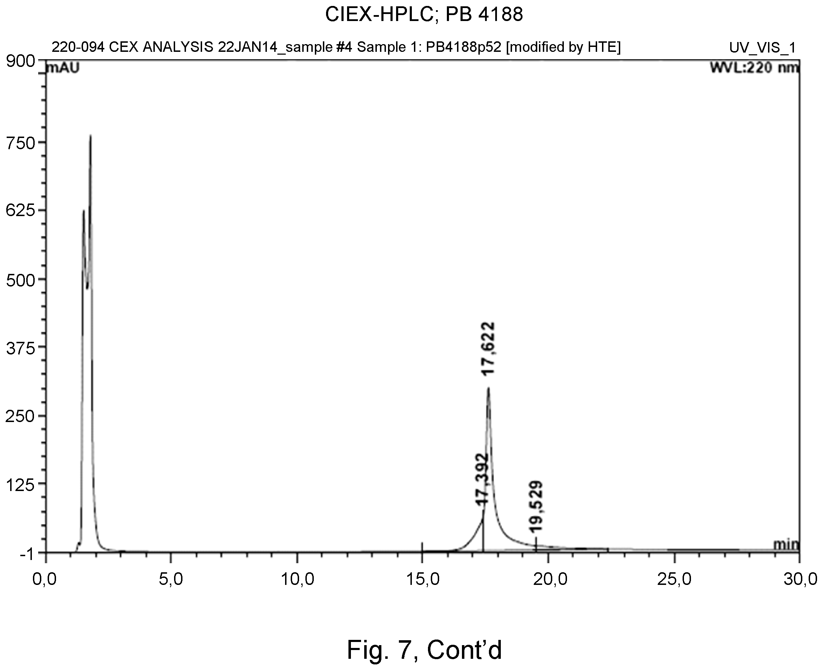

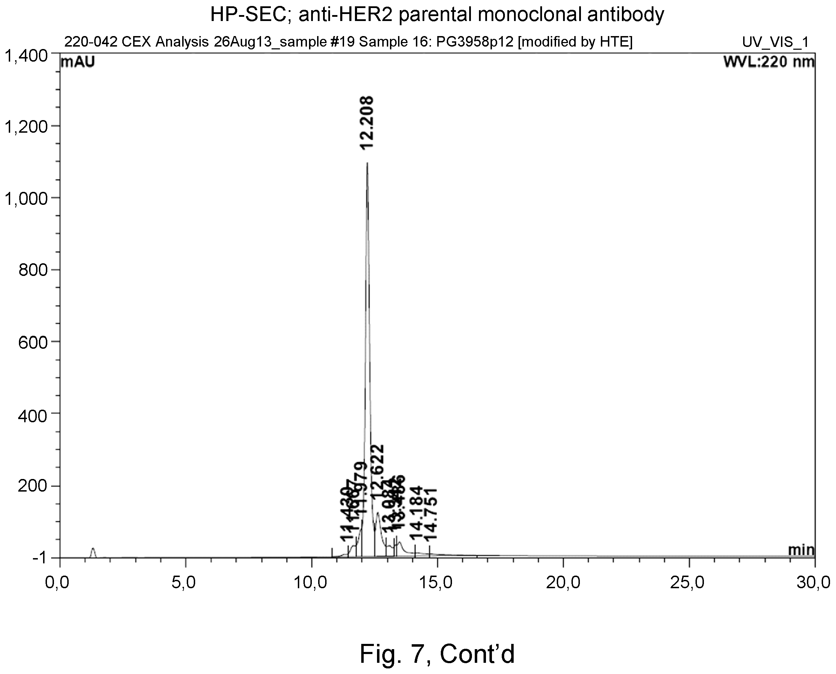

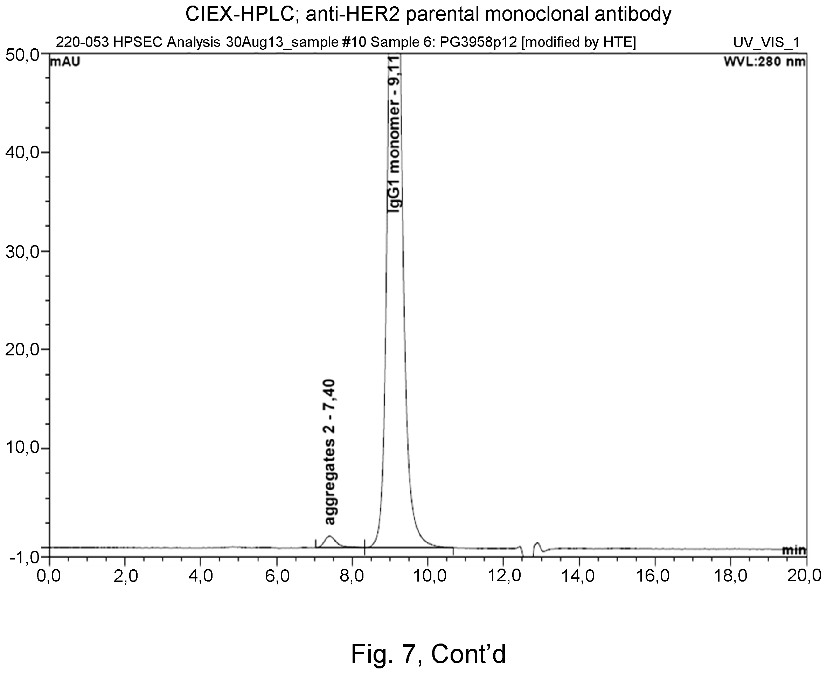

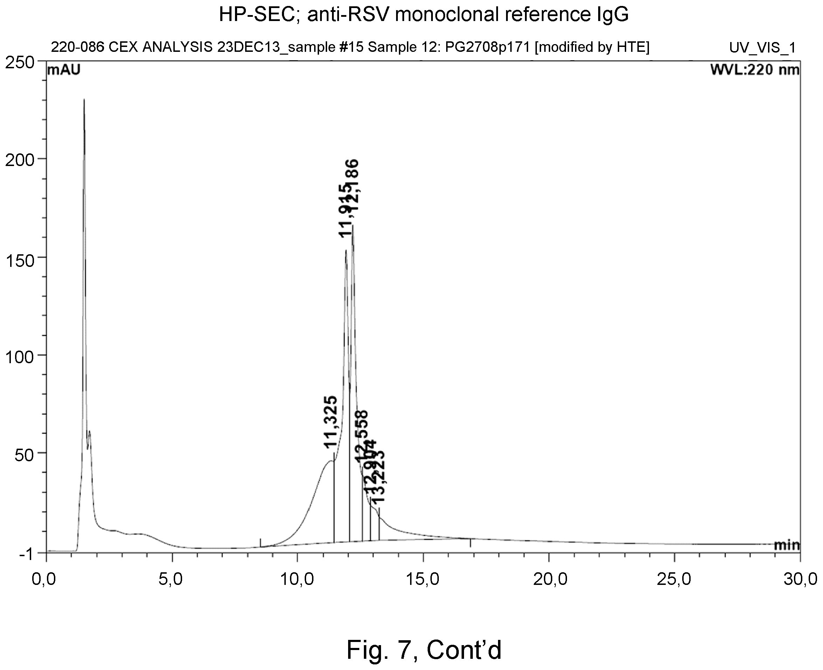

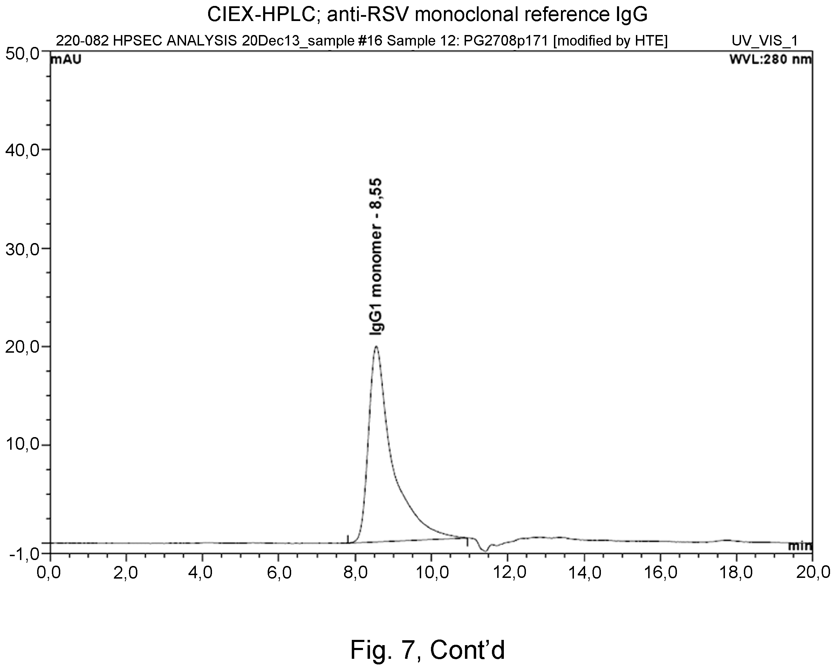

The PB4188 (MF3958xMF3178) was subjected to analysis by HP-SEC and CIEX-HPLC (TSK gel-STAT 7 µm column, 4.6 mm ID x10 cm L). The analytical profile of PB4188 was in general consistent with the behavior of normal monospecific IgG1, such as the parental HER2 arm PG3958 and the anti-RSV monoclonal control antibody (Fig. 7).

### Affinity determination

The monovalent binding affinity of PB4188 and PB3448 for recombinant HER2 and HER3 was determined by SPR (Biacore T100). Biacore^{™} T100 (GE Healthcare, Uppsala, Sweden) was used to conduct all experiments described. Sensor surface preparation and interaction analyses were performed at 25° C. Buffer and Biacore reagents were purchased from GE Healthcare. ErbB2-Fc and ERbB3-Fc(RND) was coated to the surface of a CM5 sensor chip in potassium acetate buffer (pH5.5) at the target immobilization level of 500 RU. Running buffer was HBS (hepes-buffered saline): 10 mM HEPES pH 7.4, 150 mM NaCl, 0.005% Tween-20; 0.2µm) filter-sterilized. The bispecific antibodies were diluted to 100, 50, 20, 10, 1 and 0.1 nM in HBS and run at high (30µl/min) flow rate over the antigen-coupled surface of the CM5 sensor chip. With the BIA evaluation software, a curve fitting model for 1:1 monovalent interaction allowed for determination of the HER2 arms affinities (mono-valent interaction), the affinities of the HER2 arms, could be determined. Due to the low-off rate of the HER3 arm the affinity could not be determined. To determine the affinity of the HER3 arm PB4188 was coated to a CM5 sensor chip at the target immobilization level of 500 RU. Her2-Fc and Her3-Fc antigens were diluted to 100, 50, 20, 10, 1 and 0.1nM in HBS and run at high flow rate (40µl/min) over the PB4188 surface. To determine the kₒₙ and k_{off} values, the BIA evaluation software was used in conjunction with a model that takes into account that a monovalent molecule was coated to the sensor chip surface and that the ErbB3-Fc antigen was a bivalent molecule. The affinities of PB4188 and PB3448 are shown in Table 10.

### PB4188 Affinity determination on cells

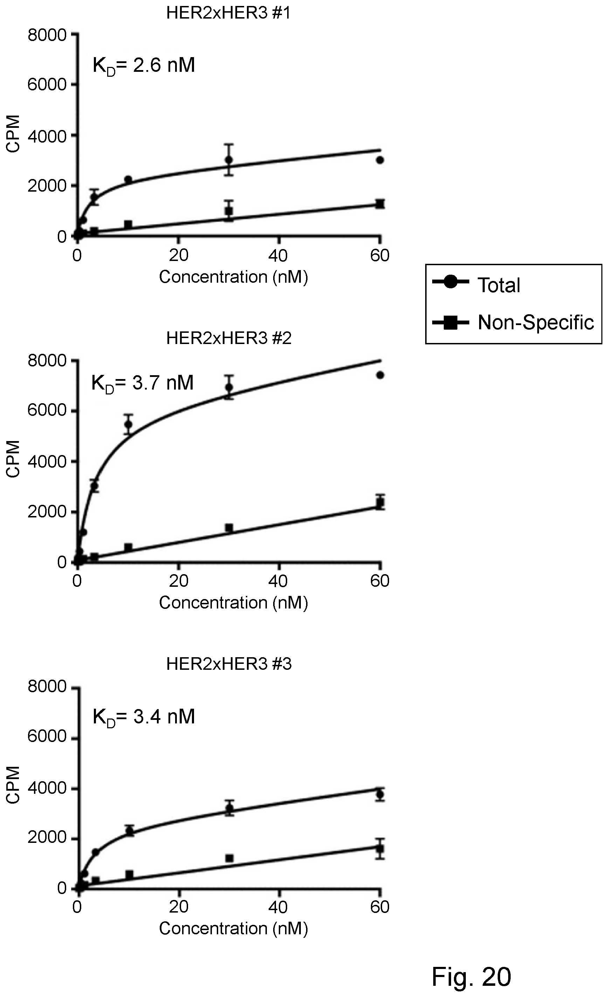

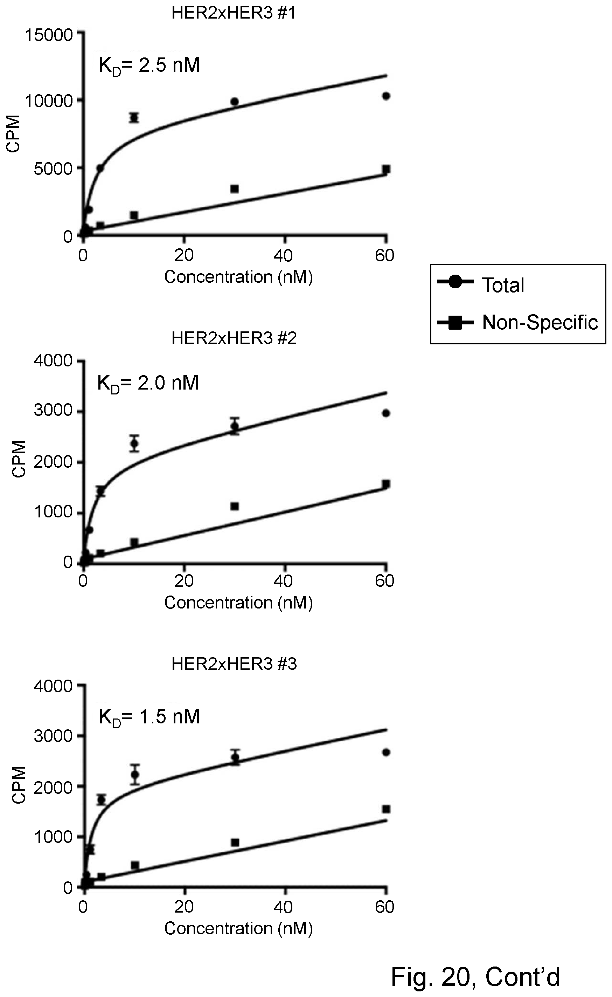

Binding affinities were also determined via steady state cell affinity measurements using BT-474 and SK-BR-3 cells. Four IgG were analyzed: 1) PB4188 (bispecific HER2xHER3), containing anti-HER2 antibody 3958 and anti-HER3 antibody 3178; 2) PB9215 (bispecific HER3xTT), containing anti-HER3 antibody 3178 and anti-TT (tetanus toxoid) antibody 1337; 3) PB9216 (bispecific HER2xTT), containing anti-HER2 antibody 3958 and anti-TT antibody 1337; 4) Herceptin (monospecific HER2). The IgG were radioactively labeled with ¹²⁵I using IODO-GEN^{®} Precoated Iodonation Tubes (Pierce) and associated instructions. The labeled IgG were diluted to an activity of ~1-2 x 10⁸ cpm/ml in 25 mM Tris-HCl, 0.4 M NaCl, 0.25% BSA, 5 mM EDTA, 0.05% NaN₃. Protein concentrations were determined with the BCA Protein Assay Kit (Pierce). Flow cytometry analysis of the labeled and non-labeled IgG using BT-474 and SK-BR-3 cells showed no or only minor signs of reduction in binding after labeling. Steady state cell affinity measurements were performed as follows. Cells were seeded in 96-well plates and incubated at 4°C with various concentrations of labeled IgG. Unbound radioactivity was removed after 4 hours and the cell-bound radioactivity was measured using a gamma well counter. Non-specific binding was measured by adding a receptor-blocking concentration (100-fold excess) of unlabeled antibody. Each condition was tested in triplicate and three independent experiments were performed per antibody. K_{D} values were calculated based on a non-linear regression model that compensates for non-specific binding, using Prism 6.0d (GraphPad Software). Graphs including fitted curves are given in Figure 20 for binding of the HER2xHER3 IgG (PB4188) to both cell lines. K_{D} data for all 24 assays, including mean values, are given in Table 12. In summary, the mean KD values as determined using BT-474 and SK-BR-3 cells were 3.2 and 2.0 nM for HER2xHER3, 3.7 and 1.3 nM for Herceptin, 3.9 and 2.3 nM for HER2xTT, and 0.23 and 0.99 nM for HER3xTT, respectively. Thus PB4188 shows a higher affinity for HER3 compared to HER2 which is in contrast to the HER2xHER3 bispecific molecule MM-111 that targets HER2 with a higher affinity compared to HER3.

### Anti-proliferative activity on HER2 amplified breast cancer cells

### JIMT-1 in soft agar

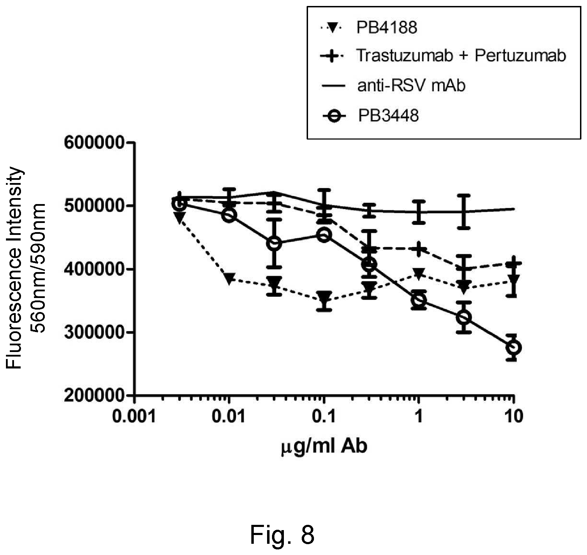

PB3448 and PB4188 were tested for their potency to inhihit the growth of the trastuzumab resistant JIMT-1 cells in soft agar. To this aim 96 well suspension cell culture plates were prepared. 100 µL of the soft agar bottom layer (0,6% final concentration in complete medium) was poured and left to solidify. 50 µL of the soft agar top layer (0,4% final concentration) containing 10.000 JIMT-1 cells/well were then added on top, solidified and such 96 well plates incubated overnight at 37°C, 10% CO2. Next day, a negative control antibody, pertuzumab + trastuzumab (1:1 v/v), PB3448 and PB4188 were added in DMEM medium in a semi-log titration ranging from 10-0,003 µg/ml. Subsequently, the assay was incubated in cell culture incubators for 8 days. Finally, the cells were incubated with Alamar Blue for 3-5 h at 37 °C and fluorescence intensity was determined (excitation: 560 nm; emission: 590 nm). An example of dose dependent inhibition of JIMT-1 proliferation by PB3448 and PB4188 is shown. (Figure 8).

### BT-474 and SKBR-3 in matrigel

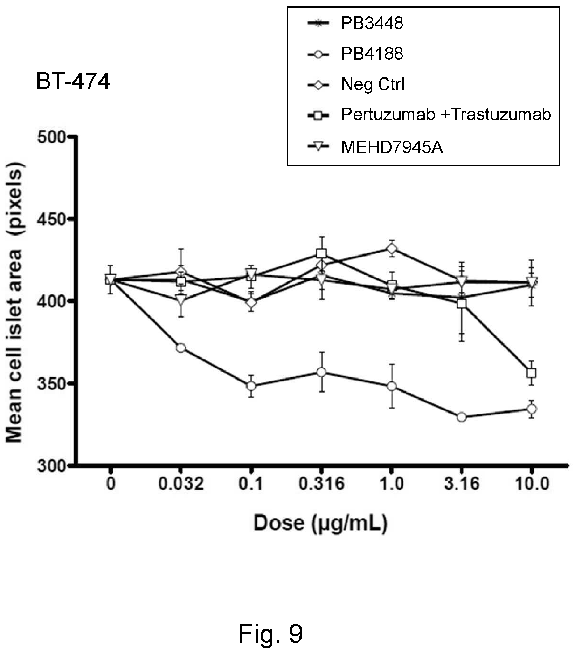

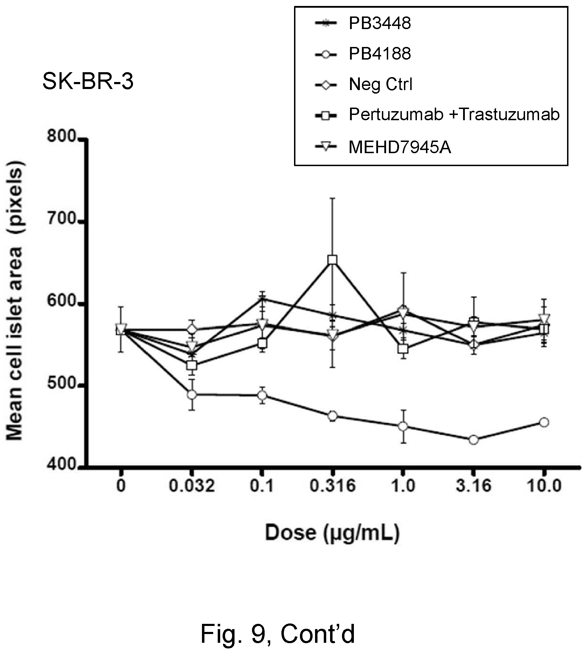

PB3448 and PB4188 were tested for their potency to inhibit the growth of BT-474 and SKBR-3 cells. The cells were tested at the company Ocello based in Leiden, the Netherlands that grows cells in three dimensional matrigel and uses principle component analysis to distinguish non-treated cells from treated cells. 2000 SK-BR-3 or 2250 BT474 cells were seeded in 15µl matrigel per well of a 384 well plate (Greiner 781091 ). The next day a semi-log titration ranging from 10 to 0.003 µg/ml of antibodies were added in culture medium in the absence or presence of 5 ng/ml HRG. The test antibodies included a negative control antibody, pertuzumab + trastuzumab (1:1 v/v), PB3448, PB4188 and the bispecific anti-EGFRxHER3 two-in-one antibody MEHD7945A. In addition a dose-dependent titration of HRG was included as a positive control. Each dose was tested in quadruplicate. Cells were incubated for 7 days in a cell culture incubator at 37°C, 5% CO2. Next, the cells were fixed and actin cytoskeleton of the cells was stained with phalloidin and the nuclei are stained with Hoechst. Next, fluorescent images were taken at different levels through the gel (Z-stack) and the images were superimposed. A broad range of morphological features were measured (800 in total). Only features that differed between medium and HRG treatments were selected for analysis. Features that were associated with growth, mean spheroid area and nuclei per spheroid were most significantly different between medium and HRG treatments. Both multiparameter and single parameter analyses were made. For single parameter measurements, t-tests were performed to compare treatments (HRG or antibody) to medium. P-values for each point were determined. Principal component analysis (PCA), a method for finding low-dimensional combinations of high-dimensional data that capture most of the variability was used in relation to antibody concentration, to plot the data. Figure 9 demonstrates the effect of pertuzumab + trastuzumab (1:1 v/v), PB3448 and PB4188 in the presence of HRG. In both HER2 amplified breast cancer cell lines PB4188 showed superior activity compared to pertuzumab + trastuzumab, PB3448 and the two-in-one antibody MEHD7945A in the presence of HRG.

### Superior anti-proliferative activity of PB4188 in the presence of HRG on HER2 amplified breast cancer cells

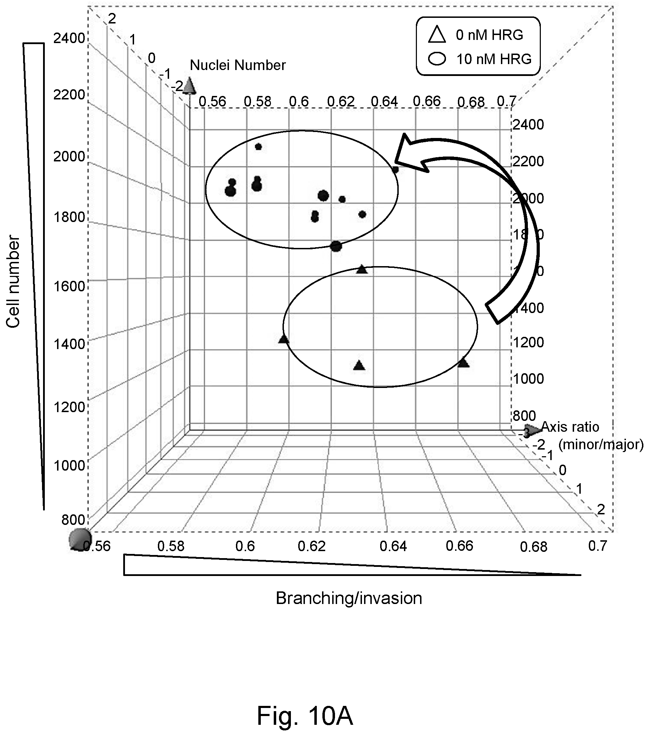

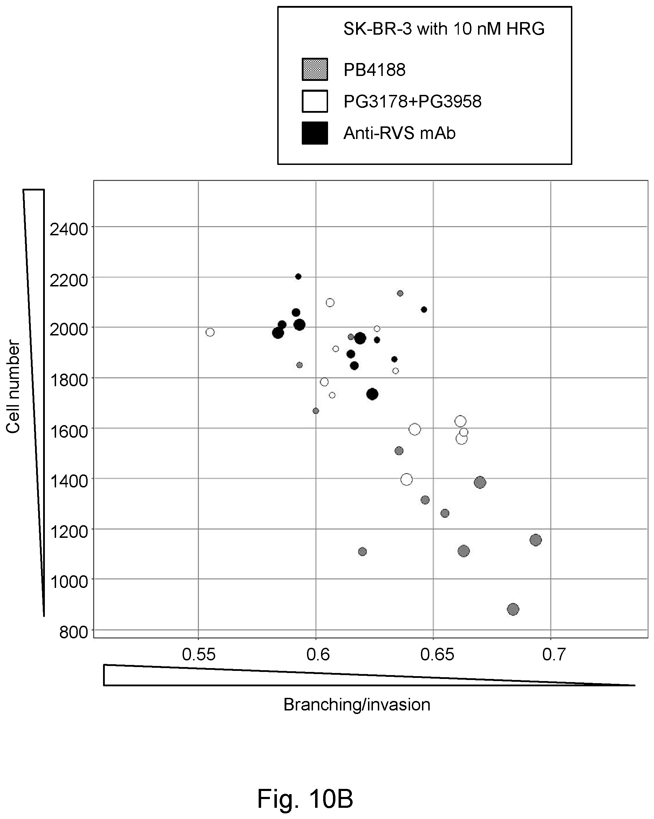

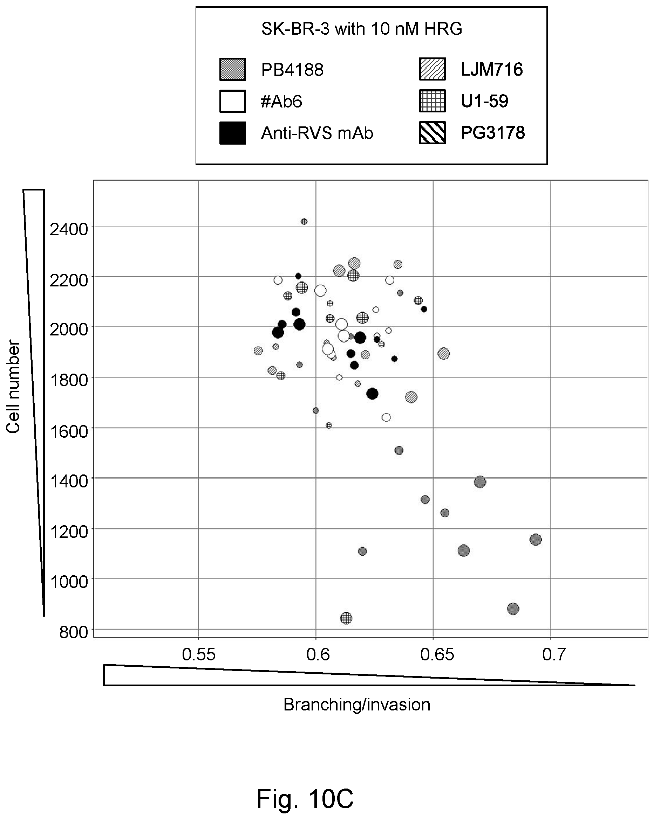

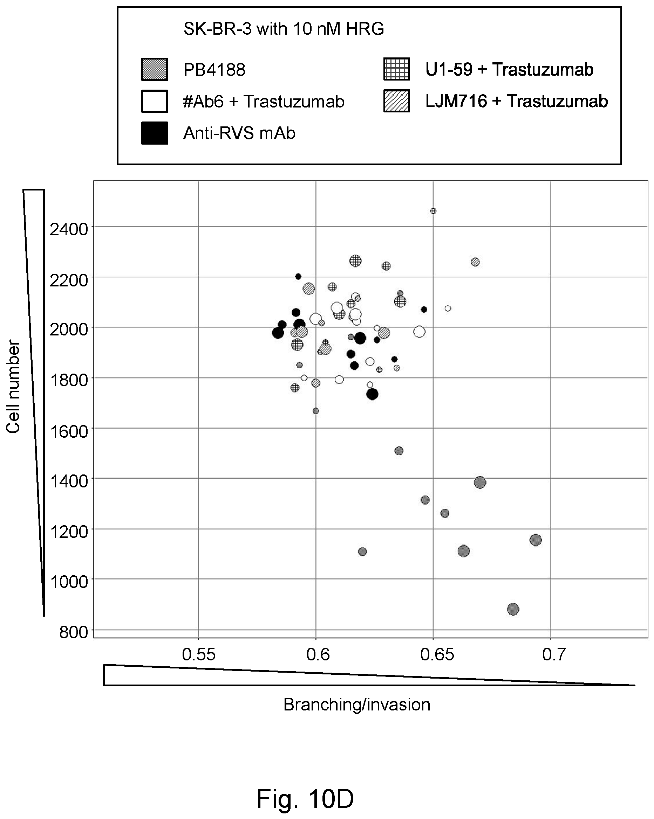

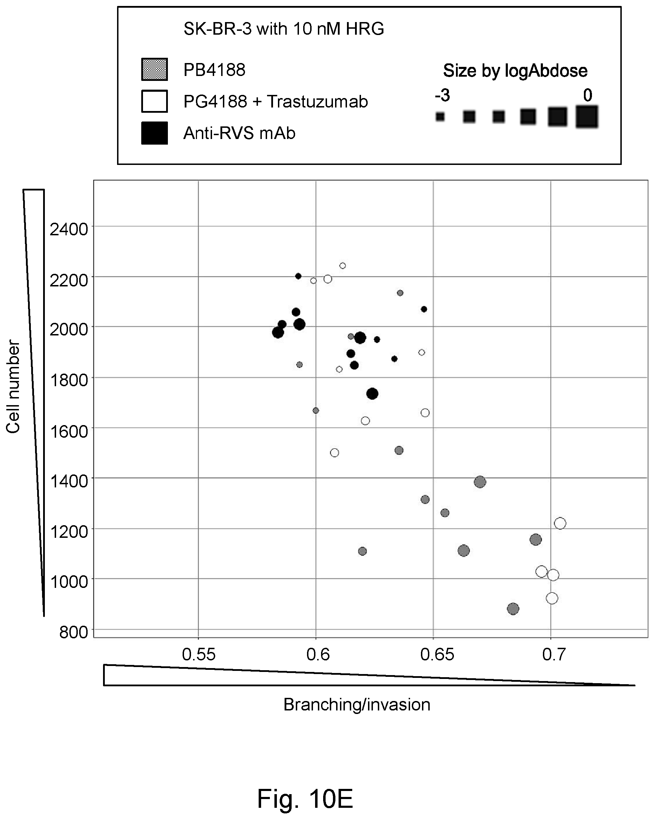

The activity of PB4188 in the presence of 10 ng/ml HRG on SKBR-3 and BT-474 was compared to a panel of HER2, HER3 antibodies and combinations thereof. The assay was performed in matrigel, as described above, and morphological features were analyzed. PCA data plotted in Figure 10a show the HRG -induced proliferation and branching/invasion of SKBR-3 cells in matrigel. Figure 10b shows that antibody PB4188 can completely revert the HRG induced phenotype, whereas the combination of the parental monoclonal antibodies (PG3958 + PG3178) has no effect. Moreover, PB4188 was far more effective compared to all anti-HER3 antibodies tested (Figure 10c). In addition, combinations of the individual anti- HER3 antibodies with trastuzumab (the current standard of care in metastatic breast cancer (mBC)) were not able to revert the HRG induced phenotype (Figure 10d). Adding trastuzumab to PB4188 in the presence of HRG reduced the proliferation and branching/ invasion of SK-BR-3 cells compared to PB4188 alone (Figure 10e).

### Superior anti-proliferative activity of PB4188 on HER2 amplified gastric cancer cells compared to HER2 and HER3 monoclonal antibodies.

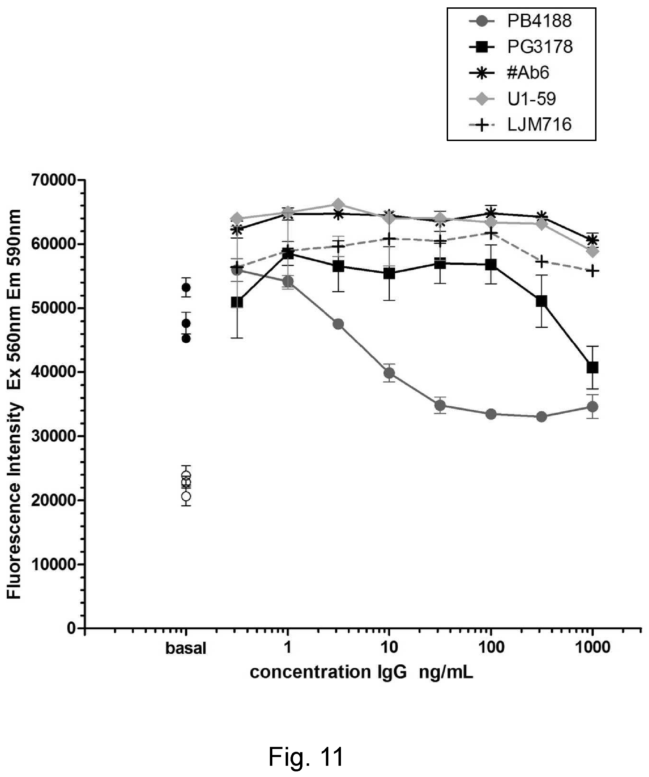

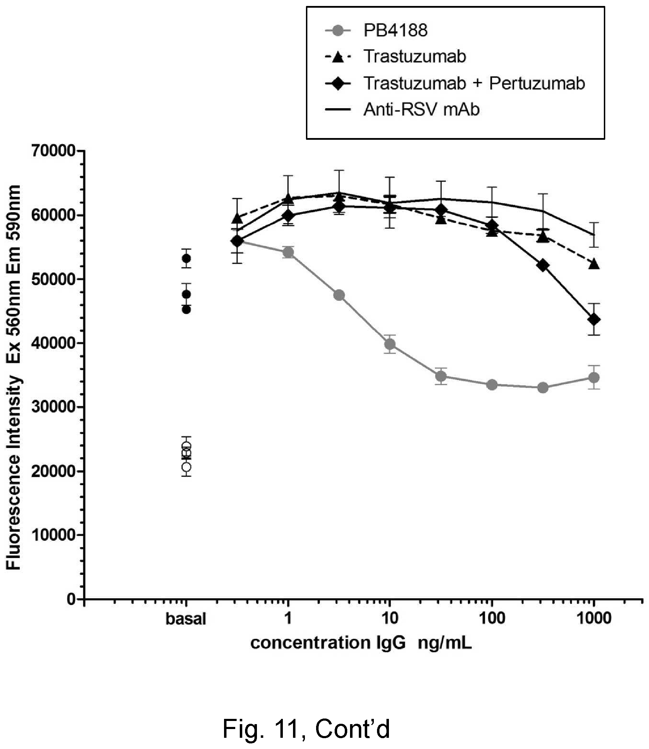

Upregulation of NRG1-β1 is a key resistance mechanism against HER2 targeted therapies (Wilson, 2012). To evaluate whether upregulation of NRG1-β1 would interfere with the anti-proliferative potency of PB4188 a panel of antibodies was tested at 100 ng/ml HRG on the N87 (HER2 amplified) gastric cancer cell line. N87 cells were cultured in RPMI 1640 supplemented with 10% heat inactivated FBS. For the proliferation assay subconfluent cell cultures of N87 cells were washed with PBS trypsinized and trypsin was inactivated by adding culture medium. Cells were washed twice in large volumes of assay medium (RPMI 1640 medium containing 0.05% BSA and 10 µg/ml Holo Transferrin). Antibodies were diluted in a semi-log titration that varied from 1-0,0001 µg/ml. Cells were added at a density of 10000 cells/well in the presence of 100 ng/ml final concentration of HRG. The cells were cultivated for 3 days at 37°C, 5% CO2, in 95% relative humidity. Alamar Blue^{™} (Invitrogen) was added according to the manufacturer's instructions and incubated for 6 hours at 37°C, 5% CO2, in 95% relative humidity in the dark. Fluorescence was measured at 550 nm excitation with 590 nm emission wavelength. PB4188 showed superior activity over anti-HER2 or anti-HER3 monoclonal antibodies (Figure 11).

### HER2XHER3 bipecific antibodies induce ADCC

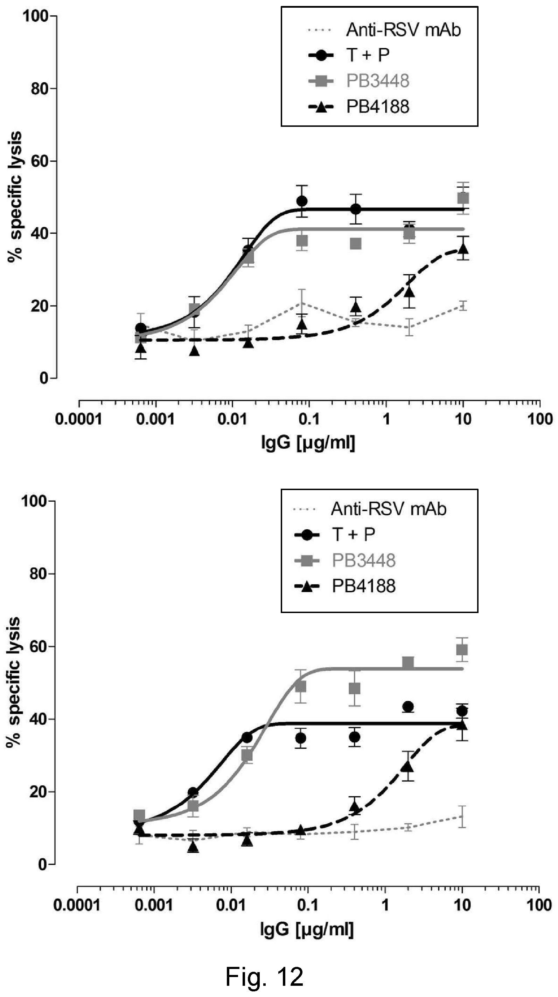

ADCC activity is an important anti-tumour mechanism of action for therapeutic antibodies in cancer. Human monoclonal antibodies directed to the HER family of receptors like cetuximab and trastuzumab induce ADCC. The baseline and enhanced ADCC activity of PB4188 and PB3448 were determined in validated *in vitro* ADCC assays. Trastuzumab and a negative control antibody were included as control antibodies in the experiment. Whole blood and PBMC fractions were obtained from healthy donors. Each antibody was tested against the HER2 high (SK-BR-3) and HER2 low (MCF-7) expressing target cells. Target cells were loaded with ⁵¹Cr (Amersham) and opsonized with the indicated concentrations of antibody. Whole-blood or PBMC fraction were used as effector cells in a 200 µL reaction in RPMI 1640 + 10% heat inactivated FCS. Cells were incubated together for 4 h, and lysis was estimated by measuring radioactivity in the supernatant using a γ-scintillator. Percentage of specific lysis was calculated as follows: (experimental cpm - basal cpm) / (maximal cpm - basal cpm) × 100, with maximal lysis determined in the presence of 5% Triton X-100 and basal lysis in the absence of antibody and effectors. As shown in Figure 12 bispecific antibody PB3448 showed similar ADCC activity compared to the combination pertuzumab + trastuzumab. Bispecific antibody PB4188 was effective at high antibody concentrations (10 µg/ml).

### HER2XHER3 bipecific antibodies show higher ADCC compared to the combination of parental antibodies

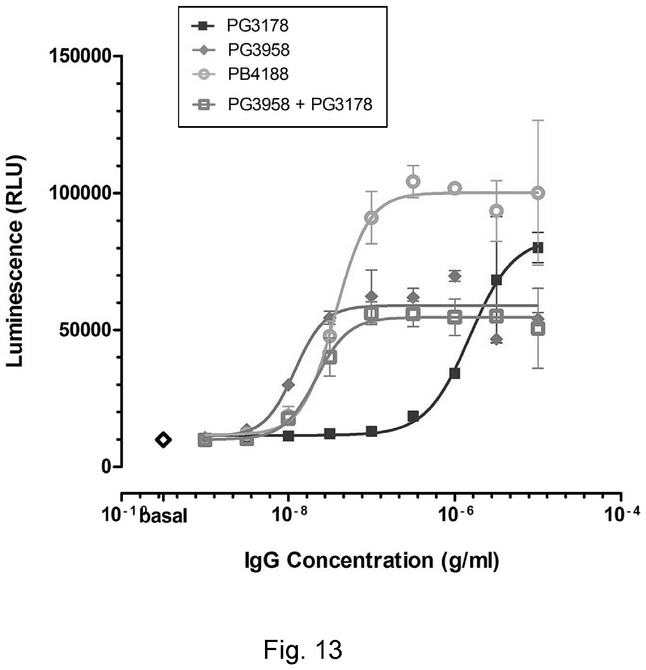

In a different ADCC setup, the ADCC Reporter Bioassay (Promega) was used. The bioassay uses engineered Jurkat cells stably expressing the FeγRIIIa receptor, V158 (high affinity) or F158 (low affinity) variant, and an NFAT response element driving expression of firefly luciferase. The assay was validated by comparing data obtained with the ADCC Reporter Bioassay to the classical ⁵¹Cr release assay. The ADCC assays were performed using the Promega ADCC Bioassay kit using 384 white well plates. In this experimental setup SKBR-3 cells were plated at a density of 1000 cells/well in 30 µl assay medium (RPMI with 4% low IgG serum) 20-24H before the bioassay. The next day, the culture medium was removed. Next, a serial dilution of antibodies, PB4188 and its parental anti-HER2 PG3958 and anti-HER3 PG3178 as well as the combination thereof was generated in duplo. 10 µl antibody dilutions were added to the wells. The starting concentration of the antibody was 10 µg/ml and a 10 points semi-log fold serial dilution was generated to provide a full dose-response curve. Finally, 5 µl of ADCC Bioassay effector cells (15000 cells/well, V158) were added. The cells were incubated for 6H at 37 °C. Next, 15 µl BIO-Glo luciferase substrate was added and 5 minutes later luminescence was detected in a plate reader. The obtained data are shown in Figure 13. The PB4188 bispecific anti-HER2xHER3 antibodies showed a higher ADCC potentency compared to the parental HER2 and HER3 monoclonals or a combination thereof.

### ADCC enhancement of PB4188

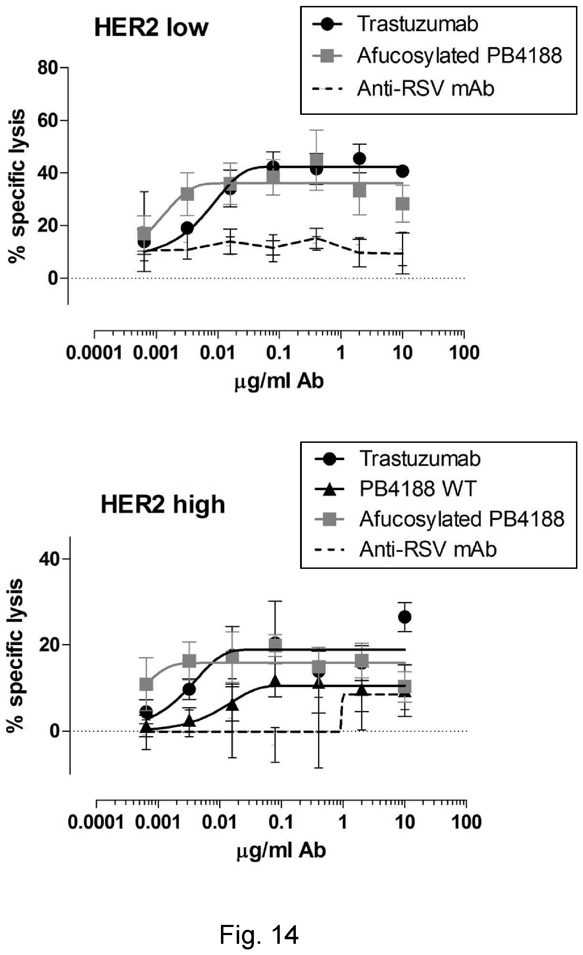

ADCC activity can be enhanced by different techniques, one of them being the removal of fucose. Removal of fucose has resulted in increased anti-tumour activity in several in vivo models [Junttila, 2010]. To maximize PB4188 activity, afucosylation technology was applied (Cheng Liu and Andreia Lee. ADCC Enhancement Technologies for Next Generation Therapeutic Antibody. Antibody therapeutics -Trends in Bio/Pharmaceutical Industry 2009 [13-17]) , thereby preventing fucosylation of the N-linked carbohydrate structure in the Fc region. The ADCC potency of afucosylated PB4188 compared to the wildtype PB4188 was determined in an ADCC ⁵¹Cr release assay using HER2 low expressing cells (MCF-7) and HER2 amplified cells (SK-BR-3). Both antibodies were applied in a serial dilution and a negative control antibody and trastuzumab were included in the assay. Figure 14 shows the increase in ADCC potency of afucosylated PB4188 compared to the wild type version and/or trastuzumab in both high and low HER2 expressing cells.

### Afucosylated PB4188 shows superior ADCC activity with low affinity FcyRIII receptors

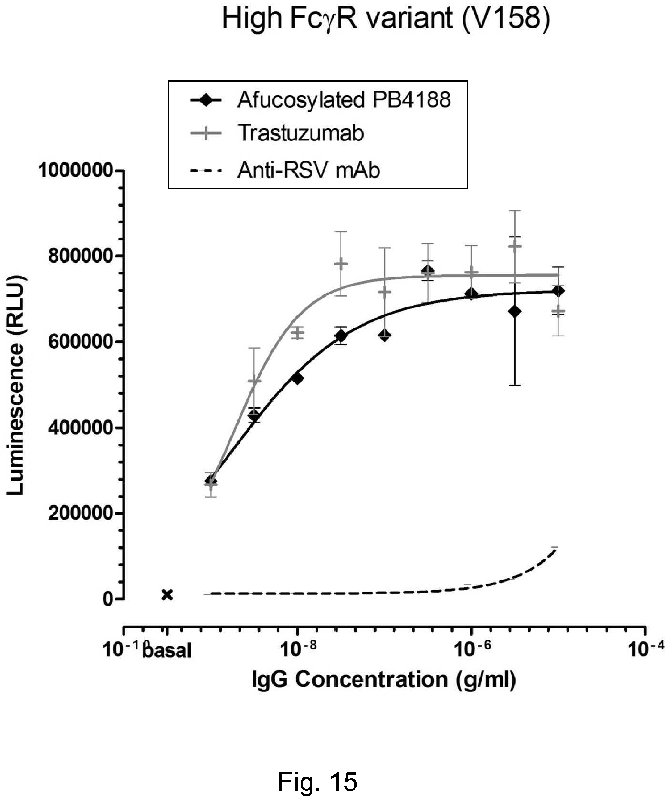

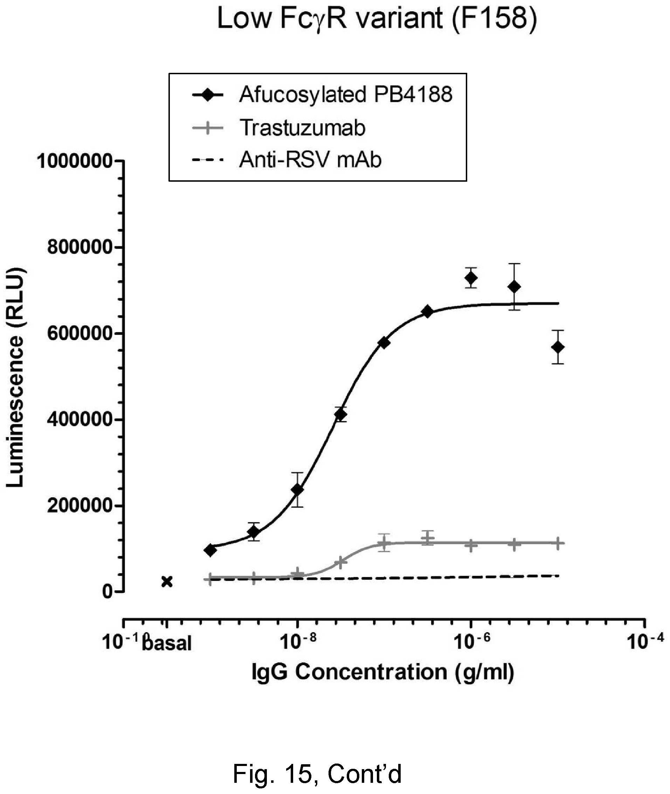

Afucosylated PB4188 activity was tested on ADCC reporter cells containing either the V158 (high affinity) FcyRIIIa receptor variant or the F158 (low affinity) FcyRIIIa receptor variant. A serial titration of antibody, i.e. control antibody, trastuzumab and afucosylated PB4188, was added in combination with ADCC reporter cells harbouring the different FcγRIIIa variants to adherent SK-BR-3 cells. ADCC activity was measured by measuring luciferase activity. Afucosylated PB4188 showed equal activity compared to trastuzumab in combination with the high affinity V158 FcyRIIIa receptor variant. In contrast afucosylated PB4188 displayed superior ADCC activity compared to trastuzumab in combination with the low affinity F158 FcyRIIIa receptor variant. (Figure 15)

### JIMT-1 xenograft study

JIMT-1 human breast carcinoma cells were grown in DMEM containing 10% fetal bovine serum, 100 units/mL penicillin G sodium, 100 µg/mL streptomycin sulfate, 25 µg/mL gentamicin, and 2 mM glutamine until the time of implantation. At the day of implantation JIMT-1 breast cells were harvested during log phase growth and resuspended in cold PBS. Female CB.17 SCID mice (Charles River) were 8 weeks old on Day 1 of the study and had a body weight range of 16.5 to 20.7 g. Each mouse was injected subcutaneously in the right flank with 5 x10⁶ tumor cells (0.2 mL cell suspension). The tumors were measured with a caliper in two dimensions to monitor size as the mean volume twice per week. Once tumors had reached approximately 100-150 mm³ in size animals were enrolled in the efficacy study. Outlier animals -tumor volume - were removed and the mice were randomly distributed into groups of 10 mice each. Mice were injected once weekly (antibody) or daily (lapatinib) for a period of four weeks. Details of the treatment groups are depicted in Table 11.

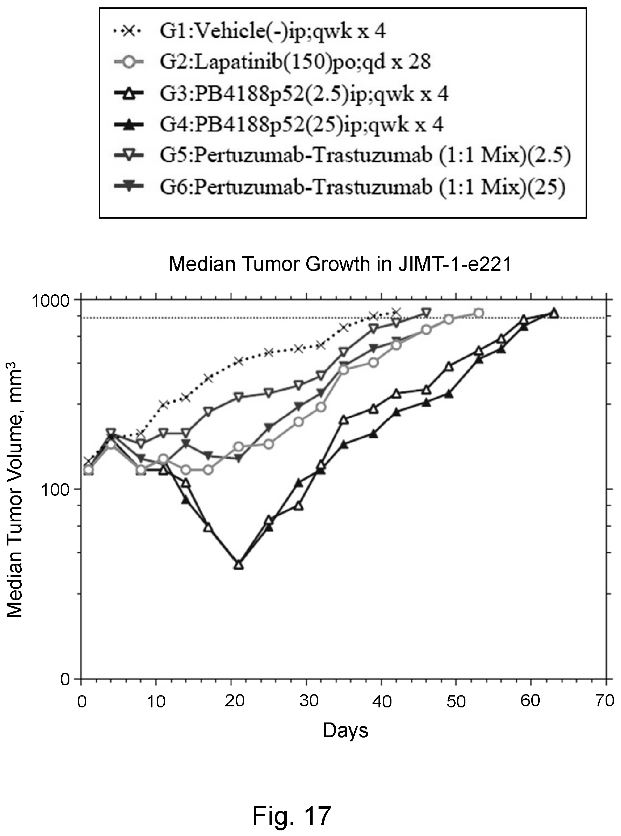

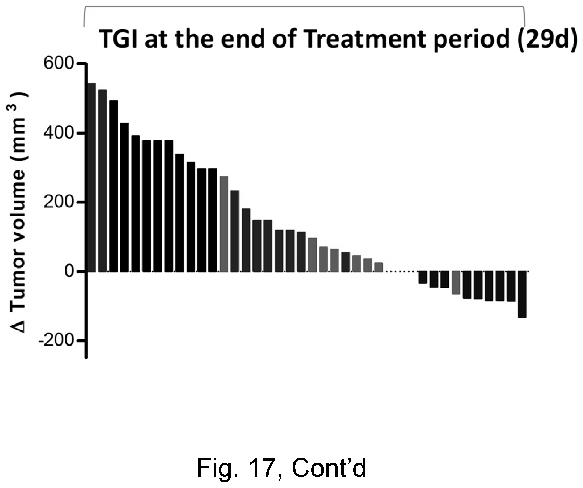

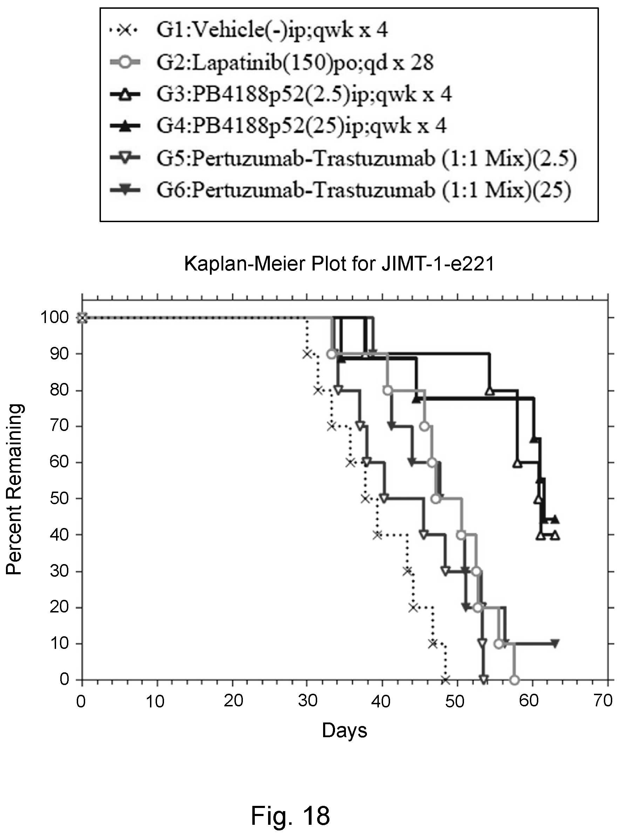

Tumor sizes were measured weekly by caliper measurement. The efficacy study revealed that PB4188 at both dosing schedules was equal effective and more potent than lapatinib or the combination pertuzumab and trastuzumab. The data are shown in Figures 17 and 18.

### PB4188 can overcome HRG mediated resistance

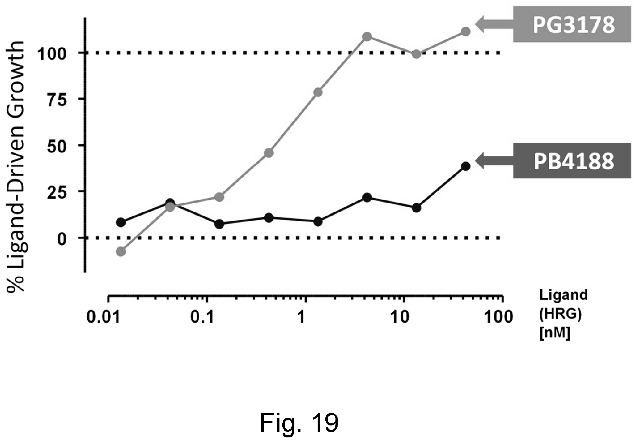

Upregulation of NRG1-β1 is a key resistance mechanism against HER2 targeted therapies (Wilson, 2012). PB4188 was tested in comparison to its parental anti-HER3 monoclonal antibody PG3178 in a serial titration in the presence of an increasing concentration of HRG (NRG1-β1 EGF). To this aim N87 cells were cultured in RPMI 1640 supplemented with 10% heat inactivated FBS. For the proliferation assay subconfluent cell cultures of N87 cells were washed with PBS trypsinized and trypsin was inactivated by adding culture medium. Cells were washed twice in large volumes of assay medium (RPMI 1640 medium containing 0.05% BSA and 10 µg/ml Holo Transferrin). Antibodies were diluted in a semi-log titration ranging from 1 to 0.0001 µg/ml. Cells were added at a density of 10000 cells/well in the presence an increasing concentration of HRG (0.04-39,5 nM). The cells were cultivated for 3 days at 37°C, 5% CO2, in 95% relative humidity. Alamar Blue^{™} (Invitrogen) was added according to the manufacturer's instructions and incubated for 6 hours at 37°C, 5% CO2, in 95% relative humidity in the dark. Fluorescence was measured at 550 nm excitation with 590 nm emission wavelength. PB4188 showed superior activity compared to the parental anti-HER3 monoclonal antibody (Figure 19).

Hence, in case of an escape mechanism, such as for instance upregulation of NRG1-β1, a bispecific antibody according to the invention is preferred.

### Epitope mapping of HER2/HER3 specific IgGs

### Shotgun mutagenesis experiments

Alanine scanning mutagenesis was used to map the epitopes of PG3958 and PG3178 for HER2 and respectively HER3. In the shotgun mutagenesis assay, clones are generated whereby each amino acid residue of the HER2/HER3 extracellular domain (ECD) is substituted for alanine. Next, a cell array was prepared by reverse transfection (patent US2011/0077163A1). Therefore, DNA of each clone was mixed with lipofectamin and the mixture was placed in a dedicated well of a 384 well plate. HEK293T cells were added to each well and expression of protein was measured 24H later. Subsequently, the reactivity of antibodies was measured by immunofluorescent staining leading to binding maps and identification of critical residues for antibody binding. Expression levels of the HER2 and HER3 ECD constructs were verified by FACS analysis using commercially available monoclonal antibodies (R&D mAb 1129 (HER2) and R&D mAb 66223 (HER3)).

### HER2

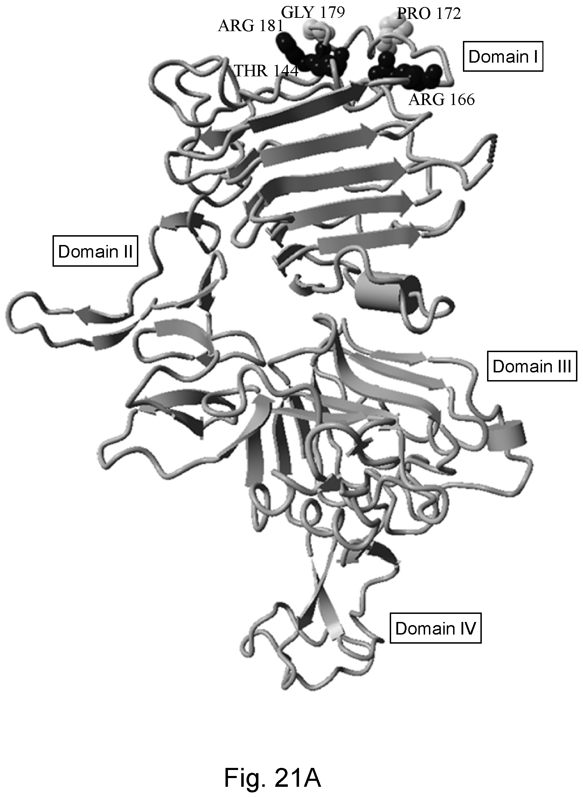

Binding of monovalent PG3958 Fab to HER2 ECD mutants was tested at a concentration of 0.25 µg/ml in the assay and stringent washing conditions were used (pH 9.0, 350 mM NaCl). This resulted in the identification of three 'critical' residues (T144, R166, R181) in HER2 that showed less than 35% residual binding of the PG3958 Fab compared to WT HER2 while retaining control mAb binding. Two residues (P172, G179) that are positioned near the critical residues in the HER2 structure showed significant, but less severe loss of binding and were designated 'secondary critical' residues (Table 13 and Figure 21A). All these surface-exposed residues are located in Domain I of HER2 and together they form a discontinuous patch on the surface of the HER2 molecule.

### Confirmation experiments HER2 epitope

Constructs encoding Wildtype (WT) HER2 ECD and the HER2 ECD variants listed in Table 13 were expressed in CHO-K1 cells. Three Domain I residues that are surface exposed and structurally near the determined critical residues were selected for further analysis. T164, S180 and D143 point mutations to tyrosine were generated in the HER2 ECD construct and the resulting constructs were also expressed in CHO-K1. The L159A HER2 ECD variant was expressed in CHO-K1 cells as control sample.

The bispecific PG3958xTT antibody tested for binding to the ECD variants in a FACS titration experiment. The anti-HER2 antibody trastuzumab which binds domain IV of HER2 was used to verify HER2 ECD expression at the cell surface. Mean MFI values were plotted and for each curve the AUC was calculated using GraphPad Prism 5 software. WT HER2 binding was used to normalize the data. The FACS data showed that in addition to T144A, R166A, R181A, P172A, G179A the mutations T164Y and S180Y resulted in significant reduction in binding of the PG3958xTT antibody (Figure 22). The D143Y mutation resulted in severe loss of expression as demonstrated by the decreased binding of the control mAb, so its potential role in the PG3958 epitope could not be determined.

### HER3

Binding analysis of PG3178 IgG at 0.25 µg/ml to HER3 ECD mutants in FACS resulted in the identification of two so-called 'critical' residues (F409, R426) for which mutation to alanine caused substantial loss of binding compared to WT HER3, while binding of the control mAb was retained (Table 14 and Figure 23). Both residues are located in Domain III of HER3 and spatially distant. Moreover, F409 is buried in the HER3 hydrophobic core, which makes it unlikely to be part of the PG3178 epitope.

### Confirmation experiments HER3 epitope

CHO-K1 cells were transfected with HER3 ECD mutation constructs (listed in Table 14), WT HER3 ECD and two control constructs (H407A and Y424A). PG3178 binding to the HER3 ECD variants was tested in a FACS titration experiment. Two control antibodies, binding Domain I (MM-121) and Domain III (MEHD7945A) of HER3 were included to verify HER3 ECD expression on the cell surface. Mean MFI values were plotted and for each curve the AUC was calculated using GraphPad Prism 5 software. WT HER3 binding was used to normalize the data. The R426A mutation was shown to be critical for PG3178 binding whereas the binding to F409A could not be confirmed due to loss of cell surface expression (Figure 24).

### PB4188 activity on cardiomyocytes in vitro

HER2 is involved in growth, repair, and survival of adult cardiomyocytes as part of a signalling network that involves the heregulin receptor complex HER2:HER4. Cardiotoxicity is a known risk factor in HER2 targeting and the frequency of complications is increased when trastuzumab is used in conjunction with anthracyclines thereby inducing cardiac stress. A model system based on human stem cell derived cardiomyocytes was used to test the potential toxicity of PB4188 and benchmark it against trastuzumab and the combination of trastuzumab and pertuzumab in the presence of the anthracyclin doxorubicin. Human stem cell derived cardiomyocytes (Pluriomics BV) were seeded at a concentration of 20.000 well in white flat-bottom assay plates (corning 655098). On day 5 of culture the medium was replaced for glucose and galactose free culture medium supplemented with 10ng/ml HRG. On day 7 test antibodies were added in combination with doxorubicin (3 µM). Cell viability was assayed on day 9 using the Promega Cell titer Glo assay. The monospecific antibodies were tested at single concentrations of 68 nM whereas PB4188 was tested at three concentrations in the presence of 3 µM doxorubicin. Figure 25 shows that the viability of the cardiomyocyte was unaffected by all PB4188 concentrations tested. In contrast, trastuzumab and the combination of trastuzumab and pertuzumab both reduced cardiomyocyte cell viability.

### PB4188 binding to cells with different HER2 levels

The binding of PB4188 in comparison to trastuzumab and the HER3 antibody U1-59 was analyzed by FACS on breast and gastric cancer cell lines expressing different levels of HER2. Cells were considered HER2+++ if they express millions of HER2 copies and/or are HER2 gene amplified. The following cell lines were used: MCF-7 (HER 2 +); MDA-MB-468 (HER2 +, MKN-45 (HER2 +), MDA-MB-175 (HER2+), MDA-MB-453 (HER2 ++), MDA-MB-361(HER2 ++), ZR-75-1(HER2 ++), JIMT-1 (HER2+++), BT-474 (HER2+++), SKBR-3 (HER2+++), SK-OV-3 (HER2+++), N87 (HER2+++). Cells of an exponentially grown culture were harvested by trypsin and diluted to 10⁶ cells/ml in FACS buffer (PBS/0.5%BSA/0.5mM EDTA). 1-2 10⁵ cells were added to each well in a U-bottom 96 well plate. Cells were centrifuged for 2 minutes at 300 g at 4°C. Supernatant was discarded by inverting plate(s) above, followed by flicking once. 50 µl of each IgG sample was added in a serial dilution from 3.16 ng- 10 µg/ml and incubated for 1H on ice. Cells were centrifuged once, supernatant was removed and cells were washed twice with FACS buffer. 50 µl diluted 1:100 mouse anti human IgG gamma PE (Invitrogen) was added and incubated for 30-60 minutes on ice in the dark. Cells were centrifuged once, supernatant was removed and cells were washed twice with FACS buffer. Cells were analysed on a FACSCanto Flow cytometer in a HTS setting. The quantity of antibody bound was was assessed by median fluorescence. Data were plotted and the area under the curve (AUC, a cumulative measurement of the median fluorescence intensity) was determined for each antibody per cell line tested (Figure 26).

From this experiment it is concluded that PB4188 has a higher binding affinity for HER2+++ cells, HER++ cells and HER+ cells as compared to trastuzumab.

### Simultaneous binding with trastuzumab

### PB4188 and trastuzumab do not compete for binding to HER2

PB4188 binds domain I of the HER2 protein whereas the binding epitope of trastuzumab is localized in domain IV. To demonstrate that both antibodies do not compete for HER2 binding, a binding assay with HER2 amplified SKBR-3 breast cells was performed. First unlabeled antibody was allowed to bind SKBR-3 at saturating concentrations. Next FITC-labeled PB4188 was added in a titration range and fluorescence was measured by FACS. Figure 27 demonstrates that PB4188^{FITC} bound as effectively to cells in the presence of trastuzumab or the negative control. Pre-incubation of SKBR-3 cells with PB4188 prevented PB4188^{FITC} from binding. Thus, trastuzumab and PB4188 do not compete for binding to HER2

### Targeting domain I of HER2 by a HER2xHER3 bispecific molecule can overcome Heregulin resistance

To test whether the orientation of PB4188 on the HER2xHER3 dimer was preferred for inhibiting cell proliferation under HRG stress conditions, bispecific antibodies were generated composed of the 3178 HER3 arm and HER2 arms targeting either domain I, II, III or IV. Two HER2xHER3 bispecific antibodies were generated for each of the HER2 domains I-IV. The HER2 arms included: MF3958 and MF3003 targeting domain I; MF2889 and MF2913 targeting domain II; MF1847 and MF3001 targeting domain III and MF1849 and MF1898 targeting domain IV. Each HER2 Fab arm was combined with the 3178 HER3 Fab arm and tested for their potency to inhibit cell proliferation in the presence of high concentrations of heregulin. Antibody titrations were performed on HER2 low expressing MCF-7 cells and the HER2 overexpresssing N87 and SK-BR-3 cells. Subconfluent cell cultures of N87, SK-BR-3, and MCF-7 cells were washed with PBS trypsinized and trypsin was inactivated by adding culture medium. Cells were washed twice in large volumes of assay medium (RPMI 1640 medium containing 0.05% BSA and 10 µg/ml Holo Transferrin). Antibodies were diluted in a semi-log titration. Cells were added at a density of 10000 cells/well (N87, SKB-BR-3) and 5000 cells/well MCF-7 in the presence the experimentally defined stress concentration of HRG (10nM SK-BR-3, 100nM N87 and MCF-7). The cells were cultivated for 3 - 4 days at 37°C, 5% CO2, in 95% relative humidity. Alamar BlueTM (Invitrogen) was added to assess the proliferation. Absorbance was measured at 550nm excitation with 590 nm emission wave length. In all assays tested, only the bispecific antibodies targeting domain I of HER2 were able to inhibit proliferation in the presence of a high heregulin concentration (Figure 28).

### Drug combinations with PB4188 in vitro.

To investigate the possibility to combine PB4188 with small molecule drugs PB4188 was combined with drugs interfering at different levels of the PI3K or MAPK pathway. Moreover, combination with chemotherapeutic drugs and cyclin inhibitors were tested. Combinations were tested on HER2 overexpressing cells growing in the presence of HRG in matrigel (SK-BR-3 and BT-474) or in the presence of HRG stress concentrations (N87 and SK-BR-3 as described in proliferation assays). The inhibitory effect of drug combinations was tested by imaging or by measuring proliferation using Alamar Blue as described herein before. First, the EC20 PB4188 and drugs tested was determined. Next, checkerboard titrations were performed with PB4188 and the drugs. Synergies were observed in all cell lines tested with tyrosine kinase inhibitors (afatinib, lapatinib, neratinib), the PI3Ka inhibitor BYL719, the Akt inhibitor MK-2206, the mTOR inhibitor everolimus, the Src inhibitor saracatinib, the microtubuli disrupting drug paclitaxel, and the HDAC inhibitor vorinostat (which is misspelled in Figure 40 as "voronistat"). Figure 29 shows an example of the synergistic combination of PB4188 with lapatinib on SKBR-3 cells grown in matrigel resulting in morphological changes and reduction of cell growth. The extent of growth inhibition obtained with each combination was calculated. Potency shifting can be shown using isobolograms (Greco et al 1995) which shows how much less drug is required in a combination to achieve a desired level when compared to the single agent required to reach that effect. The inhibition values of the combination experiments were used by CHALICE^{™} Analyzer software to generate the isobolograms. Isobolograms of the different drug combinations on HER2 amplified cells are shown in figure 40. Isobologram analysis indicated that PB4188 displayed synergistic drug combinations with afatinib, lapatinib, neratinib, BYL719 , MK-2206, everolimus, saracatinib, vorinostat and paclitaxel.

These data demonstrate that drugs acting on the PI3K pathway are particular effective in combination with PB4188. In addition, combinations with Tyrosine Kinase Inhibitors are effective. Moreover, a combination with the growth and migration/invasion drug saracatinib can be favourable in the metastatic setting.

### PB4188 In vitro inhibition of phosphorylation

Cells of an exponentially grown culture were harvested and seeded in 6 well plates (3.75 x10⁶ cells for N87 and 1.5x10⁶ cells for SKBR-3) in starvation medium (N87 cells: RPMI-1640, 0.05% BSA, 10µg/ml Holo-transferrin; SKBR-3 cells: DMEM/F-12, 2 mM L-glutamine, 0.05% BSA, 10µg/ml Holo-transferrin) and incubated incubated overnight at 37°C, 5% CO2, in 95% relative humidity. The next day, antibodies were added to a final concentration of 5 nM and cells were incubated for one hour at 37°C, 5% CO2, in 95% relative humidity. HRG was then added to a final concentration of 100 ng/ml. After 1, 3, 6 or 24 hours at 37°C, 5% CO2, in 95% relative humidity, plates were placed on ice, cells were washed twice with cold PBS. Subsequently 0.3 ml ice-cold lysis buffer was added (Cell signaling RTK # 9803 or IC # 7018) and cells were lysed for a minimum of 30 minutes on ice. Next, protein concentrations were measured using BCA (Pierce #23235). Protein concentrations were adjusted to 2 mg/ml with lysis buffer. Next, lysates were applied to PathScan RTK Signaling Antibody Arrays (Cell signaling #7949) or PathScan Intracellular Signaling Antibody Arrays. All incubations were performed with sealed wells on an orbital shaker at room temperature. Lysates (75 µl) were diluted 2 times to 0.8 mg/ml concentration with 75 µl Array Diluent Buffer supplemented with protease inhibitor cocktail and kept on ice. Array wells were blocked with 100 µl Array block buffer for 15 minutes. Block buffer was removed and Lysates were applied to the wells and allowed to incubate for 2 hours. Lysate was aspirated and wells were washed 4 times with 100 µl Wash buffer. Next, 100 µl detection antibody cocktail was added per well and incubated for 1 hour. Antibody cocktail was aspirated and wells were washed 4 times with 100 µl Wash buffer. 75 µl Dylight80^{™} Streptavidin was added to each well. Dylight80^{™} Streptavidin was aspirated and wells were washed 4 times with 100 µl Wash buffer. The multi-gasket was removed and slides were washed for 10 seconds in 10 ml in deionized water. Slides were allowed to dry and processed for imaging on an Odysee^{®}Clx. Spot fluorescence intensity was calculated using Image Studio software.

In N87 and SKBR-3, PB4188 completely blocks AKT phosphorylation during the first 6H of incubation, in contrast to the combination of trastuzumab + pertuzumab. In addition a strong inhibition is observed in ERK and S6 phosphorylation in contrast to the combination of trastuzumab + pertuzumab. PB4188 does not inhibit phosphorylation of HER2 (Figure 30)

### Western blot analyses

To verify the phosphorylation inhibition observed in the RTK and intracellular Pathscan arrays Western blots were performed of cells treated with PB4188, the combination pertuzumab and trastuzumab and a control antibody in the presence of HRG stress concentrations. Cells of an exponentially grown culture were harvested and seeded in 10 cm dishes (20x10⁶ cells for N87 and 7x10⁶ cells for SKBR-3) in starvation medium (N87 cells: RPMI-1640, 0.05% BSA, 10µg/ml Holo-transferrin; SKBR-3 cells: DMEM/F-12, 2 mM L-glutamine, 0.05% BSA, 10µg/ml Holo-transferrin). The next day, antibodies were added to a final concentration of 5 nM and cells were incubated for one hour. HRG was then added to a final concentration of 100 ng/ml. After 1, 3, 6 or 24 hours, dishes were placed on ice, cells were washed twice with cold PBS, transferred to Eppendorf tubes and lysed with 250 µl of RIPA lysis buffer (20 mM Tris-HCl pH 7.5, 150 mM NaCl, 1 mM Na2EDTA, 1 mM EGTA, 1% NP-40, 1% sodium deoxycholate, 0.1% SDS, 2.5 mM sodium pyrophosphate, 1 mM beta-glycerophosphate, 1 mM Na3VO4, 1 µg/ml leupeptin). Lysis was allowed to proceed for 30 minutes on ice. Cell lysates were centrifuged and supernatants were collected in new Eppendorf tubes. Protein concentration was determined using the BCA method (Pierce). 30 µg of the lysate was separated on a 4-12 % Bis-Tris NuPage gel (Invitrogen) and proteins on the gel were transferred to a nitrocellulose membrane. Membranes were blocked for one hour with TBS-T containing 5 % BSA and stained with the indicated antibodies according to the manufacturer's instructions (Cell Signaling Technology). Membranes were then incubated with a HRP-conjugated secondary antibody, incubated with ECL substrate and subjected to autoradiography using X-ray films (Amersham). All detection antibodies were from Cell Signaling Technology: Phospho-Akt (ser 473) #4060, Total Akt #4691, Phospho-HER2 (Tyr 1221/1222) #2243, Total HER2 #2242, Phospho-HER3 (Tyr 1289) #4791, Total HER3 #4754, Phospho-ERK1/2 (Thr 202/Tyr 204) #4377, Total ERK1/2 #4695, Phospho-S6 RP (Ser 235/236) #2211, Total S6 RP #2217, Goat anti- rabbit HRP-linked #7074.

The results show that PB4188 shows a prolonged inhibition of HER3 phosphorylation resulting in the inhibition of both the MAPK and PI3 kinase pathway with a profound effect on AKT phosphorylation inhibition (Figure 31).

### PB4188 In vivo pharmacodynamics

### Phosphoprotein analysis by Luminex

Tumors (100mm³) of JIMT-1 transplanted mice treated with 2 doses of PB4188 and 4 doses of PB4188 were removed 24H after dosing. Tumors were flash-frozen and processed to powder. Tumor lysates were prepared to a concentration of 50 mg tumor/mL using cold BioRad Lysis Buffer (supplemented with 0.4% BioRad Factor 1, 0.2% BioRad Factor 2, and 2 mM PMSF) to the frozen powder samples, incubated at 4°C on a rocker for 60 minutes to ensure complete lysis. The samples were centrifuged at 4°C for 10 minutes at 16000 x g, and aliquoted. Total protein was determined using the Biorad DC Protein Assay reagents according to manufacturer's instructions. Luminex Assay: The JIMT-1 tumor lysate samples were processed and analyzed for: Total AKT AKT(Ser473) and AKT(Thr308using commercially available Luminex kits from Millipore (Cat # 48-618MAG (Lot No. 2532050), 46-645MAG (Lot No. 46645M-1K). Each sample was tested in duplicate. Dilutions were prepared in sample diluent to load a target of approximately 25 µg protein per well for all total and phosphorylated analyte determinations. The Millipore kits were used according to the manufacturer's specifications.

Tumors treated with PB4188 showed an increase in Akt expression in comparison to untreated tumors. Phosphorylation of AKT was completely inhibited by PB4188 both after a two-weekly dose as after a four-weekly dose (Figure 32).

### Phosphoprotein analysis by VeraTag assay

Tumors (100mm³ or 400 mm³) of JIMT-1 transplanted mice treated with 1 or 2 doses doses of PB4188 were removed and fixed in 10% neutral buffered formalin. Mice bearing 100mm³ tumors were sacrificed 24H after a single PB4188 dose (25 mg/kg) whereas mice bearing 400mm³ tumors received 2 weekly dosis of 25 mg/kg and were sacrificed 4H after dosing. Next, samples were paraffin-embedded. Sections of 7 um in thickness were sliced with a microtome (LEICA) and placed on positively charged glass slides (VWR) with serial number labeled. Slides were air-dried for 30 min and then baked in a heated oven set at 60° C. Next samples were processed for different VeraTag analysis. Total HER2 analysis (HT2) according to U.S. Patent Appl. No. 12/340,436, total HER3 analysis (H3T) according to U.S. Patent No. 8,349,574; U.S. Patent Appl. No. 2013/0071859 and finally HER2-HER3 heterodimer (H23D), HER3pY1289 (H3pY1289) and HER3-PI3 kinase (H3PI3K) according to Int'l Patent Appl. No. PCT/US2014/033208. In both dosing regimens a significant PB4188 mediated reduction in HER2:HER3 dimers became apparent in comparison to untreated controls. There was no difference observed in total HER2, HER3 or phosphorylated HER3 between PB4188 treated tumors and controls. Tumors that were analyzed 4H after PB4188 dosing showed a significant reduction in HER3-p85 (PI3K) compared with untreated controls.

### PB4188 reduces cell cycle progression in HRG-stimulated cancer cells

The ability of PB4188 to influence cell cycle progression was investigated in cancer cell lines expressing various protein levels of HER2. HER2+ (MCF-7), HER2+++ (JIMT-1, SK-BR-3 and N87 cells) cells were seeded in assay medium (MCF-7 cells: RPMI-1640, 0.05 % BSA, 10 µg/ml Holo-transferrin, 1 mM sodium pyruvate, MEM NEAA; JIMT-1: DMEM, 0.05 % BSA, 10 µg/ml Holo-transferrin; SK-BR-3 cells: DMEM/F-12, 2 mM L-glutamine, 0.05 % BSA, 10 µg/ml Holo-transferrin; N87 cells: RPMI-1640, 0.05 % BSA, 10 µg/ml Holo-transferrin). Per well of 24-well plate, 300.000 MCF-7, or 400.000 N87 or 150.000 SK-BR-3 or 150.000 JIMT-1 or cells seeded in 1 ml assay medium and incubated overnight at 37°C, 5% CO2, in 95% relative humidity. The next day, PB4188 or pertuzumab + trastuzumab or PG3178 or PG1337 were added to the cells in the presence of a final concentration of HRG of 1 or 100 ng/ml. After 24 hrs (for JIMT-1, N87 or SK-BR-3 cells) or 48 hrs (for MCF-7 cells) incubation at 37°C, 5% CO2, in 95% relative humidity, cells were supplemented with EdU (10 µM final concentration) for 2 hrs before being harvested and stained for EdU incorporation using the Click-iT EdU AlexaFluor488 kit according to the manufacturer instructions (LifeTechnologies, cat.no. C10425). At least 30 min before analyzing the cells by flow cytometry on FACSCanto, cells were incubated with 200 nM FxCycle far red dye (LifeTechnologies, cat.no. F10348) and 100 µg/ml RNAse A (LifeTechnologies, cat.no. 12091-039). Events were acquired in the AlexFluor488 channel (for EdU detection) and in the APC channel (for total DNA stain with the FxCycle dye). Data were analyzed by gating single cells on a FSC-width vs FSC-height scatter plot, and subgating the G0/G1, S and G2M phases of the cell cycle on an APC vs AlexaFluor488 scatter plot, as EdU^{neg}APC^{low}, EdU^{pos} and EdU^{neg}APC^{high} populations, respectively.

Data are represented as the proliferation index calculated by dividing the percentage of cells in the S and G2/M phases by the percentage of cells in the G0/G1 phase. Figure 34 shows that PB4188 is consistently more potent than PG3178 or pertuzumab + trastuzumab in inhibiting proliferation induced by a standard (1 ng/ml) or a high (100 ng/ml) concentration of HRG. At high concentrations of HRG PB4188 still inhibits the cell cycle progression.

### PB4188 induces receptor internalization

Internalization pattern of antibodies was measured using pH-sensitive dyes. This has been described in the art in WO2013134686 A1 where such dyes, when coupled to an antibody, display an increased fluorescence signal when exposed to lower pH. This occurs when the dye-coupled antibodies internalize from the surface of target cells into mildly acidic endosomes (pH 6-6.5) to acidic lysosomes (pH lower than 5.5). To investigate whether PB4188 internalizes in cancer cells, the antibody was coupled to the pH sensor dye with succinimidyl ester reactive group (Promega, cat.no. CS1783A01) according to the manufacturer's instructions. As comparators, anti-HER2 (trastuzumab, pertuzumab, PG3958), anti-HER3 (PG3178, #Ab6) and negative control (anti-tetanus toxin, PG1337) dye labeled antibodies were included. HER2-overexpressing SKBR-3 and N87 cancer cells of an exponentially grown culture were harvested and seeded on 96 well plates (15x10³ cells per well) in 100 µl assay medium (N87 cells: RPMI-1640, 0.05 % BSA, 10 µg/ml Holo-transferrin; SKBR-3 cells: DMEM/F-12, 2 mM L-glutamine, 0.05 % BSA, 10 µg/ml Holo-transferrin) containing 1 ng/ml HRG and incubated overnight at 37°C, 5% CO2, in 95% relative humidity. The next day, 20 µl pH-sensitive dye-labelled antibodies were added to reach a final concentration of 100 nM and cells were incubated overnight at 37°C, 5% CO2, in 95% relative humidity. The next day, cells were harvested by collecting non-adherent cells and trypsinizing adherent cells. After washing cells with FACS buffer (PBS 0.5% BSA 0.1% sodium azide), cells were stained with APC-labelled anti-human IgG (Jackson Immunoresearch, cat.no. 109-136-098, 1:100 dilution). Cells were analyzed by flow cytometry on FACSCanto (BD Biosciences) measuring median fluorescence intensities (MFI) of the PE and APC channels to determine internalization and residual surface binding of antibodies, respectively. Data shown in Figure 35 show that PB4188 internalizes to the same extend as trastuzumab whereas the combination trastuzumab + pertuzumab leads to enhanced internalization. The combination of trastuzumab + pertuzumab reduces the ADCC in comparison to trastuzumab alone (Figure 36). It is therefore anticipated that the level of PB4188 internalization leaves the ADCC potency unaffected.

### Generation and characterization of anti-HER3 antibody 3178 variants

Variants of anti-HER3 antibody MF3178 were designed with the aim to improve antibody properties. Mutations were introduced in the VH gene framework region 1 (FR1), complementarity determining region 1 (CDR1), FR2, CDR2 and/or FR3, while CDR3 and FR4 were not modified. The design included, but was not limited to, mutations that were introduced to remove post-translational modification (PTM) motifs (e.g. by changing the deamidation motif NS to NQ), to reduce surface hydrophobicity (e.g. by changing I to T) or to increase the iso-electric point (pI; e.g. by changing Q to K). All 20 variants (See Figure 37) were expressed as bispecific antibody combined with a Tetanus Toxoid (TT) arm and tested in the MCF-7 functional assay and all 20 variants had a similar potency as the MF3178 antibody in this format. All 20 variants were also tested in this format in FACS in a titration for binding to MCF-7 and all variants had very similar binding profiles suggesting that the affinities of all variants are similar. Three lead variants MF6058, MF6061 and MF6065 were selected for further experiments that contain ten, three and seven amino acid mutations, respectively (see sequences in Figure 16E and Figure 37). The corresponding monospecific IgG1 PG6058, PG6061 and PG6065 were produced and purified at large scale. As shown in Figure 38, the inhibitory activity of the three variants in the HRG-dependent N87 cell line proliferation assay is similar to that of PG3178. The CIEX-HPLC profile of the three variants was similar to that of PG3178 with respect to charge heterogeneity as well as peak width and symmetry, as shown in Figure 39. The retention time (tR) of the main peak correlated roughly with the pI of the antibodies, i.e. higher pI resulted in longer retention time. In the design of bispecific antibodies or mixtures of antibodies, selecting antibody variants with optimal tR is valuable since purification of the desired antibody components using CIEX can be facilitated.

### Example 2

The efficacy of the bispecific antibody MCLA-128 directed against HER2 and HER3 in mice with intracranial PDX tumors was determined. The efficacy of MCLA-128 was compared to T-DM1. In addition, the combination of MCLA-128 and T-DM1was compared to single agent treatments.

### Animals

The study was performed in 43 (incl. 11 spare animals) female NMRI nude mice (ordered age-matched with a one-week time frame, approximately 6 weeks of age) of the stock from Janvier Labs, France.

### Animal housing and handling

### Health monitoring:

The mice were clinically examined at arrival at the Department of Experimental Medicine, Building 10.3, University of Copenhagen according to the Animal Unit Standard Procedures. Educated personnel under veterinary supervision handled the mice. All animals were healthy and no decisions concerning the welfare were made.

### Acclimatization:

An acclimatization period of 14 days was allowed, before start of experimental procedures.

### Housing and environment:

Animals were housed in an animal room/lab. The room was illuminated to give a cycle of12 hours light and 12 hours darkness. Light was on from 06:00 h to 18:00 h. Mice were housed in IVC Type III cages, Techniplast (820 cm2, height 15.5 cm, maximum 8 / minimum 2 mice per cage). Animals were monitored by animal technicians daily, whereas veterinarians monitor the animal facility every other month or upon request from the technicians. Each cage was labeled with at least study ID, group and animal numbers and test compound. Cages were equipped with a disposable plastic insert after intracranial tumor implantation.

### Bedding:

The bedding was Aspen wood, from Brogården/Finn Tapvei Oy, FIN-73620 Kortteinen, Finland. The bedding was changed every other week.

### Environmental enrichment:

The animals were offered a supply of nesting material, Brogården, at each change of bedding. Furthermore, each cage contained wooden sticks from Brogården / Finn TapveiOy, FIN-73620 Kortteinen, Finland and custom made transparent red plastic hiding.

### Diet and drinking water:

A pelleted complete diet "Altromin 1319", a maintenance diet for rats and mice, was available ad libitum and changed every 14 day. The animals had free access to tap water changed weekly. Drinking water was supplemented with estrogen after intra cranial tumor implantation.

### Humane endpoints

Animals were euthanized for humane reasons. Humane reasons for terminating an animal included, but are not limited to, cases where the animals show signs of permanent suffering, pain or fear. Specific humane endpoints for the study are governed by a scoring system listed in Table 16. When indicated the mice were euthanized by cervical dislocation.

### Methods

A time-line for the study is presented in Table 17.

### Intracranial tumor implantation

A subcutaneous ST1360B PDX tumor (4^{th} passage) grown in a NMRI nude mouse was harvested, please see Figure 41 for tumor growth curve. Mice carrying ST1360B tumors were supplemented with estrogen following tumor implantation. The tumor was washed with PBS and trimmed for residual connective tissues at the surface. The tumor was cut into small pieces and digested with Accutase and Collagenase IV to yield a suspension of single cells. The digestion was stopped by addition of media containing fetal bovine serum and the cell suspension was filtered through a 100 µm filter, washed in PBS and resuspended in PBS. The viability of the tumor cells was checked by trypan blue staining and the final concentration was 18 million viable cells/mL. The viability of total cells was greater than 80%. There was no differentiation between stromal or tumor cells and the solutions do also contain some cell debris. The ST1360B demonstrate a high tumor cellularity. The cells were kept on ice until inoculation.

Mice were anaesthetized by hypnorm/midazolam (1 ml/100 g body weight) and placed in a stereotactic frame for fixation of the head. A longitudinal incision was made in the scalp exposing the *calvarium.* A hole was drilled in the skull 1.5 mm right of the *sutura saggitalis* and 0.5 mm posterior to the *bregma* using a microdrill. Ten µl of the cell suspension (180.000 cells) was injected at a depth of 2-2.5 mm at a rate of 60 nl/sec using a 100 µl syringe with a 25-gauge needle placed in a micro infusion pump. The needle was left for 3 minutes before being withdrawn. Bupivacaine (0.2 mg/100 g bodyweight) and Lidocaine (1 mg/100 g body weight) were administrated in the incision site for local anesthetic and the skin was closed with a suture. The mice were ear punched for identification and returned to their cages where they were monitored until fully recovered from the anesthesia. The mice were monitored at least twice per week (weight and clinical signs) after tumor inoculation and more often if clinical signs or weight loss was present.

### MR imaging

Tumor development was monitored bi-weekly by T2-weighted MR imaging (axial and coronal planes). The first imaging session was 19 days after tumor inoculation. The animals were anesthetized during the MR imaging sessions (sevoflurane, 2-4% in ambient air supplemented with 100% O2 at approximately 4:1 ratio). Enrolment into study was based on two pathological MR scans showing tumor growth and a tumor volume of about 10-20 mm³. Mice that meet the enrolment criteria were randomized into one of four groups. The first 32 mice that meet the inclusion criteria were enrolled into the study. Mice were randomized so all groups presented with the same mean tumor volume at treatment initiation.

### Therapy

Mice were dosed with either, vehicle, MCLA-128, T-DM1 or MCLA-128 + T-DM1 according to Table 18. Drugs were diluted in sterile saline before each dosing. Mice were placed under a heating lamp for approximately 5-10 minutes before injection of the test compound to make the procedure as quick and easy as possible. The mouse was placed on a tail restrain box and the test compound was dosed in the lateral tail vein as a single intravenous (i.v.) bolus dose. The dosing volume was 5.0mL/kg.

### Post therapy MR imaging and weight monitoring

Tumor growth were monitored bi-weekly by T2-weighted MR imaging (axial and coronal planes) for the first two weeks after therapy initiation and weekly until 6 weeks after therapy. The animals were anesthetized during the MR imaging sessions (sevoflurane, 2-4% in ambient air supplemented with 100% O2 at approximately 4:1 ratio).

Animals were euthanized by cervical dislocation on an individual basis due to humane endpoints, Table 16. The brain with tumor was resected and preserved in 4% formaldehyde for 24-48 hours and transferred to 70% ethanol. The fixative: tissue ratio was at least 20:1.

### Image analysis

Tumor volumes were measured on the images by drawing region of interests (ROIs) on the individual slices and calculating the volume of the ROIs. ROIs were drawn on both the axial and coronal slices and the average of the tumor volume in the two planes was used as tumor volume. Edema in the brain was manually scored on a scale from 0-4, where the score 0 was no brain edema and the score 4 indicates massive brain edema, see Figure 42. Image analysis was performed using Horos. (Horos Project (2017). DICOM image viewing and measuring. [Horos]. http://www.horosproject.org/).

### Results

### Inclusion and randomization

The first animals were included into the study 23 days after intracranial tumor implantation. The dates of inclusion and inclusion tumor volume for all animals are listed in table 19. The weight and tumor volume of mice in group A-D at inclusion are shown in Figure 43. No difference in body weight or tumor volume was seen between the groups (one-way ANOVA, p = 0.43 (weight) and p= 0.92 (tumor volume).Figure 43: Body weight and tumor volume at inclusion of mice in group AD. No difference in weight or tumor volume was seen between the groups(one-way ANOVA, p = 0.43 (body weight) and p = 0.92 (tumor volume).

### Post therapy monitoring

T2-weighted MRI was performed on day 3, 7, 10, 14, 21, 28, 35 and 42 post initiation of therapy to measure intra cranial tumor volume. The mean tumor volume post initiation of therapy for each group is depicted in Figure 44. Individual tumor volumes for animals in each group are shown in Figure 46-49. Representative T2-weighted images of mice are shown in figures 50-53. Tumor growth was inhibited in mice treated with T-DM1 and T-DM1 + MCLA-128,whereas a tumor growth delay was observed in mice treated with MCLA-128 compared to vehicle treated mice. There was a significant difference in tumor volume 10 days post therapy (p=0.009, one-way ANOVA), and the mean tumor volume of T-DM1 and T-DM1+ MCLA-128 treated mice was significantly smaller compared to vehicle treated mice(p<0.05, corrected for multiple comparisons; Tukey).

The weight of the mice was closely monitored post initiation of therapy. The mean weight of mice in different groups is shown in Figure 45. Weight measurements for each mouse in the different groups are shown in Figure 54-57 both in grams and as percentage change relative to the weight at inclusion. It is evident from the individual weight measurements that the majority of mice lost weight before the humane end-points were met.

### Scoring of brain edema

Tumor stasis (presence of tumor without growth) was seen for animals in groups B and D. Evaluation of the MR images closest to the time of sacrifice showed that some animals presented brain edema. This could contribute to the deteriorating condition of the mice and the necessity for euthanasia. Edema in the brain was manually scored on a scale from 0-4, 0 indicating no brain edema and 4 indicating massive brain edema (Table 20 and Figure 58).There was a tendency towards increased edema in the groups treated with T-DM1 (group B and D). However, no significant difference in brain edema between the groups was found (non-parametric Kruskal-Wallis test). Precautions should be taken when interpreting the results, as the edema score was not performed at the same time-point or at the time of euthanasia. Also, the tumor volumes were different between the groups, which could also influence the brain edema. As such, the study was not designed to investigate the influence of treatment and brain edema in detail.

### Survival analysis

Mice were euthanized due to humane endpoints according to Table 16. Despite thorough monitoring, four mice were found dead in the cages during the study. No ex vivo tumor material was preserved from animals that were found dead in the cage. Kaplan-Meier plot of survival data for all groups are depicted in Figure 59. The survival curves were significantly different (p<0.0001, Log-rank). The median survival for vehicle, T-DM1, MCLA-128 and T-DM1 + MCLA-128 animals was 13, 19.5, 29 and 42 days post therapy initiation respectively. Pair-wise Kaplan-Meier plots are depicted in Figure 60. A significant (Log-rank) longer median survival was observed for T-DM1 vs. vehicle (19.5vs. 13 days, p=0.020), MCLA-128 vs. vehicle (29 vs. 13 days, p<0.0001), and T-DM1+MCLA-128 vs. vehicle (42 vs. 13 days, p<0.0001). No difference in median survival was seen for MCLA-128 vs. T-DM1 (29 vs. 19.5 days, p=0.10, Log-rank). Mice treated with T-DM1 + MCLA-128 had a significant (Log-rank) longer median survival compared to mice treated with T-DM1 (42 vs. 19.5 days, p=0.0005) or MCLA-128 (42 vs. 29 days, p=0.013).

### Discussion

T-DM1 and T-DM1 + MCLA-128 inhibited tumor growth, whereas MCLA-128 showed tumor growth delay determined by T2-weighted MRI. The median survival for vehicle, T-DM1, MCLA-128 and T-DM1 + MCLA-128 treated mice was 13, 19.5, 29 and 42 days post therapy initiation, respectively. Mice treated with MCLA-128 had a significantly longer survival compared to vehicle treated animals (29 vs. 13 days, p<0.0001), and mice treated with T-DM1 + MCLA-128 had a significantly longer median survival compared to mice treated with T-DM1 (42 vs. 19.5 days, p=0.0005) or MCLA-128 (42 vs. 29 days, p=0.013). A tendency towards increased edema in the groups treated with T-DM1 (group B and D) was observed. However, no significant difference in brain edema between the groups was found (non-parametric Kruskal-Wallis test). In conclusion, MCLA-128 showed efficacy on survival of mice with intracranial ST1360BPDX tumors both as a single agent and in combination with T-DM1.

### Example 3: Phase II study of MCLA-128-based combinations in metastatic breast cancer (MBC): MCLA-128/trastuzumab/chemotherapy in HER2-positive MBC

While the Example describes the administration of MCLA-128/trastuzumab/chemotherapy, the Example is not intended to be limiting to the use of this specific therapeutic agents set out, and applies to the disclosed ErbB-2 and ErbB-3 binding bispecific antibodies in combination with a ErbB-2 binding agent, including an inhibitory agent, and chemotherapy.

### OBJECTIVES

### HER2-positive/amplified MBC): MCLA-128 + trastuzumab ± vinorelbine

### Primary objective:

- Evaluate efficacy of MCLA-128 combined with trastuzumab ± vinorelbine in terms of clinical benefit rate (CBR) at 24 weeks based on RECIST 1.1 (per investigator review) in HER2-positive/amplified MBC patients who have progressed on prior HER2-directed therapy that included trastuzumab with pertuzumab, and an HER2 antibody drug conjugate (ADC)

### Secondary objectives:

- Evaluate CBR at 24 weeks based on RECIST 1.1 per central review
- Evaluate progression-free survival (PFS; per investigator and central review)
- Evaluate overall response rate (ORR) based on RECIST 1.1 (per investigator and central review).
- Evaluate duration of response (DoR) based on RECIST v1.1 (per investigator and central review)
- Evaluate overall survival (OS)
- Evaluate safety and tolerability of MCLA-128 in combination with trastuzumab ± vinorelbine
- Characterize pharmacokinetics (PK) of MCLA-128 in combination with trastuzumab ± vinorelbine
- Characterize immunogenicity of MCLA-128 in combination with trastuzumab

### Exploratory objective:

- Evaluate potential correlations between biomarkers in tumor or blood samples and antitumor activity (including HER2, HER3, HER2:HER3 dimers, heregulin and other potential biomarkers)

### STUDY DESIGN

A phase 2, open-label, multicenter international study is performed to evaluate the efficacy of MCLA-128-based combinations in two metastatic breast cancer (MBC) populations, HER2-positive/amplified. Two combination treatments are evaluated in 15-20 sites in 7 countries in Europe and the USA.

Patients with HER2-positive/amplified MBC, having confirmed HER2 overexpression by immunohistochemistry (IHC) 3+ or IHC 2+ combined with positive fluorescence *in situ* hybridization (FISH), who have progressed per RECIST v1.1 on 2 to 4 lines of HER2-directed therapy in the adjuvant/neoadjuvant, unresectable locally advanced/metastatic setting including trastuzumab with pertuzumab and an HER2 ADC are eligible. For enrollment, HER2 status is based on medical records, and eligibility is confirmed subsequently as soon as possible, by central lab review. Patients found to be ineligible retrospectively are not be evaluable for the primary objective and may be replaced. Documented imaging proof of disease progression on the last prior line of therapy should be made available when possible.

Initially MCLA-128 is administered with trastuzumab (doublet combination). Safety is reviewed by an Independent Data Monitoring Committee (IDMC). After the safety of the doublet has been assessed, MCLA-128 + trastuzumab + vinorelbine (triplet combination) is evaluated in parallel with the doublet combination (see Figure 61).

The doublet and triplet combinations are both evaluated in two steps with an initial safety run-in in 4 to 6 patients who are reviewed by the IDMC, followed by a cohort efficacy expansion, as described below. The triplet combination go/no-go decision is made after evaluation of the doublet safety run-in patients by the IDMC. The efficacy expansion of both combinations continues in parallel.

*Safety run-in:* After 4-6 patients have received at least 2 complete cycles (6 weeks) of MCLA-128 + trastuzumab, a safety review is performed by the IDMC. If the doublet combination is considered safe, the safety run-in for the triplet combination is initiated. Safety of the triplet is evaluated after 4-6 patients have received at least 2 complete cycles (6 weeks) of MCLA-128 + trastuzumab + vinorelbine by the IDMC.

Based on the observed safety in the first 4-6 patients (adverse events [AEs], serious adverse events [SAEs], relationship to study drug, and other clinically relevant parameters [e.g. laboratory parameters], available PK, immunogenicity, and cytokine data) the IDMC, investigators and Sponsor decide on a potential additional run-in period for each combination (i.e. doublet and triplet).

*Expansion:* After the safety run-in, each combination therapy considered tolerable by the IDMC is expanded to a total of up to 40 patients evaluable for efficacy.

### STUDY POPULATION

### Inclusion criteria

Patients must fulfill all of the following requirements to enter the study:
1. Signed informed consent before initiation of any study procedures.
2. Women with histologically or cytologically confirmed breast cancer with evidence of metastatic or locally advanced disease not amenable to any local therapy with curative intent:
   a. Documented HER2 overexpression/amplification, defined as immunohistochemistry (IHC) 3+ positive, or IHC 2+ combined with positive fluorescence *in situ* hybridization (FISH), based on local analysis on the most recent tumor biopsy (preferably metastatic, otherwise primary), either fresh or archival collected within 12 months before screening.
   b. Documented disease progression (by investigator assessment) on 2 to 4 lines of HER2-directed therapy administered in the adjuvant/neoadjuvant, unresectable locally advanced/metastatic setting; trastuzumab plus pertuzumab and an HER2 antibody drug conjugate (e.g. T-DM1) must all have been previously administered (in any sequence).
3. Measurable disease as defined by RECIST version 1.1 by radiologic methods on or after the most recent line of therapy.
4. Age ≥ 18 years at signature of informed consent.
5. Eastern Cooperative Oncology Group (ECOG) performance status of 0 or 1.
6. Life expectancy of ≥ 12 weeks, as per investigator.
7. Left ventricular ejection fraction (LVEF) ≥ 50% by echocardiogram (ECHO) or multiple gated acquisition scan (MUGA).
8. Adequate organ function:
   a. Absolute neutrophil count (ANC) ≥ 1.5 X 10⁹/L
   b. Hemoglobin ≥ 9 g/dL
   c. Platelets ≥ 100 x 10⁹/L
   d. Serum calcium within normal ranges (or corrected with supplements)
   e. Alanine aminotransferase (ALT), aspartate aminotransferase (AST) ≤ 2.5 x upper limit of normal (ULN) and total bilirubin ≤ 1.5 x ULN (in cases of liver involvement, ALT/AST ≤ 5 x ULN and total bilirubin within normal ranges is allowed)
   f. Serum creatinine ≤ 1.5 x ULN or creatinine clearance ≥ 60 mL/min calculated according to the Cockroft and Gault formula or MDRD formula for patients aged > 65 years
   g. Serum albumin > 3.0 g/dL

### INVESTIGATIONAL AND COMPANION THERAPIES

MCLA-128: 750 mg intravenous flat dose over 2 hours, Day 1 every 3 weeks (q3w). Premedication with paracetamol/acetaminophen, antihistamines and corticosteroids (as per standard practices) is mandatory for every MCLA-128 infusion.

Trastuzumab: 8 mg/kg intravenous loading dose over 90 minutes on Day 1 Cycle 1, then from Cycle 2, 6 mg/kg is administered intravenously over 30-90 minutes, on Day 1 of each cycle, q3w. For safety run-in patients, trastuzumab administration is delayed to Day 2 in Cycle 1.

Vinorelbine: 25 mg/m² intravenously over 10 minutes, Days 1 and 8, every 3 weeks. For safety run-in patients, vinorelbine administration is delayed to Days 2 and 9 in Cycle 1.

### TREATMENT REGIMENS

For all combinations a cycle is considered 3 weeks. A 6-hour observation period is implemented following infusion start for the initial MCLA-128 and/or trastuzumab administration, and 2 hours for all subsequent administrations.

### Doublet combination (see Figure 62):

- Safety run-in (4-6 patients): for Cycle 1, MCLA-128 is administered on Day 1, and trastuzumab on Day 2. From Cycle 2, trastuzumab is administered on Day 1, 30 minutes after the completion of the MCLA-128 administration.
- Expansion: for all cycles, MCLA-128 is administered on Day 1 followed by trastuzumab 30-minutes after the end of the MCLA-128 infusion.

### Triplet combination (see Figure 63):

- Safety run-in (4-6 patients): for Cycle 1, MCLA-128 is administered on Day 1 followed 30 minutes later by trastuzumab, and vinorelbine is administered on Days 2 and 9. From Cycle 2, vinorelbine is administered on Day 1, 30 minutes after trastuzumab, and on Day 8.
- Expansion: for all cycles, MCLA-128 is administered on Day 1 followed 30 minutes later by trastuzumab, followed by vinorelbine 30 minutes after the end of the trastuzumab infusion.

For both the doublet and the triplet combinations, if an individual patient does not tolerate all drugs on the same day, the safety run-in Cycle 1 dosing schedule is maintained for that patient.

**Treatment assignment:** the Sponsor alternately assigns eligible patients to the doublet or triplet combination, in the safety run-in or expansion as available per combination.

### Treatment adaptation

- No dose reductions are permitted for MCLA-128 or trastuzumab.
- The vinorelbine dose is decreased or interrupted in cases of decreased neutrophil counts or elevated bilirubin levels, according to the SPC, and discontinued if grade ≥ 2 neurotoxicity (NCI-CTCAE v. 4.03) occurs.
- MCLA-128 infusion is interrupted in the event of an infusion-related reaction (IRR) and must be stopped definitively for severe IRRs. For mild to moderate events the infusion can be resumed at a 50% infusion rate and infusion duration extended to 4 hours.
- MCLA-128 and trastuzumab administration can be delayed for a maximum of 6 weeks between infusions to manage AEs, specifically for clinically significant LVEF decreases, signs of congestive heart failure or persistent grade 2 or grade 3-4 diarrhea.

### Treatment duration

Study treatment is administered until confirmed progressive disease (as per RECIST 1.1), unacceptable toxicity, withdrawal of consent, patient non-compliance, investigator decision (e.g. clinical deterioration), treatment interruption > 6 consecutive weeks, withdrawal of any study drug. Patients are followed up for safety for at least 35 ± 5 days following the last study drug administration and until recovery/stabilization of related toxicities, and for disease progression and survival status for 12 months.

### PROPHYLATCTIC AND CONCOMITANT MEDICATION

### Permitted

- Administration of paracetamol/acetaminophen, antihistamines and corticosteroids is mandatory with every MCLA-128 administration. In the event of an IRR or hypersensitivity, the patient is managed according to local clinical practice, as clinically indicated.
- All medication necessary for the wellbeing of the patient and which is not expected to interfere with evaluation of the study drug, including supportive treatment of symptoms and AEs or standard treatment of concomitant conditions may be given at the investigator's discretion.

### Prohibited

- Concomitant chronic oral corticosteroids (>10 mg/day prednisone equivalent), TNF-alpha inhibitors, anti-T-cell antibodies (due to risk of immunosuppression).
- Any investigational drugs during the study or 4 weeks prior to the first dose of study treatment.
- Systemic anticancer therapy or yellow fever vaccine during the study or within 3 weeks of the first dose of study treatment.
-

### SAFETY / TOLERABILITY ASSESSMENTS

- AEs (CTCAE version 4.03), SAEs
- Lab parameters: hematology, biochemistry, coagulation, urinalysis, cytokines
- ECG, MUGA/ECHO
- Medical history, vital signs, performance status and physical exam
- Concomitant medications
- Dose modifications (reductions, interruptions, delays), discontinuation due to toxicity

### EFFICACY ASSESSMENTS

Tumor assessment is based on CT/MRI with contrast per RECIST 1.1, every 6 weeks after treatment start. Objective responses must be confirmed at least 4 weeks after first observation. Central review of imaging by an independent radiologist(s) is performed for all patients (screening and on-study). Bone scans are performed as clinically indicated for patients with bone metastases at baseline or suspected lesions on study. Tumor markers (CA15-3, CEA, CA27-29) are assessed on Day 1 every cycle.

### BIOMARKERS

Candidate exploratory biomarkers are evaluated in tumor tissue (screening, optional after 12 weeks and EOT) and blood (pre-dose on Day 1 every 4 cycles and End of Treatment).

**Tumor:** HER2, HER3, HER2:HER3 dimerization, downstream signaling proteins (eg PIK3CA), heregulin, phosphorylation of HER2, HER3 and proteins in the MAPK and AKT signaling pathway, expression of inhibitors such as PTEN, mutations in cancer-related genes including HER2 and HER3 signaling, heregulin-gene fusions. **Blood:** Fc 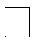 receptor polymorphism, plasma circulating tumor DNA mutations, exploratory serum biomarkers (e.g. soluble HER2, heregulin).

### PHARMACOKINETICS

Blood samples are collected to measure serum MCLA-128 and trastuzumab exposure. No PK sampling is performed for vinorelbine.

PK sampling is performed at the following time points:

### Doublet and triplet combinations: MCLA-128

- Cycle 1: Day 1, pre-dose, EOI, and at 2, 4, and 22 hours post EOI, then at any time on Day 8 (or Day 9 for safety run-in triplet patients)
- Cycle 2: Day 1, pre-dose, EOI (run-in and expansion), and at 2, 4, and 22 hours post EOI, then at any time on Day 8 (run-in only)
- Cycles 3 and 5: Day 1, pre-dose and EOI
- Every 4 cycles thereafter: pre-dose

### Doublet combination: trastuzumab

- Cycle 1 Day 1: pre-dose and EOI (expansion only)
- Cycle 1 Day 2: pre-dose and EOI (run-in only)
- Cycle 2 Day 1: pre-dose and EOI (run-in and expansion)

### Triplet combination: trastuzumab

- Cycles 1 and 2, Day 1: pre-dose and EOI (run-in and expansion)

### IMMUNOGENICITY

Blood samples (5 mL) are collected in all patients to assess serum titers of anti-MCLA-128 antibodies pre-dose on Day 1 pre-dose for Cycles 1, 3, 5, every 4 cycles thereafter, and End of Treatment.

### CYTOKINES

Blood samples are collected to analyze a serum cytokine panel (TNFα, IFNγ, IL-1β, IL-6, IL-8, IL-10) in the safety run-in patients as follows:

### Doublet combination (run-in only):

- Cycle 1: Day 1, pre-dose, 2, 4, and 22 hours post end of infusion (EOI) of MCLA-128
- Cycle 1: Day 2, pre-dose, 2 hours post-EOI of trastuzumab
- Cycle 2: Day 1, pre-dose, 2, 4, and 22 hours post-EOI of MCLA-128

### Triplet combination (run-in only):

Cycles 1 and 2: Day 1, pre-dose, 2, 4, and 22 hours post-EOI of MCLA-128

### STATISTICAL CONSIDERATIONS

### Sample size

Safety run-in: 4 to 6 evaluable patients in the safety run-in has power to detect an AE with a true incidence of 33% is 80 to 90%.

Efficacy expansion: 40 evaluable patients in the doublet or triplet combination have adequate precision to exclude 30% (lower limit of 90% CI > 30%). The threshold for the CBR rate at 24 weeks is defined based on the assumption that PFS follows an exponential distribution with a median of 5 months (clinically relevant) and 3.5 months (not clinically relevant).

The final number of patients depends on the safety and efficacy outcomes during the study. Up to ~130 patients are anticipated, allowing for a total of 40 patients in each of the two planned combination regimens and a ~10% rate of non-evaluable patients.

### Definitions

All efficacy endpoints are defined and analyzed based on tumor assessment by RECIST 1.1.

CBR: the proportion of patients with a best overall response of CR, PR or SD ≥ 24 weeks.

ORR: the proportion of patients with best overall response of CR or PR.

PFS: the time from treatment start until radiologic progression or death due to any cause.

PFS ratio: the ratio of PFS with the previous regimen to PFS on study treatment.

DoR: the time from response (CR or PR) until progression or death due to underlying cancer.

OS: the time from treatment start until death due to any cause.

### Endpoints

### Primary

CBR per investigator radiologic review at 24 weeks

### Key secondary

CBR at 24 weeks per central review, and ORR, PFS, and DoR per investigator and central review

### Other secondary:

*Safety:* Incidence, severity and relationship of AEs, laboratory abnormalities, SAEs, ECG and LVEF measurements and vital signs
*Tolerability:* discontinuations due to AEs, dose modifications due to AEs, immunogenicity, and cytokine assessments
*Other efficacy:* OS
*Pharmacokinetics:* Cₘₐₓ, C₀ₕ, AUC, CL, Vₛₛ, tₘₐₓ and t_{1/2} for MCLA-128, and C_{EOI} and C₀ₕ for trastuzumab.

### Analysis populations

Treated population: patients who receive at least one dose of MCLA-128. Evaluable for efficacy: patients who receive at least 2 complete cycles (6 weeks) of treatment and have undergone baseline assessment and one on-study tumor assessment, or who discontinue early due to disease progression.

### Analyses

Patient disposition and demographics are analyzed in the treated population, efficacy is analyzed in the evaluable for efficacy population, and safety is analyzed in the treated population.

Quantitative variables are summarized using descriptive statistics. Continuous variables are presented as N, mean and/or median, standard deviation, range. Categorical variables are presented using frequencies and percentage.

Criteria for success primary endpoint: A median PFS of 5 months is assumed as relevant, with the activity threshold for CBR at 24 weeks set to 45%.

CBR and ORR are summarized with accompanying 90% exact binomial CI.

For PFS, OS and DoR the survival function is estimated using the Kaplan-Meier product limit method; probability estimates and 90% CI is provided at specified time points; median duration and 90% CI is also be provided. DoR is estimated for responders only.

AEs are tabulated by the Medical Dictionary for Regulatory Activities (MedDRA^{®}) preferred term and by organ class according to incidence and severity. Severity of AEs is based on CTCAE 4.03.

PK, immunogenicity, cytokines and biomarkers are analyzed centrally and reported separately.

### Tables:

**Serum titers of the different cohorts of immunized mice as determined by FACS. D=day of antibody titer determination. Table 1: response against HER2. Table 2: response against HER3. Cell lines used are indicated (MCF7, SKBR3, BT474). The different mice are in the columns**

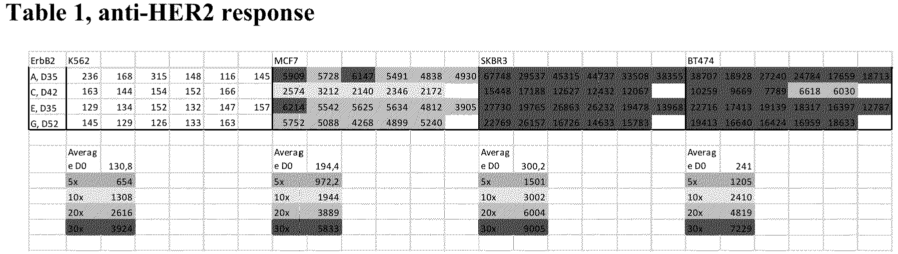

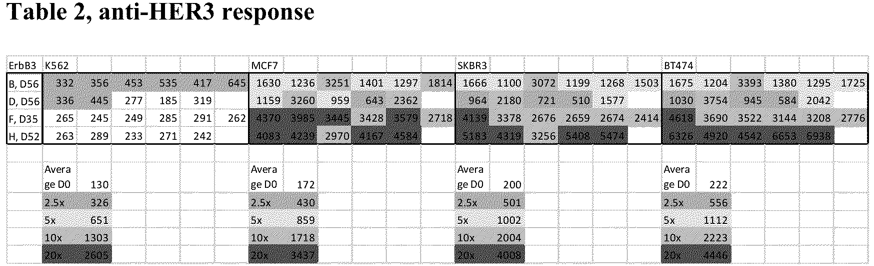

**Table 3**

| Binning of HER2 antibodies depending on their reactivity with chicken-human-HER2 chimera's and reactivity to mouse HER2. 'Number' indicates the number of unique antibodies in each group | | |
|---|---|---|
| **Group** | **Domain reactivity** | **Number** |
| 1 | Domain I specific | 25 |
| 2 | Domain II specific | 2 |
| 3 | Domain III specific | 23 |
| 4 | Domain IV specific | 7 |
| 5 | Domain IV specific + murine cross-reactive | 2 |
| 6 | Reactive to all constructs | 2 |
| 7 | Human WT reactive only | 4 |

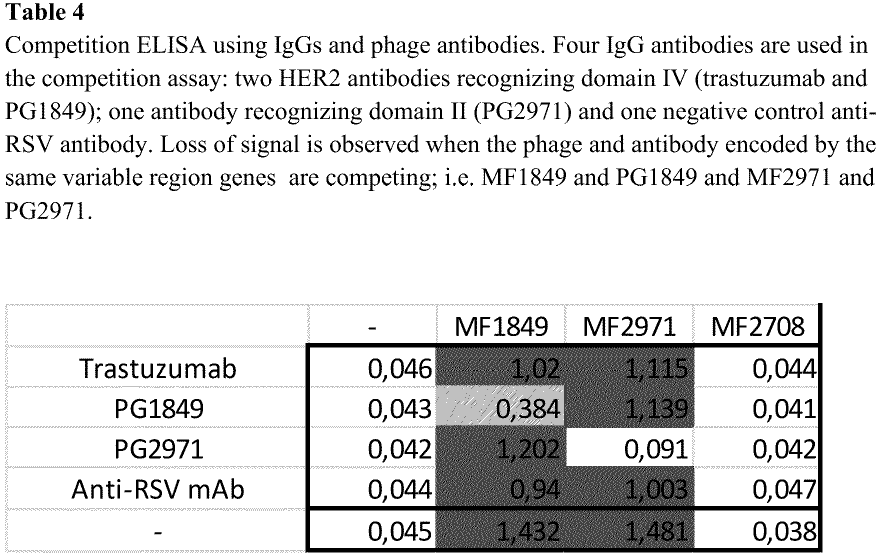

**Table 5**

| Binning of HER3 antibodies depending on their reactivity with rat-human-HER2 chimera's and reactivity to HER3 and HER3 of other species. 'Number' indicates the number of unique antibodies in each group | | |
|---|---|---|
| **Group** | **Reactivity** | **Number** |
| 1 | High Domain III reactivity, rat and mouse reactive and minor reactivity to domain IV | 8 |
| 2 | High Domain III reactivity, rat, human and cyno reactive, minor reactivity to domain IV | 8 |
| 3 | Reactivity to rat, cyno and human HER3 | 43 |
| 4 | Reactive to human HER3 | 32 |
| 5 | Reactive to all constructs | 33 |

**Table 6**

| Functional activity of the most potent HER2 monoclonals at 1 µg/ml IgG. Percentage activity compared to reference antibodies, i.e. trastuzumab in SKBR-3 and #Ab6 in MCF-7. For HER2 antibodies the domains of all antibodies except PG2926 were mapped to domains I, III or IV | | | | | |
|---|---|---|---|---|---|
| **PG ID nr** | **Target** | **Epitope Bin** | **HER2 domain** | **SKBR-3** | **MCF-7** |
| **PG2916** | HER2 | 1 | I | 58% | 30% |
| **PG2973** | HER2 | 1 | I | 49% | 58% |
| **PG3004** | HER2 | 1 | I | 49% | 56% |
| **PG1849** | HER2 | 5 | IV | 42% | 22% |
| **PG3025** | HER2 | 1 | I | 38% | 28% |
| **PG2971** | HER2 | 1 | I | 25% | 51% |
| **PG3031** | HER2 | 1 | I | 33% | 38% |
| **PG2926** | HER2 | 7 | NA | 0% | 35% |
| **PG2930** | HER2 | 3 | III | 0% | 7% |

**Table 7**

| Functional activity of the most potent HER3 monoclonals at 1 µg/ml IgG in a HRG dependent MCF-7 assay. Percentage activity compared to reference antibody #Ab6. | | | |
|---|---|---|---|
| **PG ID nr** | **Target** | **Epitope group** | **MCF-7** |
| **PG3178** | HER3 | 5 | 162% |
| **PG3163** | HER3 | 5 | 119% |
| **PG3176** | HER3 | 5 | 68% |
| **PG3099** | HER3 | 3 | ND |

**Table 8**

| FACS stainings of HER2 antibodies whereby the HER2 VH is combined with a different light chain than the common light chain indicated in figure 16. MFI, indicates Mean Fluorescence Intensity in FACS. The HER2 MF number is indicated in between brackets, HER2 binding clones in the context of the different light chain are indicated in gray. | | |
|---|---|---|
| **PGnumber** | **MFI K562 cells (neg control)** | **MFI K562 HER2** |
| PG4462 (MF2971) | 267 | 14900 |
| PG4463 (MF3958) | 248 | 15600 |
| PG4474 (MF2916) | 254 | 14700 |
| PG4478 (MF2973) | 254 | 18000 |
| PG4481 (MF3004) | 267 | 16200 |
| PG4482 (MF3025) | 299 | 12000 |
| PG4483 (MF3031) | 260 | 14900 |
| PG4465 (MF1849) | 270 | 249 |
| Anti-HER2 mAb | 309 | 7618 |
| Anti-RSV mAb | 263 | 276 |

**Table 9**

| Functional activity of lead HER2 x HER3 bispecific antibodies (indicated using the PB prefix; each PB comprises an HER2 arm and an HER3 arm as indicated in the table) compared to comparator antibodies in the HRG dependent MCF-7 and BxPC3 assays. Based on binding profiles using chimeric constructs HER2 and HER3 antibodies could be separated over different bins. For HER2 antibodies the domains all antibodies except PG2926 could be mapped to domains I, III or IV. | | | | | | |
|---|---|---|---|---|---|---|
| **Name** | **HER2 arm** | **HER2 domain** | **HER3 arm** | **HER3 bin** | **MCF-7** | **BxPC3** |
| | | | | | IC50 (pM) | % Inhibition |
| **PB3441** | 2926 | NA | 3178 | 5 | 51,7 | -24% |
| **PB3443** | 2930 | III | 3178 | 5 | 136 | -31% |
| **PB3448** | 1849 | IV | 3178 | 5 | 371 | -22% |
| **PB3565** | 2973 | I | 3178 | 5 | 30,9 | -19% |
| **PB3566** | 3004 | I | 3178 | 5 | 7,9 | -20% |
| **PB3567** | 2971 | I | 3178 | 5 | 46,5 | -17% |
| **PB3709** | 3025 | I | 3178 | 5 | 34,5 | -19% |
| **PB3710** | 2916 | I | 3178 | 5 | 74,2 | -19% |
| **PB3883** | 2971 | I | 3176 | 5 | 113 | -19% |
| **PB3986** | 3025 | I | 3163 | 5 | 30,7 | -21% |
| **PB3990** | 2971 | I | 3163 | 5 | 13 | -18% |
| **PB4011** | 2971 | I | 3099 | 3 | 40,2 | ND |
| **PB3437** | 3031 | I | 3178 | 5 | 14 | -10% |
| **PG3178** | NA | NA | 3178 | 5 | 139 | -17% |
| ***#Ab6*** | | | | | *504* | -7% |
| ***trastuz. + pertuz*** | | | | | *352* | *ND* |
| ***trastuzumab*** | | | | | *500* | -3% |

**Table 10**

| Monovalent binding affinities of PB4188 and PB3448 for HER2 and HER3 as measured in biacore. Both bispecific antibodies share the same HER3 arm. ND, not done. | | |
|---|---|---|
| **PB** | **KD on Her2 (nM)** | **KD on Her3 (nM)** |
| **PB3448** | 5.4* | ND |
| **PB4188** | 0.16* | 3.9 |

**Table 11**

| **JIMT-1 xenograft study treatment groups** | | | | | | |
|---|---|---|---|---|---|---|
| Gr. | N | **Regimen 1** | | | | |
| | | Agent | Vehicle | mg/kg | Route | Schedule |
| 1^{#} | 10 | PBS | X | - | ip | qwk x 4 (start on day 1) |
| 2 | 10 | lapatinib | - | 150 | po | qd x 28 (start on day 1) |
| 3 | 10 | PB4188 | - | 2.5 | ip | qwk x 4 (start on day 1) |
| 4 | 10 | PB4188 | - | 25 | ip | qwk x 4 (start on day 1) |
| 5 | 10 | Pertuzumab + Trastuzumab | - | 2.5 | ip | qwk x 4 (start on day 1) |
| 6 | 10 | Pertuzumab + Trastuzumab | - | 25 | ip | qwk x 4 (start on day 1) |

**Table 12.**

| Affinities of ¹²⁵I-labeled IgG HER2xHER3 IgG (PB4188), HER3xTT (PB9215), HER2xTT (PB9216) and Herceptin (monospecific for HER2), as determined using steady state cell affinity measurements with BT-474 cells and SK-BR-3 cells. Data were obtained from three independent experiments. | | |
|---|---|---|
| | BT-474 | SK-BR-3 |
| Herceptin | 3.7 ± 0.5 nM | 1.3 ± 0.1 nM |
| PB4188 | 3.2 ± 0.5 nM | 2.0 ± 0.4 nM |
| HER2xTT | 3.9 ± 0.6 nM | 2.3 ± 0.7 nM |
| HER3xTT | 0.23 ± 0.08 nM | 0.99 ± 0.4 nM |

**Table 13. The mean binding protein reactivities (and ranges) listed for all critical residues identified. Critical residues involved in PG3958Fab binding were identified as those mutated in clones that were negative for PG3958Fab binding (<35% WT) but positive for the control mAb 1129 binding (>80% WT). Two additional critical residues were identified which did not meet the threshold guidelines, but whose mutation reduced antibody binding by a lesser extent. Residue numbering is that of PDB ID #1S78.**

| **HER2 Residue** | **Mutation** | **PG3958 Fab binding** | **Control mAb binding** | **Designation** |
|---|---|---|---|---|
| | | % of wt binding (range) | % of wt binding (range) | |
| 144 | T144A | 31.9 (11) | 82.1 (13) | Critical |
| 166 | R166A | 32.2 (5) | 93.7 (17) | Critical |
| 181 | R181A | 10.1 (5) | 98.6 (34) | Critical |
| 172 | P172A | 52.5 (2) | 94.9 (24) | Secondary |
| 179 | G179A | 41.7 (18) | 87.9 (25) | Secondary |

**Table 14. The mean binding protein reactivities (and ranges) are listed for both critical residues. Critical residues involved in PG3178 binding were identified as those mutated in clones that were negative for PG3178 mAb binding (<20% WT) but positive for the control mAb 66223 binding (>70% WT). Residue numbering is that of PDB ID #4P59.**

| **HER3 Residue** | **Mutation** | **PG3178 binding** | **Control mAb binding** | **Designation** |
|---|---|---|---|---|
| | | % of wt binding (range) | | |
| | | | % of wt binding (range) | |
| 409 | F409A | 16.74 (8) | 79.63 (0) | Critical |
| 426 | R426A | 3.17 (5) | 93.08 (36) | Critical |

**Table 15. List of exposed residues within 11.2 Å radius of Arg 426 in HER3:**

| | |
|---|---|
| Leu 423 | L423 |
| Tyr 424 | Y424 |
| Asn 425 | N425 |
| Gly 427 | G427 |
| Gly 452 | G452 |
| Arg 453 | R453 |
| Tyr 455 | Y455 |
| Glu 480 | E480 |
| Arg 481 | R481 |
| Leu 482 | L482 |
| Asp 483 | D483 |
| Lys 485 | K485 |

**Table 16: Scoring system for monitoring of humane endpoints**

| **Variable** | **Score** |
|---|---|
| **Body weight changes** | |
| <20% | 0 |
| >20% | 3 |

| **Physical appearance** | |
|---|---|
| Normal | 0 |
| Lack of grooming | 1 |
| Small bites or scratches. Nasal/ocular discharge | 2 |
| Serious bites or scratches. Abnormal posture, limb, tremor etc. | 3 |

| **Unprovoked behavior** | |
|---|---|
| Normal | 0 |
| Minor changes | 1 |
| Abnormal, reduced mobility, decreased alertness, inactive | 2 |
| Unsolicited vocalizations, self-mutilation, either very restless or immobile | 3 |

| **Behavioral responses to external stimuli** | |
|---|---|
| Normal | 0 |
| Minor depression/exaggeration of response | 1 |
| Moderately abnormal responses | 2 |
| Violent reactions or comatose | 3 |

| **Occipital tumor** | |
|---|---|
| None | 0 |
| Palpable | 1 |
| Total score* | |

| | |
|---|---|
| * Euthanasia of the mouse at - Total score > 5, or - Score of 3 in any one variable, regardless of the total score. | |

**Table 17: Time-line for animal experimentation.**

| **Date** | **Procedure** |
|---|---|
| 03/08/16 | Arrival |
| 10/08/16 | End of acclimatization |
| 17/08/16 | Intracranial tumor inoculation |
| 05/09/16 | First pre therapy MR imaging sessions |
| 09/09/16 | First enrolment and start of therapy |
| 04/10/16 | Last enrolment |
| 25/10/16 | Last day of therapy |
| 12/11/16 | Last animal euthanized |

**Table 18: Overview of study groups and dosing**

| **Group** | **# of mice** | **Compound** | **Dose** |
|---|---|---|---|
| A | 8 | Vehicle (saline) | 4x twice wk, 5 mL/kg i.v. |
| B | 8 | T-DM1 | 4x qwk, 10 mg/kg diluted in saline, 5 mL/kg i.v. |
| C | 8 | MCLA-128 | 4x twice wk, 25 mg/kg diluted in saline, 5 mL/kg i.v. |
| D | 8 | T-DM1+MCLA-128 | T-DM1, 10 mg/kg + MCLA, 25 mg/kg diluted in saline: 4x qwk, 5 mL/kg i.v. MCLA-128: 25 mg/kg diluted in saline: 4x qwk, 5 mL/kg i.v. |

**Table 19: Inclusion date and tumor volume at inclusion.**

| **Group** | **Mouse ID** | **Inclusion date** | **Tumor volume (mm3)** |
|---|---|---|---|
| A | 16 | 09/09/2016 | 15,9 |
| A | 17 | 09/09/2016 | 11,1 |
| A | 21 | 13/09/2016 | 17,6 |
| A | 23 | 13/09/2016 | 13,5 |
| A | 26 | 13/09/2016 | 11,6 |
| A | 11 | 16/09/2016 | 11,0 |
| A | 36 | 20/09/2016 | 9,3 |
| A | 1 | 27/09/2016 | 9,5 |
| B | 9 | 09/09/2016 | 12,3 |
| B | 14 | 09/09/2016 | 16,7 |
| B | 25 | 13/09/2016 | 18,6 |
| B | 35 | 13/09/2016 | 9,2 |
| B | 24 | 16/09/2016 | 10,0 |
| B | 5 | 20/09/2016 | 15,7 |
| B | 32 | 23/09/2016 | 10,1 |
| B | 34 | 04/10/2016 | 14,4 |
| C | 18 | 09/09/2016 | 13,7 |
| C | 43 | 09/09/2016 | 13,2 |
| C | 7 | 13/09/2016 | 11,2 |
| C | 29 | 13/09/2016 | 11,4 |
| C | 42 | 13/09/2016 | 16,6 |
| C | 30 | 20/09/2016 | 9,8 |
| C | 37 | 20/09/2016 | 14,8 |
| C | 27 | 04/10/2016 | 9,5 |
| D | 8 | 09/09/2016 | 21,7 |
| D | 41 | 09/09/2016 | 12,5 |
| D | 38 | 13/09/2016 | 15,9 |
| D | 39 | 13/09/2016 | 10,6 |
| D | 40 | 13/09/2016 | 9,7 |
| D | 3 | 16/09/2016 | 11,6 |
| D | 31 | 20/09/2016 | 9,8 |
| D | 19 | 23/09/2016 | 13,8 |

**Table 20: Scoring of brain edema**

| **Group Mouse ID** | | **Brain edema score** | | | | | | | | | **final score** |
|---|---|---|---|---|---|---|---|---|---|---|---|
| | | **-1** | **3** | **7** | **10** | **14** | **21** | **28** | **35** | **42** | |
| A | 01 | 1 | 2 | 3 | 3 | 3 | | | | | 3 |
| A | 11 | 0 | 1 | 1 | 2 | 3 | | | | | 3 |
| A | 16 | 0 | 0 | 1 | 1 | | | | | | 1 |
| A | 17 | 0 | 0 | 0 | 1 | 2 | | | | | 2 |
| A | 21 | 1 | 2 | 2 | 2 | | | | | | 2 |
| A | 23 | 1 | 1 | 1 | 2 | | | | | | 2 |
| A | 26 | 1 | 2 | 2 | 3 | | | | | | 3 |
| A | 36 | 2 | 2 | 2 | 2 | | | | | | 2 |
| B | 05 | 0 | 1 | 2 | 2 | 2 | | | | | 2 |
| B | 09 | 1 | 1 | 2 | 3 | | | | | | 3 |
| B | 14 | 0 | 0 | 1 | 2 | 3 | | | | | 3 |
| B | 24 | 1 | 2 | 2 | 3 | 3 | 4 | 4 | | | 4 |
| B | 25 | 0 | 0 | 1 | 0 | 2 | | | | | 2 |
| B | 32 | 0 | 0 | 1 | 1 | 2 | 2 | 3 | | | 3 |
| B | 34 | 2 | 2 | 3 | 3 | 3 | | | | | 3 |
| B | 35 | 0 | 0 | 1 | 1 | 2 | 2 | 3 | | | 3 |
| C | 07 | 1 | 2 | 2 | 2 | 1 | 2 | 3 | 3 | 3 | 3 |
| C | 18 | 0 | 0 | 0 | 0 | 0 | 1 | | | | 1 |
| C | 27 | 1 | 1 | 2 | 2 | 1 | 1 | | | | 1 |
| C | 29 | 1 | 1 | 2 | 2 | 3 | 3 | 4 | | | 4 |
| C | 30 | 2 | 2 | 3 | 3 | 3 | 3 | 3 | 3 | | 3 |
| C | 37 | 1 | 1 | 2 | 2 | 3 | 3 | 3 | 3 | | 3 |
| C | 42 | 0 | 1 | 1 | 1 | 1 | 1 | | | | 1 |
| C | 43 | 0 | 0 | 0 | 0 | 1 | 2 | 3 | | | 3 |
| D | 03 | 0 | 0 | 1 | 2 | 2 | 3 | 3 | 4 | 4 | 4 |
| D | 08 | 0 | 0 | 0 | 1 | 2 | 3 | 4 | | | 4 |
| D | 19 | 0 | 0 | 0 | 0 | 1 | 1 | 1 | 1 | NA | 1 |
| D | 31 | 2 | 3 | 4 | 4 | 4 | 4 | 4 | 4 | | 4 |
| D | 38 | 0 | 1 | 2 | 3 | 3 | 3 | 4 | 4 | | 4 |
| D | 39 | 0 | 0 | 0 | 0 | 1 | 2 | 3 | 3 | 4 | 4 |
| D | 40 | 1 | 1 | 1 | 2 | 2 | 3 | 3 | 3 | 4 | 4 |
| D | 41 | 0 | 0 | 0 | 0 | 0 | 0 | 0 | | | 0 |

### References

Arteaga CL, Sliwkowski MX, Osborne CK, Perez EA, Puglisi F, Gianni L. 2011. Treatment of HER2-positive breast cancer: current status and future perspectives. Nat Rev Clin Oncol. 2011 Nov 29;9(1):16-32.
Balko JM, Miller TW, Morrison MM, Hutchinson K, Young C, Rinehart C, Sánchez V, Jee D, Polyak K, Prat A, Perou CM, Arteaga CL, Cook RS. 2012. The receptor tyrosine kinase ErbB3 maintains the balance between luminal and basal breast epithelium. Proc Natl Acad Sci U S A. Jan 3;109(1):221-6.
Baselga J, Cortés J, Kim SB, Im SA, Hegg R, Im YH, Roman L, Pedrini JL, Pienkowski T, Knott A, Clark E, Benyunes MC, Ross G, Swain SM. 2012. Pertuzumab plus trastuzumab plus docetaxel for metastatic breast cancer. N Engl J Med. Jan 12;366(2):109-19.
de Kruif et al. Mol. Biol. (1995), 248, 97-105
Ewer MS, Ewer SM. Cardiotoxicity of anticancer treatments: What the cardiologist needs to know. Nat Rev Cardiol 2010;7:564-75
Guarneri Jain KK, Casper ES, Geller NL, et al. A prospective randomized comparison of epirubicin and doxorubicin in patients with advanced breast cancer; J Clin Oncol 1985;3:818-26
Junttila TT, Akita RW, Parsons K, Fields C, Lewis Phillips GD, Friedman LS, Sampath D, Sliwkowski MX. 2009. Ligand-independent HER2/HER3/PI3K complex is disrupted by trastuzumab and is effectively inhibited by the PI3K inhibitor GDC-0941. Cancer Cell. May 5;15(5):429-40.
Junttila, T. T., K. Parsons, et al. (2010). "Superior In vivo Efficacy of Afucosylated Trastuzumab in the Treatment of HER2-Amplified Breast Cancer." Cancer Research 70(11): 4481-4489
Merchant et al. Nature Biotechnology, Vol. 16 July 1998 pp 677-681
Nissim A, Hoogenboom HR, Tomlinson IM, Flynn G, Midgley C, Lane D, Winter G. 1994. Antibody fragments from a 'single pot' phage display library as immunochemical reagents. EMBO J. 1994 Feb 1;13(3):692-8.
Ocana A, Vera-Badillo F, Seruga B, Templeton A, Pandiella A, Amir E. 2013. HER3 overexpression and survival in solid tumors: a meta-analysis. J Natl Cancer Inst. Feb 20;105(4):266-73.
Sergina NV, Rausch M, Wang D, Blair J, Hann B, Shokat KM, Moasser MM. 2007. Escape from HER-family tyrosine kinase inhibitor therapy by the kinase-inactive HER3. Nature. Jan 25;445(7126):437-41.
Schaefer et al. Cancer Cell 20, 472-486, October 2011
Schoeberl B, Faber AC, Li D, Liang MC, Crosby K, Onsum M, Burenkova O, Pace E, Walton Z, Nie L, Fulgham A, Song Y, Nielsen UB, Engelman JA, Wong KK. 2010. An ErbB3 antibody, MM-121, is active in cancers with ligand-dependent activation. Cancer Res. Mar 15;70(6):2485-94.
Shames et al. PLOS ONE, February 2013, Vol.8, Issue 2, pp 1-10
Tanner M, Kapanen AI, Junttila T, Raheem O, Grenman S, Elo J, Elenius K, Isola J. 2004. Characterization of a novel cell line established from a patient with Herceptin-resistant breast cancer. Mol Cancer Ther. 2004 Dec;3(12):1585-92. Yarden Y, Pines G.2012. The ERBB network: at last, cancer therapy meets systems biology. Nat Rev CancerJul 12;12(8):553-63.
Thery J.-C. et al., Resistance to human epidermal growth factor receptor type 2-targeted therapies, Eur J Cancer (2014), Vol. 50, Issue 5, pages 892-901
Wadhwa D, Fallah-Rad N, Grenier D, et al. Trastuzumab mediated cardiotoxicity in the setting of the adjuvant chemotherapy for breast cancer: A retrospective study. Breast Cancer Res Treat 2009;117:357-64.
Wehrman TS, Raab WJ, Casipit CL, Doyonnas R, Pomerantz JH, Blau HM. 2006. A system for quantifying dynamic protein interactions defines a role for Herceptin in modulating ErbB2 interactions. Proc Natl Acad Sci U S A. Dec 12;103(50):19063-8.
Wilson TR, Fridlyand J, Yan Y, Penuel E, Burton L, Chan E, Peng J, Lin E, Wang Y, Sosman J, Ribas A, Li J, Moffat J, Sutherlin DP, Koeppen H, Merchant M, Neve R, Settleman J. 2012. Widespread potential for growth-factor-driven resistance to anticancer kinase inhibitors. Nature. Jul 26;487(7408):505-9.
Yonesaka et al., Sci.transl.Med., Vol.3, Issue 99 (2011); pp1-11
Zhang H, Berezov A, Wang Q, Zhang G, Drebin J, Murali R, Greene MI. 2007. ErbB receptors: from oncogenes to targeted cancer therapies. J Clin Invest. Aug;117(8):2051-8.
Greco, Bravo, Parsons (1995) The search for synergy: a critical review from a response surface perspective. Pharmacol. Rev 47 (2): 331-85.

## Claims

1. A combination of
- an inhibitor of ErbB-2, wherein the inhibitor of ErbB-2 is a bivalent monospecific antibody that comprises antigen binding sites that can bind an epitope on an extracellular part of ErbB-2, and wherein the monospecific antibody comprises a drug conjugate and
- a bispecific antibody that comprises
a first antigen-binding site comprising a heavy chain variable region with a CDR1, CDR2 and CDR3 having the sequences AYYIN, RIYPGSGYTSYAQKFQG, and PPVYYDSAWFAY, respectively, and that binds an epitope on domain I of ErbB-2, and
a second antigen-binding site comprising a heavy chain variable region with a CDR1, CDR2 and CDR3 having the sequences GYYMH, WINPNSGGTNYAQKFQG, and DHGSRHFWSYWGFDY respectively, and that binds an epitope on domain III of ErbB-3,
wherein said first antigen binding site and said second antigen binding site of said bispecific antibody comprise a light chain variable region of the sequence DIQMTQSPSSLSASVGDRVTITCRASQSISSYLNWYQQKPGKAPKLLIYAASSLQ SGVPSRFSGSGSGTDFTLTISSLQPEDFATYYCQQSYSTPPTFGQGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC or
a light chain variable region of the sequence DIQMTQSPSSLSASVGDRVTITCRASQSISSYLNWYQQKPGKAPKLLIYAASSLQ SGVPSRFSGSGSGTDFTLTISSLQPEDFATYYCQQSYSTPPTFGQGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC with 1, 2, 3, 4 or 5 amino acid insertions, deletions, substitutions or a combination thereof, wherein said insertions, deletions, or substitutions are not in a CDR1, CDR2 or CDR3 region or FR4 region,
for use in a method treatment of an individual that has an ErbB-2, ErbB-3 or ErbB-2/ErbB-3 positive tumor or is at risk of developing said tumor.

2. The combination for use of claim 1, wherein said treatment is for breast cancer.

3. The combination for use of claim 1, wherein said treatment is for metastatic breast cancer.

4. A pharmaceutical composition comprising
- an inhibitor of ErbB-2, wherein the inhibitor of ErbB-2 is a bivalent monospecific antibody that comprises antigen binding sites that can bind an epitope on an extracellular part of ErbB-2, and wherein the monospecific antibody comprises a drug conjugate and
- a bispecific antibody that comprises
a first antigen-binding site comprising a heavy chain variable region with a CDR1, CDR2 and CDR3 having the sequences AYYIN, RIYPGSGYTSYAQKFQG, and PPVYYDSAWFAY, respectively, and that binds an epitope on domain I of ErbB-2, and
a second antigen-binding site comprising a heavy chain variable region with a CDR1, CDR2 and CDR3 having the sequences GYYMH, WINPNSGGTNYAQKFQG, and DHGSRHFWSYWGFDY respectively, and that binds an epitope on domain III of ErbB-3,
wherein said first antigen binding site and said second antigen binding site of said bispecific antibody comprise a light chain variable region of the sequence DIQMTQSPSSLSASVGDRVTITCRASQSISSYLNWYQQKPGKAPKLLIYAASSLQ SGVPSRFSGSGSGTDFTLTISSLQPEDFATYYCQQSYSTPPTFGQGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC or
a light chain variable region of the sequence DIQMTQSPSSLSASVGDRVTITCRASQSISSYLNWYQQKPGKAPKLLIYAASSLQ SGVPSRFSGSGSGTDFTLTISSLQPEDFATYYCQQSYSTPPTFGQGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC with 1, 2, 3, 4 or 5 amino acid insertions, deletions, substitutions or a combination thereof, wherein said insertions, deletions, or substitutions are not in a CDR1, CDR2 or CDR3 region or FR4 region.

5. A kit of parts comprising
- an inhibitor of ErbB-2, wherein the inhibitor of ErbB-2 is a bivalent monospecific antibody that comprises antigen binding sites that can bind an epitope on an extracellular part of ErbB-2, and wherein the monospecific antibody comprises a drug conjugate and
- a bispecific antibody that comprises
a first antigen-binding site comprising a heavy chain variable region with a CDR1, CDR2 and CDR3 having the sequences AYYIN, RIYPGSGYTSYAQKFQG, and PPVYYDSAWFAY, respectively, and that binds an epitope on domain I of ErbB-2, and
a second antigen-binding site comprising a heavy chain variable region with a CDR1, CDR2 and CDR3 having the sequences GYYMH, WINPNSGGTNYAQKFQG, and DHGSRHFWSYWGFDY respectively, and that binds an epitope on domain III of ErbB-3,
wherein said first antigen binding site and said second antigen binding site of said bispecific antibody comprise a light chain variable region of the sequence DIQMTQSPSSLSASVGDRVTITCRASQSISSYLNWYQQKPGKAPKLLIYAASSLQ SGVPSRFSGSGSGTDFTLTISSLQPEDFATYYCQQSYSTPPTFGQGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC or
a light chain variable region of the sequence DIQMTQSPSSLSASVGDRVTITCRASQSISSYLNWYQQKPGKAPKLLIYAASSLQ SGVPSRFSGSGSGTDFTLTISSLQPEDFATYYCQQSYSTPPTFGQGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC with 1, 2, 3, 4 or 5 amino acid insertions, deletions, substitutions or a combination thereof, wherein said insertions, deletions, or substitutions are not in a CDR1, CDR2 or CDR3 region or FR4 region.

6. The combination for use, the pharmaceutical composition or the kit of parts of claim according to any one of the preceding claims, wherein the monospecific antibody can bind an epitope on ErbB-2 extracellular domain IV, domain III and/or domain II.

7. The combination for use, the pharmaceutical composition or the kit of parts of claim according to any one of the preceding claims, the pharmaceutical composition, or the kit of parts of any one of claims 5-6, wherein the drug conjugate comprises emtansine.

8. The combination for use, the pharmaceutical composition or the kit of parts of claim according to any one of the preceding claims, wherein the monospecific antibody is Trastuzumab.

9. The combination for use, the pharmaceutical composition or the kit of parts of claim according to any one of the preceding claims, wherein the monospecific antibody is Trastuzumab emtansine.

10. The combination for use, the pharmaceutical composition or the kit of parts of claim according to any one of the preceding claims, wherein said first antigen binding site and said second antigen binding site of said bispecific antibody comprise the sequence DIQMTQSPSSLSASVGDRVTITCRASQSISSYLNWYQQKPGKAPKLLIYAASSLQ SGVPSRFSGSGSGTDFTLTISSLQPEDFATYYCQQSYSTPPTFGQGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC with 1, 2, 3, 4 or 5 amino acid insertions, deletions, substitutions or a combination thereof, wherein said insertions, deletions, or substitutions are not in a CDR1, CDR2 or CDR3 region or FR4 region.

11. The combination for use, the pharmaceutical composition or the kit of parts of claim according to any one of the preceding claims, wherein said first antigen binding site and said second antigen binding site of said bispecific antibody comprise the sequence

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPPTFGQGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC.

12. The combination for use, the pharmaceutical composition or the kit of parts of claim according to any one of the preceding claims, wherein the antibody is a bispecific IgG antibody, such as a human IgG1.

13. The combination for use, pharmaceutical composition, or kit of parts of any one of the preceding claims, wherein the bispecific antibody comprises a heavy chain variable region comprising the sequence QVQLVQSGAEVKKPGASVKLSCKASGYTFTAYYINWVRQAPGQGLEWIGRIYPG SGYTSYAQKFQGRATLTADESTSTAYMELSSLRSEDTAVYFCARPPVYYDSAWF AYWGQGTLVTVSS and a second heavy chain variable region comprising the sequence

QVQLVQSGAEVKKPGASVKVSCKASGYTFTGYYMHWVRQAPGQGLEWMGWI
NPNSGGTNYAQKFQGRVTMTRDTSISTAYMELSRLRSDDTAVYYCARDHGSRH
FWSYWGFDYWGQGTLVTVSS.

14. The combination for use, pharmaceutical composition, or kit of parts of any one of the preceding claims, wherein the bispecific antibody comprises a heavy chain comprising the sequence
QVQLVQSGAEVKKPGASVKLSCKASGYTFTAYYINWVRQAPGQGLEWIGRIYPG SGYTSYAQKFQGRATLTADESTSTAYMELSSLRSEDTAVYFCARPPVYYDSAWF AYWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVD KRVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKE YKCKVSNKALPAPIEKTISKAKGQPREPQVYTDPPSREEMTKNQVSLTCEVKGF YPSDIAVEWESNGQPENNYKTTPPVLDSDGSFFLYSKLTVDKSRWQQGNVFSC SVMHEALHNHYTQKSLSLSPG and a heavy chain having the sequence

QVQLVQSGAEVKKPGASVKVSCKASGYTFTGYYMHWVRQAPGQGLEWMGWI
NPNSGGTNYAQKFQGRVTMTRDTSISTAYMELSRLRSDDTAVYYCARDHGSRH
FWSYWGFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKRVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTKPPSREEMTKNQVS

LKCLVKGFYPSDIAVEWESNGQPENNYKTTPPVLDSDGSFFLYSKLTVDKSRW
QQGNVFSCSVMHEALHNHYTQKSLSLSPG.

15. The combination for use, pharmaceutical composition, or kit of parts of any one of the preceding claims, wherein the bispecific antibody is afucosylated.
